# EUROPEAN PATENT APPLICATION

(11) **EP 3 845 662 A2**
(43) Date of publication of application: **07.07.2021**
(21) Application number: 20177051.8
(22) Date of filing: 09.11.2016
(51) Int. Cl.: C12Q 1/02, A61K 39/395, A61K 48/00, A61K 31/4196, A61K 31/7048, A61K 36/07, C12Q 1/18, G01N 33/50

(54) **NOVEL KINASE FOR TREATING AND PREVENTING FUNGAL INFECTIONS, AND USE THEREOF**

(30) Priority: 09.11.2015 KR 20150157021
(62) Divisional of application: 16864542.2
(71) Applicant: Amtixbio Co., Ltd., Seoul 05836 (KR)
(72) Inventor: BAHN, Yong-Sun, Seoul 05538 (KR); YANG, Dong-Hoon, Gyeonggi-do 12109 (KR); LEE, Kyung-Tae, Seoul 08643 (KR); SO, Yee-Seul, Gyeonggi-do 14086 (KR)
(74) Representative: Steffan & Kiehne Patentanwälte PartG mbB

(57) **Abstract**

The present invention relates to a use of kinases for treating and preventing fungal meningoencephalitis by pathogenic fungi of the genus Cryptococcus. Specifically, the present invention relates to a method for screening an antifungal agent characterized by measuring the amount or activity of a pathogenic-regulatory kinase protein of *Cryptococcus neoformans,* or the expression level of a gene encoding the protein; and an antifungal pharmaceutical composition comprising an inhibitor against a pathogenic-regulatory kinase protein of *Cryptococcus neoformans* or a gene encoding the same. An antifungal agent for treating meningoencephalitis, etc. can be effectively screened by using the method for screening an antifungal agent according to the present invention, and meningoencephalitis, etc. can be effectively treated by using the antifungal pharmaceutical composition according to the present invention. Thus, the present invention can be widely used in related industrial fields such as pharmaceutical and biotechnology fields.

## Description

### [Technical Field]

The preset invention relates to novel kinases for preventing and treating pathogenic fungal infection and the use thereof. Moreover, the present invention relates to a method for screening an antifungal agent, which comprises measuring the amount or activity of a *Cryptococcus neoformans* pathogenicity-regulating kinase protein or the expression level of a gene encoding the protein and to an antifungal pharmaceutical composition comprising an inhibitor against a *Cryptococcus neoformans* pathogenicity-regulating kinase protein or a gene encoding the protein.

### [Background Art]

*Cryptococcus neoformans* is a pathogenic fungus which is ubiquitously distributed in diverse natural environments, including soil, tree and bird guano, and uses various hosts ranging from lower eukaryotes to aquatic and terrestrial animals (Lin, X. & Heitman, J. The biology of the Cryptococcus neoformans species complex. Annu. Rev. Microbiol. 60, 69-105, 2006). *Cryptococcus neoformans* is the leading cause of fungal meningoencephalitis deaths and is known to cause approximately one million new infections and approximately 600,000 deaths worldwide each year (Park, B. J. et al. Estimation of the current global burden of cryptococcal meningitis among persons living with HIV/AIDS. AIDS 23, 525-530, doi:10.1097/QAD.0b013e328322ffac, 2009). However, limited therapeutic options are available for treatment of systemic cryptococcosis (Perfect, J. R. et al. Clinical practice guidelines for the management of cryptococcal disease: 2010 update by the infectious diseases society of America. Clin Infect Dis 50, 291-322, doi:10.1086/649858, 2010). Meanwhile, *C. neoformans* is regarded as an ideal fungal model system for basidiomycetes, owing to the availability of completely sequenced and well-annotated genome databases, a classical genetic dissection method through sexual differentiation, efficient methods of reverse and forward genetics, and a variety of heterologous host model systems (Idnurm, A. et al. Deciphering the model pathogenic fungus Cryptococcus neoformans. Nat. Rev. Microbiol. 3, 753-764, 2005).

Extensive studies have been conducted over several decades to understand the mechanisms underlying the pathogenicity of *C. neoformans.* Besides efforts to analyze the functions of individual genes and proteins, recent large-scale functional genetic analyses have provided comprehensive insights into the overall biological circuitry of *C. neoformans.* However, the signaling and metabolic pathways responsible for the general biological characteristics and pathogenicity of *C. neoformans* have not yet been fully elucidated. This is mainly because the functions of kinases, which have a central role in signaling pathways and are responsible for the activation or expression of transcription factors (TFs), have not been fully characterized on a genome-wide scale. In general, kinases play pivotal roles in growth, cell cycle control, differentiation, development, the stress response and many other cellular functions, affecting about 30% of cellular proteins by phosphorylation (Cohen, P. The regulation of protein function by multisite phosphorylation-a 25 year update. Trends Biochem Sci 25, 596-601, 2000). Furthermore, kinases are considered to be a protein class representing a major target in drug development, as their activity is easily inhibited by small molecules such as compounds, or antibodies (Rask-Andersen, M., Masuram, S. & Schioth, H. B. The druggable genome: Evaluation of drug targets in clinical trials suggests major shifts in molecular class and indication. Annu Rev Pharmacol Toxicol 54, 9-26, doi:10.1146/annurev-pharmtox-011613-135943, 2014). Therefore, the systematic functional profiling of fungal kinases in human fungal pathogens is in high demand to identify virulence-related kinases that could be further developed as antifungal drug targets.

Accordingly, the present inventors performed systematic functional profiling of the kinome networks in *C. neoformans* and Basidiomycetes by constructing a high-quality library of 226 signature-tagged gene-deletion strains through homologous recombination methods for 114 putative kinases, and examining their phenotypic traits under 30 distinct *in vitro* growth conditions, including growth, differentiation, stress responses, antifungal resistance and virulence-factor production(capsule, melanin and urease). Furthermore, the present inventors investigated their pathogenicity and infectivity potential in insect and murine host models.

### [Disclosure]

### [Technical Problem]

It is an object of the present invention to provide novel kinases for prevention and treatment of pathogenic fungal infection and the use thereof. Furthermore, the present invention is intended to provide a method of screening an antifungal agent by measuring the amount or activity of a *Cryptococcus neoformans* pathogenicity-regulating kinase protein or the expression level of a gene encoding the protein. The present invention is also intended to provide an antifungal pharmaceutical composition comprising an inhibitor and/or activator of a *Cryptococcus neoformans* pathogenicity-regulating kinase protein or a gene encoding the protein. The present invention is also intended to provide a method for screening a drug candidate for treating and preventing cryptococcosis or meningoencephalitis. The present invention is also intended to provide a pharmaceutical composition for treatment and prevention of cryptococcosis or meningoencephalitis. The present invention is also intended to provide a method for diagnosing fungal infection.

### [Technical Solution]

To achieve the above objects, the present invention provides novel pathogenicity-regulating kinase proteins. Specifically, the novel pathogenicity-regulating kinase proteins according to the present invention include, but are not limited to, Fpk1, Bck1, Gal83, Kic1, Vps15, Ipkl, Mecl, Urk1, Yak1, Pos5, Irk1, Hs1101, Irk2, Mps1, Sat4, Irk3, Cdc7, Irk4, Swe102, Vrk1, Fbp26, Psk201, Ypk101, Pan3, Ssk2, Utr1, Pho85, Bud32, Tco6, Arg5, 6, Ssn3, Irk6, Dak2, Riml5, Dak202a, Snf101, Mpk2, Cmk1, Irk7, Cbkl, Kic102, Mkk2, Ckal, and Bub1.

The present invention also provides a method for screening an antifungal agent, comprising the steps of: (a) bringing a sample to be analyzed into contact with a cell containing a pathogenicity-regulating kinase protein; (b) measuring the amount or activity of the protein; and (c) determining that the sample is an antifungal agent, when the amount or activity of the protein is measured to be downregulated or up-regulated.

The present invention also provides a method for screening an antifungal agent, comprising the steps of: (a) bringing a sample to be analyzed into contact with a cell containing a gene encoding a pathogenicity-regulating kinase protein; (b) measuring the expression level of the gene; and (c) determining that the sample is an antifungal agent, when the expression level of the gene is measured to be downregulated or up-regulated.

In the present invention, the cell that is used in screening of the antifungal agent may be a fungal cell, for example, a *Cryptococcus neoformans* cell.

In the present invention, the antifungal agent may be an agent for treating and preventing meningoencephalitis or cryptococcosis, but is not limited thereto.

In the present invention, a BLAST matrix for 60 pathogenicity-related kinases was constructed using the CFGF (Comparative Fungal Genomics Platform) (http://cfgp.riceblast.snu.ac.kr) database, and the pathogenicity-related 60 kinase protein sequence was queried. As a result, orthologue proteins were retrieved and matched from the genome database from the 35 eukaryotic species. To determine the orthologue proteins, each protein sequence was analyzed by BLAST and reverse-BLAST using genome databases (CGD; *Candida* genome database for *C. albicans,* Broad institute database for *Fusarium graminearum* and *C. neoformans*). 21 kinases were related to pathogenicity in both *F. graminearum* and *C. neoformans.* 13 kinases were related to pathogenicity of *C. neoformans* and *C. albicans.* Among them, five kinases, including Sch9, Snf1, Pka1, Hog1 and Swe1, were related to virulence of all the three fungal pathogenic strains. Genes in the pathogenicity network according to the present invention were classified by the predicted biological functions listed in the information of their Gene Ontology (GO) term. Six kinases (Arg5/6, Ipkl, Irk2, Irk4, Irk6 and vrk1) did not have any functionally related genes in CryptoNet (http://www.inetbio.org/cryptonet).

As used herein, the term "sample" means an unknown candidate that is used in screening to examine whether it influences the expression level of a gene or the amount or activity of a protein. Examples of the sample include, but are not limited to, chemical substances, nucleotides, antisense-RNA, siRNA (small interference RNA) and natural extracts.

The term "antifungal agent" as used herein is meant to include inorganic antifungal agents, organic natural extract-based antifungal agents, organic aliphatic compound-based antifungal agents, and organic aromatic compound-based antifungal agents, which serve to inhibit the propagation of bacteria and/or fungi. Examples of the inorganic antifungal agents include, but are not limited to, chlorine compounds (especially sodium hypochlorite), peroxides (especially hydrogen peroxide), boric acid compounds (especially boric acid and sodium borate), copper compounds (especially copper sulfate), zinc compounds (especially zinc sulfate and zinc chloride), sulfur-based compounds (especially sulfur, calcium sulfate, and hydrated sulfur), calcium compounds (especially calcium oxide), silver compounds (especially thiosulfite silver complexes, and silver nitrate), iodine, sodium silicon fluoride, and the like. Examples of the organic natural extract-based antifungal agents include, but are not limited to, hinokithiol, *Phyllostachys pubescens* extracts, creosote oil, and the like.

In the present invention, measurement of the expression level of the gene may be performed using various methods known in the art. For example, the measurement may be performed using RT-PCR (Sambrook et al, Molecular Cloning. A Laboratory Manual, 3rd ed. Cold Spring Harbor Press, 2001), Northern blotting (Peter B. Kaufma et al., Molecular and Cellular Methods in Biology and Medicine, 102-108, CRCpress), hybridization using cDNA microarray (Sambrook et al, Molecular Cloning. A Laboratory Manual, 3rd ed. Cold Spring Harbor Press, 2001) or *in situ* hybridization (Sambrook et al., Molecular Cloning. A Laboratory Manual, 3rd ed. Cold Spring Harbor Press, 2001). Where the measurement is performed according to RT-PCR protocol, total RNA is isolated from cells treated with a sample, and then single-stranded cDNA is synthesized using dT primer and reverse transcriptase. Subsequently, PCR is performed using the single-stranded cDNA as a template and a gene-specific primer set. The gene-specific primer sets used in the present invention are shown in Tables 2 and 3 below. Next, the PCR amplification product is amplified, and the formed band is analyzed to measure the expression level of the gene.

In the present invention, measurement of the amount or activity of the protein may be performed by various immunoassay methods known in the art. Examples of the immunoassay methods include, but are not limited to, radioimmunoassay, radio-immunoprecipitation, immunoprecipitation, ELISA (enzyme-linked immunosorbentassay), capture-ELISA, inhibition or competition assay, and sandwich assay. The immunoassay or immunostaining methods are described in various literatures (Enzyme Immunoassay, E. T. Maggio, ed., CRC Press, Boca Raton, Florida, 1980; Gaastra, W., Enzymelinked immunosorbent assay(ELISA), in Methods in Molecular Biology, Vol. 1, Walker, J.M. ed., Humana Press, NJ, 1984; and Ed Harlow and David Lane, Using Antibodies: A Laboratory Manual, Cold Spring Harbor Laboratory Press, 1999). For example, when radioimmunoassay is used, protein-specific antibodies labeled with radioisotopes (e.g., C14, I125, P32 and S35) may be used.

When ELISA is used in one embodiment of the present invention, it comprises the steps of: (i) coating an extract of sample-treated cells on the surface of a solid substrate; (ii) incubating the cell extract with a kinase protein-specific or labeled protein-specific antibody as a primary antibody; (iii) incubating the resultant of step (ii) with an enzyme-conjugated secondary antibody; and (iv) measuring the activity of the enzyme. Suitable examples of the solid substrate include hydrocarbon polymers (e.g., polystyrene and polypropylene), glass, metals or gels. Most preferably, the solid substrate is a microtiter plate. The enzyme conjugated to the secondary antibody includes an enzyme that catalyzes a color development reaction, a fluorescent reaction, a luminescent reaction, or an infrared reaction, but is not limited. Examples of the enzyme include alkaline phosphatase, β-galactosidase, horseradish peroxidase, luciferase, and cytochrome P450. When alkaline phosphatase is used as the enzyme conjugated to the secondary antibody, bromochloroindolylphosphate (BCIP), nitro blue tetrazolium (NBT), naphthol-AS-B1-phosphate and ECF (enhanced chemifluorescence) may be used as substrates for color development reactions. When horseradish peroxidase is the enzyme, chloronaphthol, aminoethylcarbazol, diaminobenzidine, D-luciferin, lucigenin (bis-N-methylacridinium nitrate), resorufin benzyl ether, luminol, Amplex Red reagent (10-acetyl-3,7-dihydroxyphenoxazine), TMB (3,3,5,5-tetramethylbenzidine), ABTS (2,2'-azine-di[3-ethylbenzthiazoline sulfonate]) and o-phenylenediamine (OPD) may be used as substrates. The final measurement of the activity or signal of the enzyme in the ELISA assay may be performed according to various conventional methods known in the art. When biotin is used as a label, the signal can be easily detected with streptavidin, and when luciferase is used as a label, the signal can be easily detected with luciferin.

In one embodiment, the present invention provides an antifungal pharmaceutical composition comprising an agent (inhibitor or activator) for a fungal pathogenicity-regulating kinase protein. In another embodiment, the fungus is *Cryptococcus neoformans.*

In one embodiment, the present invention provides an antifungal pharmaceutical composition comprising an agent (inhibitor or activator) for a gene encoding a fungal pathogenicity-regulating kinase protein. In another embodiment, the fungus is *Cryptococcus neoformans.*

In the present invention, the pharmaceutical composition may be a composition for treating meningoencephalitis or cryptococcosis, but is not limited.

In the present invention, the agent may be an antibody. In one embodiment, the inhibitor may be an inhibitor that inhibits the activity of the protein by binding to the protein, thereby blocking signaling of the protein. For example, it may be a peptide or compound that binds to the protein. This peptide or compound may be selected by a screening method including protein structure analysis or the like and designed by a generally known method. In addition, when the inhibitor is a polyclonal antibody or monoclonal antibody against the protein, it may be produced using a generally known antibody production method.

As used herein, the term "antibody" may be a synthetic antibody, a monoclonal antibody, a polyclonal antibody, a recombinantly produced antibody, an intrabody, a multispecific antibody (including bi-specific antibody), a human antibody, a humanized antibody, a chimeric antibody, a single-chain Fv (scFv) (including bi-specific scFv), a BiTE molecule, a single-chain antibody, a Fab fragments, a F(ab') fragment, a disulfide-linked Fv (sdFv), or an epitope-binding fragment of any of the above. The antibody in the present invention may be any of an immunoglobulin molecule or an immunologically active portion of an immunoglobulin molecule. Furthermore, the antibody may be of any isotype. In addition, the antibody in the present invention may be a full-length antibody comprising variable and constant regions, or an antigen-binding fragment thereof, such as a single-chain antibody or a Fab or Fab'2 fragment. The antibody in the present invention may also be conjugated or linked to a therapeutic agent, such as a cytotoxin or a radioactive isotope.

In the present invention, the agent for the gene may be an antisense oligonucleotide, siRNA, shRNA, miRNA, or a vector comprising the same, but is not limited thereto.

In the present invention, the inhibitor may be an inhibitor that blocks signaling by inhibiting expression of the gene, or interferes with transcription of the gene by binding to the gene, or interferes with translation of mRNA by binding to mRNA transcribed from the gene. In one embodiment, the inhibitor may be, for example, a peptide, a nucleic acid, a compound or the like, which binds to the gene, and it may be selected through a cell-based screening method and may be designed using a generally known method. For example, the inhibitor for the gene may be an antisense oligonucleotide, siRNA, shRNA, miRNA, or a vector comprising the same, which may be constructed using a generally known method.

As used herein, the term "antisense oligonucleotide" means DNA, RNA, or a derivative thereof, which has a nucleic acid sequence complementary to the sequence of specific mRNA. The antisense oligonucleotide binds to a complementary sequence in mRNA and acts to inhibit the translation of the mRNA to a protein. In one embodiment, the length of the antisense oligonucleotide is 6 to 100 nucleotides, preferably 8 to 60 nucleotides, more preferably 10 to 40 nucleotides. In one embodiment of the present invention, the antisense oligonucleotide may be modified at one or more nucleotide, sugar or backbone positions in order to enhance their effect (De Mesmaeker et al., Curr Opin Struct Biol., 5(3) :343-55, 1995). The nucleic acid backbone may be modified with a phosphorothioate linkage, a phosphotriester linkage, a methyl phosphonate linkage, a short-chain alkyl intersugar linkage, a cycloalkyl intersugar linkage, a short-chain heteroatomic intersugar linkage, a heterocyclic intersugar linkage or the like. The antisense oligonucleotide may also include one or more substituted sugar moieties. The antisense oligonucleotide may include modified nucleotides. The modified nucleotides include hypoxanthine, 6-methyladenine, 5-Me pyrimidine (particularly, 5-methylcytosine, 5-hydroxymethylcytosine (HMC), glycosyl HMC, gentiobiosyl HMC, 2-aminoadenine, 2-thiouracil, 2-thiothimine, 5-bromouracil, 5-hydroxymethyluracil, 8-azaguanine, 7-deazaguanine, N6 (6-aminohexyl) adenine, 2,6-diaminopurine, and the like. In addition, the antisense oligonucleotide in the present invention may be chemically linked to one or more moieties or conjugates in order to enhance its activity or cellular uptake. In one embodiment of the present invention, the moiety may be a lipophilic moiety such as a cholesterol moiety, a cholesteryl moiety, cholic acid, thioether, thiocholesterol, an aliphatic chain, phospholipid, polyamine, a polyethylene glycol chain, adamantane acetic acid, a palmityl moiety, octadecylamine, or hexylamino-carbonyl-oxycholesterol moiety, but is not limited thereto. Oligonucleotides comprising lipophilic moieties, and methods for preparing such oligonucleotides, are well known in the field to which the present invention pertain (see US Patent Nos. 5,138,045, 5,218,105 and 5,459,255). In one embodiment of the present invention, the modified nucleic acid may increase resistance to nuclease and increase the binding affinity between antisense nucleic acid and the target mRNA. In one embodiment, the antisense oligonucleotide may generally be synthesized *in vitro* and administered *in vivo,* or synthesized *in vivo.* In an example of synthesizing the antisense oligonucleotide *in vitro,* RNA polymerase I is used. In an example of synthesizing the antisense RNA *in vivo,* a vector having origin of recognition region (MCS) in opposite orientation is used to induce transcription of antisense RNA. The antisense RNA preferably includes a translation stop codon for inhibiting translation to peptide.

As used herein, the term "siRNA" means is a nucleic acid molecule capable of mediating RNA interference or gene silencing (see WO 00/44895, WO 01/36646, WO 99/32619, WO 01/29058, WO 99/07409 and WO 00/44914). The siRNA can inhibit expression of a target gene, and thus provide an effective gene knock-down method or gene therapy method. In the present invention, the siRNA molecule may consist of a sense RNA strand (having a sequence corresponding to mRNA) and an antisense RNA strand (having a sequence complementary to mRNA) and form a duplex structure. In the present invention, the siRNA molecule may have a single-strand structure comprising self-complementary sense and antisense strands. In one embodiment of the present invention, the siRNA is not restricted to a RNA duplex of which two strands are completely paired, and it may comprise non-paired portion such as mismatched portion with non-complementary bases and bulge with no opposite bases. In one embodiment of the present invention, the overall length of the siRNA may be 10-100 nucleotides, preferably 15-80 nucleotides, more preferably 20-70 nucleotides. In the present invention, the siRNA may comprise either blunt or cohesive end, as long as it can silence gene expression. The cohesive end may have a 3'-end overhanging structure or a 5'-end overhanging structure. In the present invention, the siRNA molecule may have a structure in which a short nucleotide sequence (e.g., about 5-15 nt) is inserted between self-complementary sense and antisense strands. In this case, the siRNA molecule formed by expression of the nucleotide sequence forms a hairpin structure by intramolecular hybridization, resulting in the formation of a stem-and-loop structure.

As used herein, the term "shRNA" refers to short hairpin RNA. When an oligo DNA that connects a 3-10-nucleotide linker between the sense and complementary nonsense strands of the target gene siRNA sequence is synthesized and then cloned into a plasmid vector, or when shRNA is inserted and expressed in retrovirus, lentivirus or adenovirus, a looped hairpin shRNA is produced and converted by an intracellular dicer to siRNA that exhibits the RNAi effect. The shRNA exhibits the RNAi effect over a longer period of time than the siRNA.

As used herein, the term "miRNA (microRNA)" refers to an 18-25-nt single-stranded RNA molecule which controls gene expression in eukaryotic organisms. It is known that the miRNA binds complementarily to the target mRNA, acts as a posttranscriptional gene suppressor, and functions to suppress translation and induce mRNA destabilization.

As used herein, the term "vector" refers to a gene structure comprising a foreign DNA inserted into a genome encoding a polypeptide, and includes a DNA vector, a plasmid vector, a cosmid vector, a bacteriophage vector, a yeast vector, or a virus vector.

In one embodiment of the present invention, the pharmaceutical composition may be administered in combination with at least one azole-based antifungal agent selected from the group consisting of fluconazole, itraconazole, voriconazole and ketoconazole, or may be administered in combination with at least one non-azole-based antifungal agent selected from the group consisting of amphotericin B, natamycin, rimocidin, nystatin, flucytosine and fludioxonil.

In the present invention, the antifungal pharmaceutical composition may comprise a pharmaceutically suitable and physiologically acceptable adjuvant in addition to the active ingredient. This adjuvant may be an excipient, a disintegrant, a sweetening agent, a binder, a coating agent, a swelling agent, a lubricant, a flavoring agent, a solubilizing agent or the like.

The antifungal pharmaceutical composition according to the present invention may comprise, in addition to the active ingredient, at least one pharmaceutically acceptable carrier. In one embodiment, when the pharmaceutical composition is formulated as a liquid solution, a carrier may be used, such as saline, sterile water, Ringer's solution, buffered saline, albumin injection solution, dextrose solution, malto-dextrin solution, glycerol, ethanol, or a mixture of two or more thereof, which is sterile and physiologically suitable. If necessary, other conventional additives may be added, including antioxidants, buffers, bacteriostatic agents or the like.

In one embodiment of the present invention, the antifungal pharmaceutical composition may be formulated as injectable formulations such as aqueous solutions, suspensions, emulsions or the like, pills, capsules, granules or tablets, by use of a diluent, a dispersing agent, a surfactant, a binder or a lubricant. Furthermore, the composition may preferably be formulated using a suitable method as disclosed in Remington's Pharmaceutical Science, Mack Publishing Company, Easton PA, depending on each disease or components. In one embodiment of the present invention, the pharmaceutical composition may be formulated in the form of granules, powders, coated tablets, tablets, capsules, suppositories, syrups, juices, suspensions, emulsions, drops, injectable liquid formulations, or sustained-release formulations of the active ingredient, or the like. The pharmaceutical composition of the present invention may be administered in a conventional manner by an intravenous, intra-arterial, intraperitoneal, intramuscular, intrasternal, transdermal, intranasal, inhalation, topical, intrarectal, oral, intraocular or intradermal route.

In the present invention, the effective amount of the active ingredient in the pharmaceutical composition of the present invention means an amount required to prevent or treat a disease. Thus, the effective amount may be adjusted depending on various factors, including the kind of disease, the severity of the disease, the kinds and contents of the active ingredient and other ingredients contained in the composition, the type of formulation, the patient's age, weight, general health state, sex and diet, the period of administration, the route of administration, the secretion rate of the composition, treatment time, and concurrently used drugs.

### [Advantageous Effects]

According to the present invention, novel antifungal agent candidates can be effectively screened using kinases. In addition, using an antifungal pharmaceutical composition comprising an agent (antagonist or antagonist) for kinase according to the present invention, fungal infection can be effectively prevented, treated and/or diagnosed.

### [Description of Drawings]

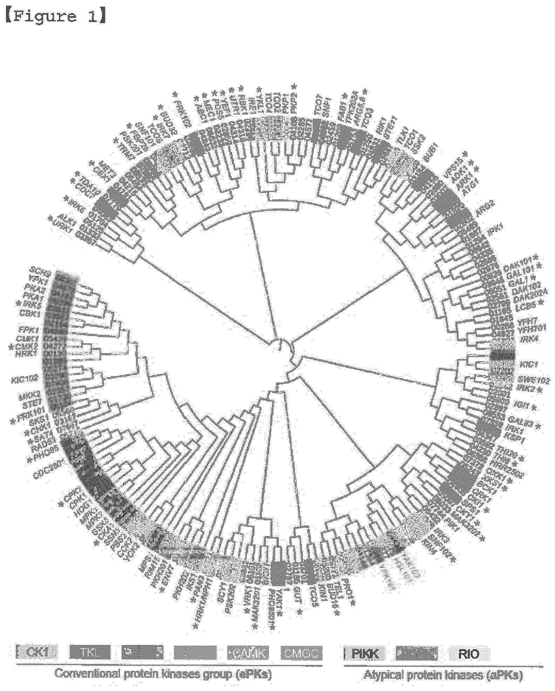

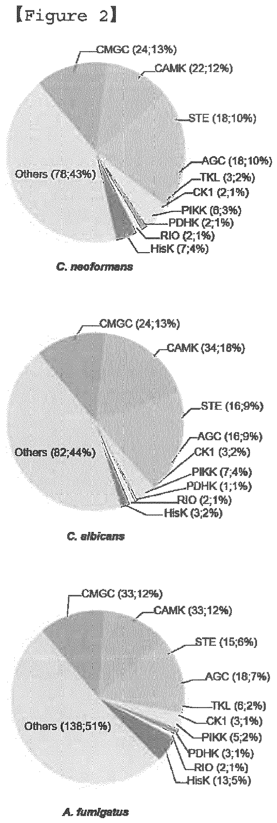

FIG. 1 shows the phylogenetic correlation among protein kinases in *Cryptococcus neoformans,* and FIG. 2 shows a comparison of major kinases in *Cryptococcus neoformans, C. albicans* and *A. fumigatus.* Regarding FIG. 1, protein sequence-based alignment was performed using ClustalX2 (University College Dublin). Using this alignment data, the phylogenetic tree was illustrated by Interactive Tree Of Life (http://itol.embl.de) (Letunic, I. & Bork, P. Interactive Tree Of Life v2: online annotation and display of phylogenetic trees made easy. Nucleic Acids Res 39, W475-478, doi:10.1093/nar/gkr201 (2011)). Among the 183 kinases found in *C. neoformans,* the present inventors constructed 114 gene-deletion kinases, and the kinases named based on the nomenclature rules for *S. cerevisiae* genes. The different colour codes represent the different classes of protein kinases predicted by Kinomer 1.0 (http://www.compbio.dundee.ac.uk/kinomer) (Martin, D. M., Miranda-Saavedra, D. & Barton, G. J. Kinomer v. 1.0: a database of systematically classified eukaryotic protein kinases. Nucleic Acids Res 37, D244-250, doi:10.1093/nar/gkn834 (2009)). Red marked genes indicate the 60 pathogenicity-related kinases, and the distribution of these kinases for total kinases and various classes. FIG. 2 is a Pie-chart for the kinase classes predicted by Kinomer 1.0 to reveal the relative portion of protein kinase classes in human infectious fungal pathogens, *C. neoformans, Candida albicans* and *Aspergillus fumigatus.*

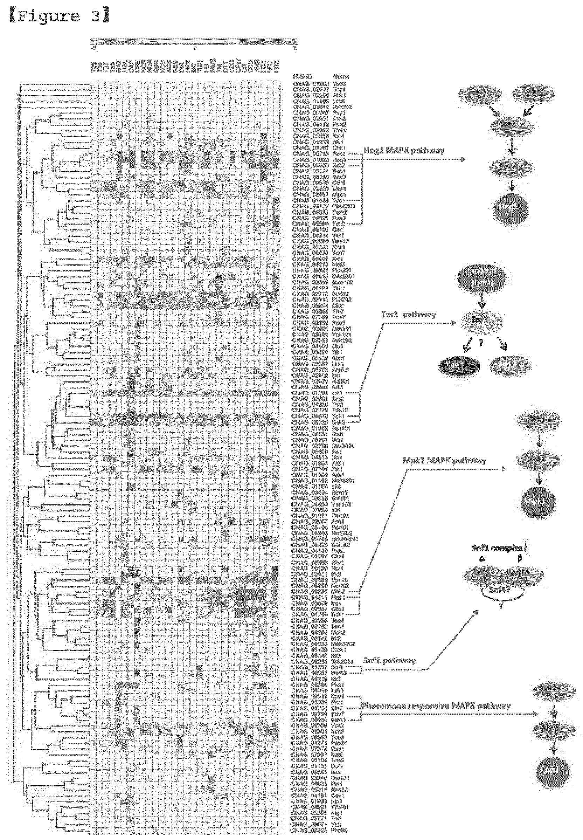

FIG. 3 shows phenotypic clustering of protein kinases in *Cryptococcus neoformans.* The phenotypes were scored by seven grades (-3: strongly sensitive/reduced, -2: moderately sensitive/reduced, -1: weakly sensitive/reduced, 0: wild-type like, +1: weakly resistant/increased, +2: moderately resistant/increased, +3: strongly resistant/increased). The excel file containing the phenotype scores of each kinase mutant was loaded by Gene-E software (http://www.broadinstitute.org/cancer/software/GENE-E/) and then the kinase phenome clustering was drawn using one minus Pearson correlation. The abbreviations used in FIG. 3 have the following meanings: [T25: 25°C, T30: 30°C, T37: 37°C, T39: 39°C, CAP: capsule production; MEL: melanin production; URE: urease production; MAT: mating filamentation, HPX: hydrogen peroxide, TBH: tert-butyl hydroperoxide, MD: menadione, DIA: diamide, MMS: methyl methanesulfonate, HU: hydroxyurea, 5FC: 5-flucytosine, AMB: amphotericin B, FCZ: fluconazole, FDX: fludioxonil, TM: tunicamycin, DTT: dithiothreitol, CDS: cadmium sulfate, SDS: sodium dodecyl sulfate, CR: Congo red, CFW: calcofluor white, KCR: YPD + KCl, NCR: YPD + NaCl, SBR: YPD + sorbitol, KCS: YP + KCl, NCS: YP + NaCl, SBS: YP + sorbitol].

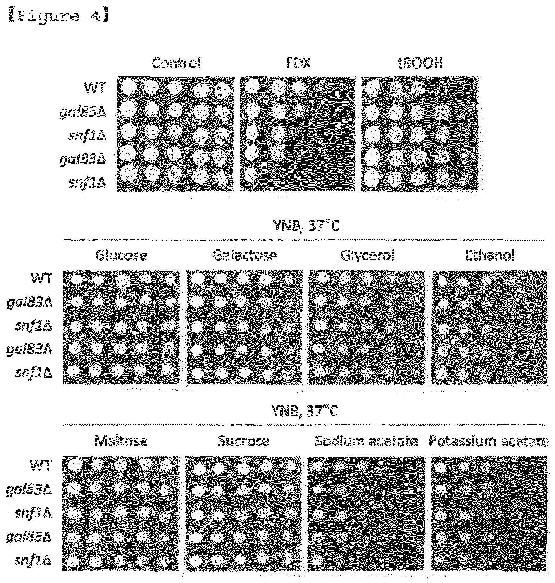

FIG. 4 shows the phenotypic traits of *gal83Δ* mutant and *snf1Δ* mutant. FIG. 4a shows the results of comparing the phenotypic traits between a wild-type strain and *snf1Δ* and *ga183Δ* mutants under various stress conditions, and indicates that in 1 µg/ml fludioxonil (FDX), the *snf1Δ* and *gal83Δ* mutants showed increased susceptibility compared to the wild-type strain, and in 0.65 mM tert-butyl hydroperoxide (tBOOH), the *snf1Δ* and *gal83Δ* mutants showed increased resistance compared to the wild-type strain. FIG. 4b shows the results of comparing carbon source utilization between a wild-type strain and *snf1Δ* and *ga183A* mutants. An experiment was performed under the conditions of 2% glucose, 2% galactose, 3% glycerol, 3% ethanol, 2% maltose, 2% sucrose, 2% sodium acetate, and 1% potassium acetate, and the experimental results indicated that the *snf1Δ* and *ga183Δ* mutants required ethanol, sodium acetate and potassium acetate as carbon sources.

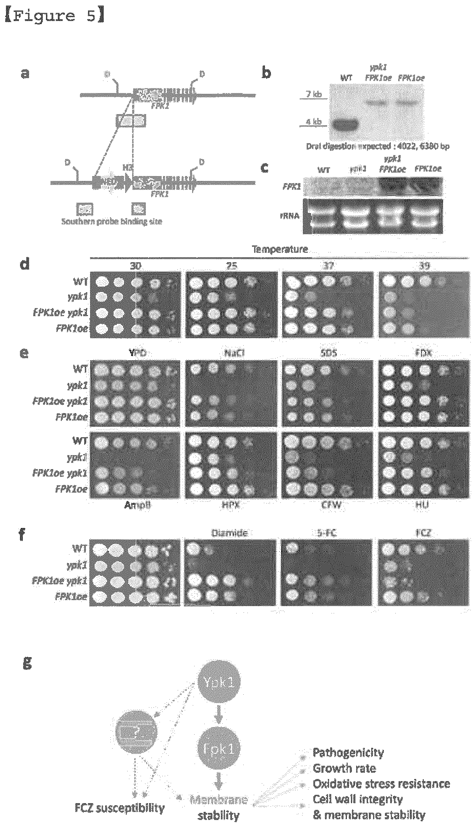

FIG. 5 shows the results of an experiment performed to examine whether Fpk1 regulates Ypk1-dependent phenotypes in the pathogenicity of *Cryptococcus neoformans.* (a) A scheme for the replacement of the FPK1 promoter with histone H3 promoter to construct an FPK1-overexpressing strain. (b) The *FPK1* overexpressing strain was analyzed by Southern blot analysis, and YSB3986 and YSB3981 strains were produced by overexpressing *FPK1* using a *ypk1Δ* mutant as a parent strain. (c) Overexpression of FPK1 was verified by Northern blot analysis. rRNA was used as a loading control. (d) WT strain (H99S), *ypk1Δ* (YSB1736) mutant, and FPK1 overexpression strains (YSB3986 and YSB3981) were cultured in YPD liquid medium for 16 hours, spotted on YPD medium, and incubated at the indicated temperature to observe the degree of growth. (e and f) The strains were tested on YPD medium containing 1.5 M NaCl, 0.04% sodium dodecyl sulphate, 1 µg/ml fluorodioxonil, 1 µg/ml amphotericin B, 3 mM hydrogen peroxide, 3 mg/ml calcofluor white, 100 mM hydroxyurea, 2 mM diamide, 300 µg/ml flucytosine and 5 mg/ml fluconazole. Cells were further incubated at 30°C for 3 days and photographed. (g) The regulatory model for Ypk1 and Fpkl kinases in C. neoformans, which can be proposed based on the experimental results.

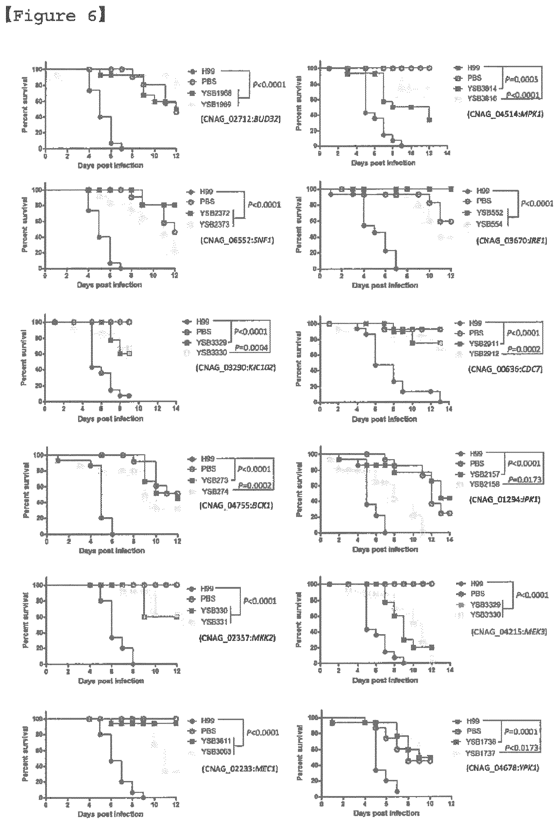

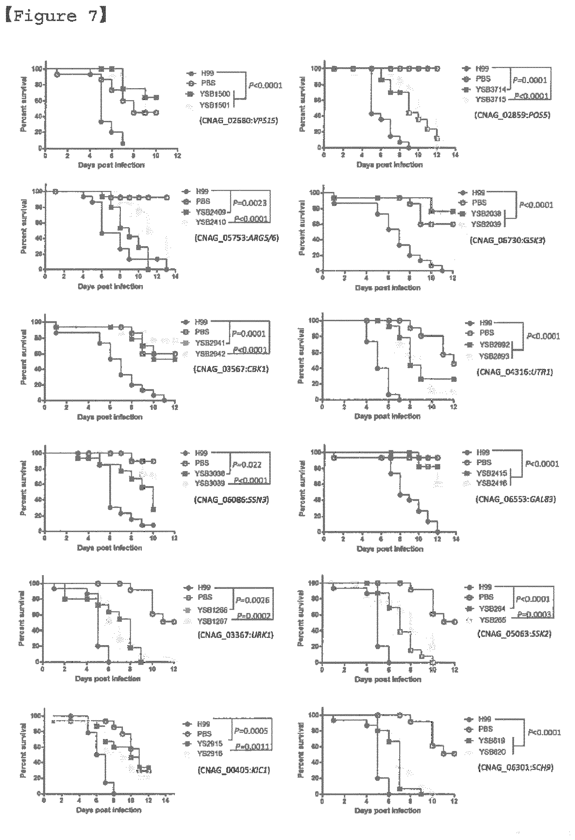

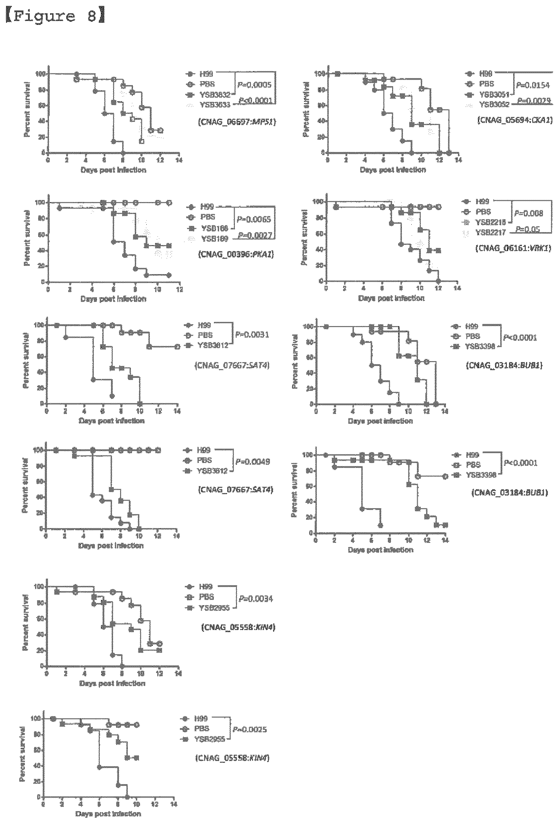

FIGS. 6, 7 and 8 show the results of identifying pathogenic kinases by insect killing assay. Each mutant was grown for 16 hours in liquid YPD medium, washed three times with PBS buffer, and then inoculated into *G. mellonella* larva using 4,000 mutant cells per larva (15 larvae per group). The infected larvae were incubated at 37°C and monitored for their survival each day. Statistical analysis of the experimental results was performed using the Log-rank (Mantel-Cox) test. FIGS. 6, 7 and 8a show the survival data of two independent mutants for each kinase. FIG. 8b shows the results of two repeated experiments for kinases from which only one mutant was produced.

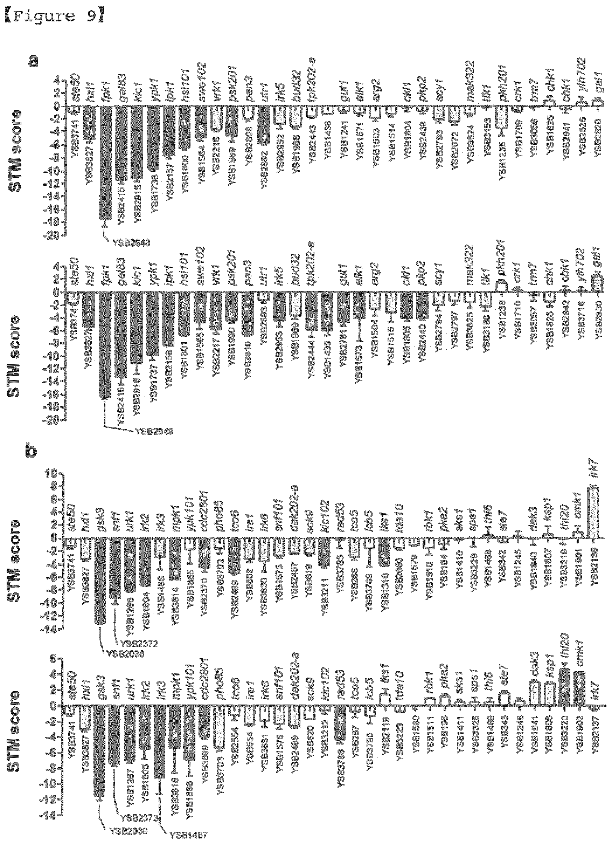

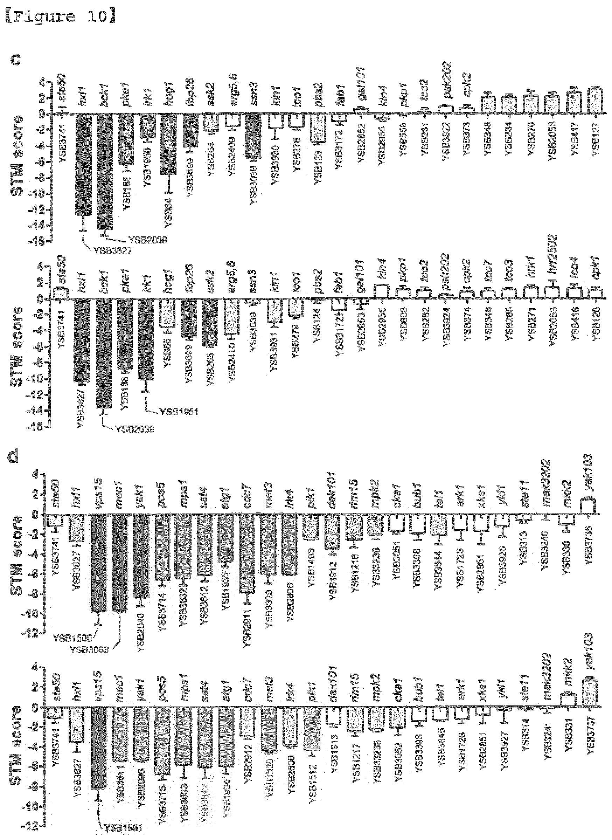

FIGS. 9 and 10 shows the results of a signature-tag mutagenesis (STM)-based murine model virulence test. In the STM study, *ste50Δ* and *hxl1Δ* strains were used as virulent and non-virulent control strains. STM scores were measured by using qPCR analysis using the STM-specific primers listed in Table 2 below for three-independent biological replicates. (a-d) All the kinase mutants were divided into four sets. The genes of each set consisted of two-independent mutants, and when one mutant was present, two independent experiments were performed.

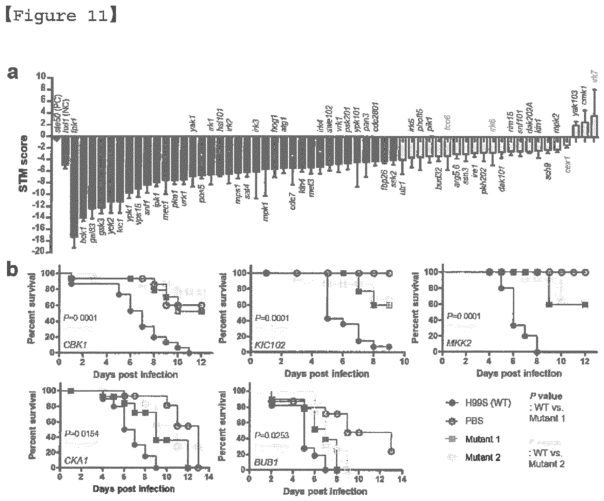

FIG. 11 summarizes the pathogenicity-related kinases in *Cryptococcus neoformans.* STM scores were calculated by the quantitative PCR method, arranged numerically and coloured in gradient scales (FIG. 11a). Red marked letters show the novel infectivity-related kinases revealed by this analysis. Gene names for the 25 kinases that were co-identified by both insect killing and STM assays were depicted below the STM zero line. The *P*-value between control and mutant strains was determined by one-way analysis of variance (ANOVA) employing Bonferroni correlation with three mice per each STM set. Each set was repeated twice using independent strains. For single strain mutants, two independent experiments were repeatedly performed using each single strain. In the STM study, the roles of a total of 54 kinases in the infectivity of C. neoformans were analyzed. Referring to FIGS. 5 to 8, a total of 6 kinases were not shown to be involved in pathogenicity regulation in the murine model infectivity test, but were shown to be pathogenicity-related kinases by the wax moth killing assay (FIG. 11b). For bub1 and kin4 single mutant strains, the experiment was repeated twice.

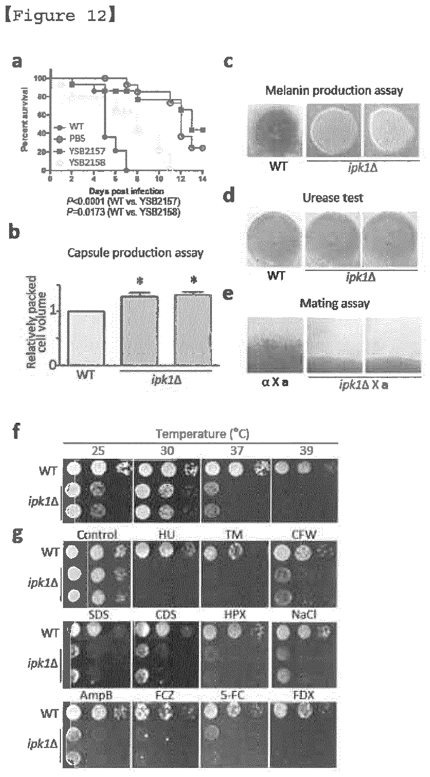

FIG. 12 shows the pleiotropic roles of Ipk1 in *Cryptococcus neoformans.* Using WT (wild-type) and *ipk1Δ* mutants (YSB2157 and YSB2158), various experiments were performed. In FIG. 12a, *ipk1*Δ mutants (YSB2157 and YSB2158) showed attenuated virulence in the insect-based *in vivo* virulence assay. In this assay, WT and PBS were used as controls. In FIG. 12b, ipk1Δ mutants showed increased capsule production. Cells, incubated overnight, were placed on a DME plate at 37°C for 2 days. 50 µl of 1.5 X 10⁸ cells were packed into each capillary tube, and the packed cell volume was monitored every day. After 3 days when the cells were precipitated by gravity, the packed cell volume in the total volume was calculated and normalized to WT. The P value of each strain was less than 0.05. (*) Error bars indicate SEM. In FIG. 12c, *ipk1Δ* mutants show melanin-deficient phenotypes. Melanin production was assayed on Niger seed plates containing 0.2% glucose after 3 days. In FIG. 12d, *ipk1Δ* deletion mutants show defects in urease production. Urease production was assayed on Christensen's agar media at 30°C after 2 days. In FIG. 12e, ipk1Δ mutants display severe defects in mating. Mating was assayed on V8 media (pH 5, per L: V8 juice 50 ml (Campbell), KH₂PO₄ (Bioshop, PPM302) 0.5 g, agar (Bioshop, AGR001.500) 40 g) plate for 9 days. FIGS. 12f and 12g are micrographs obtained from 10-fold diluted spot analysis (10² to 10⁵-fold dilution). Growth rate was measured under various growth conditions indicated on the photographs. For analysis of chemical susceptibility, YPD medium was treated with the following chemicals: HU; 100 mM hydroxyurea as DNA damage reagent, TM; 0.3 ug/ml tunicamycin as ER (endoplasmic reticulum) stress inducing reagent, CFW; 3 mg/ml calcofluor white as cell wall damage reagent, SDS; 0.03% sodium dodecyl sulfate for membrane stability testing, CDS; 30 M CdSO₄ as heavy metal stress reagent, HPX; 3 mM hydrogen peroxide as oxidizing reagent, 1M NaCl for osmotic shock, and 0.9 ml/mg AmpB (amphotericin B), 14 µg/ml FCZ (fluconazole), 300 µg/ml 5-FC (flucytosine), and 1 µg/ml FDX (fludioxonil) for analysis of antifungal agent susceptibility.

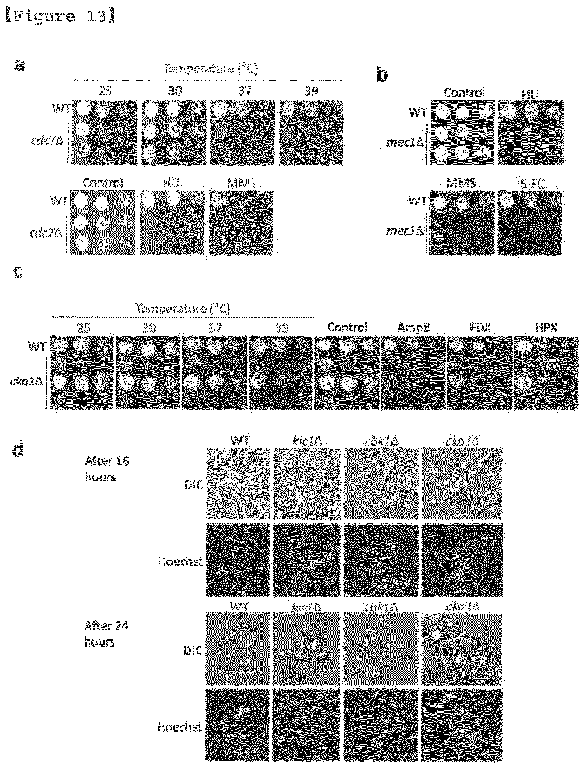

FIG. 13 shows the results of experiments using *cdc7Δ*, *cbk1Δ* and *kic1Δ* mutants. (a-c) *cdc7Δ* mutants (YSB2911, YSB2912), *met1Δ* mutants (YSB3063, YSB3611) and cka1 (YSB3051, YSB3052) were grown overnight in YPD medium, diluted 10-fold serially, and spotted on solid YPD medium and a YPD medium containing 100 mM hydroxyurea (HU), 0.06% methyl methanesulphonate (MMS), 1 µg/ml amphotericin B (AmpB), 1 µg/ml fludioxonil (FDX), 3 mM hydrogen peroxide (HPX) and 300 ug/ml flucytosine (5-FC). The spotted cells were further incubated at 30°C or the indicated temperatures for 3 days and then photographed, (d) Wild-type and *kic1Δ* (YSB2915, YSB2916), *cbk1Δ* (YSB2941, YSB2942) and *cka1Δ* (YSB3051, YSB3052) mutants were incubated in YPD medium for 16 hours or more, and then fixed with 10% paraformaldehyde for 15 minutes and washed twice with PBS solution. The fixed cells were stained with 10 µg/ml Hoechst solution (Hoechst 33342, Invitrogen) for 30 minutes, and then observed with a fluorescence microscope (Nikon eclipse Ti microscope).

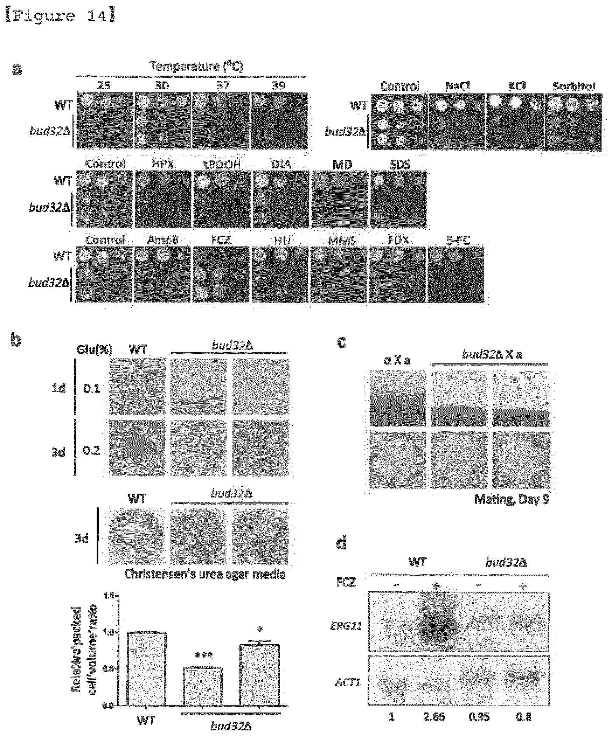

FIG. 14 shows the results of experiments on *bud32Δ* mutants. (a) Wild-type and *bud32Δ* mutants (YSB1968, YSB1969) were incubated overnight in YPD medium, diluted 10-fold serially, and then spotted on YPD medium containing the following chemicals, and observed for their growth rate under various growth conditions: 1.5 M NaCl, 1.5 M KCl, 2 M sorbitol, 1 µg/ml amphotericin B (AmpB), 14 µg/ml fluconazole (FCZ), 1 µg/ml fludioxonil (FDX), 300 µg/ml flucytosine, 100 mM hydroxyurea (HU), 0.04% methyl methanesulphonate (MMS), 3 mM hydrogen peroxide (HPX), 0.7 mM tert-butyl hydroperoxide (tBOOH), 2 mM diamide (DIA), 0.02 mM menadione (MD), and 0.03% sodium dodecyl sulphate (SDS). The cells spotted on the YPD medium containing these chemicals were further incubated at 30°C, and then photographed. (b) Melanin production of wild-type and *bud32Δ* mutants was assayed on Niger seed plates containing 0.1% glucose, and urease production was assayed after incubation on Christensen's agar media at 30°C. To examine capsule production, cells incubated overnight were placed on a DME plate at 37°C for 2 days. 50 µl of 1.5 X 10⁸ cells were packed into each capillary tube, and after 3 days, the packed cell volume was monitored every day by gravity. The packed cell volume in the total volume was calculated and normalized to WT. The results were analyzed by one-way analysis of variance (ANOVA) employing Bonferroni correlation, and the analysis was repeated three times. (c) To examine the mating efficacy, wild-type and *bud32Δ* mutants were spotted onto V8 mating medium and then incubated at room temperature in the dark for 9 days. (d) WT and *bud32Δ* mutants grown at 30°C to the logarithmic phase and then were treated with or without fluconazole (FCZ) for 90 min. Total RNA was extracted from each sample, and the expression level of *ERG11* was analyzed by Northern blotting.

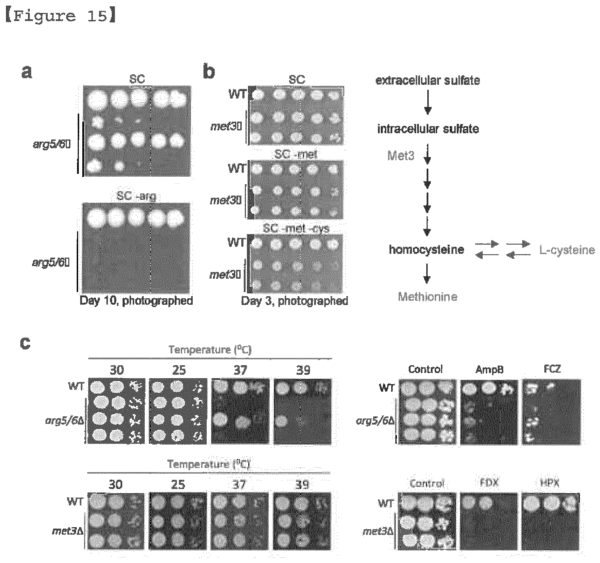

FIG. 15 shows the results of experiments on *arg5,6Δ* mutants and *met3Δ.* (a, b) Wild-type (H99S), *arg5,6Δ* mutants (YSB2408, YSB2409, YSB2410) and *met3Δ* mutants (YSB3329, YSB3330) were incubated overnight in YPD medium and then washed with PBS. The washed cells were diluted 10-fold serially and spotted on solid synthesis complete medium. [SC; yeast nitrogen base without amino acids (Difco) supplemented with the indicated concentration of the following amino acids and nucleotides: 30 mg/l L-isoleucine, 0.15 g/l L-valine, 20 mg/l adenine sulphate, 20 mg/l L-histidine-HCl, 0.1 g/l L-leucine, 30 mg/l L-lysine, 50 mg/l L-phenylalanine, 20 mg/l L-tryptophan, 30 mg/l uracil, 0.4 g/l L-serine, 0.1 g/l glutamic acid, 0.2 g/l L-threonine, 0.1 g/l L-aspartate, 20 mg/l L-arginine, 20 mg/l L-cysteine, and 20 mg/l L-methionine]. SC-arg (a), SC-met and SC-met-cys (b) media indicate the SC medium lacking arginine, methionine and/or cysteine supplements. (b) A schematic view showing methionine and cysteine biosynthesis pathways. (c) Wild-type, arg5,6Δ mutants and met3Δ mutants were incubated overnight in YPD medium, diluted 10-fold serially, and then spotted on YPD medium containing the following chemicals, and observed for their growth rate under various growth conditions: 1 ug/ml amphotericin B (AmpB), 14 µg/ml fluconazole (FCZ), 1 µg/ml fludioxonil (FDX), and 3 mM hydrogen peroxide (HPX). The spotted cells were incubated at 30°C or indicated temperature for 3 days, and then photographed.

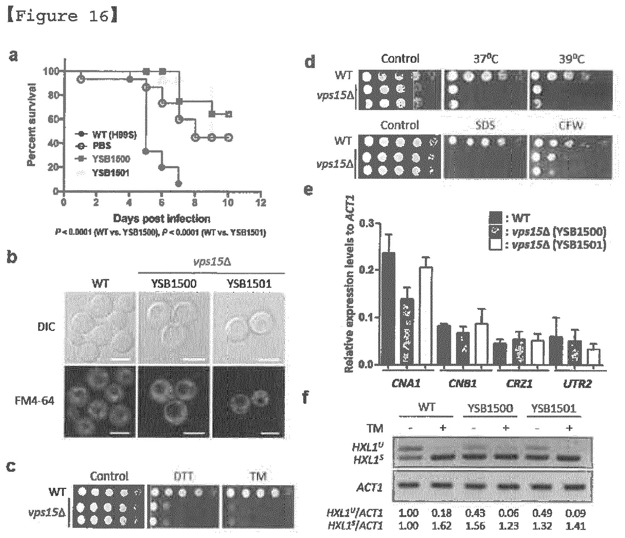

FIG. 16 shows retrograde vacuole trafficking that controls the pathogenicity of *Cryptococcus neoformans.* Retrograde vacuole trafficking controls the pathogenicity of *Cryptococcus neoformans.* Various tests were performed using WT and *vps15Δ* mutants [YSB1500, YSB1501] . In FIG. 16a, Vps15 is required for virulence of *C. neoformans.* WT and PBS were used as positive and negative virulence controls, respectively. In FIG. 16b, *vps15*Δ mutants display enlarged vacuole morphology. Scale bars indicate 10µm. In FIG. 16c, *vps15Δ* mutants show significant growth defects under ER stresses. Overnight cultured cells were spotted on the YPD medium containing 15mM dithiothreitol (DTT) or 0.3 µg/ml tunicamycin (TM), further incubated at 30°C for 3 days, and photographed. In FIG. 16d, *vps15Δ* mutants show significant growth defects at high temperature and under cell membrane/wall stresses. Overnight cultured cells were spotted on the YPD medium and further incubated at the indicated temperature or spotted on the YPD medium containing 0.03% SDS or 5mg/ml calcofluor white (CFW) and further incubated at 30 °C. Plates were photographed after 3 days. In FIG. 16e, Vps15 is not involved in the regulation of the calcineurin pathway in *C. neoformans.* For quantitative RT-PCR (qRT-PCR), RNA was extracted from three biological replicates with three technical replicates of WT and *vps15*Δ mutants. CNA1, CNB1, CRZ1, UTR2 expression levels were normalized by *ACT1* expression levels as controls. Data were collected from the three replicates. Error bars represent SEM (standard error of means). In FIG. 16f, Vps15 negatively regulates the *HXL1* splicing. For RT-PCR, RNA was extracted from WT and *vps15*Δ mutants and cDNA was synthesized. *HXL1* and *ACT1*-specific primer pairs were used for RT-PCR (Table 3). This experiment was repeated twice and one representative experiment is presented.

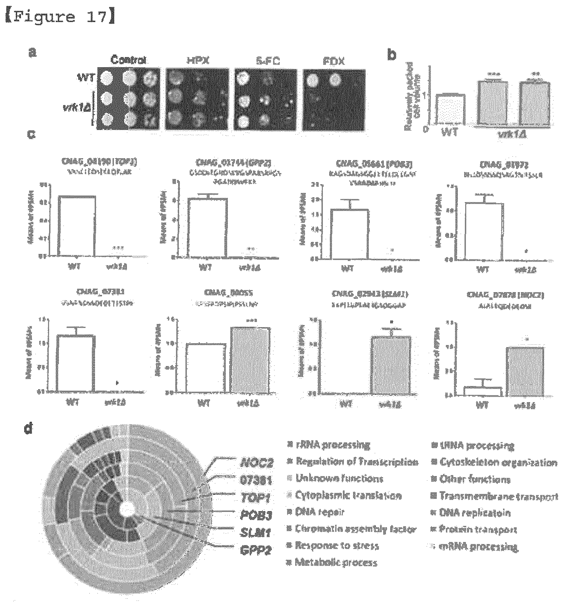

FIG. 17 shows the results of experiments on *vrk1Δ* mutants. FIG. 17a shows the results of spotting WT and *vrk1Δ* strains on YPD medium and on YPD medium containing 2.5 mM hydrogen peroxide (HPX), 600 µg/ml flucytosine (5-FC) or 1 µg/ml fludioxonil (FDX). The strains were incubated at 30°C for 3 days and photographed. FIG. 17b shows the results of relative quantification of the packed cell volume. Three independent measurements shows a significant difference between WT and *vrk1Δ* strains (***; 0.0004 and **; 0.0038, s.e.m). FIG. 17c shows relative quantification of Vrk1-mediated phosphorylation. Peptide samples were analyzed three times on average, and peptides were obtained from two independent experiments. The data is the mean ± s.e.m of two independent experiments. Student's unpaired t-test was applied for determination of statistical significance. ***P<0.001, **P<0.01, *P<0.05. PSMs represent peptide spectrum matching.

### [Best Mode]

In one embodiment of the present invention, there is provided a method for screening an antifungal agent, comprising the steps of: (a) bringing a sample to be analyzed into contact with a cell containing a pathogenicity-regulating kinase protein or a gene encoding the protein; (b) measuring the amount or activity of the protein or the expression level of the gene; and (c) determining that the sample is an antifungal agent, when the amount or activity of the protein or the expression level of the gene is measured to be downregulated or up-regulated.

In the method for screening the antifungal agent, the pathogenicity-regulating kinase protein may be one or more selected from the group consisting of BUD32, ATG1, CDC28, KIC1, MEC1, KIN4, MKK1/2, BCK1, SNF1, SSK2, PKA1, GSK3, CBK1, KIN1, SCH9, RIM15, HOG1, YAK1, IPK1, CDC7, SSN3, CKA1, MEC1, ARG5,6P, MET3, VPS15 and VRK1.

In another embodiment of the present invention, the cell used in screening of the antifungal agent is a *Cryptococcus neoformans* cell, and the antifungal agent is an antifungal agent for treating meningoencephalitis or cryptococcosis.

In another embodiment of the present invention, there is provided an antifungal pharmaceutical composition comprising an antagonist or inhibitor of the *Cryptococcus neoformans* pathogenicity-regulating kinase protein or an antagonist or inhibitor of the gene encoding the protein. In this regard, the pathogenicity-regulating kinase protein may be one or more selected from the group consisting of BUD32, ATG1, CDC28, KIC1, MEC1, KIN4, MKK1/2, BCK1, SNF1, SSK2, PKA1, GSK3, CBK1, KIN1, SCH9, RIM15, HOG1, YAK1, IPK1, CDC7, SSN3, CKA1, MEC1, ARG5, 6P, MET3, VPS15 and VRK1.

In still another embodiment of the present invention, the antifungal pharmaceutical composition is for treating meningoencephalitis or cryptococcosis, and the antagonist or inhibitor may be a small molecule; an antibody against the protein; or an antisense oligonucleotide, siRNA, shRNA, miRNA, or a vector comprising one or more of these, against the gene.

In yet another embodiment of the present invention, the antifungal pharmaceutical composition is an antifungal pharmaceutical composition to be administered in combination with an azole-based or non-azole-based antifungal agent. The azole-based antifungal agent may be at least one selected from the group consisting of fluconazole, itraconazole, voriconazole and ketoconazole. In addition, the non-azole-based antifungal agent may be at least one selected from the group consisting of amphotericin B, natamycin, rimocidin, nystatin and fludioxonil.

### [Mode for Invention]

Hereinafter, the present invention will be described in further detail with reference to examples. It will be obvious to those skilled in the art that these examples are for illustrative purposes and are not intended to limit the scope of the present invention.

Animal care and all experiments were conducted in accordance with the ethical guidelines of the Institutional Animal Care and Use Committee (IACUC) of Yonsei University. The Yonsei University IACUC approved all of the vertebrate studies.

### Examples

### Example 1: Identification of Protein Kinases in Cryptococcus neoformans

To select the putative kinase genes in the genome of *C. neoformans* var. *grubii* (H99 strain), two approaches were used. The first approach used was Kinome v. 1.0 database (www.compbio.dundee.ac.uk/kinomer/) which systematically predicts and classifies eukaryotic protein kinases based on a highly sensitive and accurate hidden Markov model (HMM)-based method (Martin, D. M., Miranda-Saavedra, D. & Barton, G. J. Kinomer v. 1.0: a database of systematically classified eukaryotic protein kinases. Nucleic Acids Res 37, D244-250, doi:10.1093/nar/gkn834, 2009). Through the Kinome database, 97 putative kinases in the genome of serotype D *C. neoformans* (JEC21 strain) were predicted. The ID of each JEC21 kinase gene was mapped with the H99 strain based on the most recent genome annotation (version 7), 95 putative kinases were queried. However, it was shown that this Kinome list was incomplete, because it failed to present all histidine kinases and some known kinases such as Hog1. For this reason, the present inventors surveyed a curated annotation of kinases in the H99 genome database provided by the Broad Institute (www.broadinstitute.org/annotation/genome/cryptococcus_neoform ans) and the JEC21 genome database within the database of the National Center for Biotechnology Information. For each gene that had a kinase-related annotation, the present inventors performed protein domain analyses using Pfam (http://pfam.xfam.org/) to confirm the presence of kinase domains and to exclude the genes with annotations such as phosphatases or kinase regulators. Through this analysis, 88 additional putative kinases genes were queried. As a result, 183 putative kinase genes in *C. neoformans* were retrieved. The phylogenetic relationship thereof is shown in FIG. 1.

Eukaryotic protein kinase superfamilies are further classified into six conventional protein kinase groups (ePKs) and three atypical groups (aPKs) (Miranda-Saavedra, D. & Barton, G. J. Classification and functional annotation of eukaryotic protein kinases. Proteins 68, 893-914, doi:10.1002/prot.21444, 2007). ePKs include the AGC group (including cyclic nucleotide and calcium-phospholipid-dependent kinases, ribosome S6-phosphoprylated kinases, G protein-linked kinases and all similar analogues of these sets), CAMKs (calmodulin-regulated kinases); the CK1 group (casein kinase 1, and similar analogues), the CMGC group (including cyclin-dependent kinases, mitogen-activated protein kinases, glycogen synthase kinases and CDK-like kinases), the RGC group (receptor guanylate cyclase), STEs (including many kinase functions in the MAP kinase cascade), TKs (tyrosine kinases) and TKLs (tyrosine kinase-like kinases) (FIGS. 1 and 2). The aPKs include the alpha-kinase group, PIKK (phosphatidylinositol 3-kinase-related kinase group), RIO and PHDK (pyruvate dehydrogenase kinase group). To classify 183 *C. neoformans* protein kinases based on these criteria, the present inventors queried their amino acid sequences in the Kinomer database. Some of the previously classified kinases (Martin, D. M., Miranda-Saavedra, D. & Barton, G. J. Kinomer v. 1.0: a database of systematically classified eukaryotic protein kinases. Nucleic Acids Res 37, D244-250, doi:10.1093/nar/gkn834, 2009) were classified otherwise (14 out of 95), presumably due to sequence differences between JEC21 and H99. Most of other kinases identified by annotation did not correspond to the previous category (82 out of 88), and were classified as "others". Therefore, it was found that the C. *neoformans* genome consists of 89 ePKs (18 AGC, 22 CAMK, 2 CK1, 24 CMGC, 2 PDHK, 18 STE, 3 TKL), 10 aPKs (2 PDHK, 6 PIKK, 2 RIO), and 84 "others" (FIG. 1). The others include 7 histidine kinases (FIGS. 1 and 2). Based on prediction by the HMMER sequence profiles of Superfamily (version 1.73) (Wilson, D. et al. SUPERFAMILY--sophisticated comparative genomics, data mining, visualization and phylogeny. Nucleic Acids Res 37, D380-386, doi:10.1093/nar/gkn762 (2009)), it was shown that two human fungal pathogens, *C. albicans* and *A. fumigatus,* have 188 and 269 protein kinases, respectively. Among pathogenic fungal protein kinases, CMGC (12-13%), CAMK (12-18%), STE (6-10%) and AGC (6-10%) kinases appear to be the most common clades (FIGS. 1 and 2).

Given that most eukaryotic genomes are predicted to contain kinase at a ratio of about 1-2% of the genome, the protein kinase ratio of *C. neoformans* (~2.6%) was higher than expected. This indicates that *C. neoformans* has both saprobic and parasitic life cycles in which pathogenic yeast is in contact with more diverse environmental signals and host signals. Nevertheless, it is still necessary to explain whether all these predicted kinases have biologically significant kinase activity. The phylogenetic comparison of 183 putative kinases in *C. neoformans* with those in other strains and higher eukaryotes suggest that kinases much more evolutionarily conserved than transcription factors (TFs) in strains and other eukaryotes. In conclusion, the kinome network appears to be evolutionarily conserved in at least sequence similarity among fungi, which is in sharp contrast to evolutionary distribution of TF networks.

### Example 2: Construction of Kinase Gene-Deletion Mutant Library in C. neoformans

To gain insights into the biological functions of *Cryptococcus* kinome networks and the complexity thereof, the present inventors constructed gene-deletion mutants for each kinase and functionally characterized them. Among the kinases analyzed here, mutants for 22 kinases (TCO1, TCO2, TCO3, TCO4, TCO5, TCO7, SSK2, PBS2, HOG1, BCK1, MKK1/2, MPK1, STE11, STE7, CPK1, PKA1, PKA2, HRK1, PKP1, IRE1, SCH9, and YPK1) were already functionally characterized in part by the present inventor. (Bahn, Y. S., Geunes-Boyer, S. & Heitman, J. Ssk2 mitogen-activated protein kinase governs divergent patterns of the stress-activated Hog1 signaling pathway in Cryptococcus neoformans. Eukaryot. Cell 6, 2278-2289 (2007); Bahn, Y. S., Hicks, J. K., Giles, S. S., Cox, G. M. & Heitman, J. Adenylyl cyclase-associated protein Aca1 regulates virulence and differentiation of Cryptococcus neoformans via the cyclic AMP-protein kinase A cascade. Eukaryot. Cell 3, 1476-1491 (2004); Bahn, Y. S., Kojima, K., Cox, G. M. & Heitman, J. Specialization of the HOG pathway and its impact on differentiation and virulence of Cryptococcus neoformans. Mol. Biol. Cell 16, 2285-2300 (2005); Bahn, Y. S., Kojima, K., Cox, G. M. & Heitman, J. A unique fungal two-component system regulates stress responses, drug sensitivity, sexual development, and virulence of Cryptococcus neoformans. Mol. Biol. Cell. 17, 3122-3135 (2006); Kim, H. et al. Network-assisted genetic dissection of pathogenicity and drug resistance in the opportunistic human pathogenic fungus Cryptococcus neoformans. Scientific reports 5, 8767, doi:10.1038/srep08767 (2015); Kim, M. S., Kim, S. Y. , Yoon, J. K., Lee, Y. W. & Bahn, Y. S. An efficient gene-disruption method in Cryptococcus neoformans by double-joint PCR with NAT-split markers. Biochem. Biophys. Res. Commun. 390, 983-988, doi:S0006-291X(09)02080-4 [pii]10.1016/j.bbrc.2009.10.089 (2009); Kim, S. Y. et al. Hrk1 plays both Hogl-dependent and - independent roles in controlling stress response and antifungal drug resistance in Cryptococcus neoformans. PLoS One 6, e18769, doi:doi:10.1371/journal.pone.0018769 (2011); Kojima, K., Bahn, Y. S. & Heitman, J. Calcineurin, Mpk1 and Hog1 MAPK pathways independently control fludioxonil antifungal sensitivity in Cryptococcus neoformans. Microbiology 152, 591-604 (2006); Maeng, S. et al. Comparative transcriptome analysis reveals novel roles of the Ras and cyclic AMP signaling pathways in environmental stress response and antifungal drug sensitivity in Cryptococcus neoformans. Eukaryot. Cell 9, 360-378, doi:EC.00309-09 [pii];10.1128/EC.00309-09 (2010); Cheon, S. A. et al. Unique evolution of the UPR pathway with a novel bZIP transcription factor, Hxl1, for controlling pathogenicity of Cryptococcus neoformans. PLoS Pathog. 7, e1002177, doi:10.1371/journal.ppat.1002177 (2011)).

For the remaining 161 kinases, the present inventors constructed gene-deletion mutants by using large-scale homologous recombination and by analyzing their *in vitro* and *in vivo* phenotypic traits. The constructed mutant was deposited (accession number: KCCM 51297).

In order to perform a large-scale virulence test in mouse hosts, dominant nourseothricin-resistance markers (NATs) containing a series of signature tags (Table 1) were employed. Southern blot analysis was performed to verify both the accurate gene deletion and the absence of any ectopic integration of each gene-disruption cassette. Table 1 below shows 26 kinase gene-deletion strains.

**Table 1**

| CNAG_Num. | GENE NAME | YSB# | GENOTYPE |
|---|---|---|---|
| CNAG_00047 | *PKP1* | *558, 608* | *MATα* pkp1Δ*::NAT-STM#224* |
| CNAG_00106 | *TC05* | *286, 287* | *MATα* tco5Δ*::NAT-STM#125* |
| CNAG_00130 | *HRK1* | *270,271* | *MATα* hrk1Δ*::NAT-STM#58* |
| CNAG_00363 | *TC06* | *2469, 2554* | *MATα* tco6Δ*::NAT-STM#58* |
| CNAG_00396 | *PKA1* | *188, 189* | *MATα* pka1Δ*::NAT-STM#191* |
| CNAG_00403 | *KIC1* | *2915, 2916* | *MATα* kic1Δ*::NAT-STM#201* |
| CNAG_00415 | *CDC2801* | *2370,3699* | *MATα* cdc2801Δ*::NAT-STM#191* |
| CNAG-00636 | *CDC7* | *2911, 2912* | *MATα* cdc7Δ*::NAT-STM#213* |
| CNAG_00745 | *HRK1*/*NP* H1 | *1438, 1439* | *MATα* hrk1/mph1Δ *::NAT-STM#210* |
| CNAG_00769 | *PBS2* | *123, 124* | *MATα* pbs2Δ*::NAT-STM#213* |
| CNAG_00782 | *SPS1* | *3229, 3325* | *MATα* sps1Δ*::NAT-STM#288* |
| CNAG_00826 | *DAK2* | *1912, 1913* | *MATα dak2Δ::NAT-STM#282* |
| CNAG-01062 | *PSK201* | *1989, 1990* | *MATα* psk201Δ*::NAT-STM#191* |
| CNAG_01155 | *GUTI* | *1241, 2761* | *MATα gut1*Δ*::NAT-STM#242* |
| CNAG_01 162 | *MAK322* | *3824, 3825* | *MATα* mak322Δ*::NAT-STM#159* |
| CNAG_01165 | *LCB5* | *3789, 3790* | *MATα* 1cb5Δ*::NAT-STM#213* |
| CNAG_01209 | *FAB1* | *3172* | *MATα* fab1Δ*::NAT-STM#169* |
| CNAG_01294 | *IPK1* | *2157, 2158* | *MATα* ipk1Δ*::NAT-STM#184* |
| CNAG_01333 | *ALK1* | *1571, 1573* | *MATα* alk1Δ*::NAT-STM#122* |
| CNAG_01523 | *HOG1* | *64, 65* | *MATα* hog1Δ*::NAT-STM#177* |
| CNAG_01612 | *PSK202* | *3922, 3924* | *MATα* psk202Δ*::NAT-STM#208* |
| CNAG_01704 | *IRK6* | *3830, 3831* | *MATα* irk6Δ*::NAT-STM#5* |
| CNAG_01730 | *STE7* | *342, 343* | *MATα* ste7Δ*::NAT-STM#225* |
| CNAG_01850 | *TCO1* | *278, 279* | *MATα* yco1Δ*::NAT-STM#102* |
| CNAG_01905 | *KSP1* | *1807, 1808, 1809* | *MATα* ksp1Δ*::NAT-STM#159* |
| CNAG_01938 | *KIN1* | *3930, 3931* | *MATα* kin1Δ*::NAT-STM#6* |
| CNAG_01988 | *TCO3* | *284, 285* | *MATα* tco3Δ*::NAT-STM#119* |
| CNAG_02233 | *MEC1* | *3063, 3611* | *MATα* mec 1 Δ*::NAT-STM#204* |
| CNAG_02296 | *RBK1* | *1510, 1511* | *MATα* rbk1A*:.-NAT-STM#219* |
| CNAG_02357 | *MKK2* | *330, 331* | *MATα* mkk2Δ*::NAT-STM#224* |
| CNAG_02389 | *YPK101* | *1885, 1886* | *MATα ypk101*Δ*::NAT-STM#242* |
| CNAG_02511 | *CPK1* | *127, 128* | *MATα* cpk1Δ*::NAT-STM#184* |
| CNAG_02531 | *CPK2* | *373, 374* | *MATα* cpk2Δ*::NAT-STM#122* |
| CNAG_02542 | *IRK2* | *1904, 1905* | *MATα irk2*Δ*::NAT-STM#232* |
| CNAG_02551 | *DAK3* | *1940, 1941* | *MATα* dak3Δ*::NAT-STM#295* |
| CNAG_02675 | *HSL101* | *1800, 1801* | *MATα* hs1101Δ*::NAT-STM#146* |
| CNAG_02680 | *VPS15* | *1500, 1501* | *MATα* vps15Δ*::NAT-STM#123* |
| CNAG_02712 | *BUD32* | *1968, 1969* | *MATα* bud32Δ*::NAT-STM#295* |
| CNAG_02799 | *DAK202A* | *2487, 2489* | *MATα* dak202aΔ*::NAT-STM#119* |
| CNAG_02802 | *ARG2* | *1503, 1504* | *MATα* arg2Δ*::NAT-STM#125* |
| CNAG_02820 | *PKH201* | *1234, 1235, 1236* | *MATα* pkh201Δ*::NAT-STM#219* |
| CNAG_02859 | *P0S5* | *3714, 3715* | *MATα* pos5Δ*::NAT-STM#58* |
| CNAG_02947 | *SCY1* | *2793, 2794* | *MATα* scy1Δ:*:NAT-STM#150* |
| CNAG_03024 | *RIM15* | *1216, 1217* | *MATα* rim15Δ*::NAT-STM#191* |
| CNAG_03048 | *IRK3* | *1486, 1487* | *MATα irk3Δ::NAT-STM#273* |
| CNAG_03167 | *CHK1* | *1825, 1828* | *MATα* chk1Δ*::NAT-STM#205* |
| CNAG_03184 | *BUB1* | *3398* | *MATα* bub1Δ*::NAT-STM#201* |
| CNAG_03216 | *SNF101* | *1575, 1576* | *MATα* snf101Δ*::NAT-STM#146* |
| CNAG_03258 | *TPK202A* | *2443, 2444* | *MATα* psk202aΔ*::NAT-STM#208* |
| CNAG_03290 | *KIC102* | *3211, 3212* | *MATα* kic102Δ*::NAT-STM#201* |
| CNAG_03355 | *TC04* | *417, 418* | *MATα* tco4Δ*::NAT-STM#123* |
| CNAG_03367 | *URKI* | *1266, 1267* | *MATα* urk1Δ*::NAT-STM#43* |
| CNAG_03369 | *SWE102* | *1564, 1565* | *MATα* swe102Δ*::NAT-STM#169* |
| CNAG_03567 | *CBK1* | *2941, 2942* | *MATα cbk1*Δ*::NAT-STM#232* |
| CNAG_03592 | *THI20* | *3219, 3220* | *MATα TH120Δ::NAT-STM#-231* |
| CNAG_03670 | *IRE1* | *552, 554* | *MATα* ire1Δ*::NAT-STM#224* |
| CNAG_03811 | *IRK5* | *2952, 2953* | *MATα* irk5Δ*::NAT-STM#213* |
| CNAG_03843 | *ARK1* | *1725, 1726* | *MATα* ark1Δ*::NAT-STM#43* |
| CNAG_03946 | *GAL302* | *2852, 2853* | *MATα* gal302Δ*::NAT-STM#218* |
| CNAG_04040 | *FPK1* | *2948, 2949* | *MATα* fpk1Δ*::NAT-STM#211* |
| CNAG_04108 | *PKP2* | *2439, 2440* | *MATα* pkp2Δ*::NAT-STM#295* |
| CNAG_04162 | *PKA2* | *194, 195* | *MATα* pka2Δ*::NAT-STM#205* |
| CNAG-04197 | *YAK1* | *2040, 2096, 4139* | *MATα* yak1 *Δ::NAT-STM#184* |
| CNAG_04215 | *MET3* | *3329, 3330* | *MATα* met3Δ:*:NAT-STM#205* |
| CNAO_04221 | *FBP26* | *3669* | *MATα* fbp26Δ*::NAT-STM#146* |
| CNAG_04230 | *TH16* | *1468, 1469* | *MATα* thi6Δ*::NAT-STM#290* |
| CNAG_04282 | *MPK2* | *3236, 3238* | *MATα* mpk2Δ*::NAT-STM#102* |
| CNAG_04316 | *UTR1* | *2892, 2893* | *MATα* utr1Δ*::NAT-STM#5* |
| CNAG_04408 | *CK11* | *1804, 1805* | *MATα* cki1Δ*::NAT-STM#218* |
| CNAG_04433 | *YAK103* | *3736, 3737* | *MATα YAK103*Δ*::NAT-STM#231* |
| CNAG_04514 | *MPK1* | *3814, 3816* | *MATα mpk1Δ::NAT-STM#240* |
| CNAG_04631 | *RIK1* | *1579, 1580* | *MATα* CNAG_04631Δ *::NAT-STM#150* |
| CNAG_04678 | *YPK1* | *1736, 1737* | *MATα* ypk 1 Δ*::NAT-STM#58* |
| CNAG_04755 | *BCK1* | *273, 274* | *MATα* bck1Δ*::NAT-STM#43* |
| CNAG_04821 | *PAN3* | *2809, 2810* | *MATα* pan3Δ*::NAT-STM#204* |
| CNAG_04927 | *YFH702* | *2826, 3716* | *MATα* yfh702Δ*::NAT-SYM#-220* |
| CNAG_05005 | *ATG1* | *1935, 1936* | *MATα* atg1Δ*::NAT-STM#288* |
| CNAG_05063 | *SSK2* | *264, 265* | *MATα* ssk2Δ*::NAT-STM#210* |
| CNAG_05097 | *CKY1* | *1245, 1246* | *MATα CNAG_*05097Δ *::NAT-STM#282* |
| CNAG_05216 | *RAD53* | *3785, 3786* | *MATα* rad53Δ*::NAT-STM#184* |
| CNAG_05220 | *TLK1* | *3153, 3188* | *MATα* tlk1Δ*::NAT-STM#116* |
| CNAG_05243 | *XKS1* | *2851* | *MATα* xks1Δ*::NAT-STM#125* |
| CNAG_05439 | *CMK1* | *1883, 1901, 1902* | *MATα* cmk1Δ*::NAT-STM#227* |
| CNAG_05558 | *KIN4* | *2955* | *MATα* kin4Δ*::NAT-STM#225* |
| CNAG_05590 | *TCO2* | *281, 282* | *MATα* tco2Δ*::NAT-STM#116* |
| CNAG_05600 | *IGI1* | *1514, 1515* | *MATα CNAG_*05600Δ*. :: NAT-STM#230* |
| CNAG_05694 | *CKAI* | *3051, 3052, 3053* | *MATα* cka1Δ*::NAT-STM#6* |
| CNAG_05753 | *ARG5,6* | *2408, 2409, 2410* | *MATα* arg5/6Δ*::NAT-STM#220* |
| CNAG_05771 | *TEL1* | *3844, 3845* | *MATα* tc11Δ*::NAT-STM#225* |
| CNAG_05965 | *IRK4* | *2806, 2808* | *MATα* irk4Δ*::NAT-STM#211* |
| CNAG_06033 | *MAK32* | *3240, 3241* | *MATα* mak32Δ*::NAT-STM#169* |
| CNAG_06051 | *GAL1* | *2829, 2830* | *MATα* gal1Δ*::NAT-STM#224* |
| CNAG_06086 | *SSN3* | *3038, 3039* | *MATα* ssn3Δ*::NAT-STM#219* |
| CNAG_06161 | *VRK1* | *2216, 2217* | *MATα* vrk1Δ*::NAT-STM#123* |
| CNAG_06193 | *CRK1* | *1709, 1710* | *MATα* crk1*Δ::NAT-STM#43* |
| CNAG_06278 | *TCO7* | *348* | *MATα* tco7Δ*::NAT-STM#209* |
| CNAG_06301 | *SCH9* | *619, 620* | *MATα* sch9Δ*::NAT-STM#169* |
| CNAG_06310 | *IRK7* | *2136, 2137* | *MATα* irk7Δ*::NAT-STM#208* |
| CNAG-06366 | *HRR2502* | *2053* | *MATα hrr*2502Δ*::NAT-STM#125* |
| CNAG_06552 | *SNF1* | *2372, 2373* | *MATα* snf1Δ*::NAT-STM#204* |
| CNAG_06553 | *GAL83* | *2415, 2416* | *MATα* gal83Δ*::NAT-STM#288* |
| CNAG_06568 | *SKS1* | *1410, 1411* | *MATα* sks1Δ*::NAT-STM#211* |
| CNAG_06632 | *ABC1* | *2072, 2797* | *MATα* CNAG_06632Δ *::NAT-STM#119* |
| CNAG_06671 | *YKL1* | *3926, 3927* | *MATα* CNAG_06671A *::NAT-STM#122* |
| CNAG_06697 | *MPS1* | *3632, 3633* | *MATα* mps1Δ*::NAT-STM#116* |
| CNAG_06730 | *GSK3* | *2038, 2039* | *MATα* gsk3Δ*::NAT-STM#123* |
| CNAG_06809 | *IKS1* | *1310, 2119* | *MATα* iks1Δ*::NAT-STM#116* |
| CNAG_06980 | *STE11* | *313, 314* | *MATα ste11*Δ*::NAT-STM#242* |
| CNAG_07359 | *IRK1* | *1950, 1951* | *MATα* irk1Δ*::NAT-STM#5* |
| CNAG_07580 | *TRM7* | *3056, 3057* | *MATα* trm7Δ*::NAT-STM#102* |
| CNAG_07667 | *SAT4* | *3612* | *MATα* sat4Δ*::NAT-STM#212* |
| *CNAG_07744 PIK1* | *PIK1* | *1493, 1494* | *MATα* pik*1*Δ*::NAT-STM#227* |
| CNAG_07779 | *TDA10* | *2663, 3223* | *MATα* tda10Δ*::NAT-STM#102* |
| CNAG_08022 | *PHO85* | *3702, 3703* | *MATα* pho85Δ*::NAT-STM#218* |

| | | | |
|---|---|---|---|
| *CNAG: Abbreviation for Cryptococcus neoformans serotype A genome database, which is the H99 genomic database gene number provided by the Broad Institute. | | | |

For gene-deletion through homologous recombination, gene-disruption cassettes containing the nourseothricin-resistance marker (NAT; nourseothricin acetyl transferase) with indicated signature-tagged sequences were generated by using conventional overlap PCR or NAT split marker/double-joint (DJ) PCR strategies (Davidson, R. C. et al. A PCR-based strategy to generate integrative targeting alleles with large regions of homology. Microbiology 148, 2607-2615 (2002); Kim, M. S., Kim, S. Y., Jung, K. W. & Bahn, Y. S. Targeted gene disruption in Cryptococcus neoformans using double-joint PCR with split dominant selectable markers. Methods Mol Biol 845, 67-84, doi:10.1007/97S-1-61779-539-8_5 (2012) (Table 1). To validate a mutant phenotype and to exclude any unlinked mutational effects, more than two independent deletion strains were constructed for each kinase mutant (see Table 1). When two independent kinase mutants exhibited inconsistent phenotypes (inter-isolate inconsistency), more than three mutants were constructed. As a result, the present inventors successfully generated 220 gene deletion mutants representing 114 kinases (including those that were previously reported) (Table 1). For 106 kinases, two or more independent mutants were constructed. Some kinases that had been previously reported by others were independently deleted here with unique signature-tagged markers to perform parallel *in vitro* and *in vivo* phenotypic analysis. When two independent kinase mutants exhibited inconsistent phenotypes (known as inter-isolate inconsistency), the present inventors attempted to generate more than three mutants.

For the remaining 69 kinases, the present inventors were not able to generate mutants even after repeated attempts. In many cases, the present inventors either could not isolate a viable transformant, or observed the retention of a wild-type allele along with the disrupted allele. The success level for mutant construction of the kinases (114 out of 183 (62%)) was lower than that for transcription factors (TFs) that the present inventors previously reported (155 out of 178 (87%)) (Jung, K. W. et al. Systematic functional profiling of transcription factor networks in Cryptococcus neoformans. Nat Comms 6, 6757, doi:10.1038/ncomms7757, 2015). This is probably because among fungi, kinases are generally much more evolutionarily conserved than TFs, and a greater number of essential or growth-related genes appeared to exist. In fact, 24 (35%) of the kinases are orthologous to kinases that are essential for the growth of *Saccharomyces cerevisiae.* Notably, 8 genes (RAD53, CDC28, CDC7, CBK1, UTR1, MPS1, PIK1, and TOR2) that are known to be essential in *S. cerevisiae* were successfully deleted in *C. neoformans,* suggesting the presence of functional divergence in some protein kinases between ascomycete and basidiomycete fungi.

In the first round of PCR, the 5'- and 3'-flanking regions for the targeted kinase genes were amplified with primer pairs L1/L2 and R1/R2, respectively, by using H99S genomic DNA as a template. For the overlap PCR, the whole NAT marker was amplified with the primers M13Fe (M13 forward extended) and M13Re (M13 reverse extended) by using a pNAT-STM plasmid (obtained from the Joeseph Heitman Laboratory at Duke University in USA) containing the NAT gene with each unique signature-tagged sequence. For the split marker/DJ-PCR, the split 5'- and 3'-regions of the NAT marker were amplified with primer pairs M13Fe/NSL and M13Re/NSR, respectively, with the plasmid pNAT-STM. In the second round of overlap PCR, the kinase gene-disruption cassettes were amplified with primers L1 and R2 by using the combined first round PCR products as templates. In the second round of split marker/DJ-PCR, the 5'- and 3'-regions of NAT-split gene-disruption cassettes were amplified with primer pairs L1/NSL and R2/NSR, respectively, by using combined corresponding first round PCR products as templates. For transformation, the H99S strain (obtained from the Joeseph Heitman Laboratory at Duke University in USA) was cultured overnight at 30°C in the 50ml yeast extract-peptone-dextrose (YPD) medium [Yeast extract (Becton, Dickison and company #212750), Peptone (Becton, Dickison and company #211677), Glucose (Duchefa,#G0802)], pelleted and re-suspended in 5ml of distilled water. Approximately 200µl of the cell suspension was spread on YPD solid medium containing 1M sorbitol and further incubated at 30°C for 3hours. The PCR-amplified gene disruption cassettes were coated onto 600 pg of 0.6µm gold microcarrier beads (PDS-100, Bio-Rad) and biolistically introduced into the cells by using particle delivery system (PDS-100, Bio-Rad). The transformed cells were further incubated at 30°C for recovery of cell membrane integrity and were scraped after 3 hours. The scraped cells were transferred to the selection medium (YPD solid plate containing 100µg/ml nourseothricin; YPD+NAT). Stable nourseothricin-resistant (NATr) transformants were selected through more than two passages on the YPD+NAT plates. All NAT^{r} strains were confirmed by diagnostic PCR with each screening primer listed in Table 2 below. To verify accurate gene deletion, Southern blot analysis was finally performed (Jung, K. W., Kim, S. Y. , Okagaki, L. H., Nielsen, K. & Bahn, Y. S. Ste50 adaptor protein governs sexual differentiation of Cryptococcus neoformans via the pheromone-response MAPK signaling pathway. Fungal Genet. Biol. 48, 154-165, doi:S1087-1845(10)00191-X [pii] 10.1016/j.fgb.2010.10.006 (2011). Table 2 below lists primers used in the construction of the kinase mutant library.

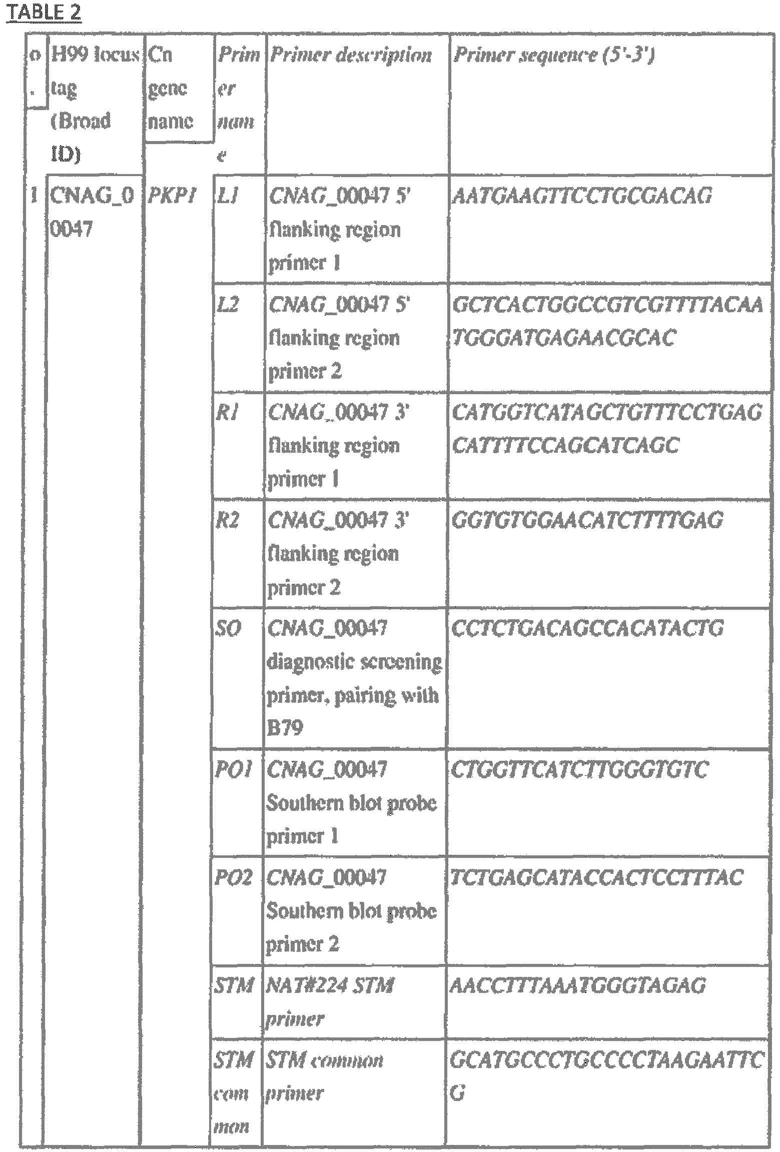

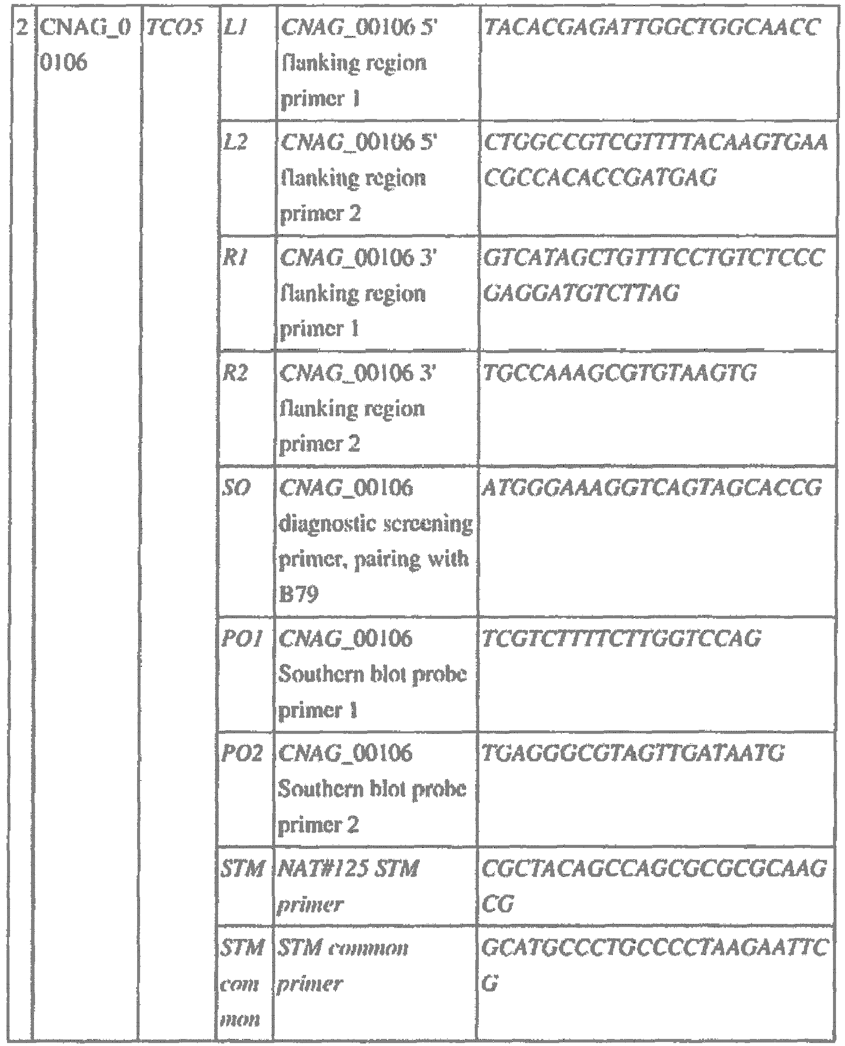

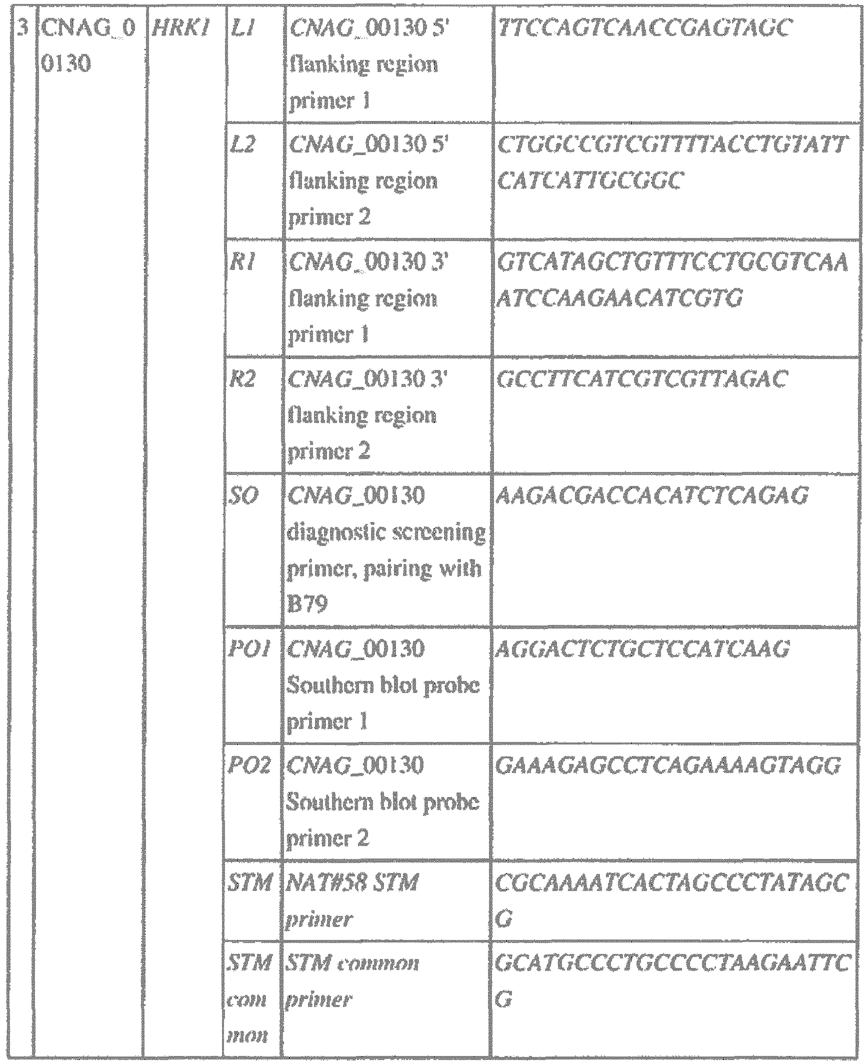

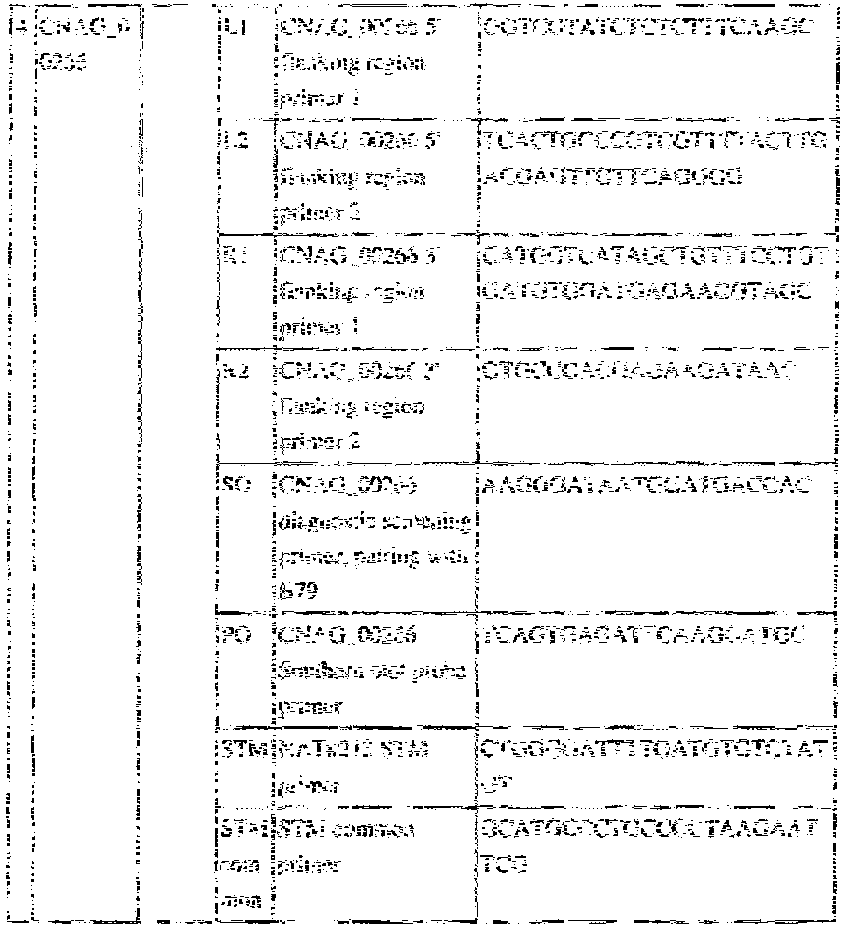

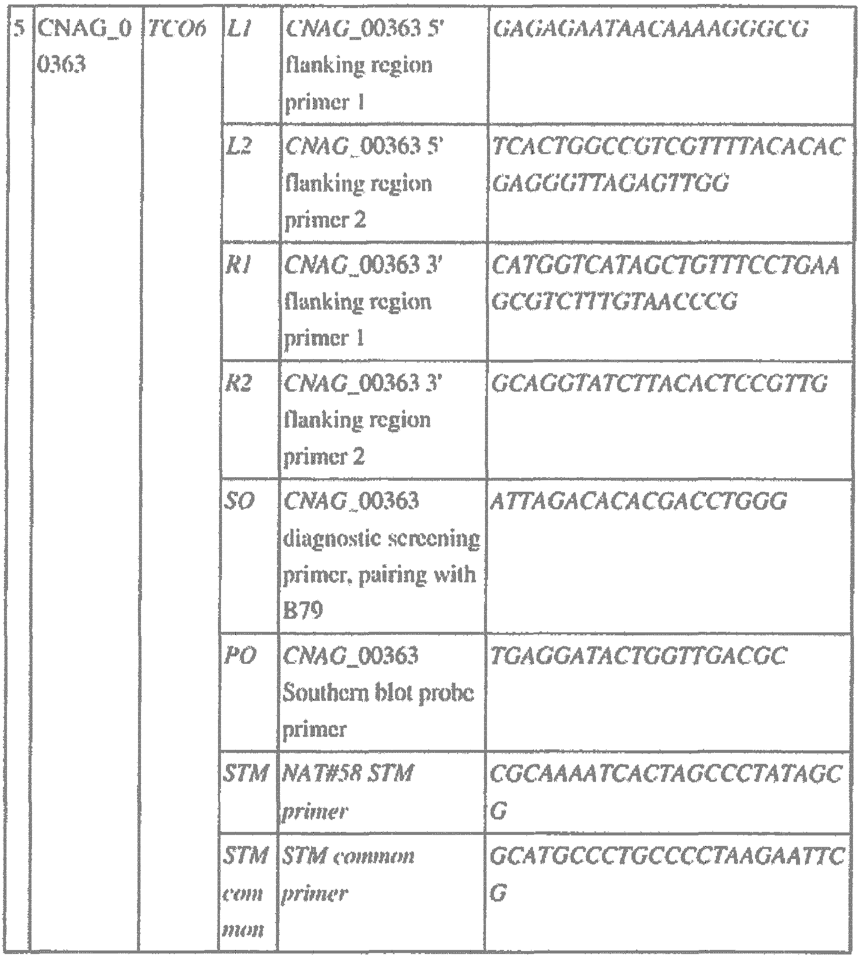

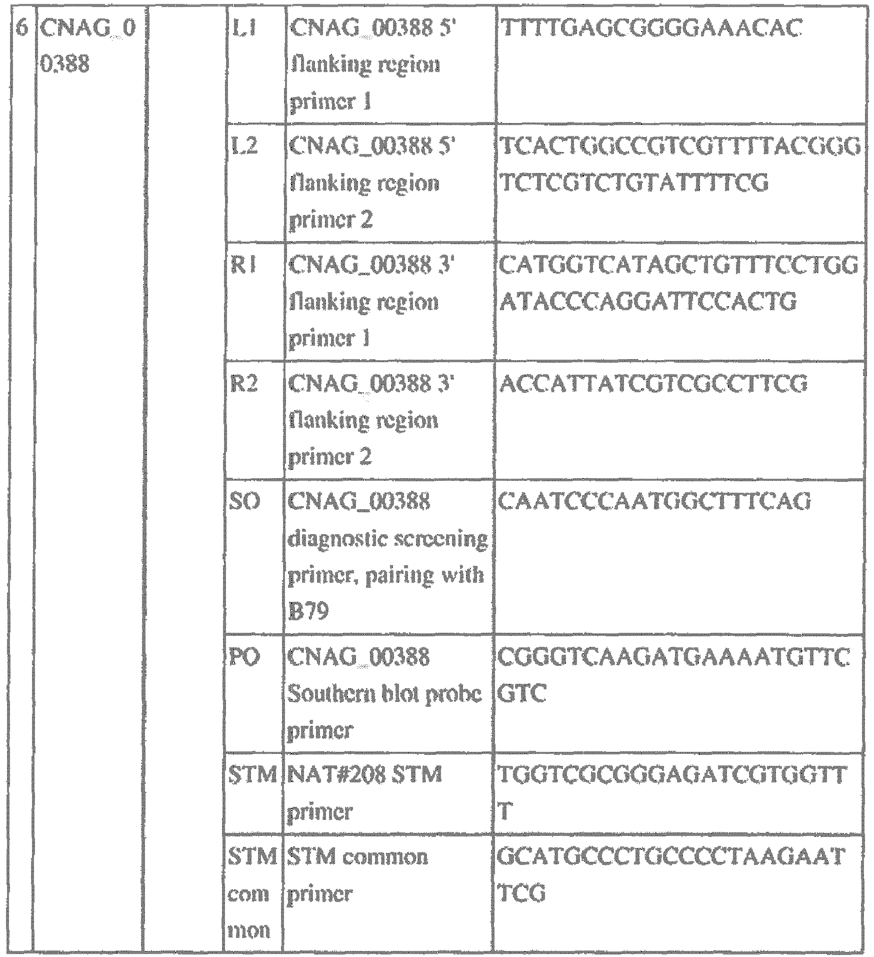

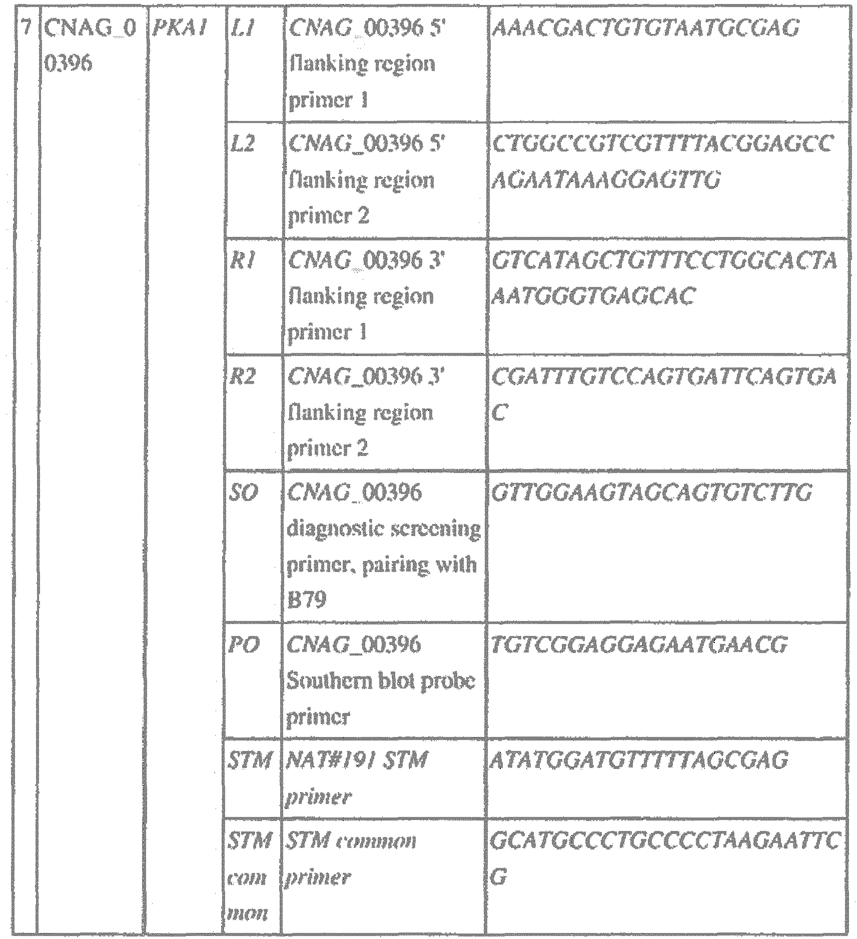

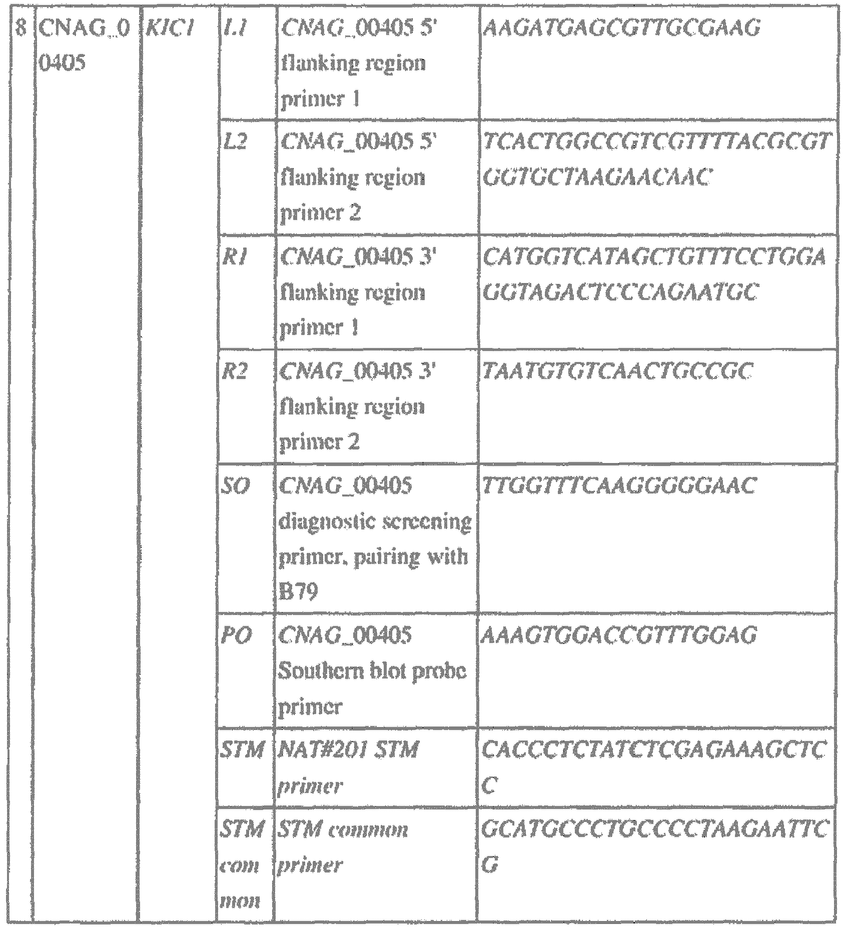

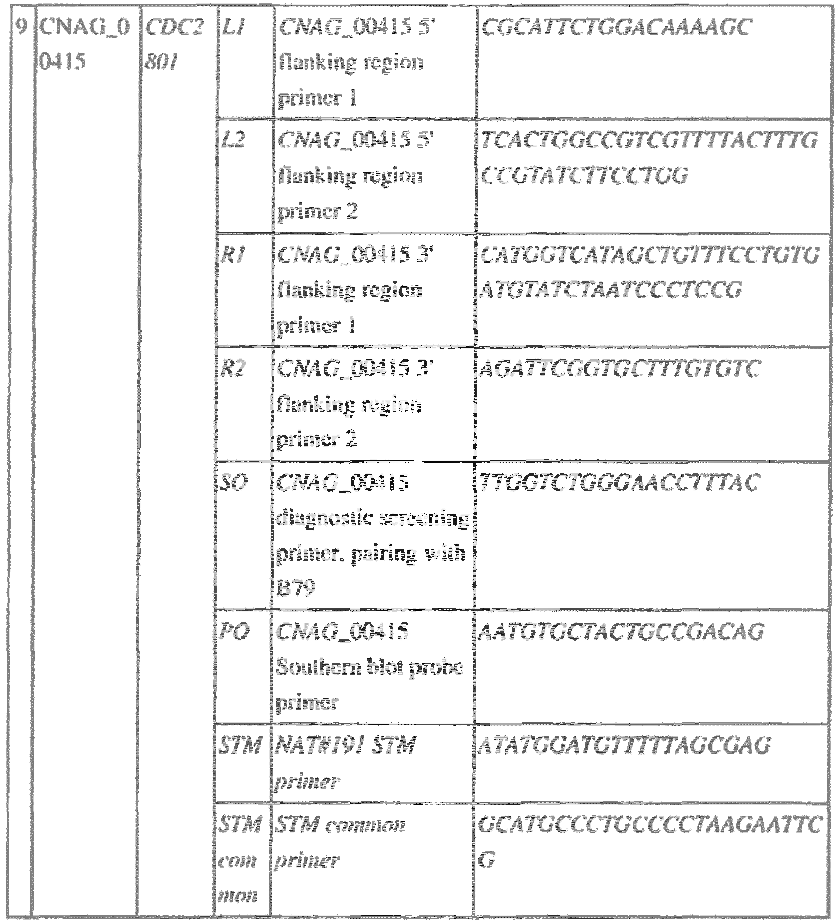

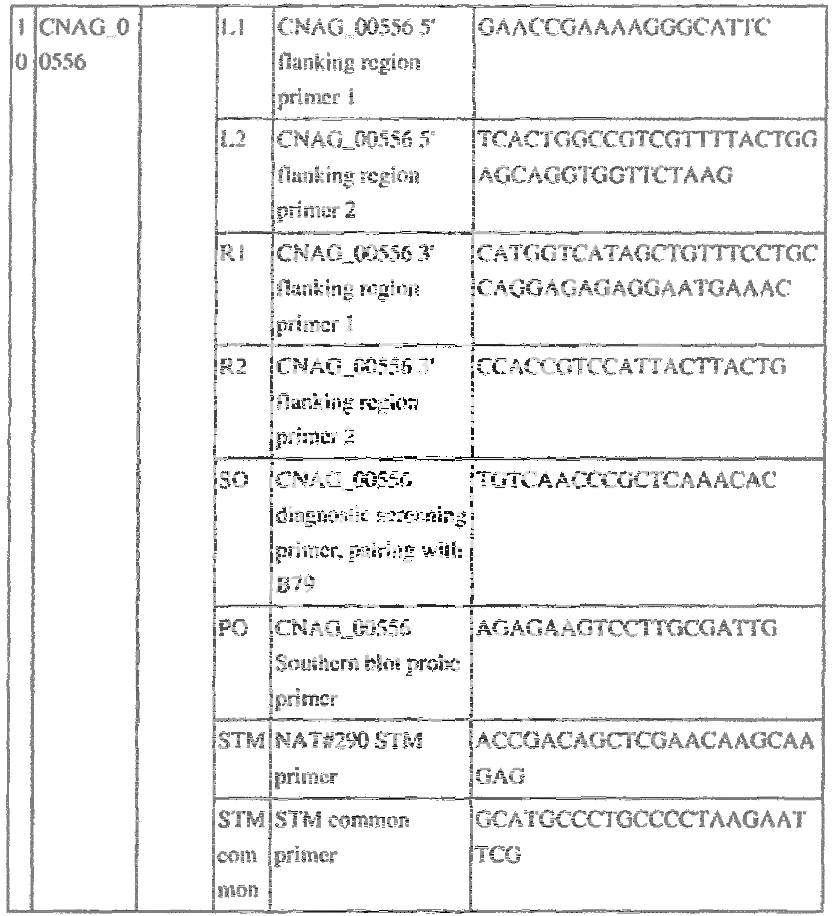

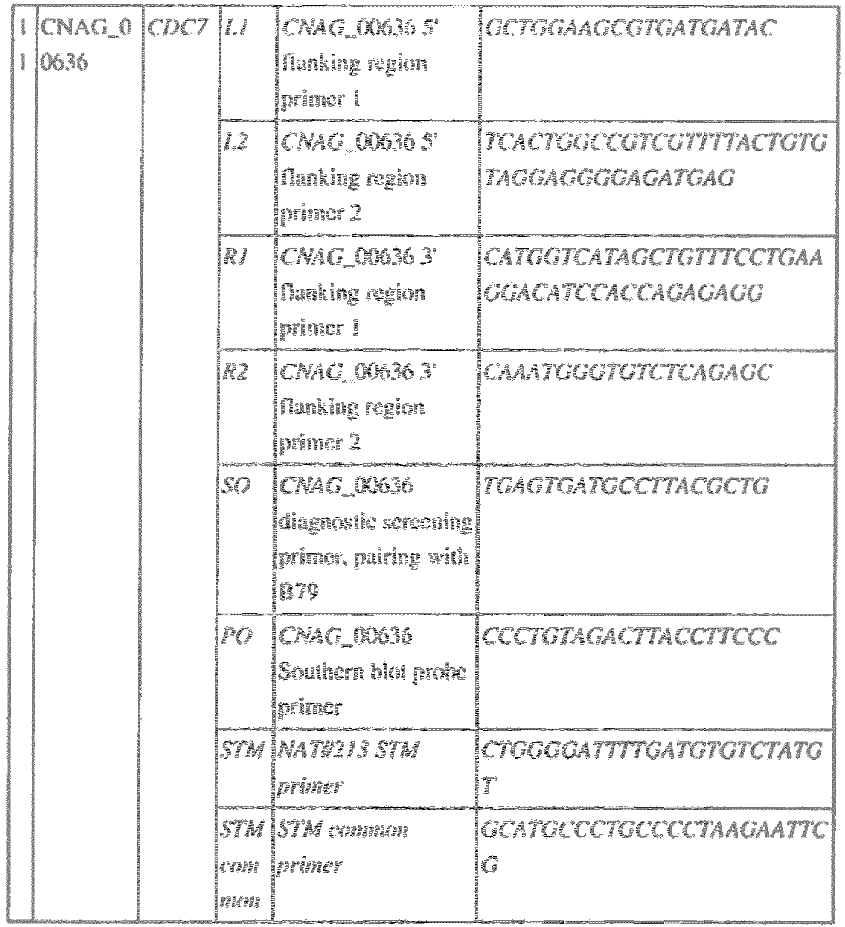

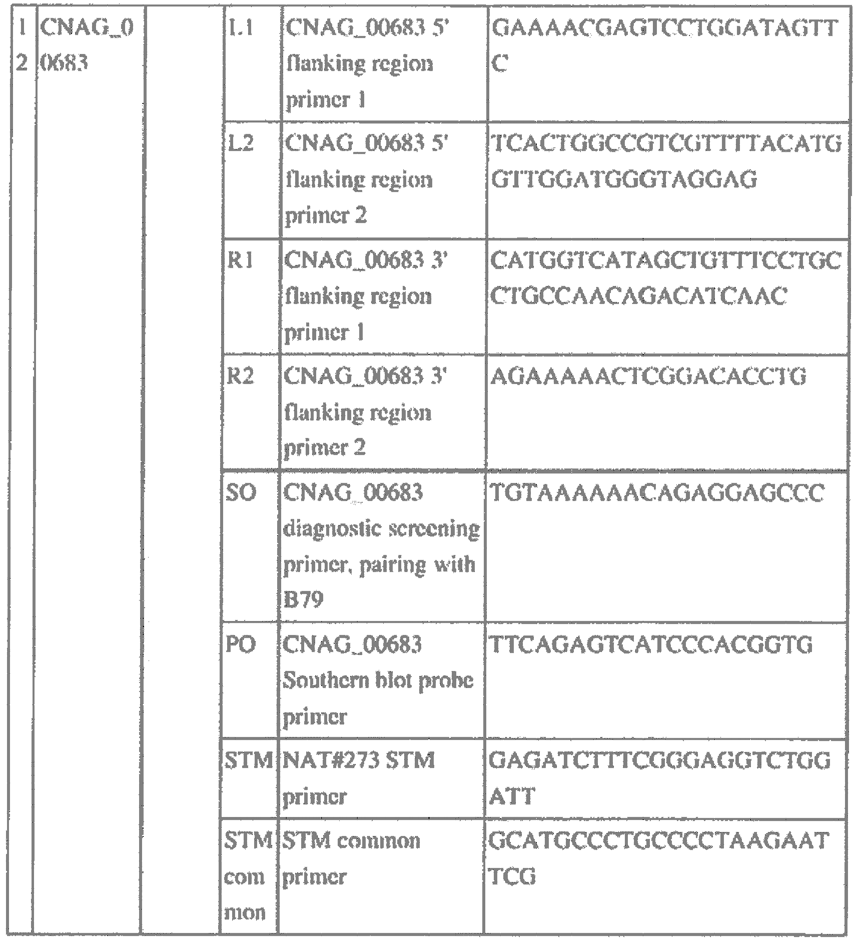

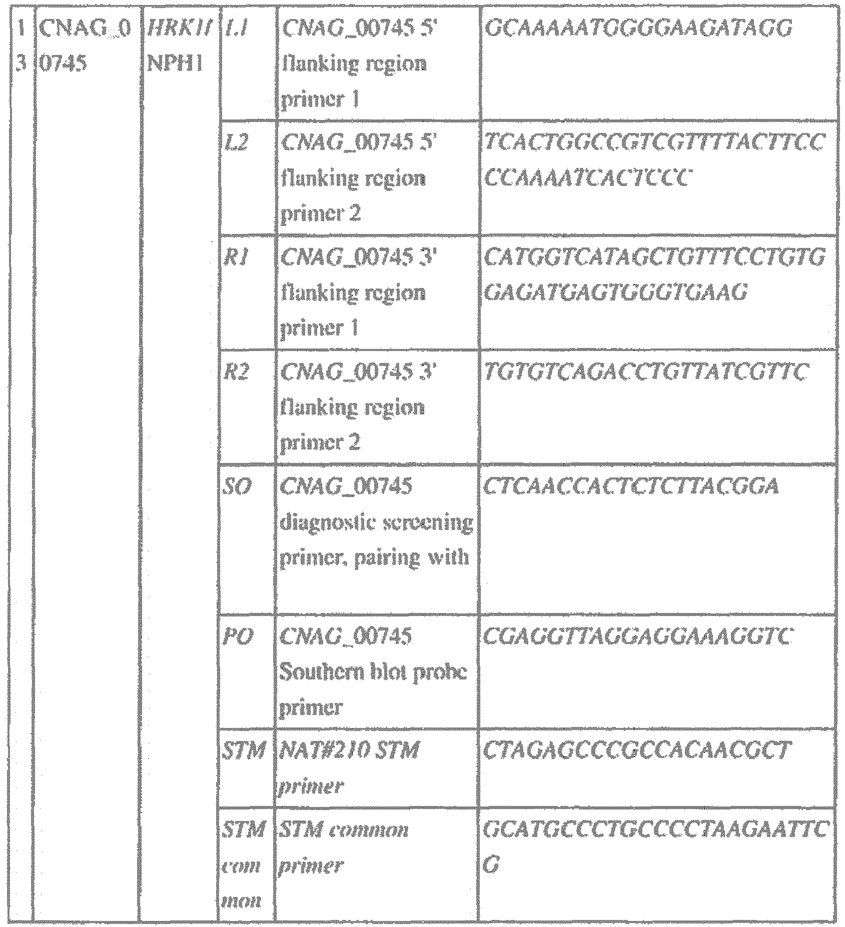

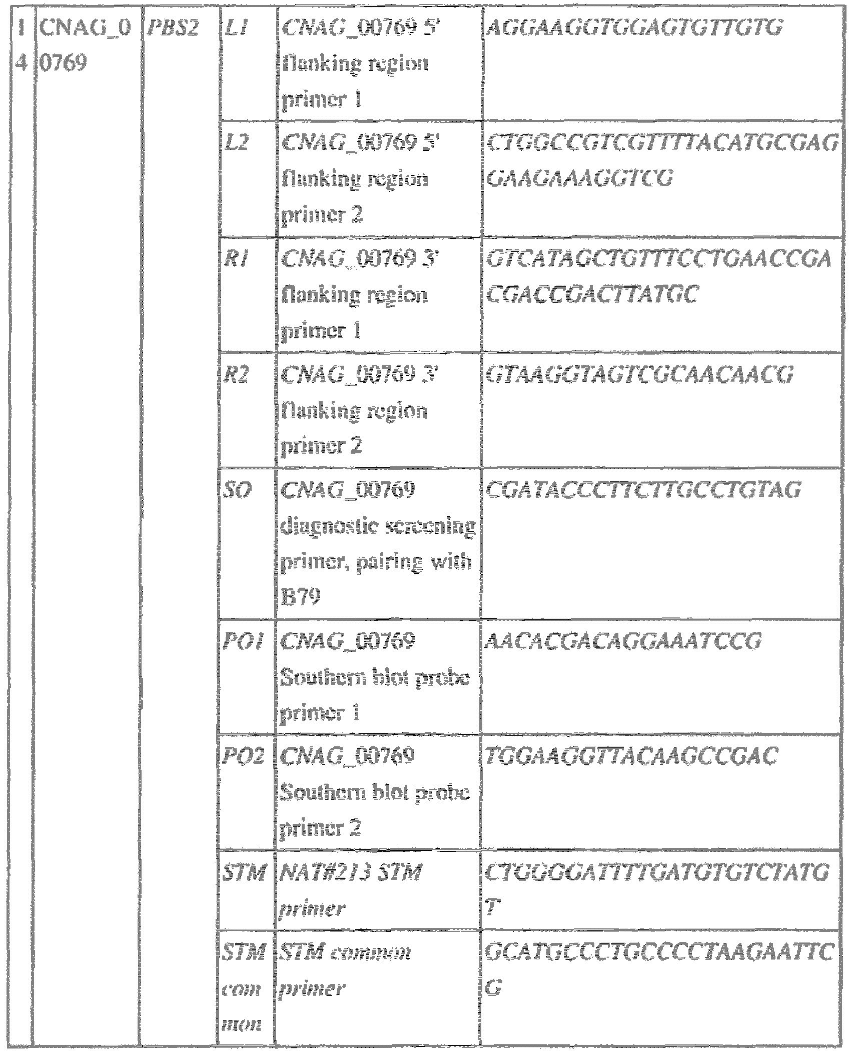

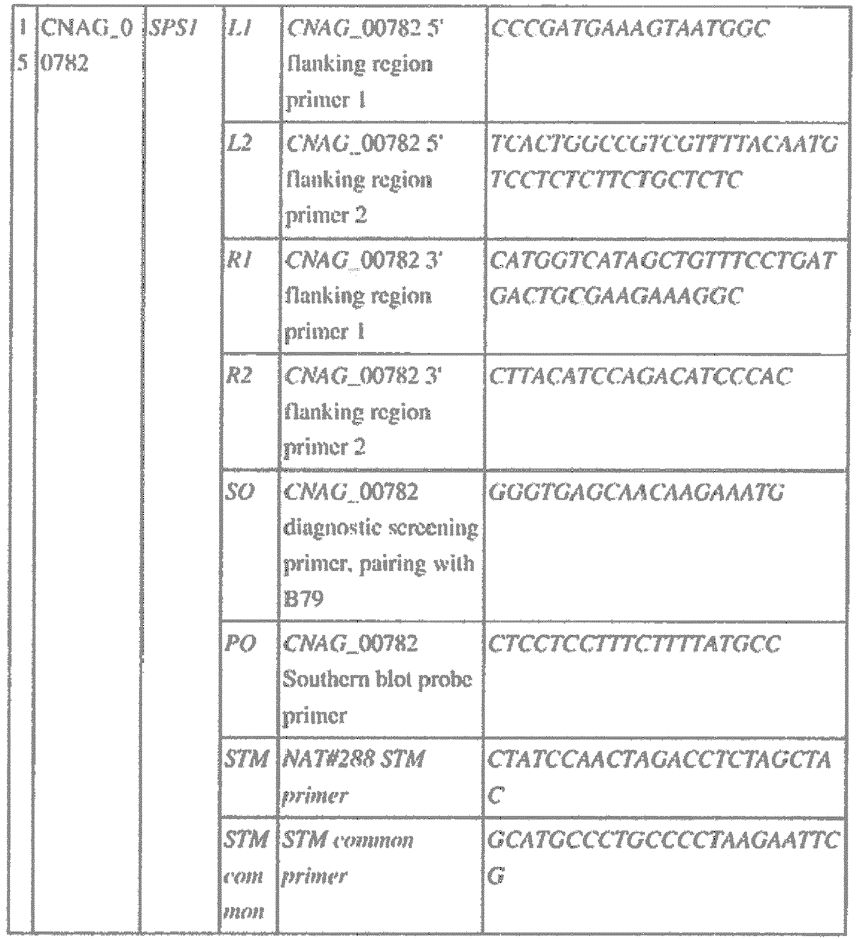

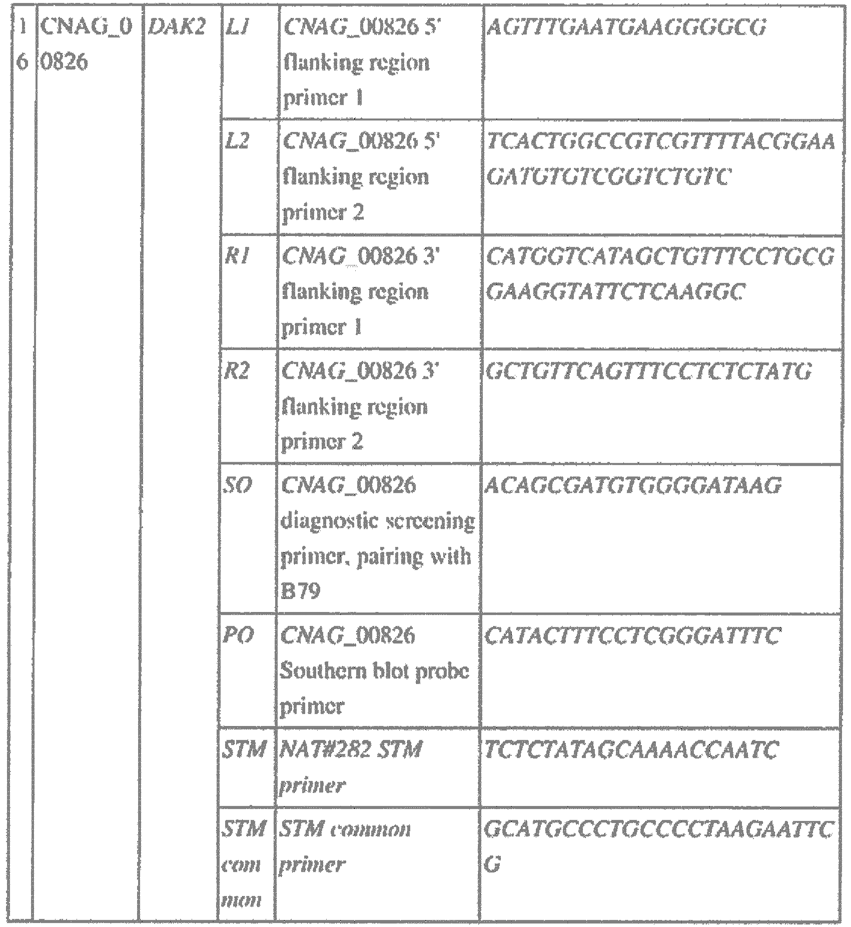

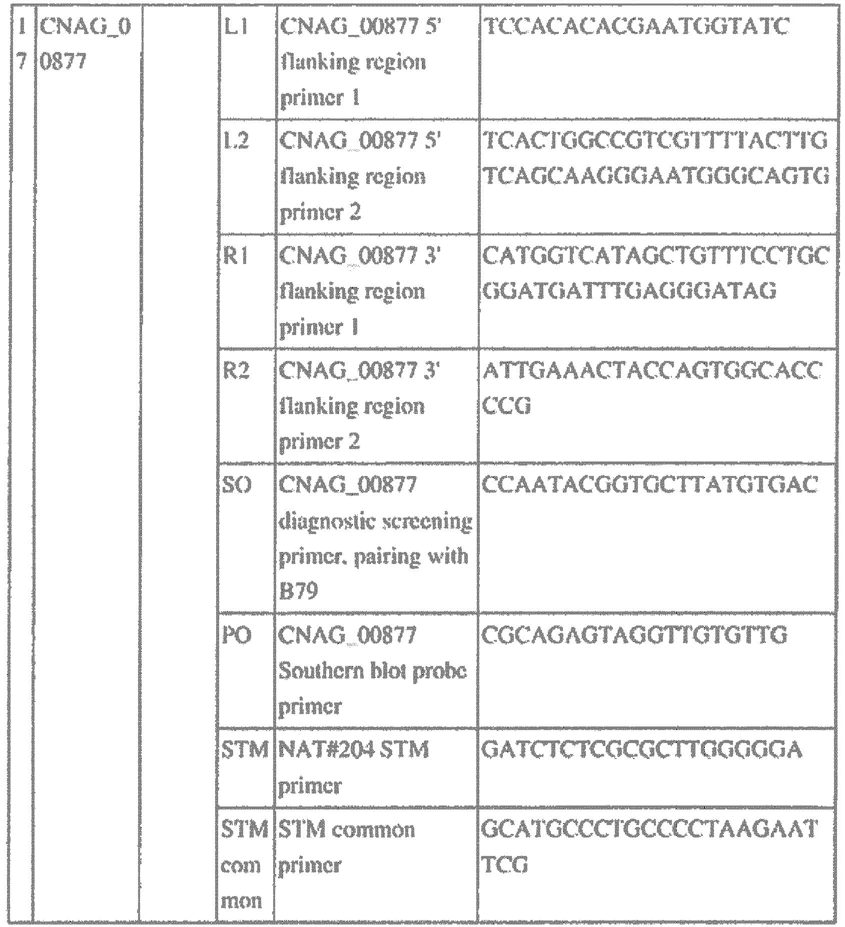

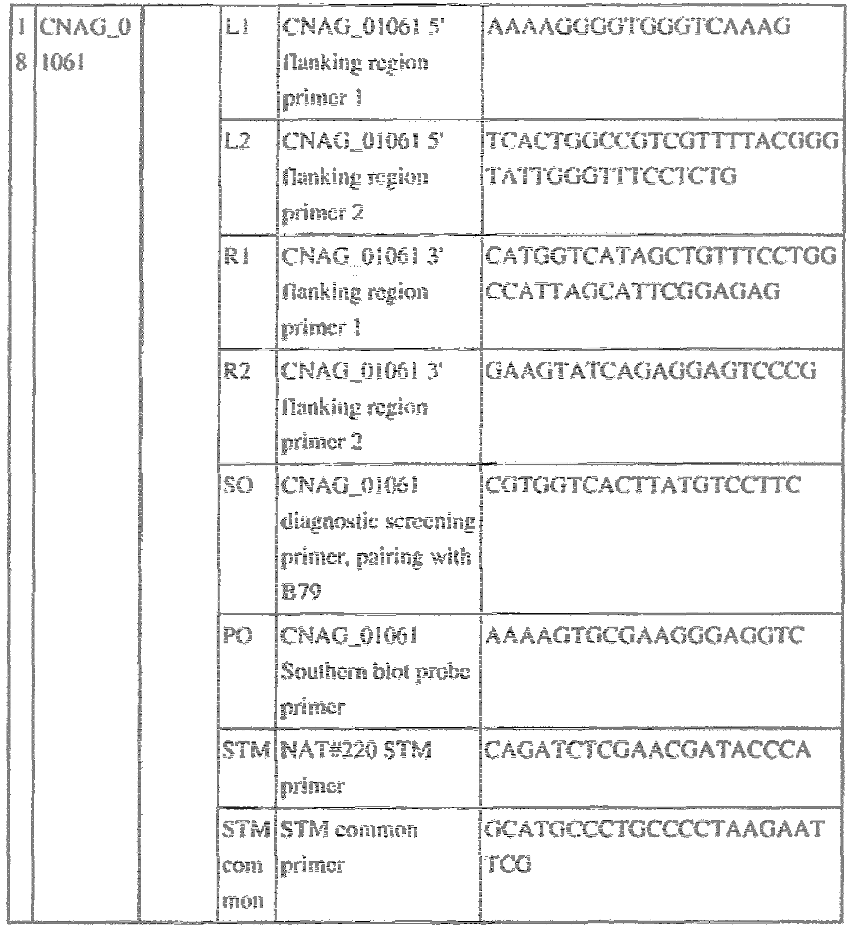

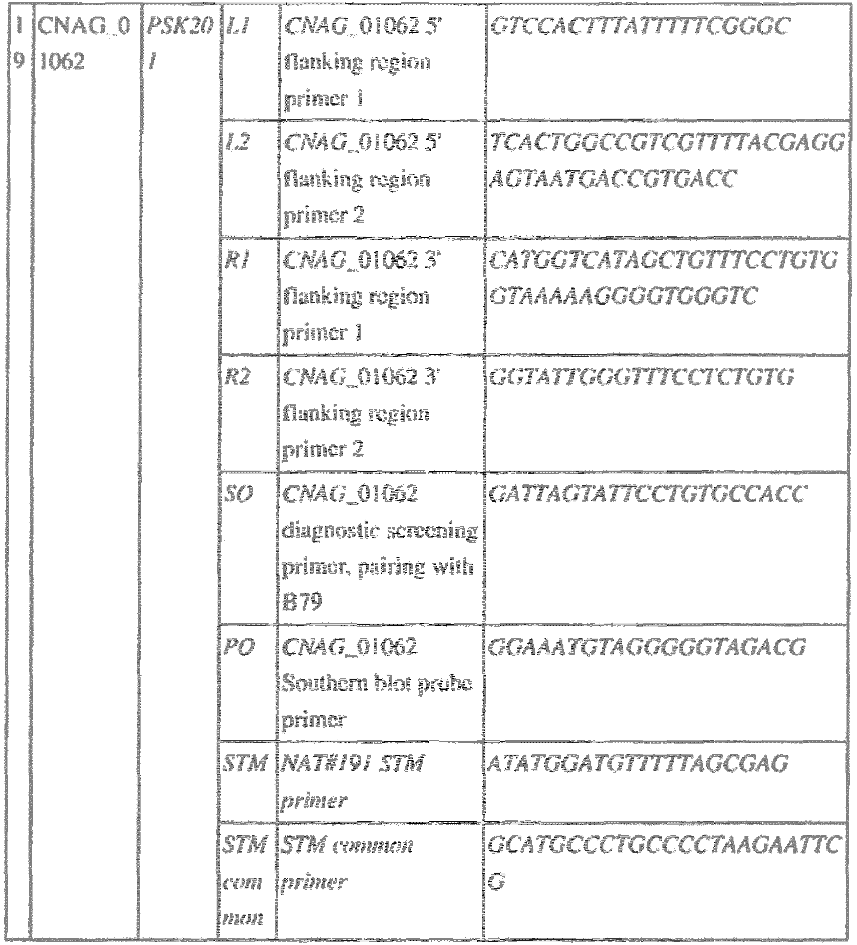

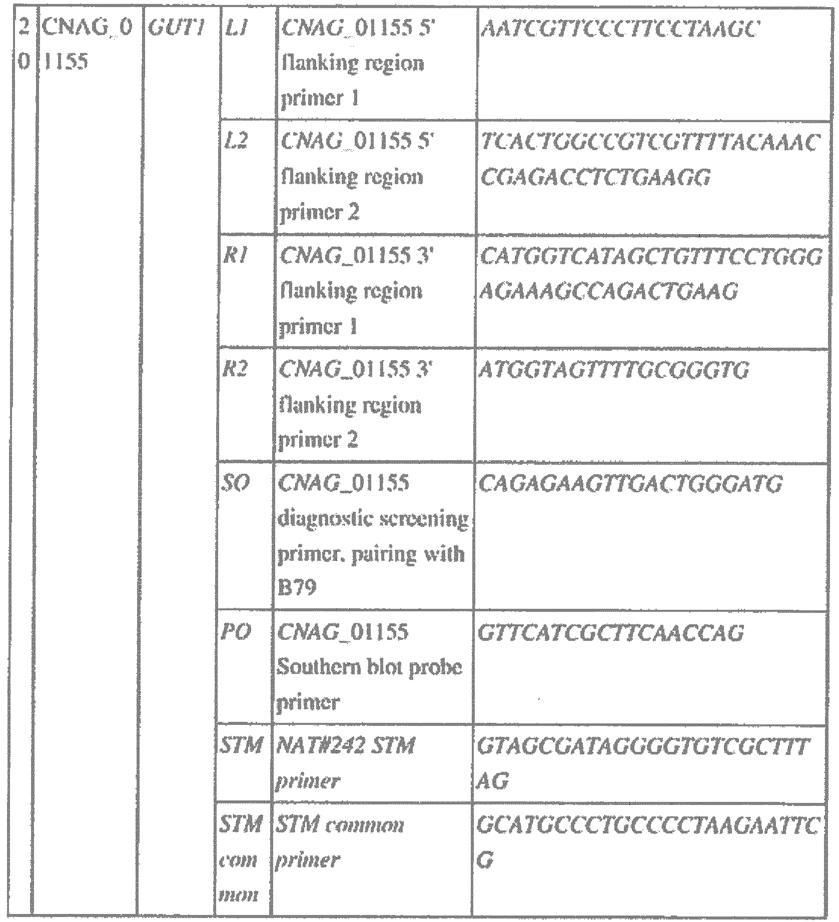

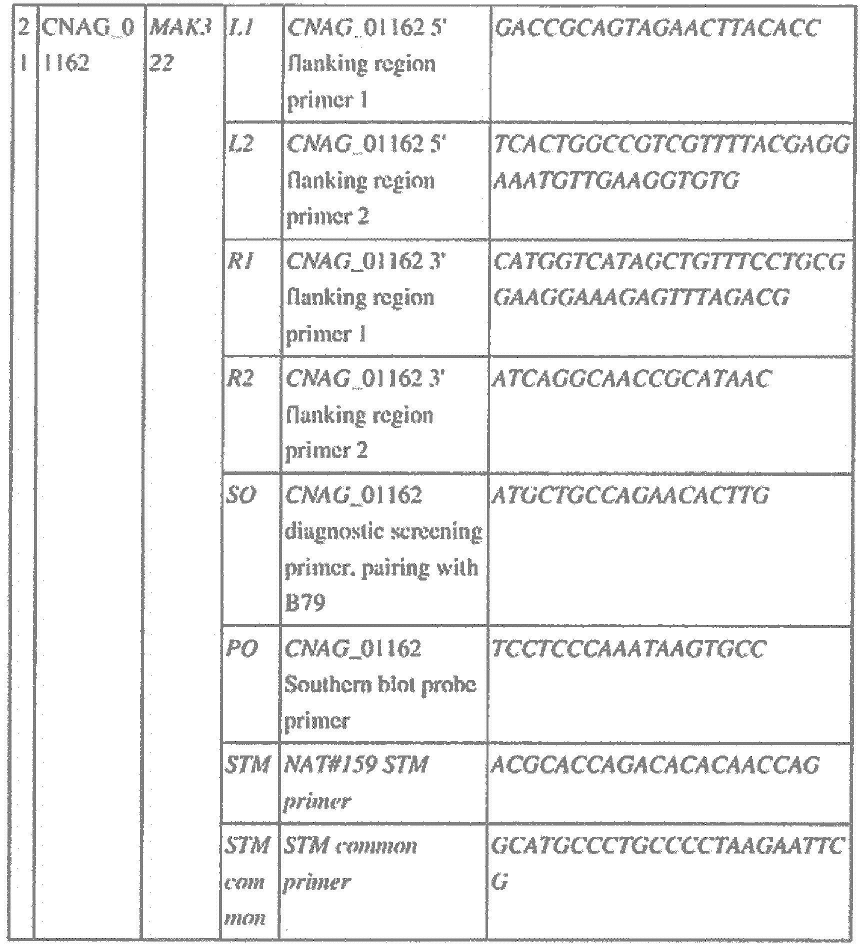

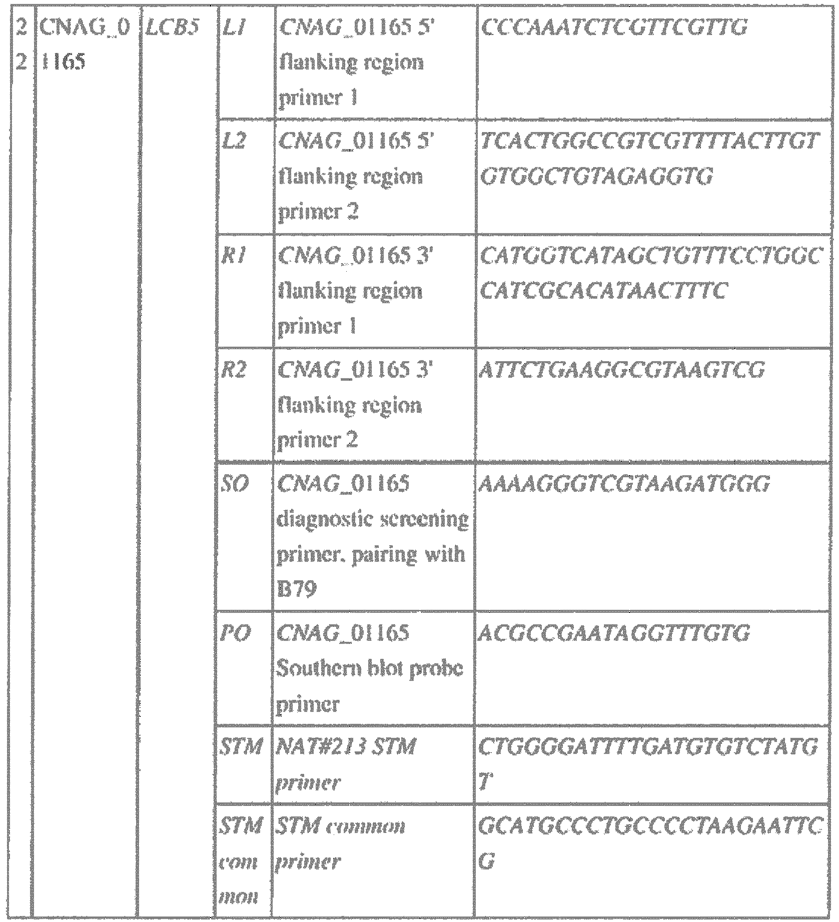

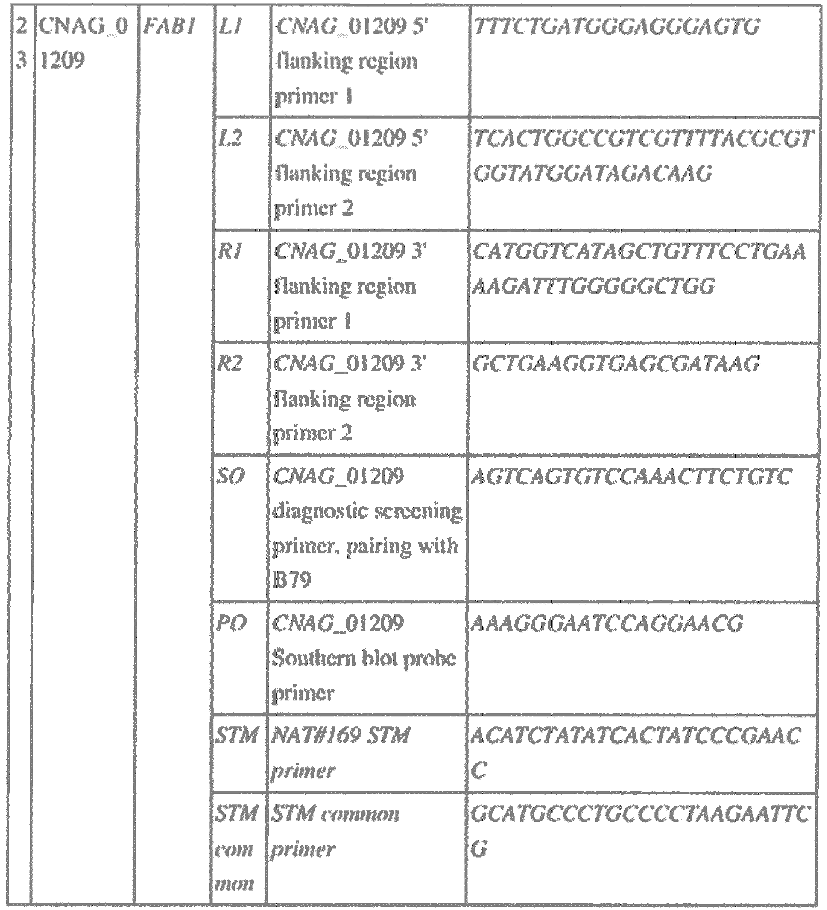

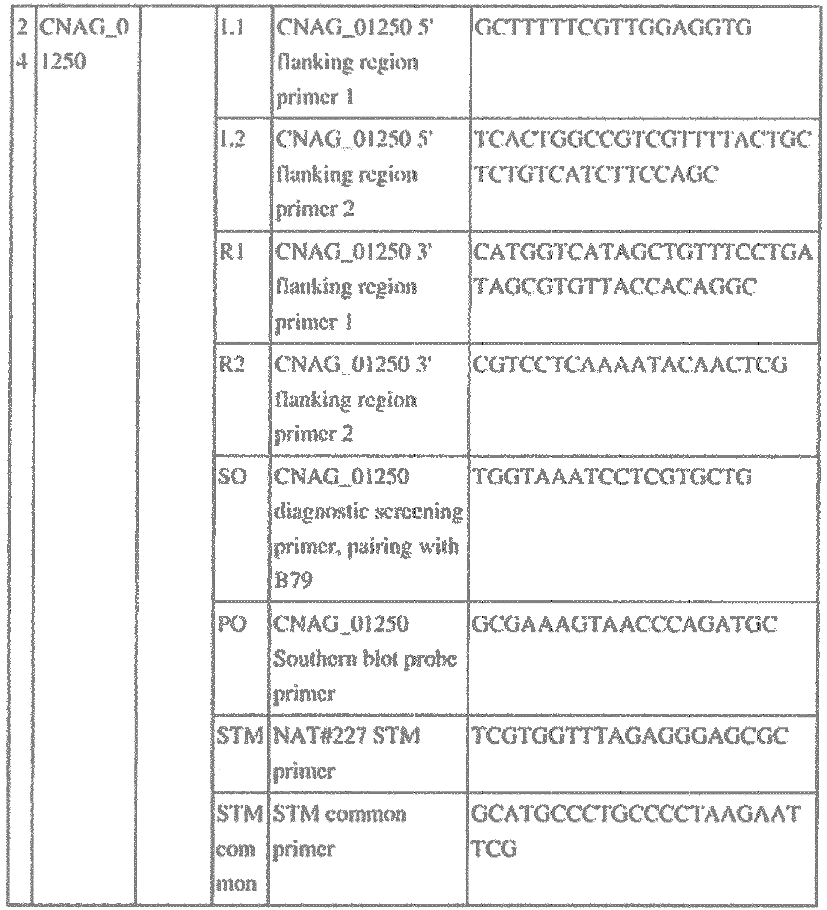

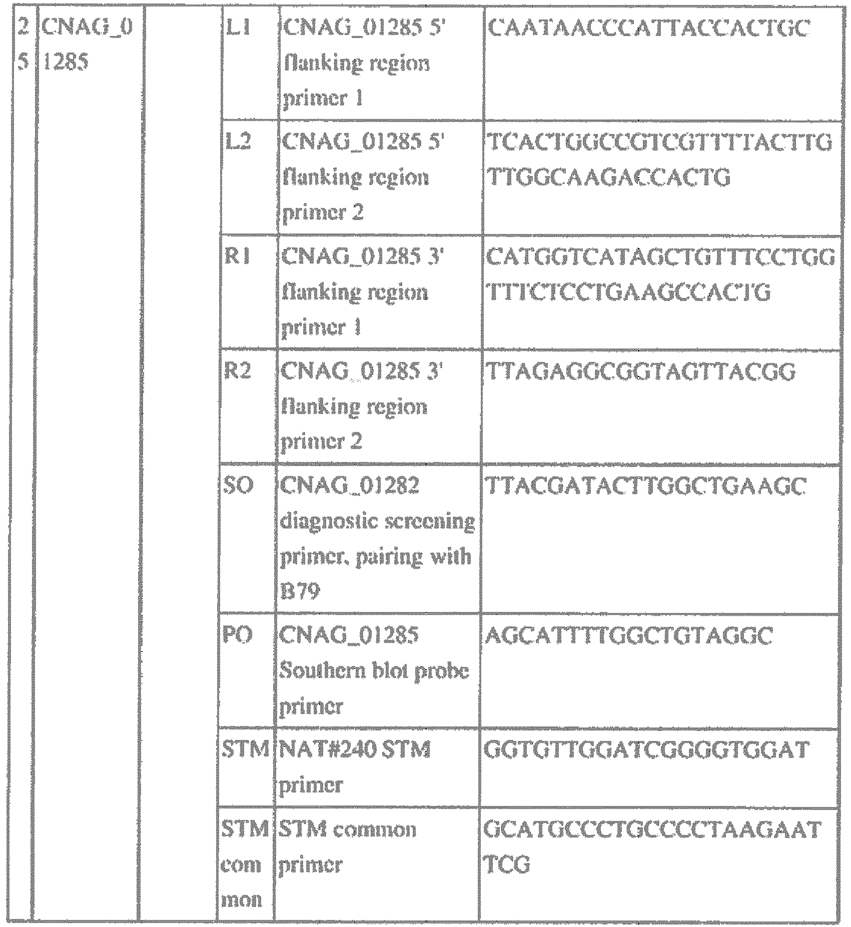

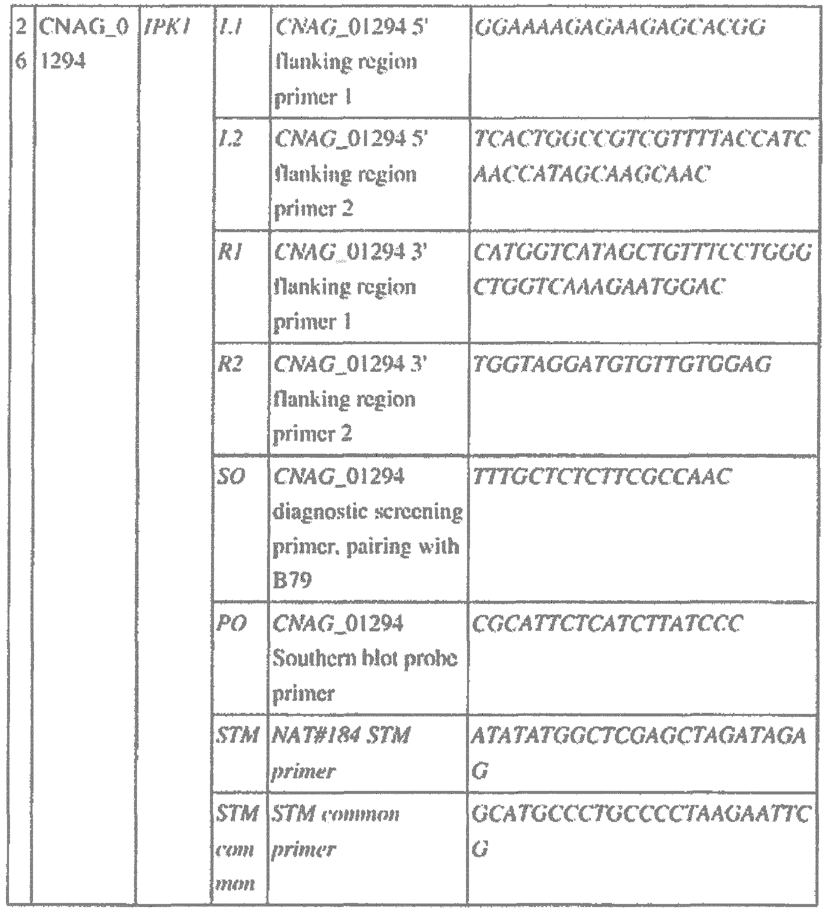

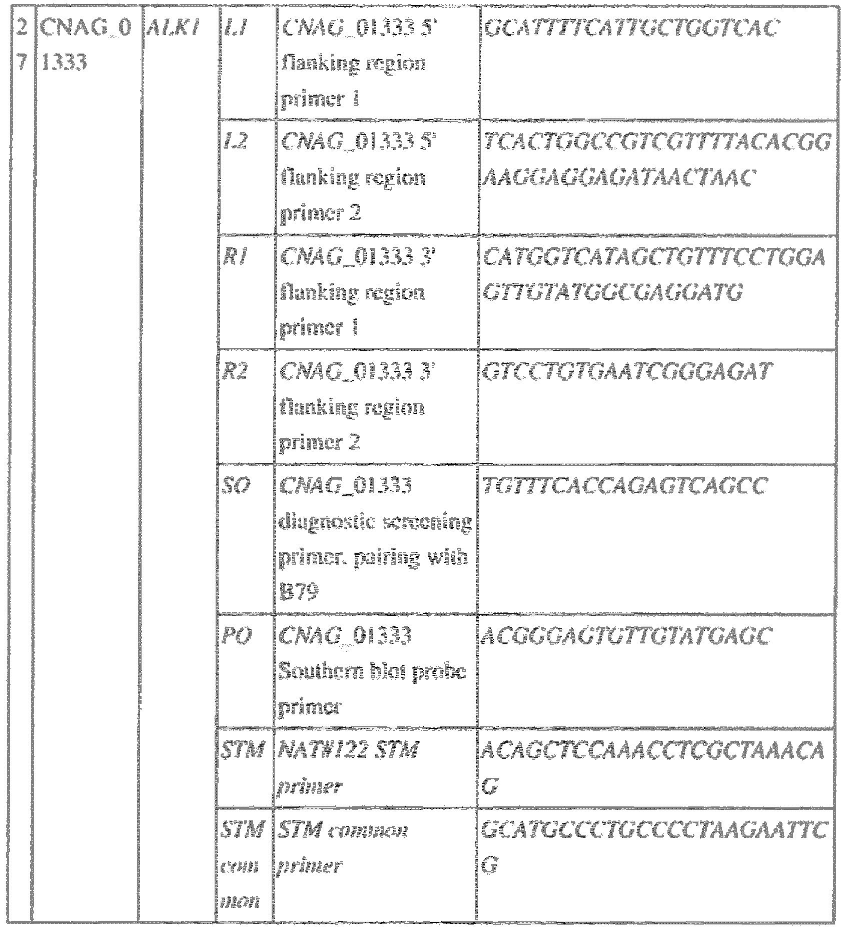

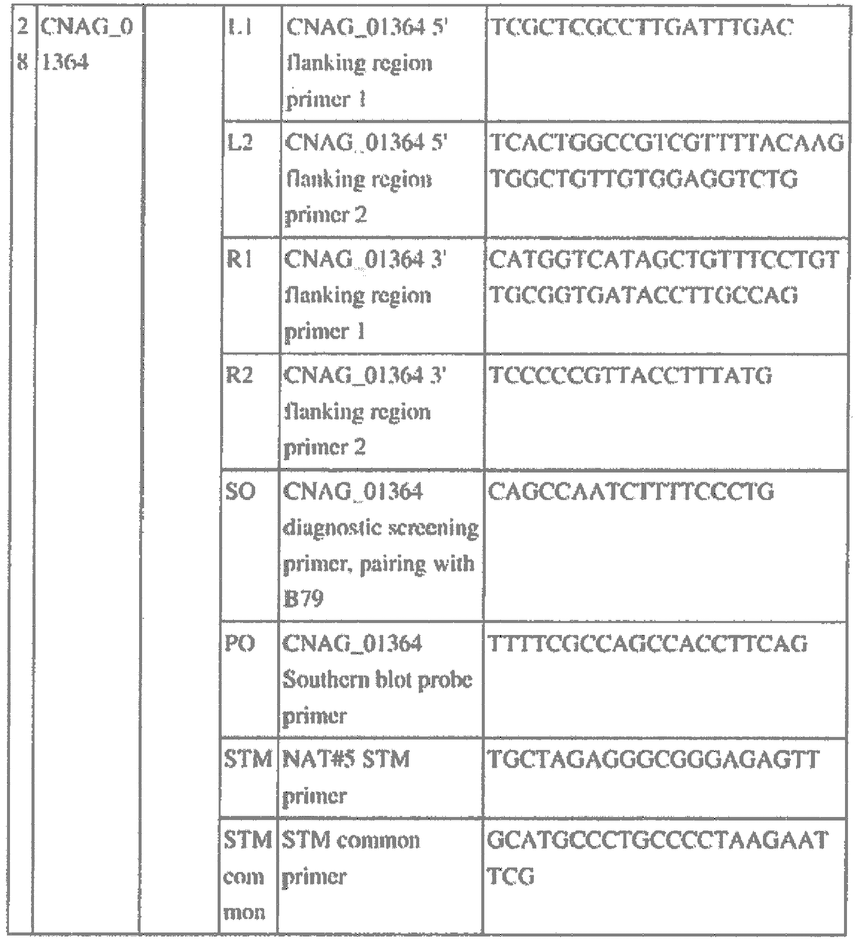

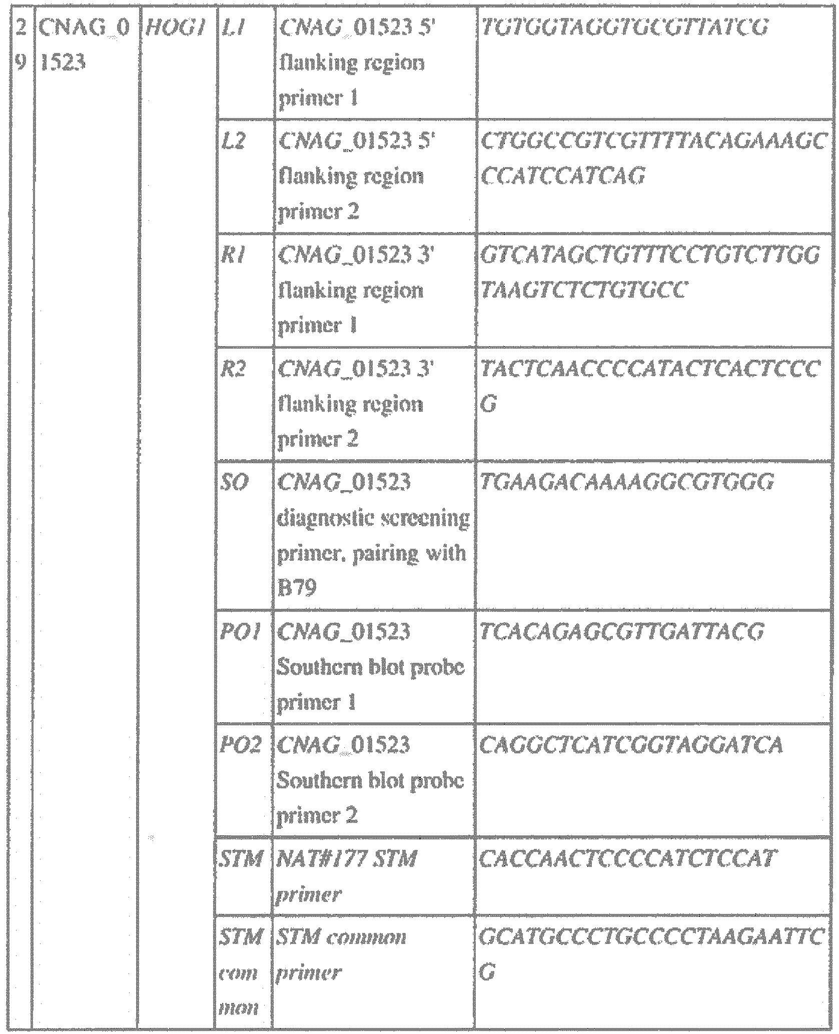

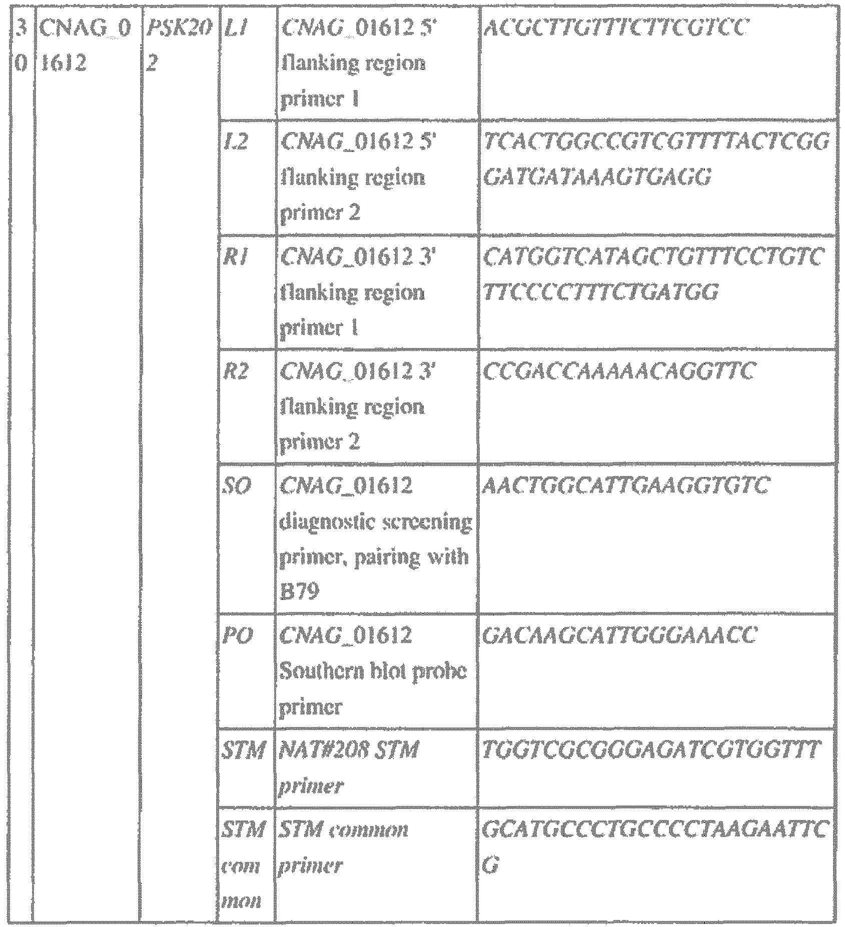

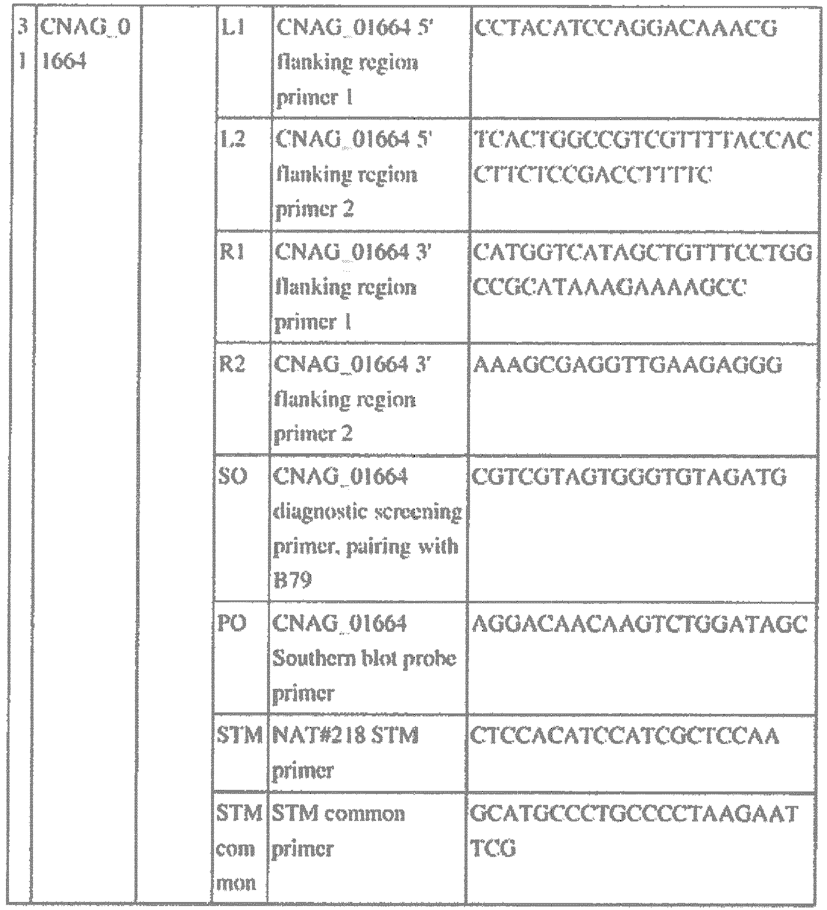

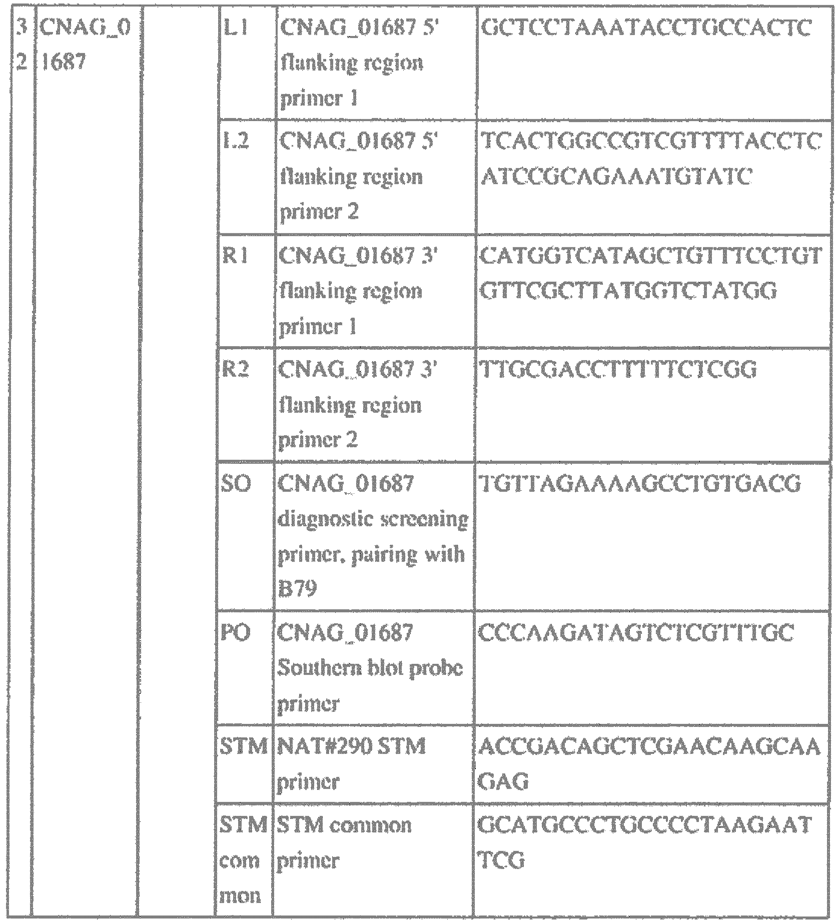

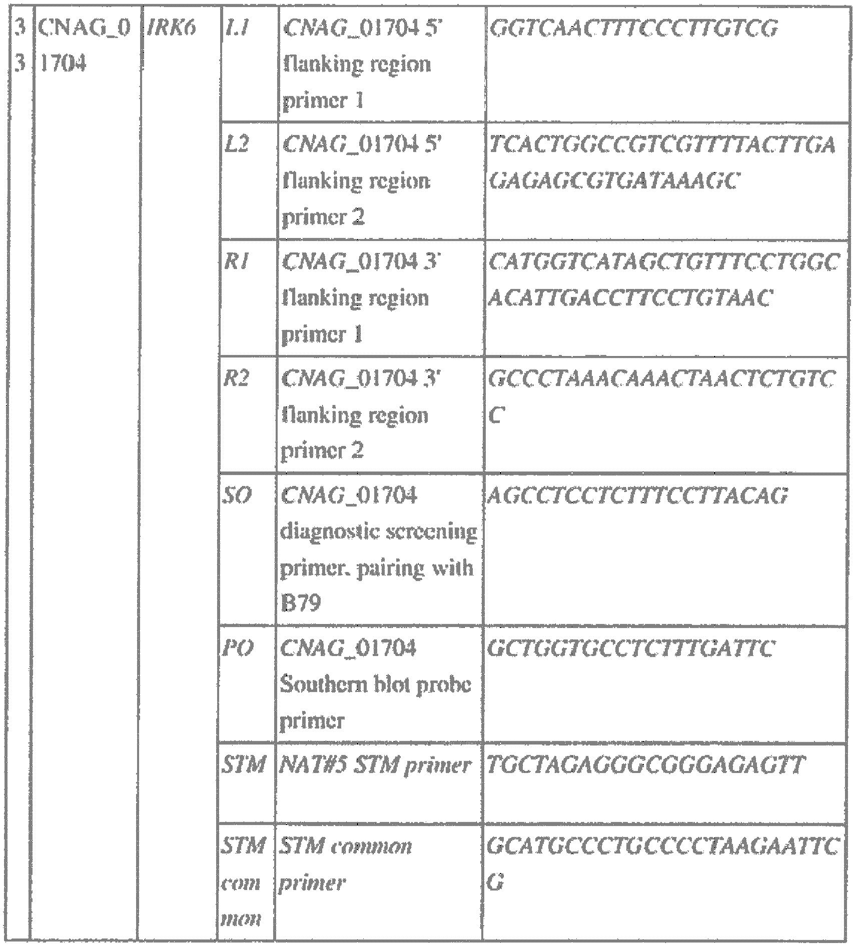

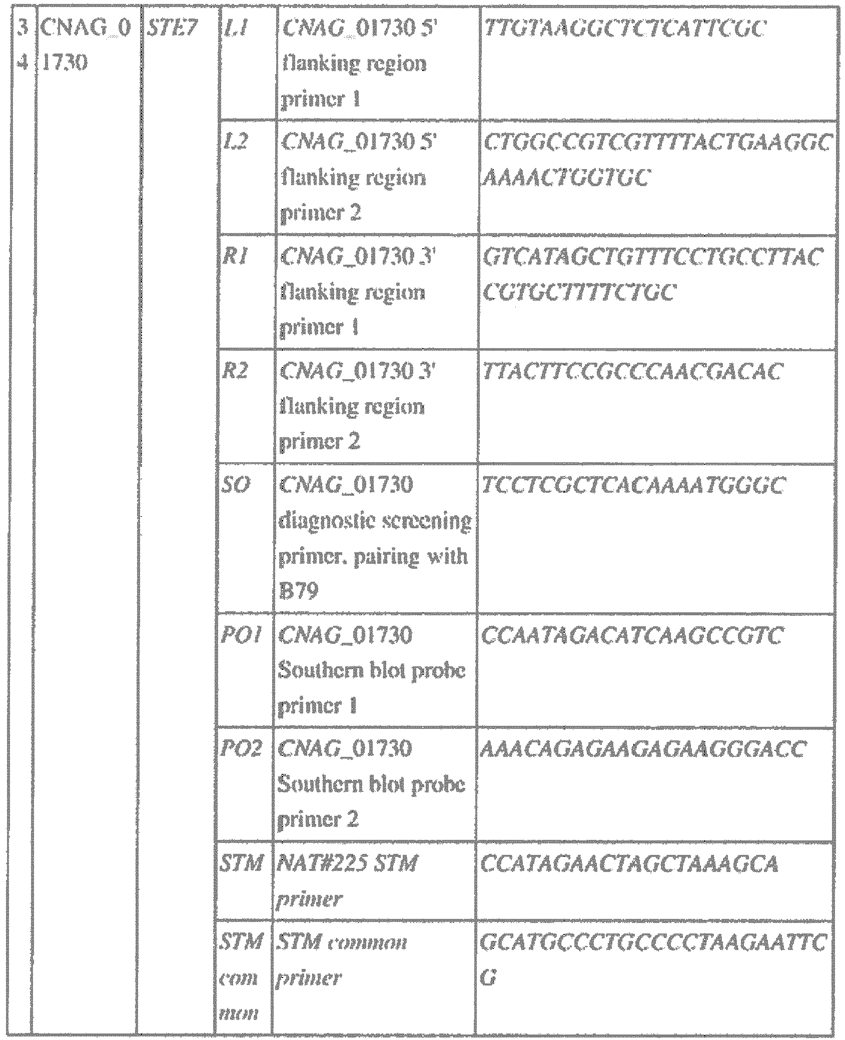

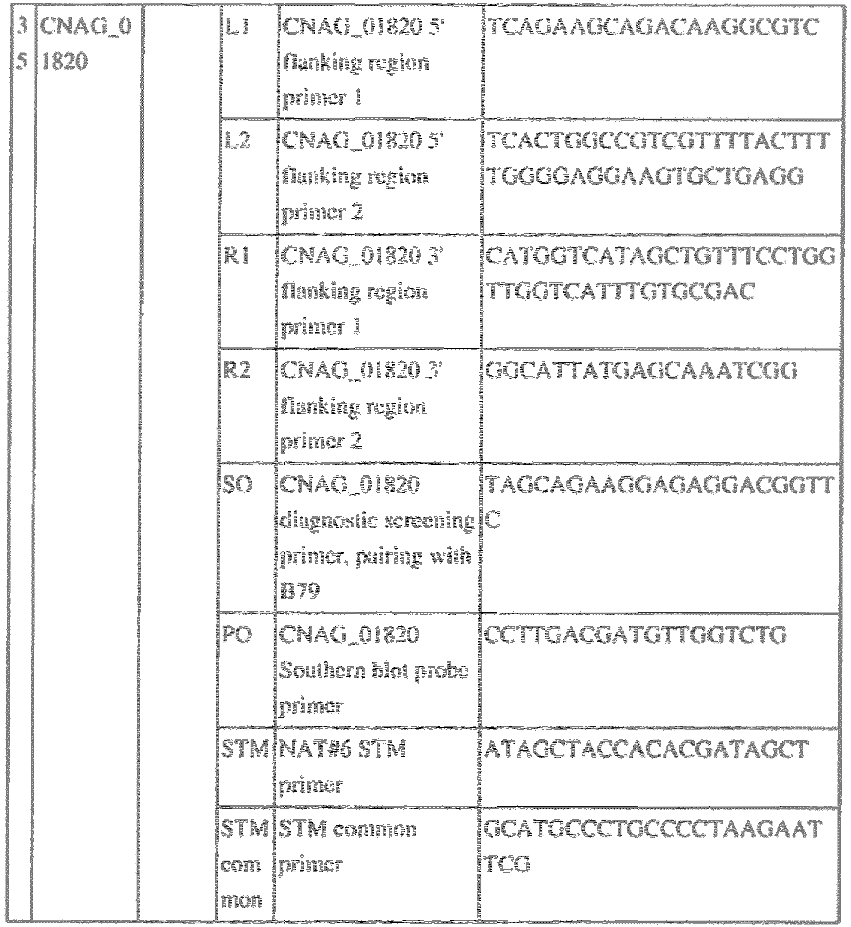

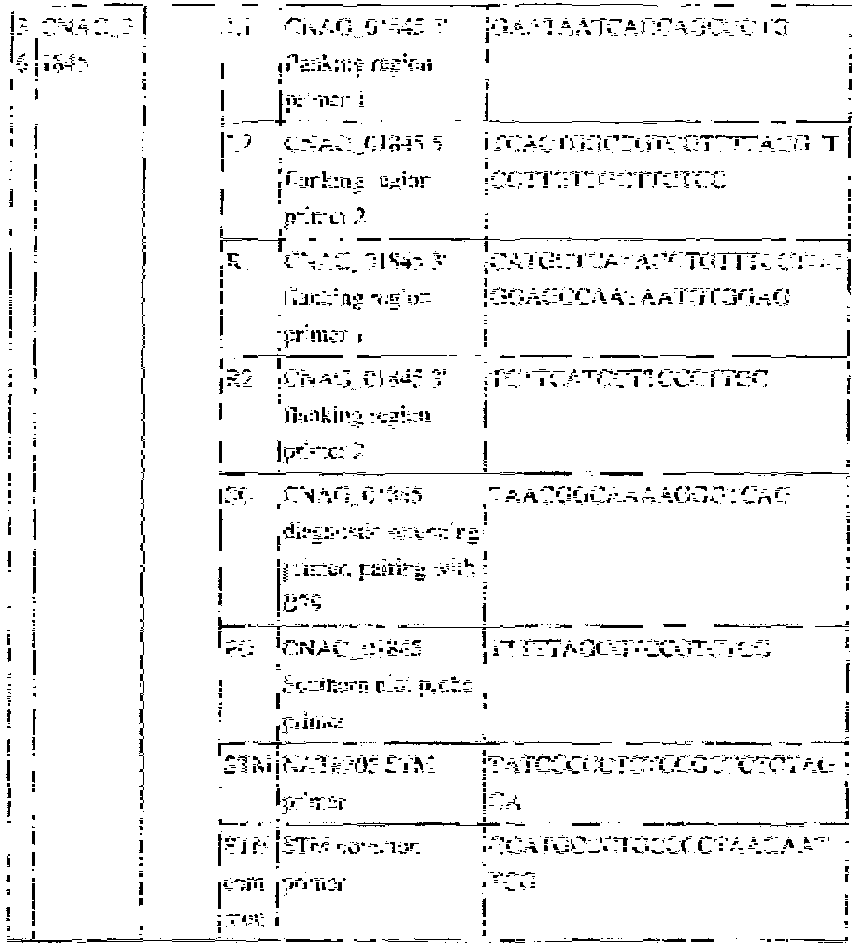

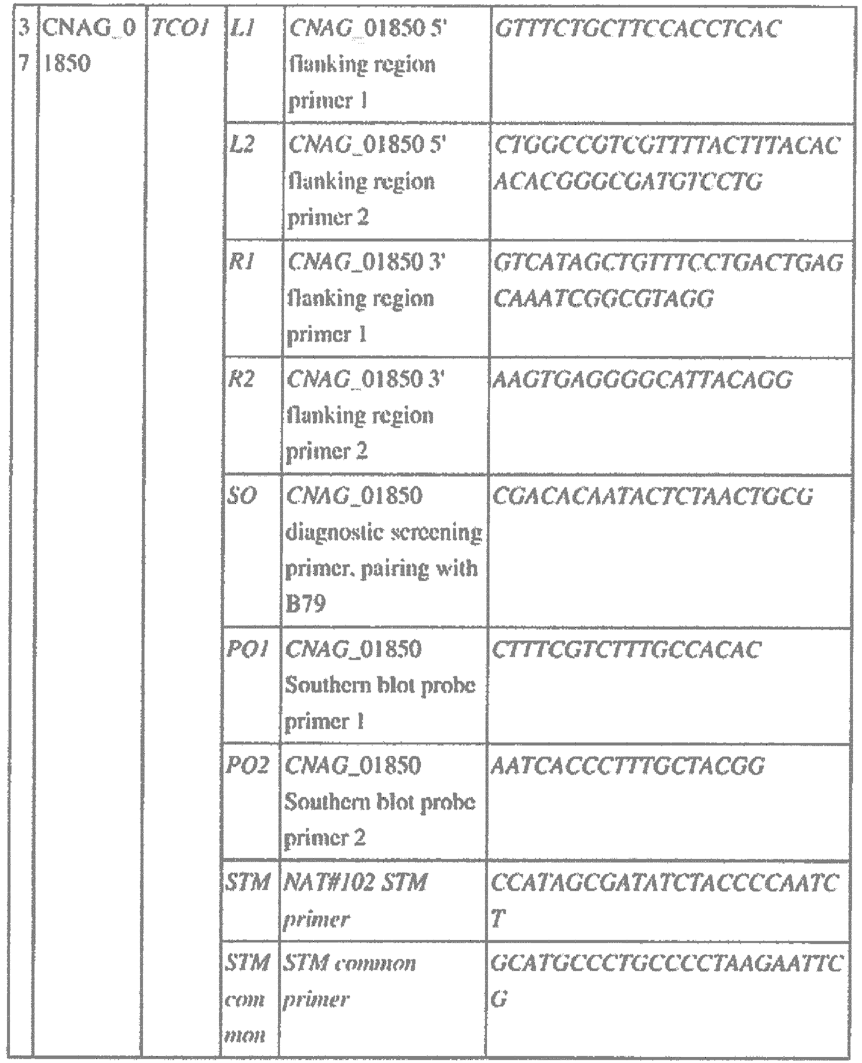

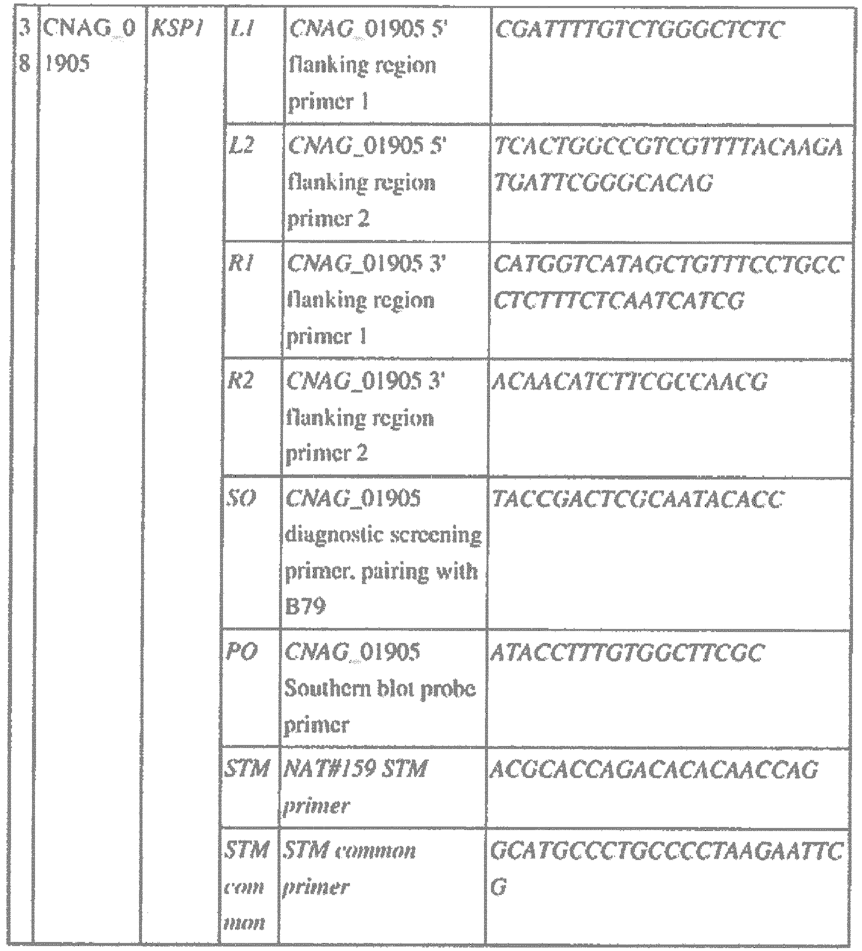

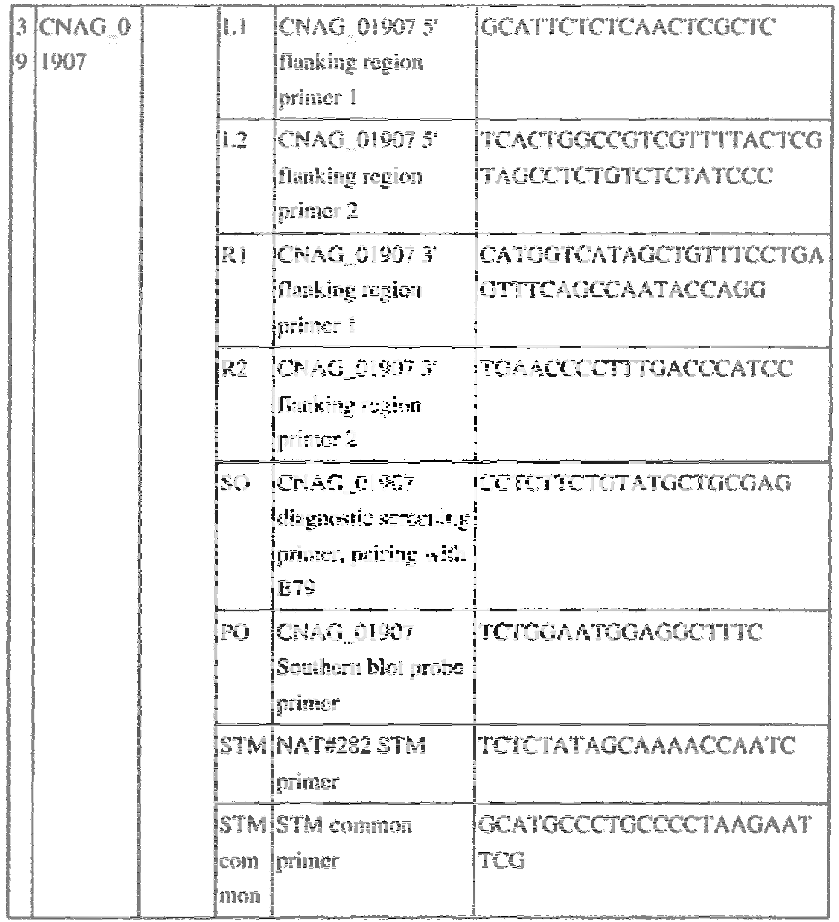

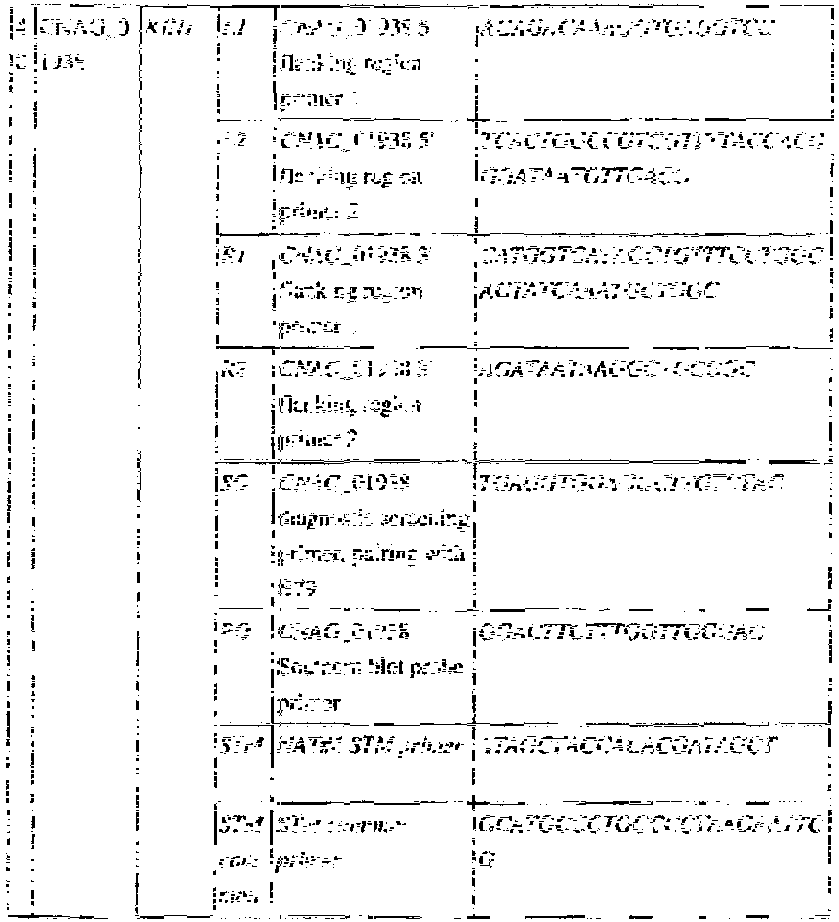

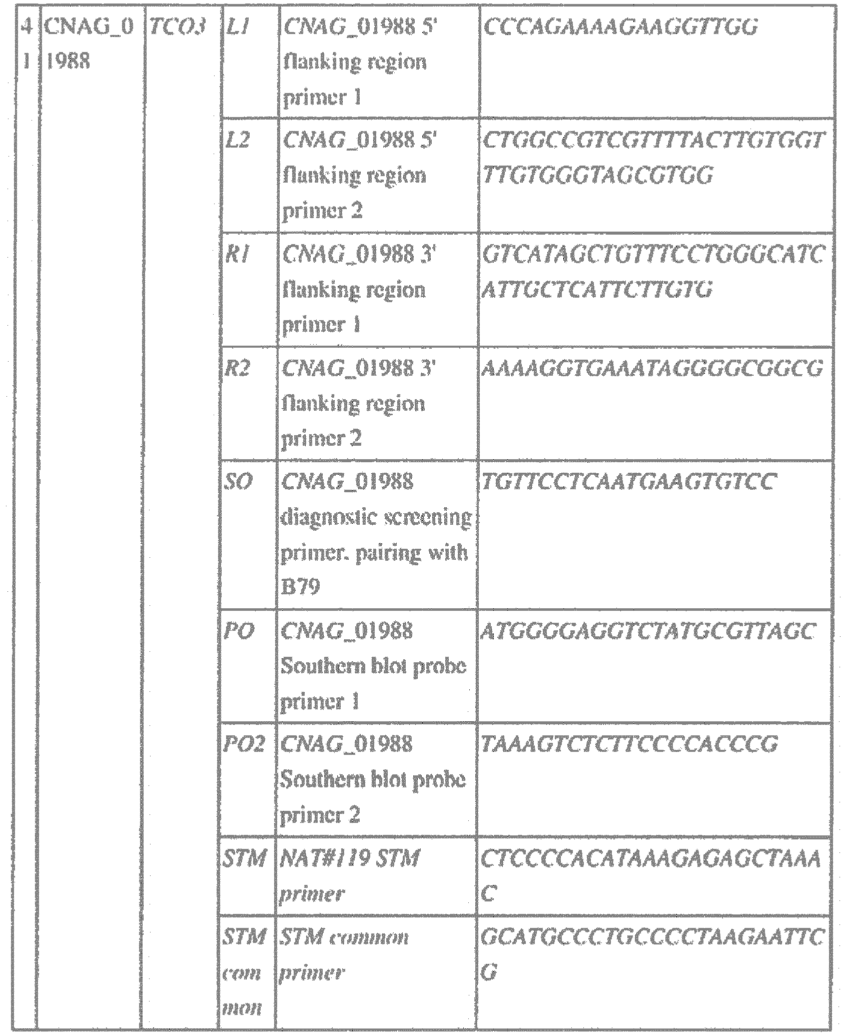

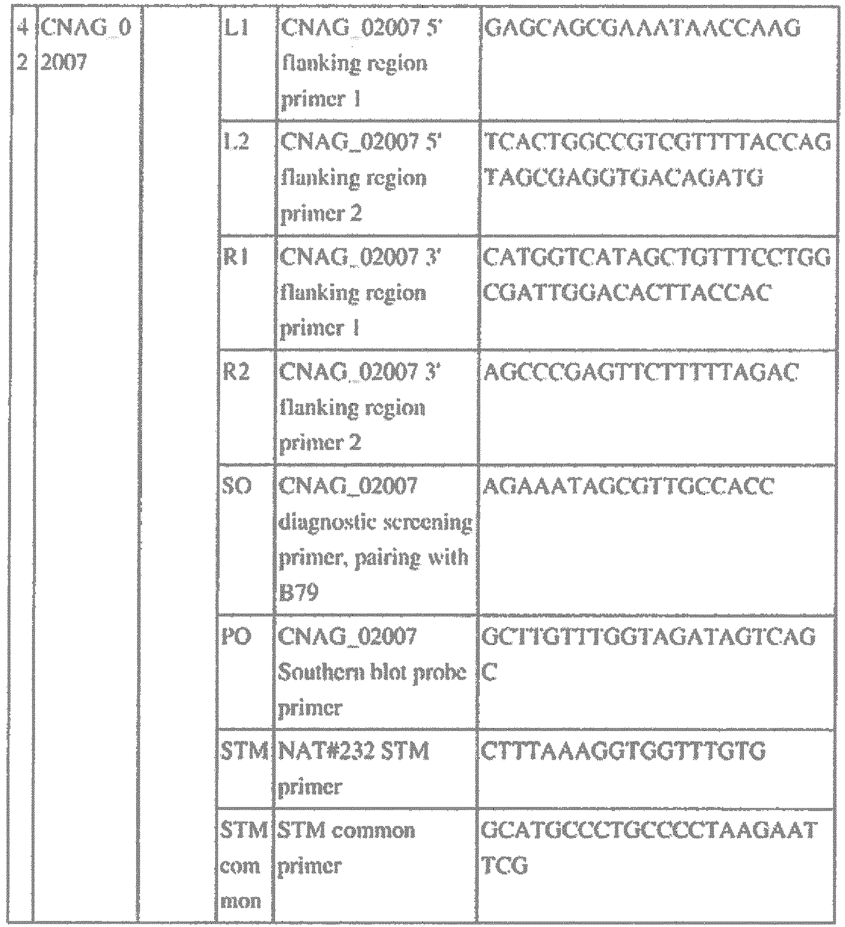

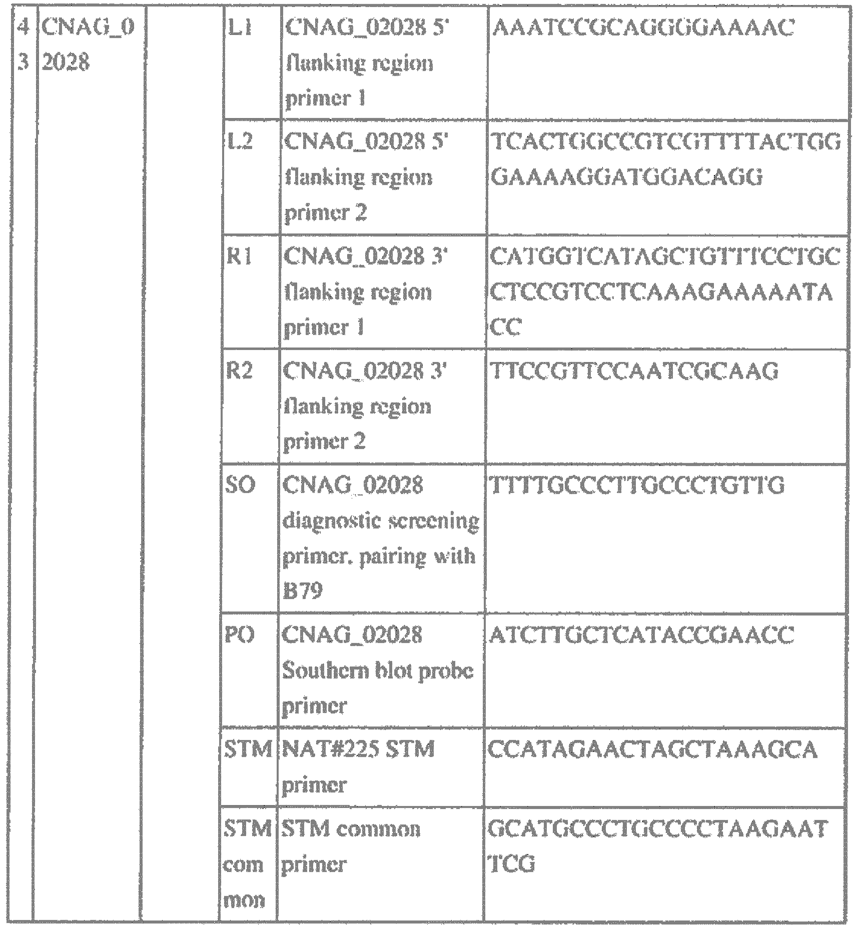

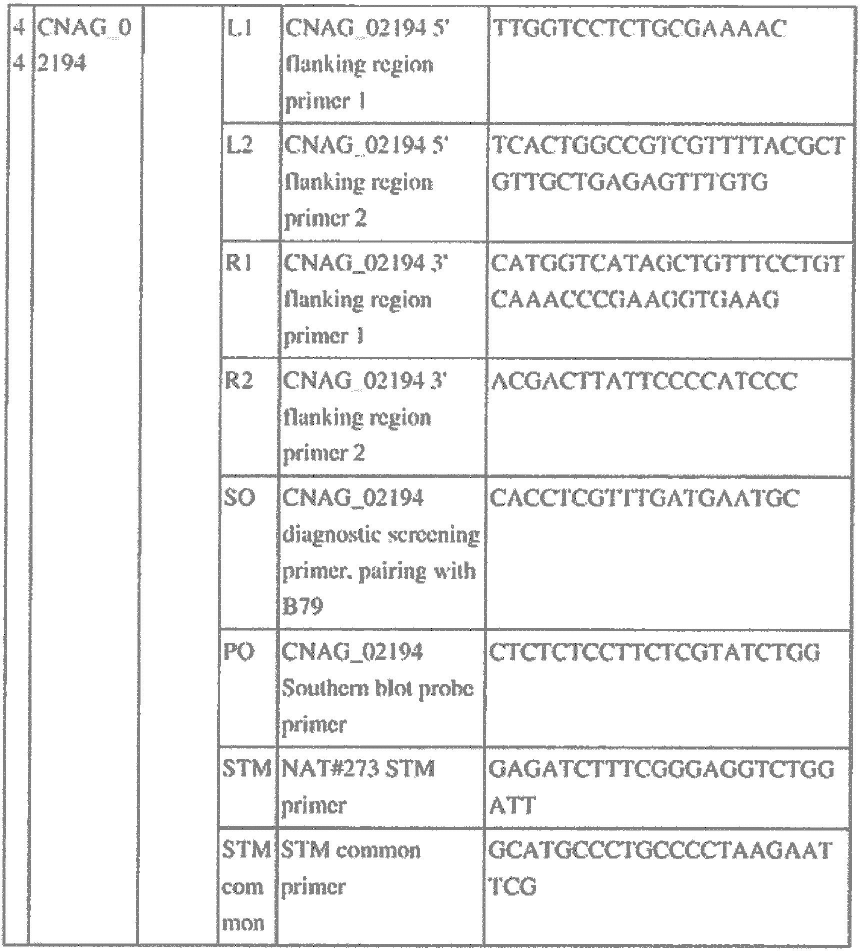

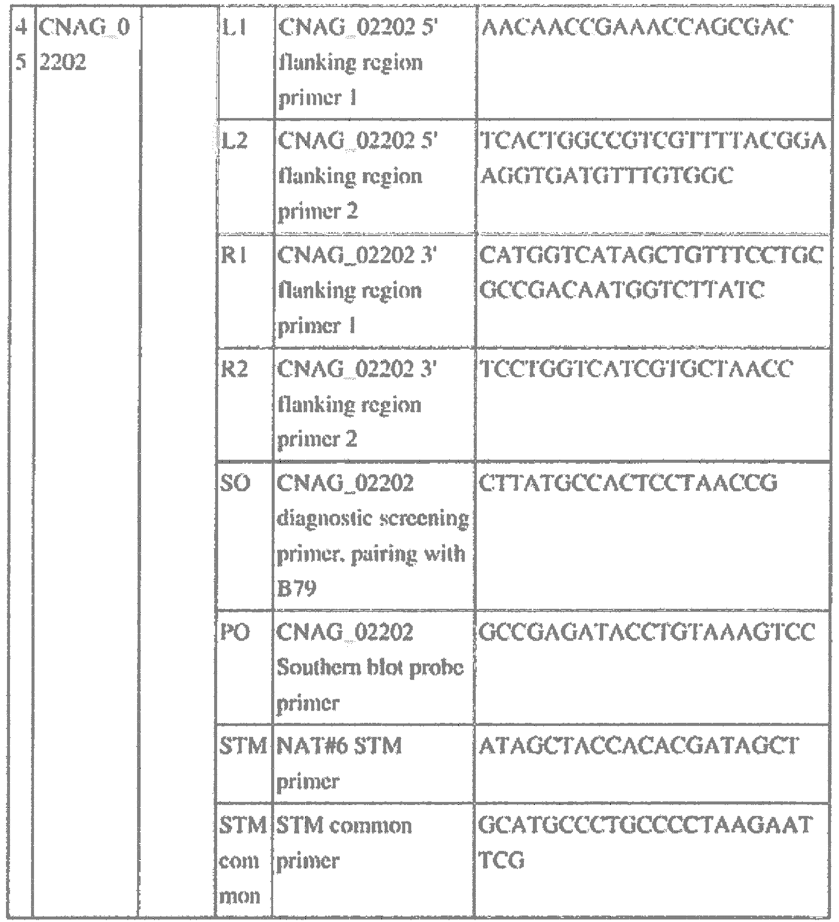

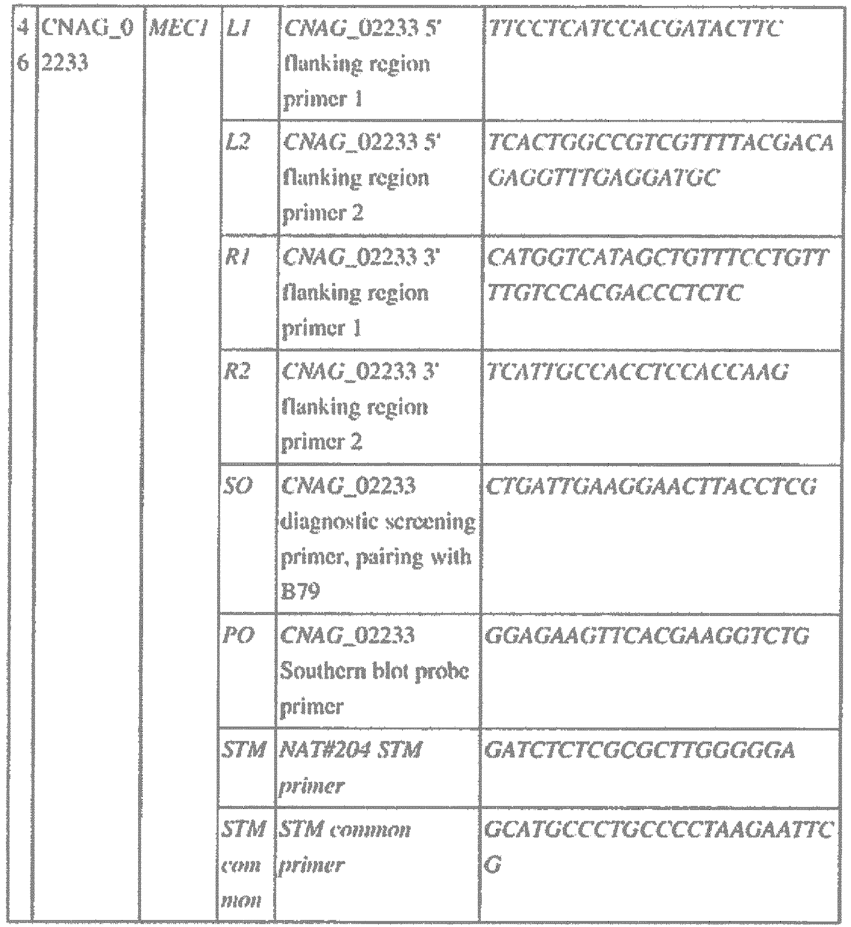

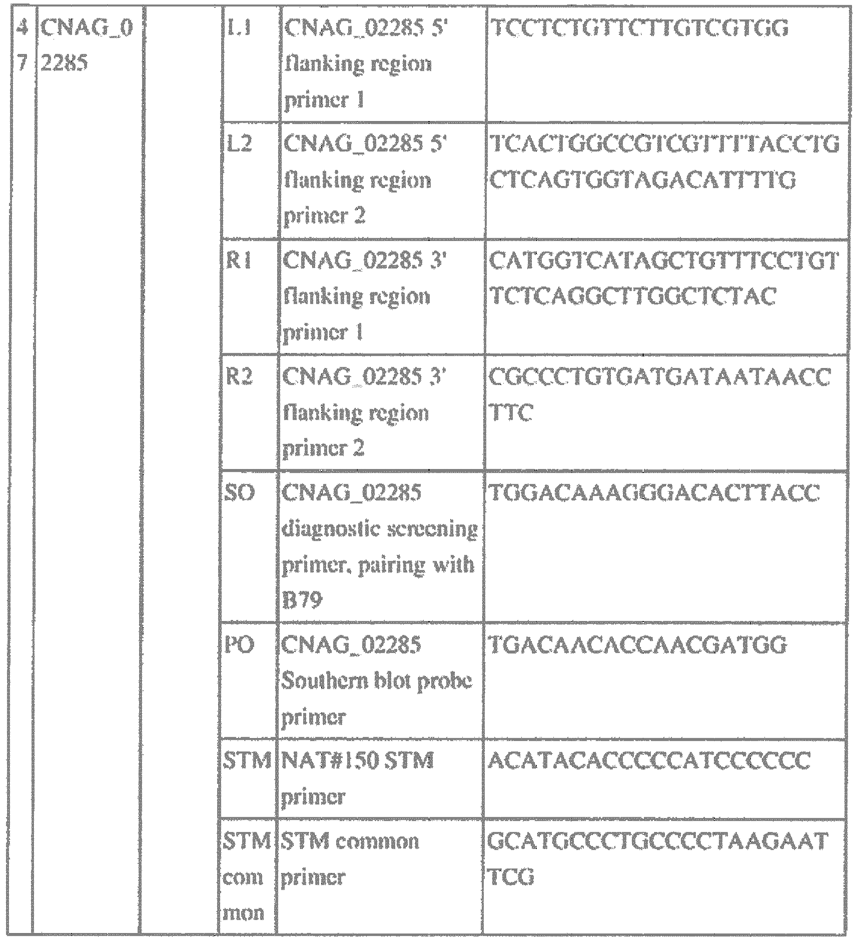

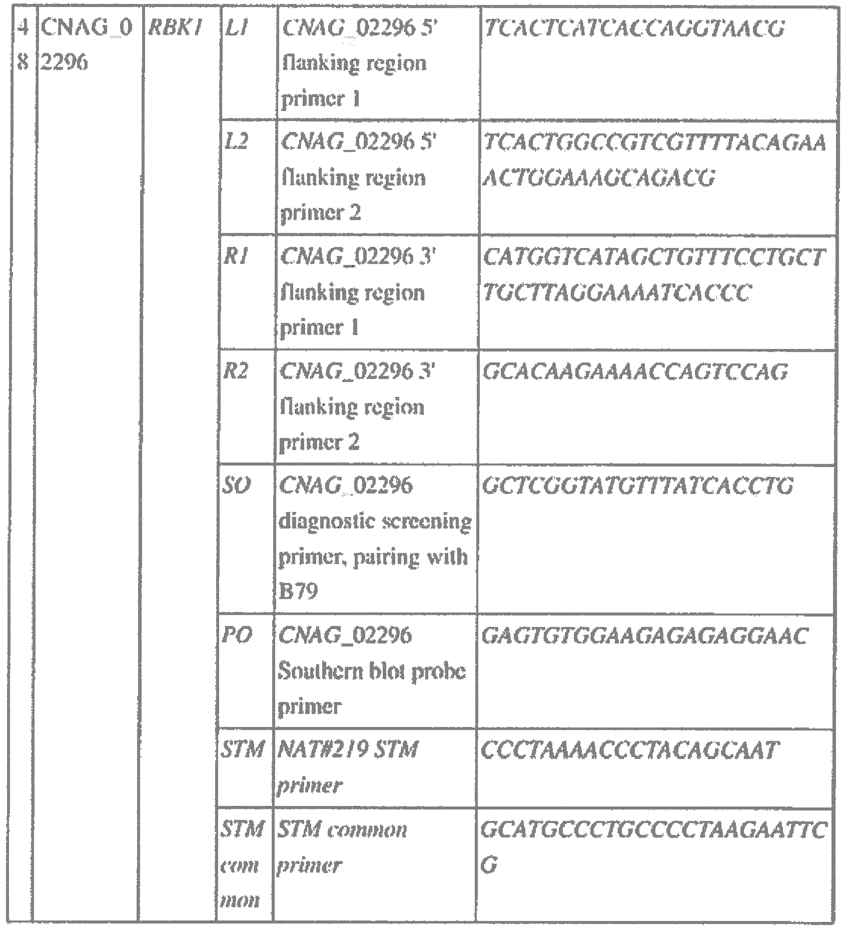

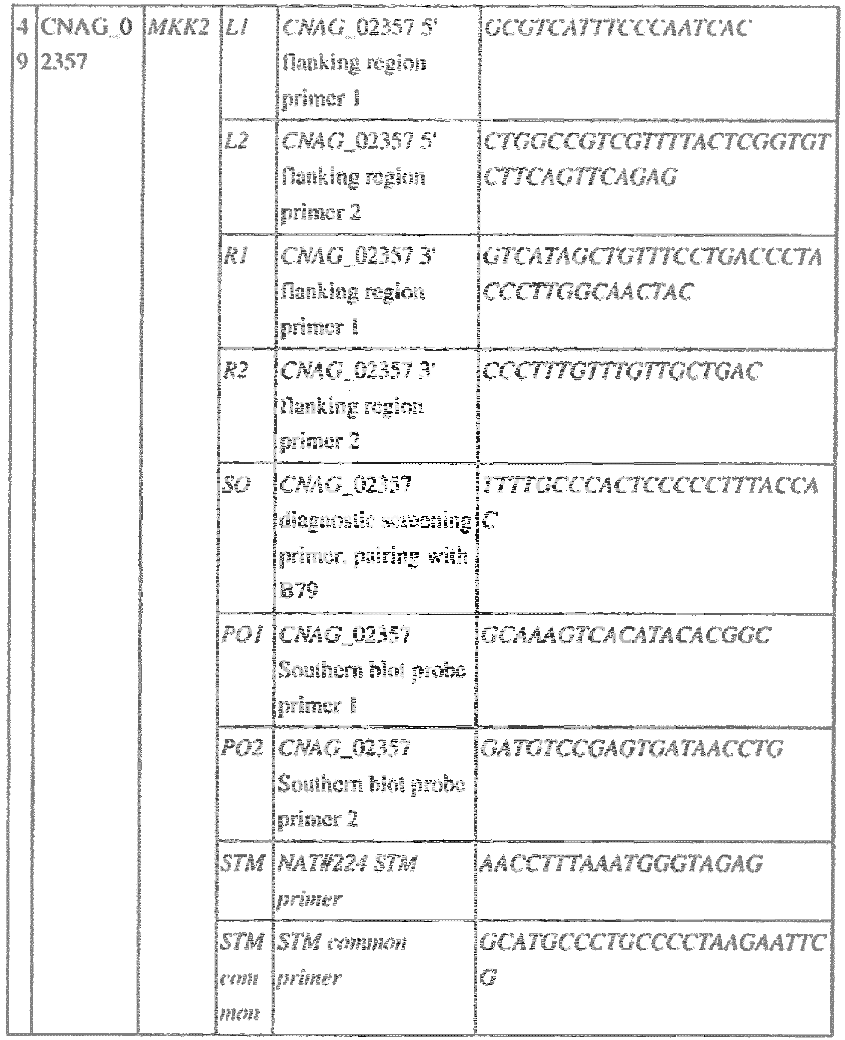

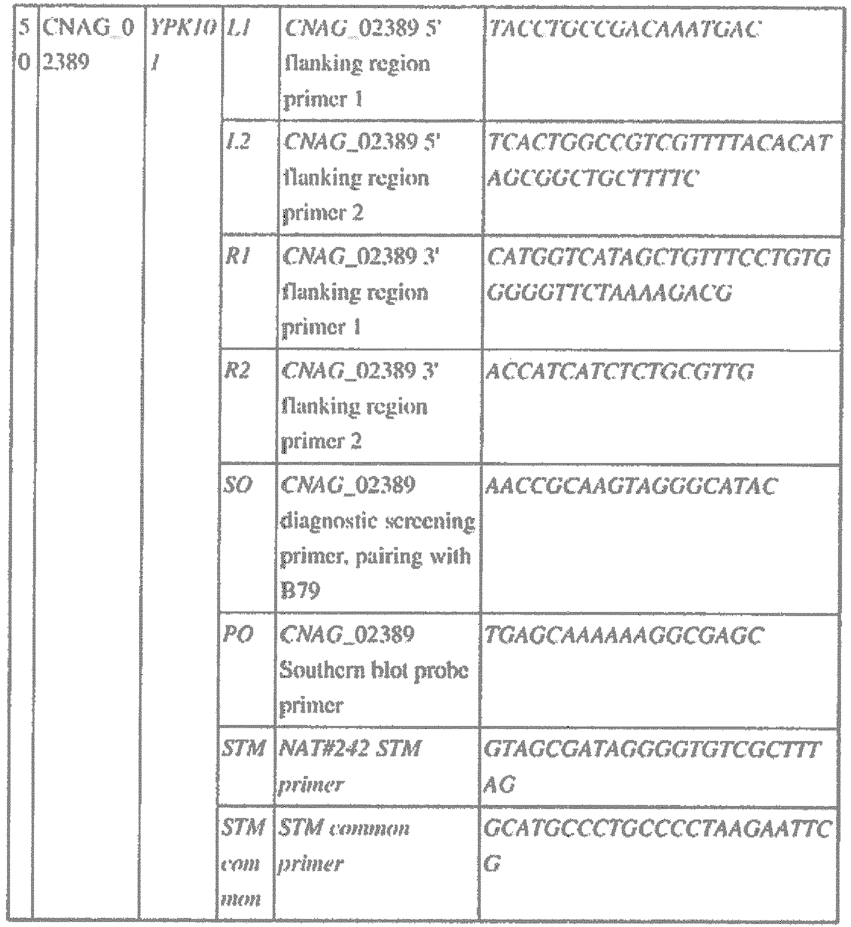

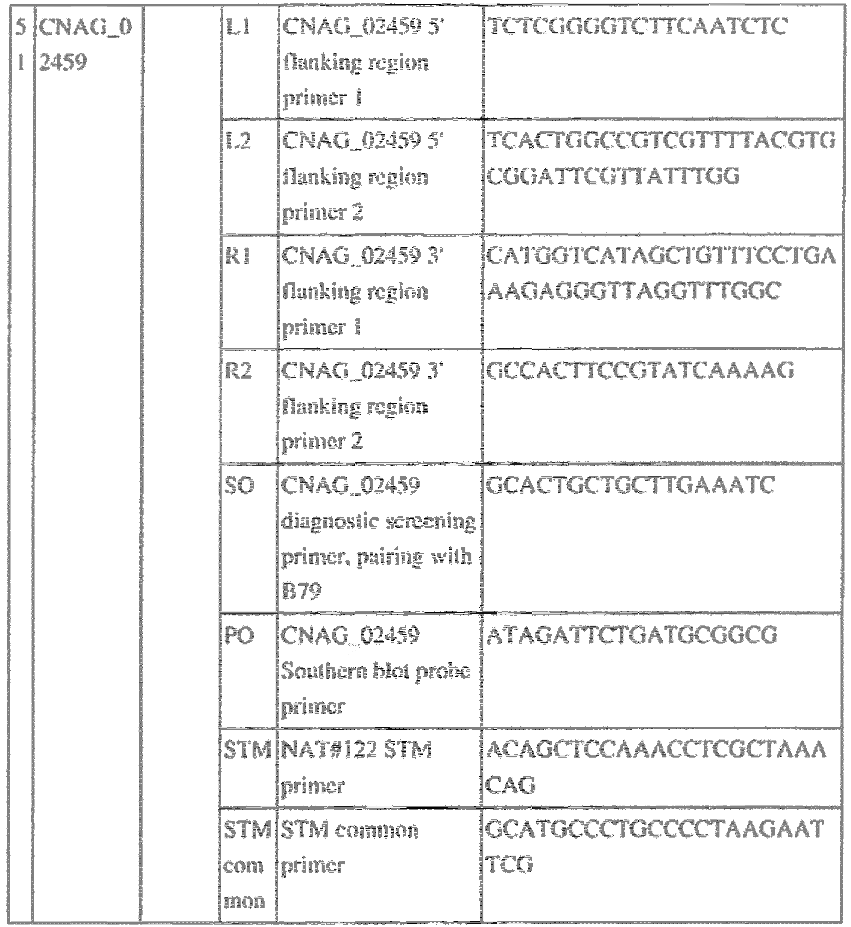

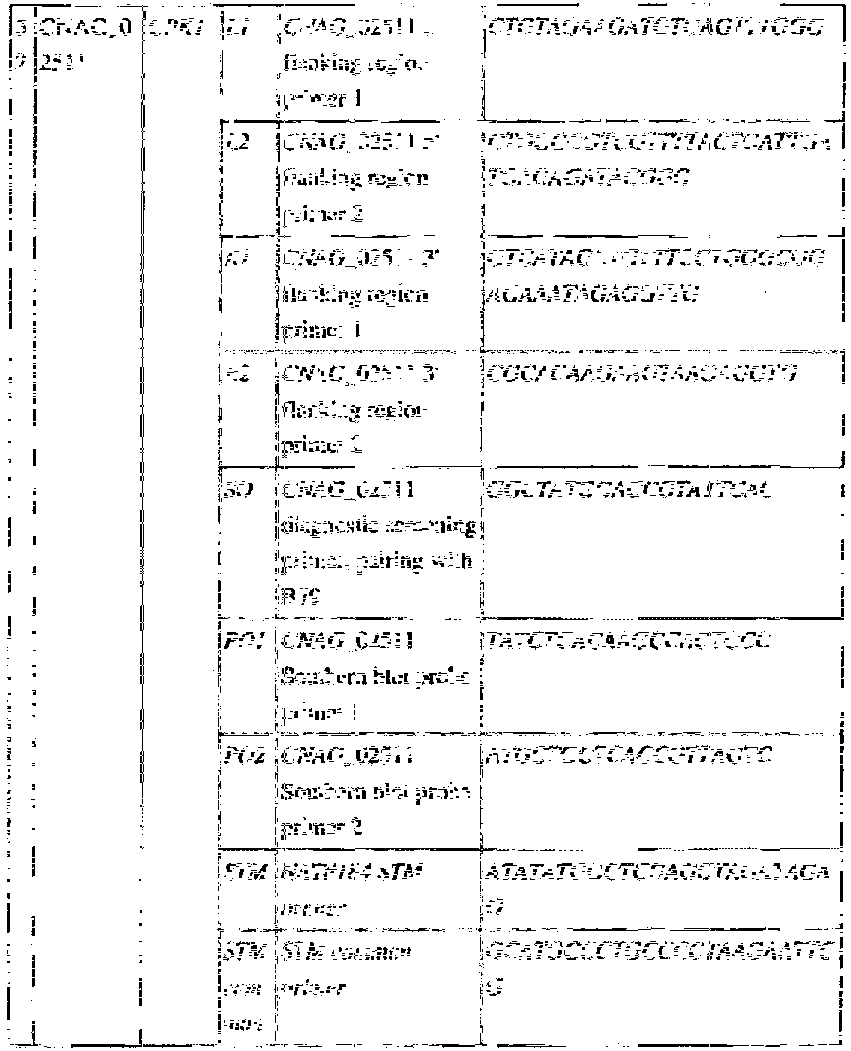

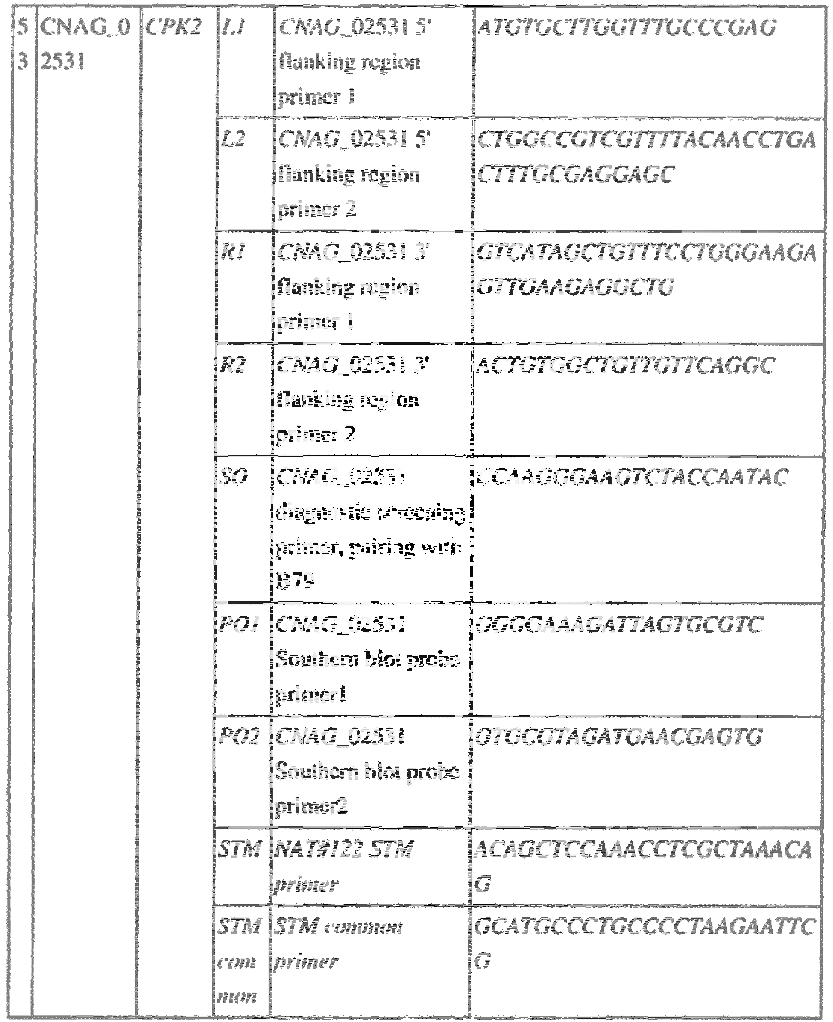

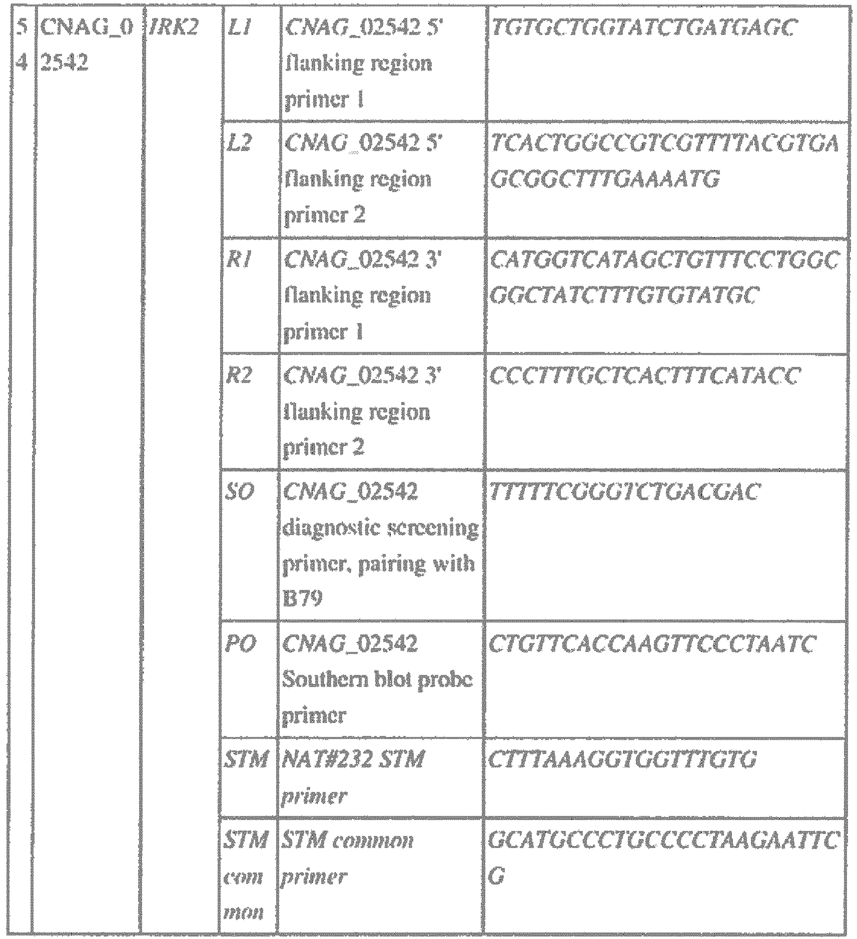

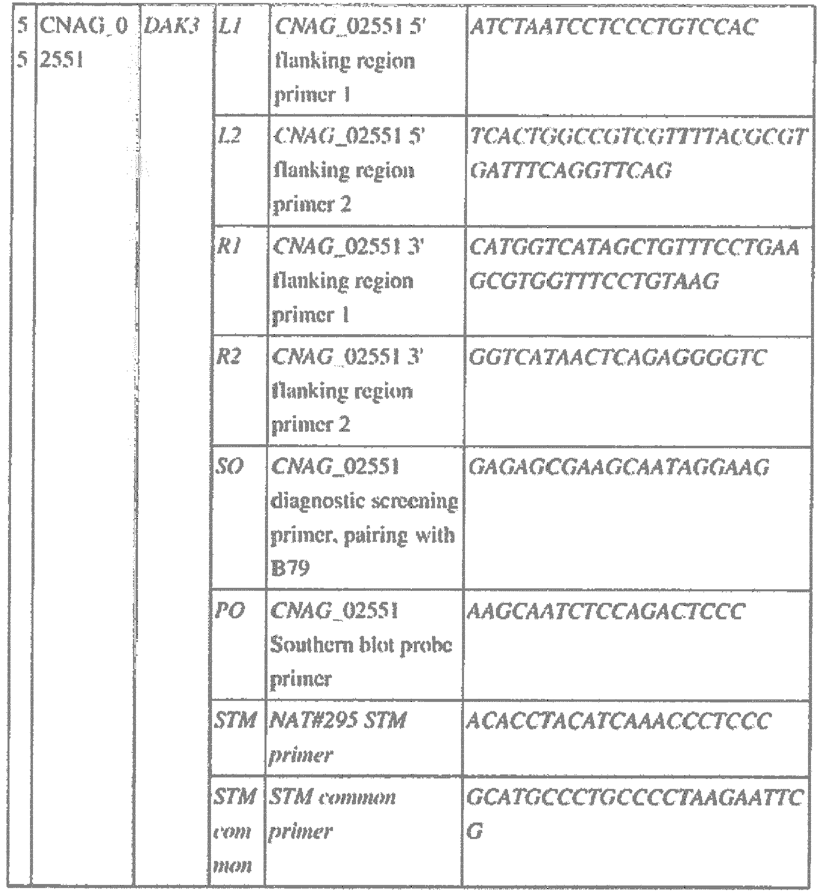

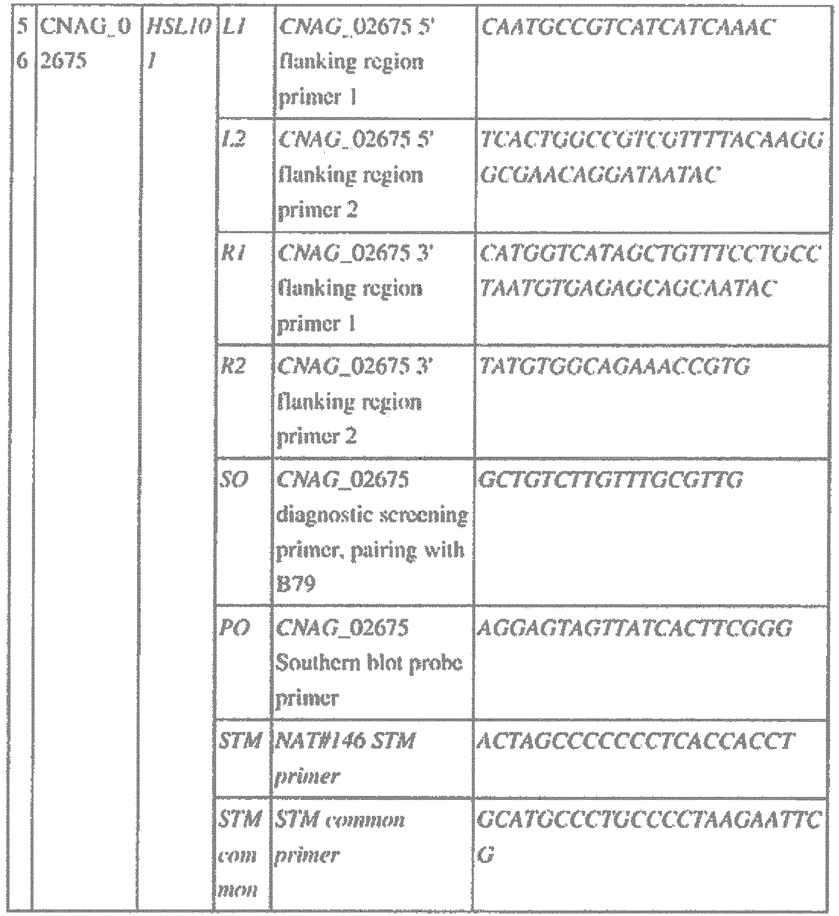

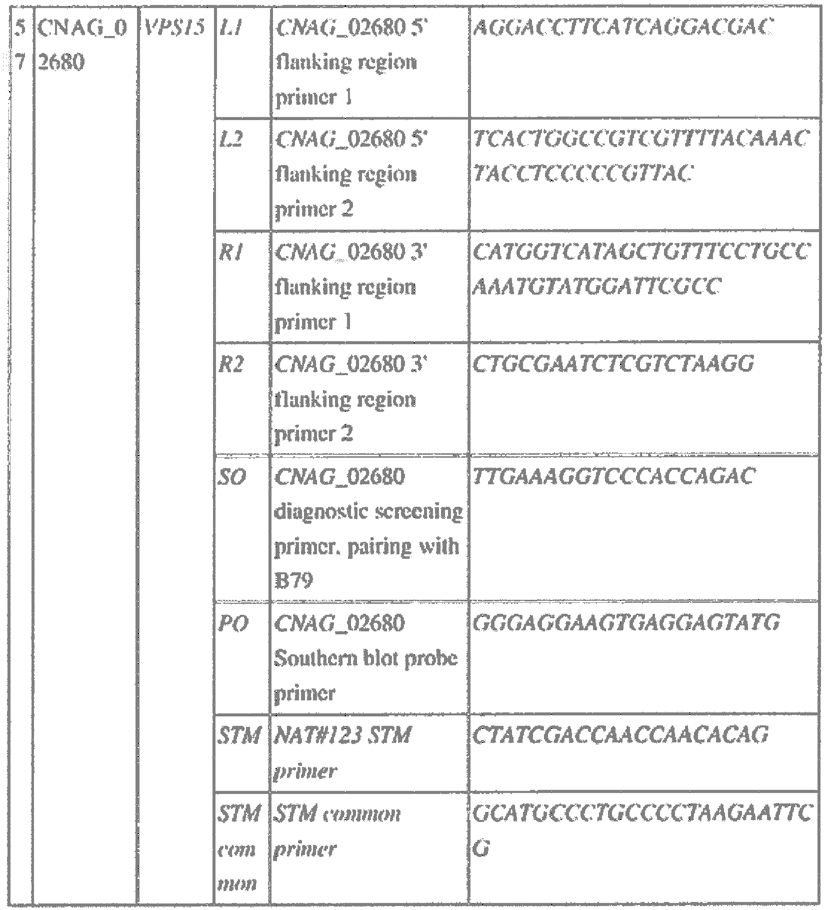

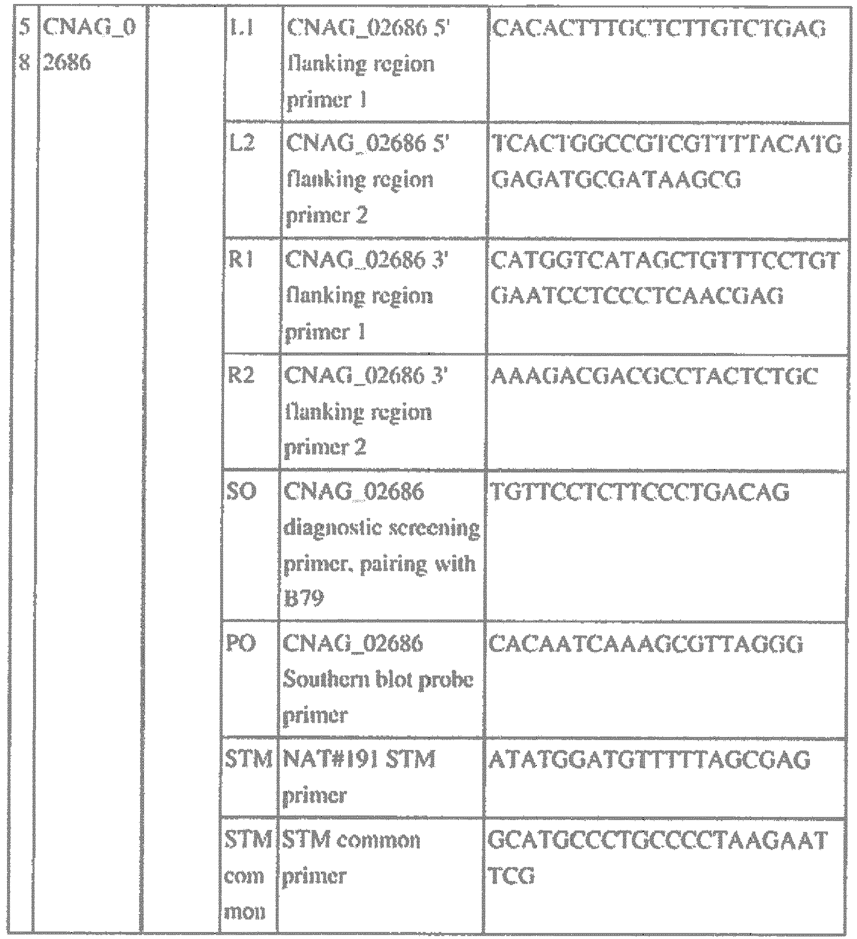

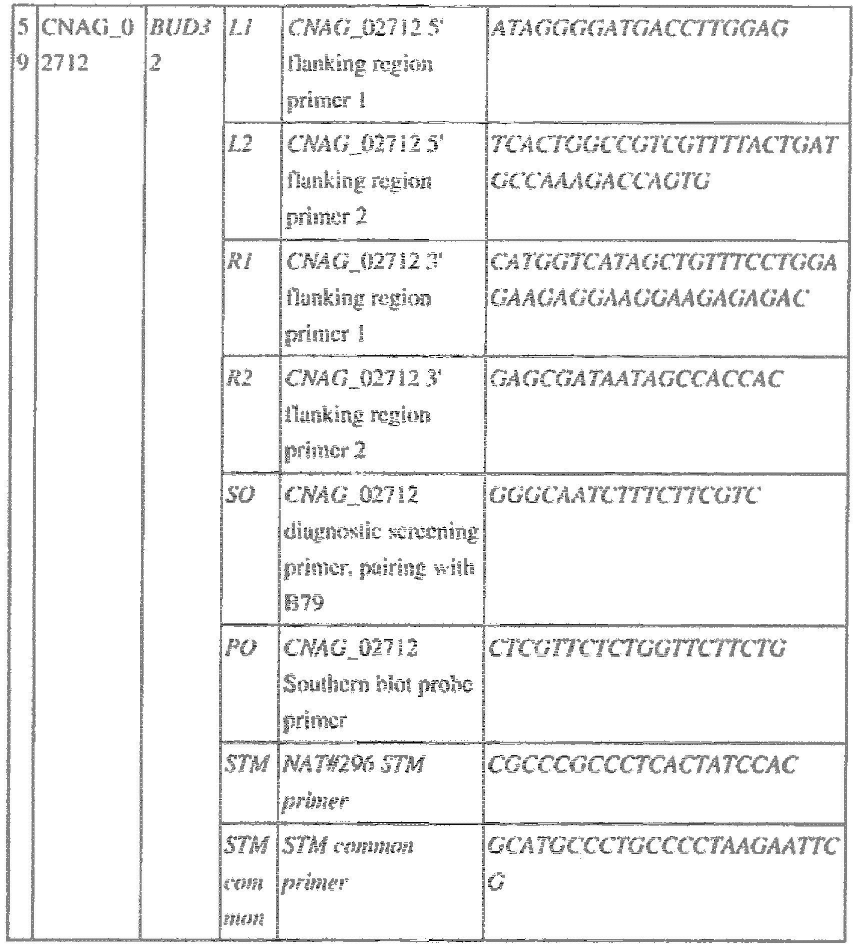

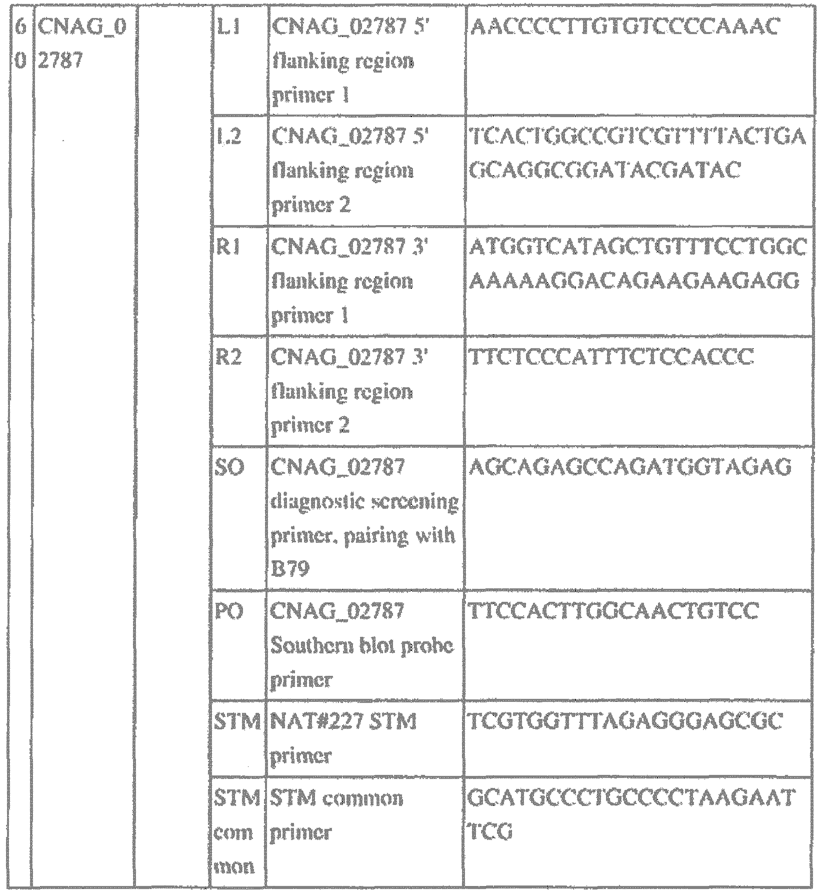

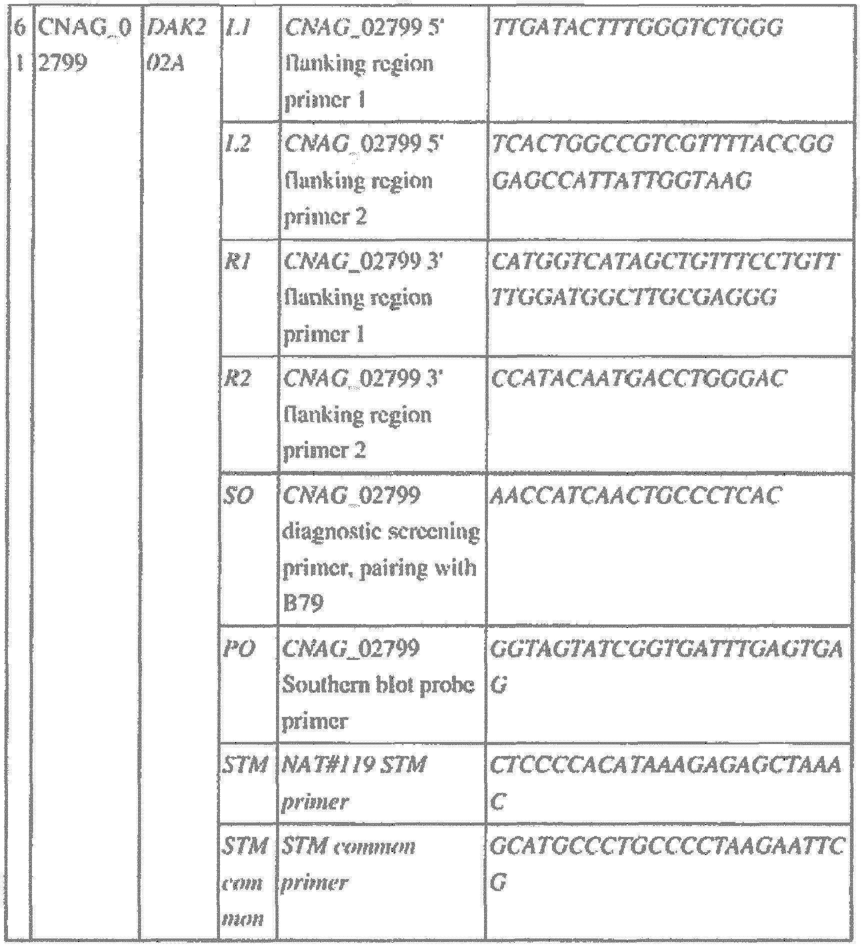

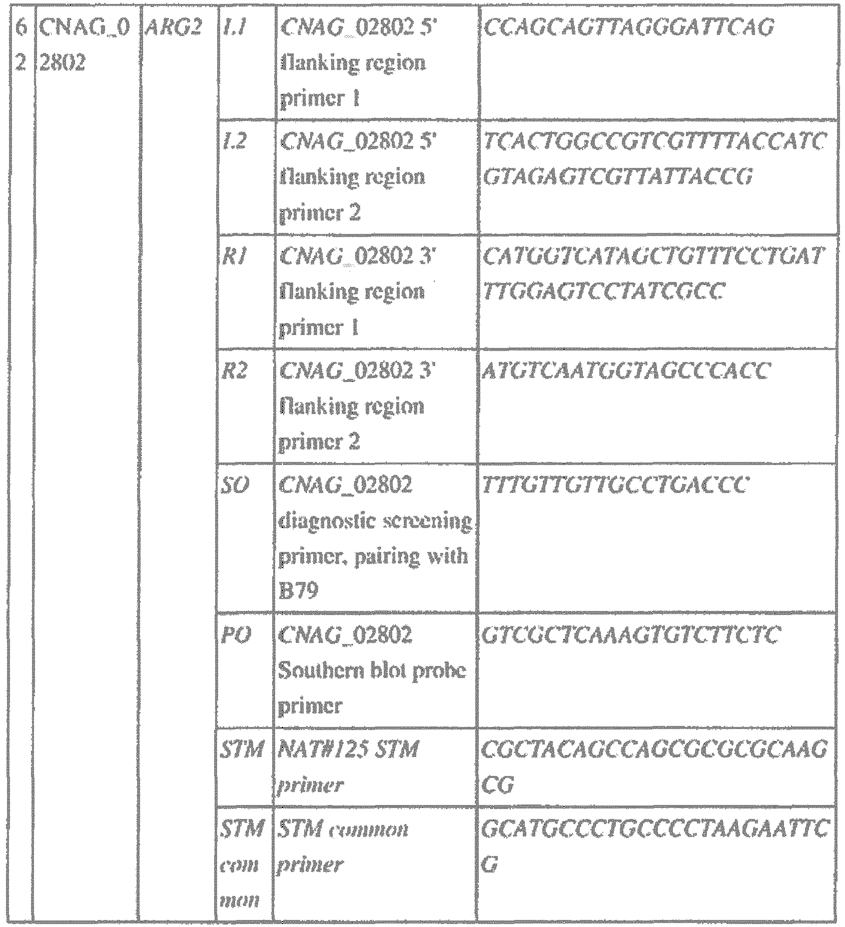

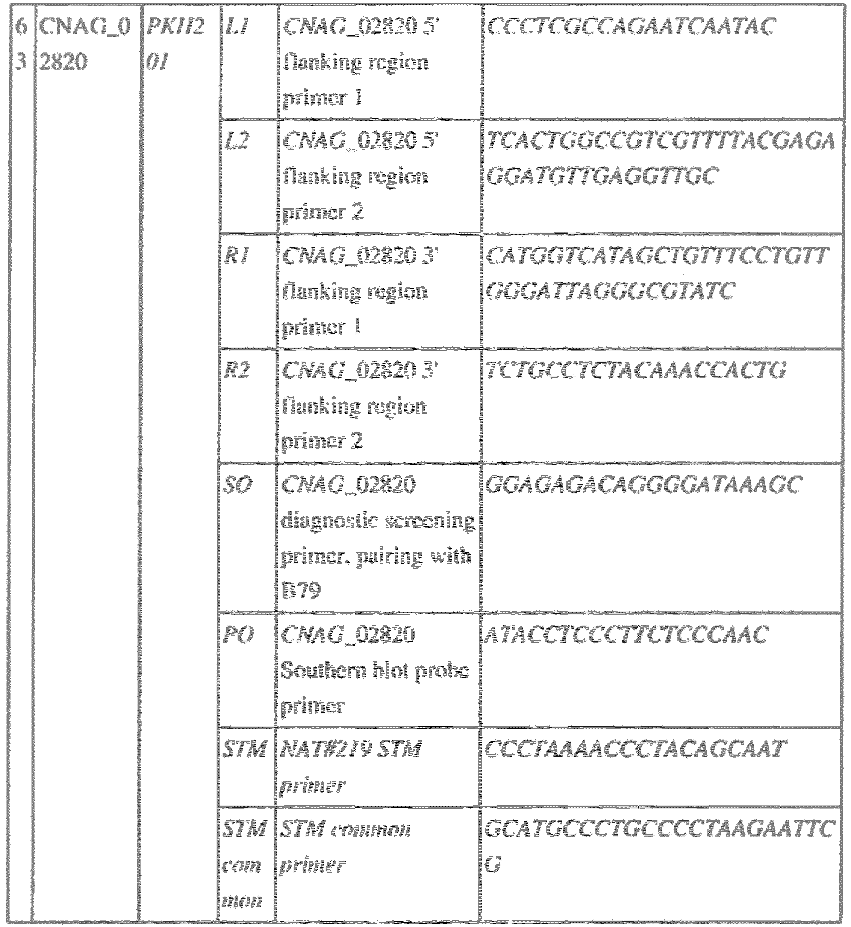

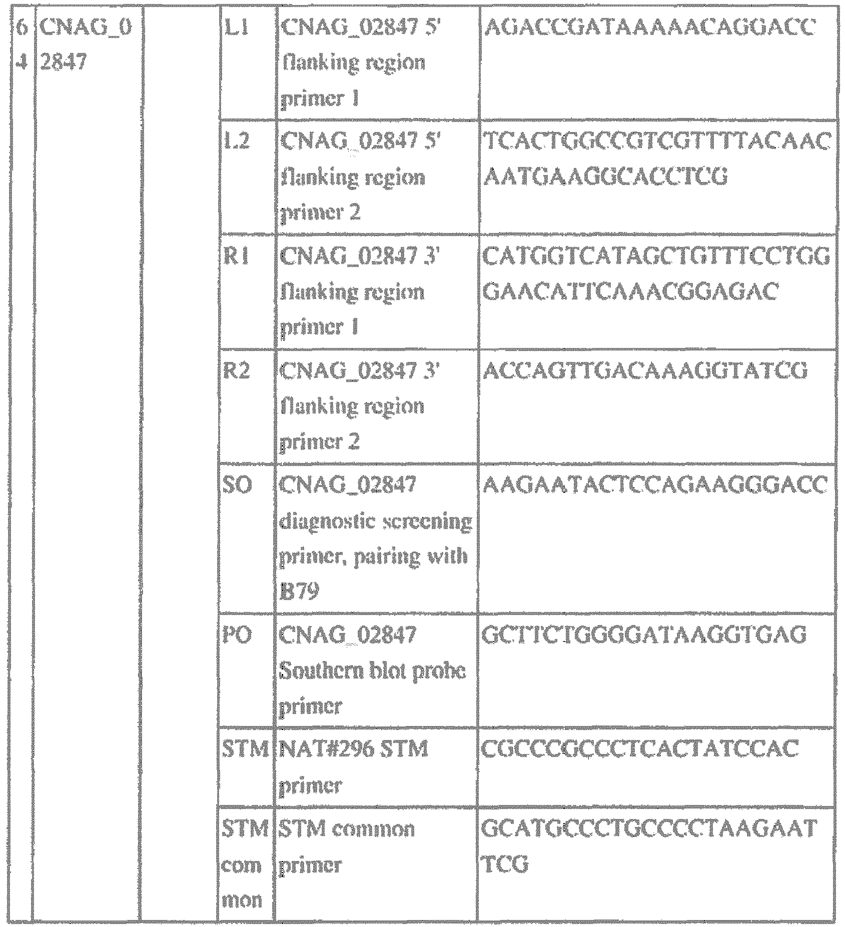

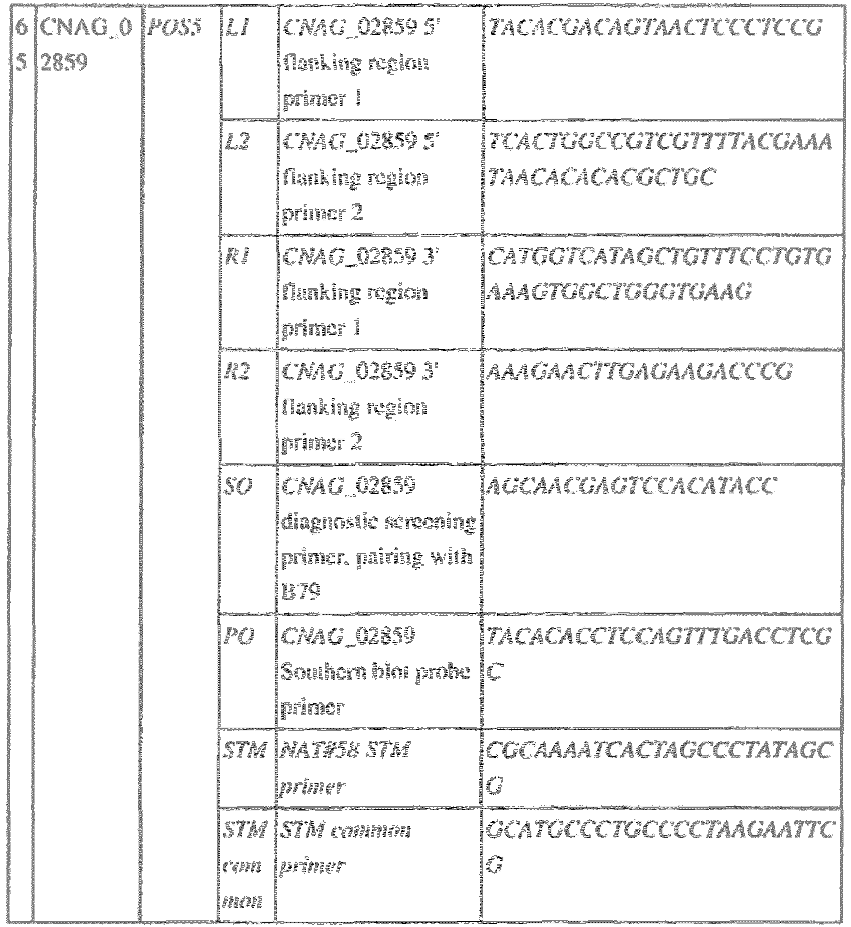

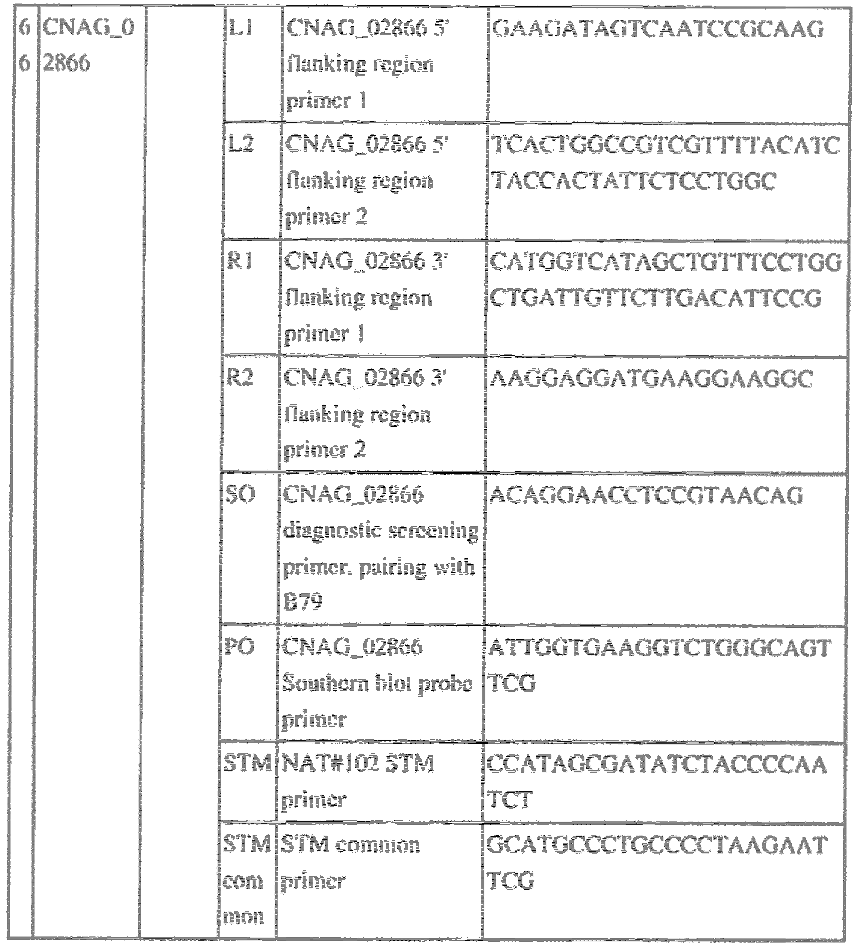

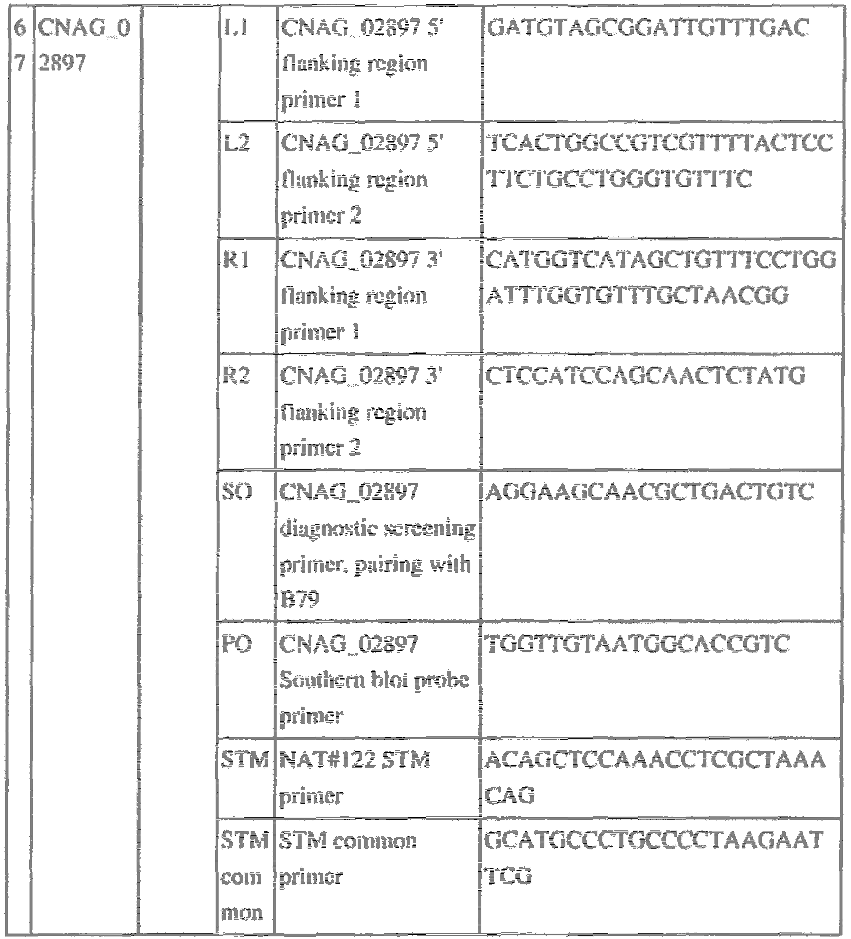

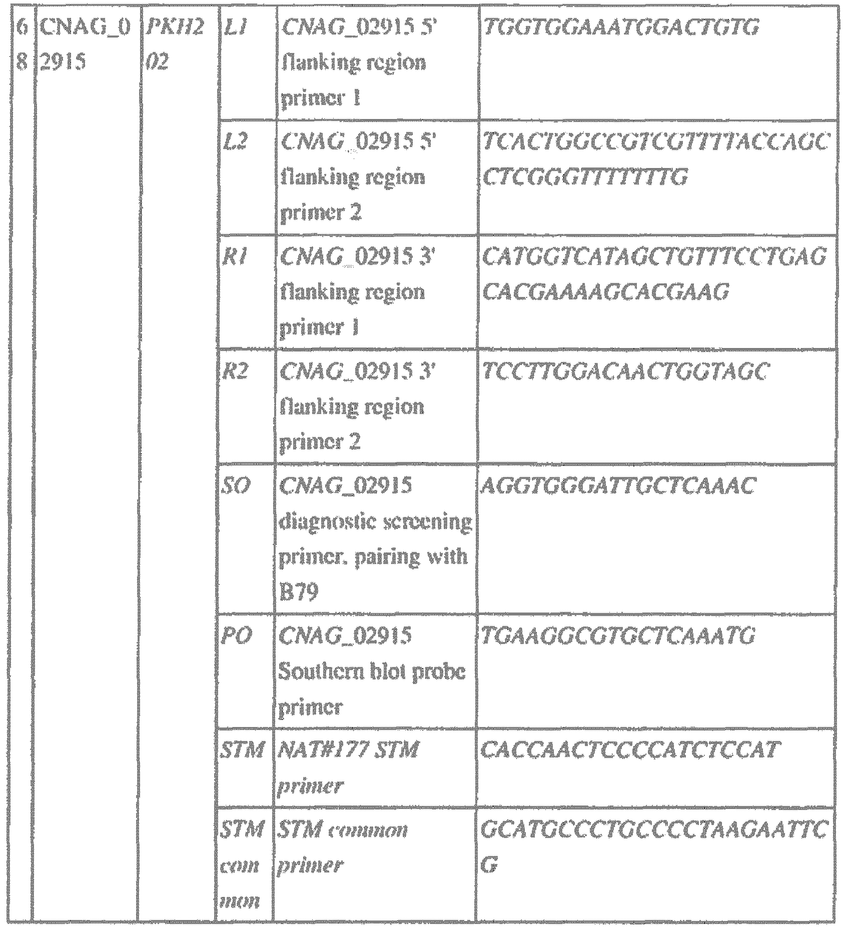

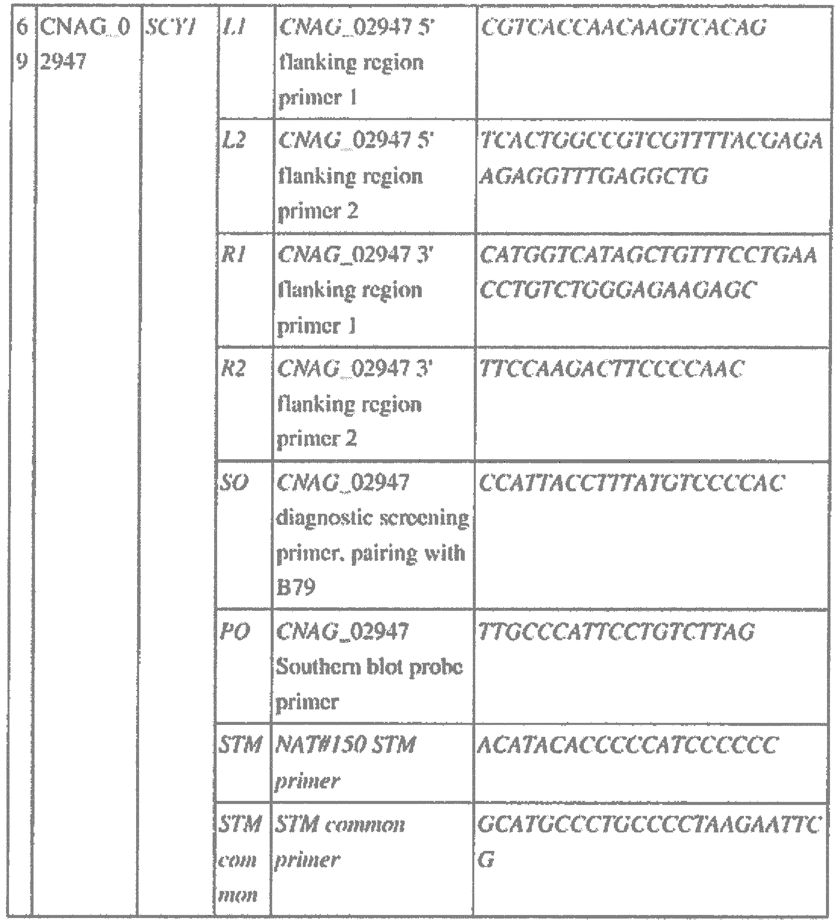

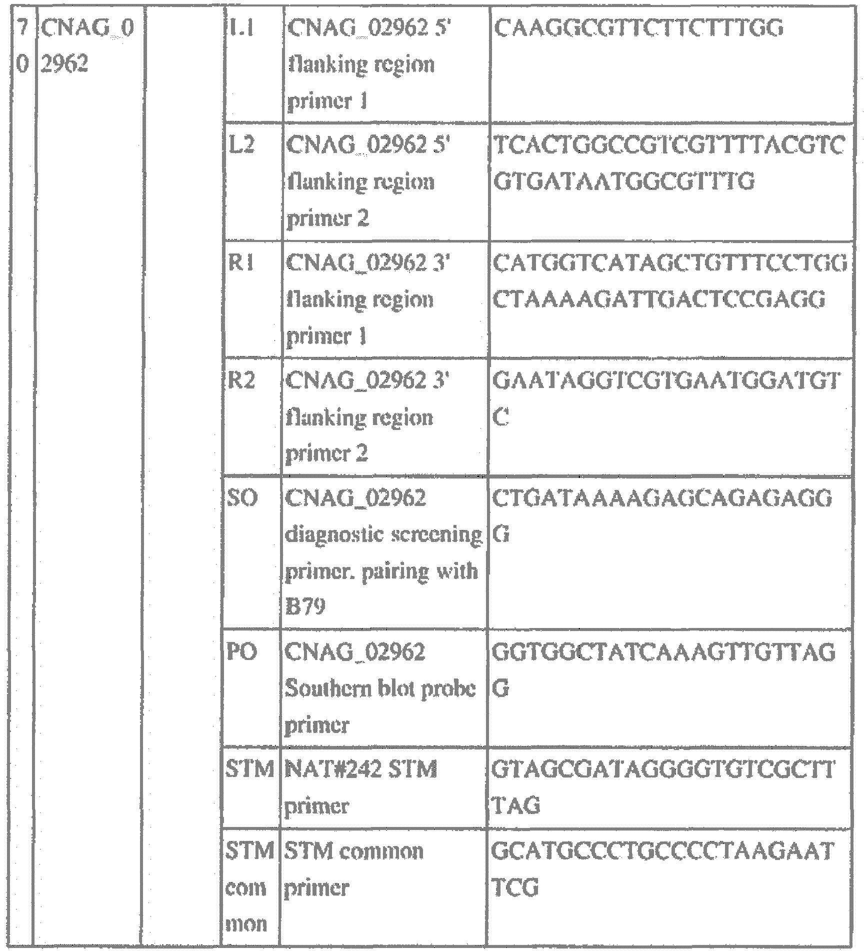

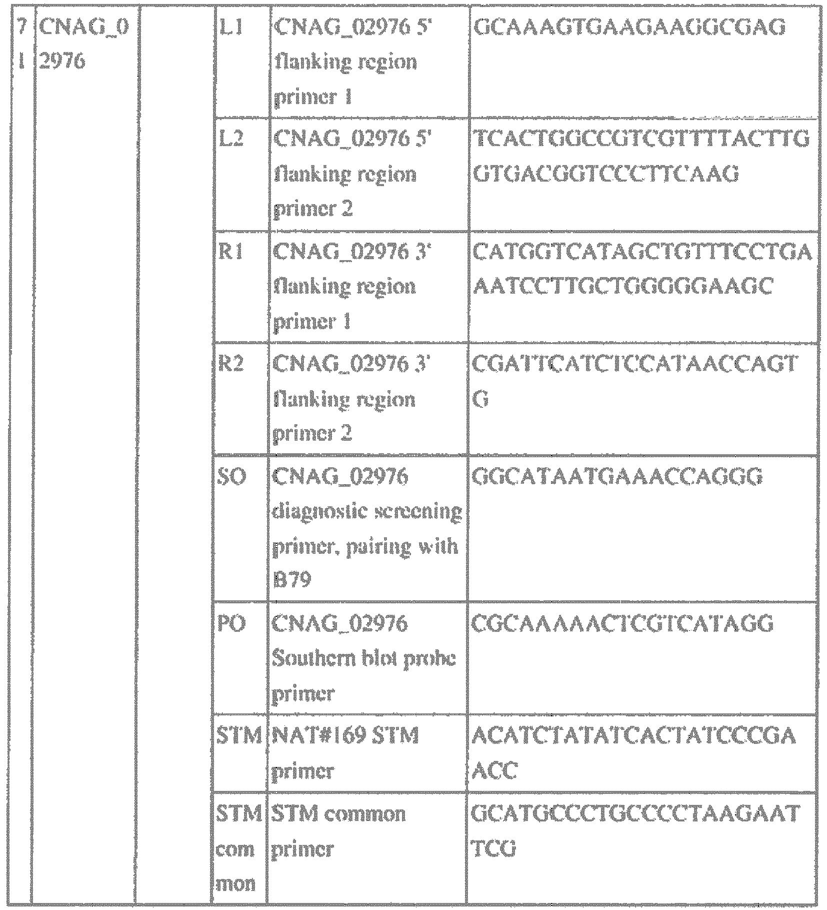

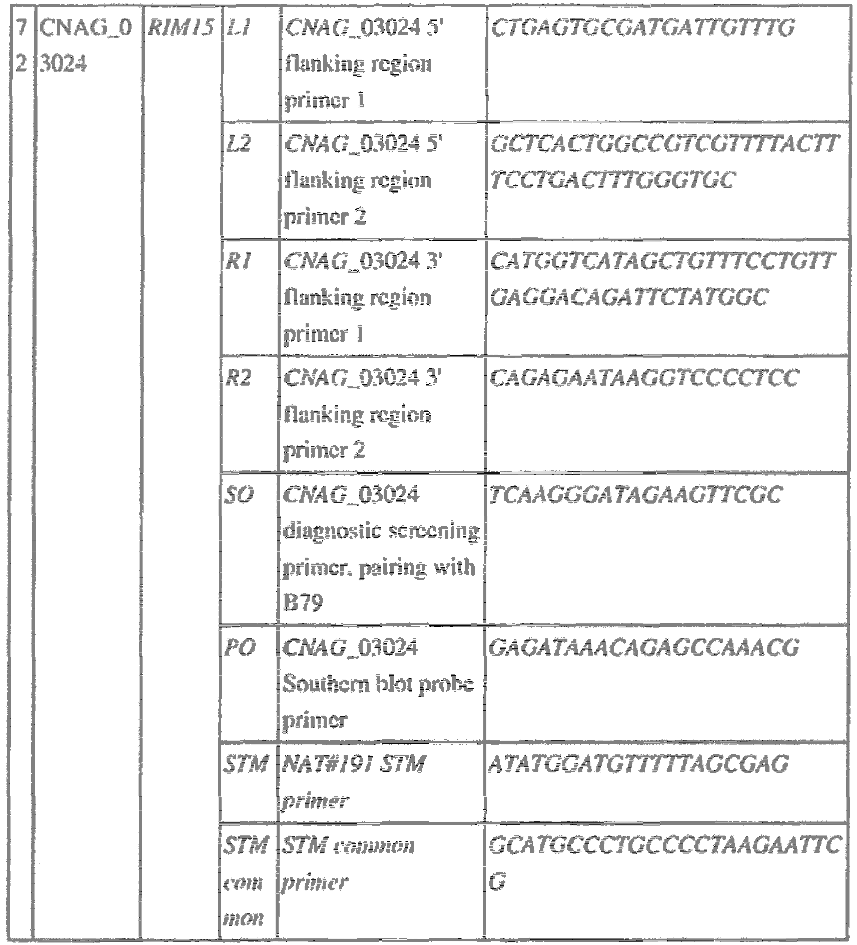

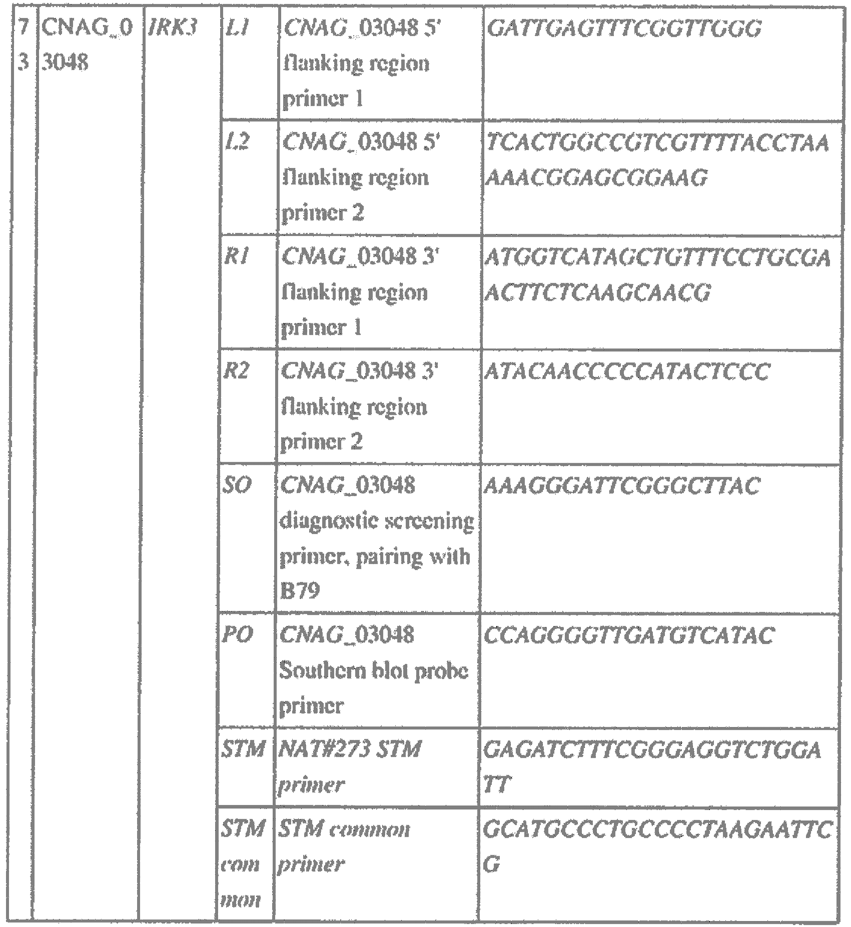

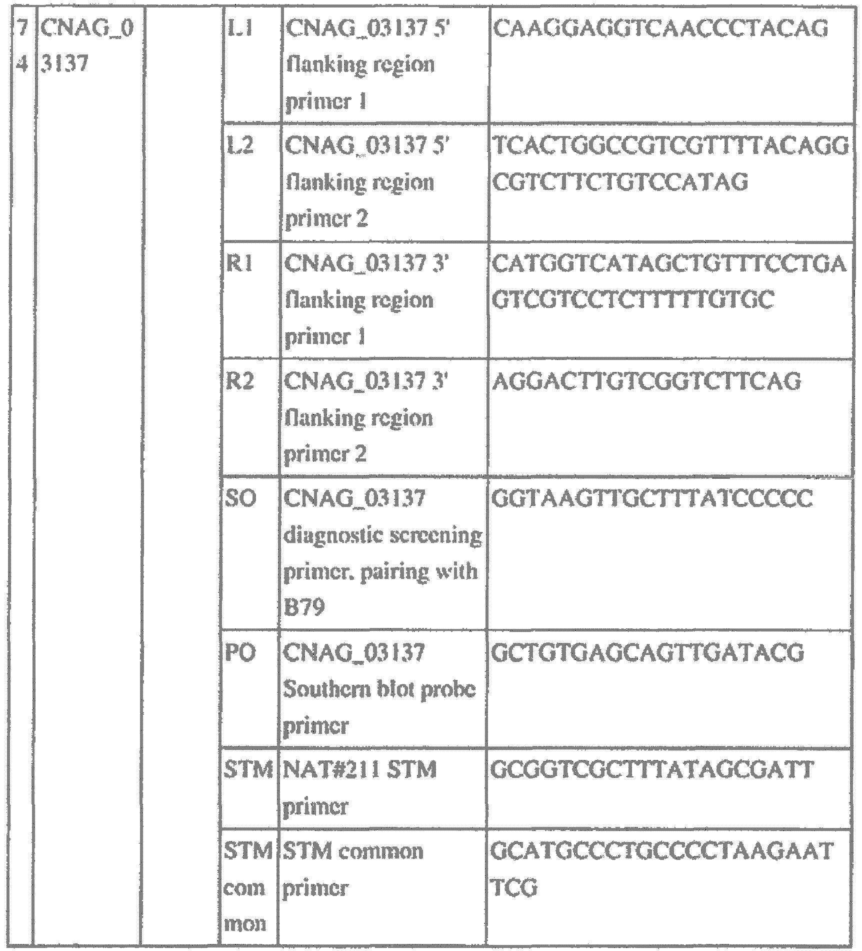

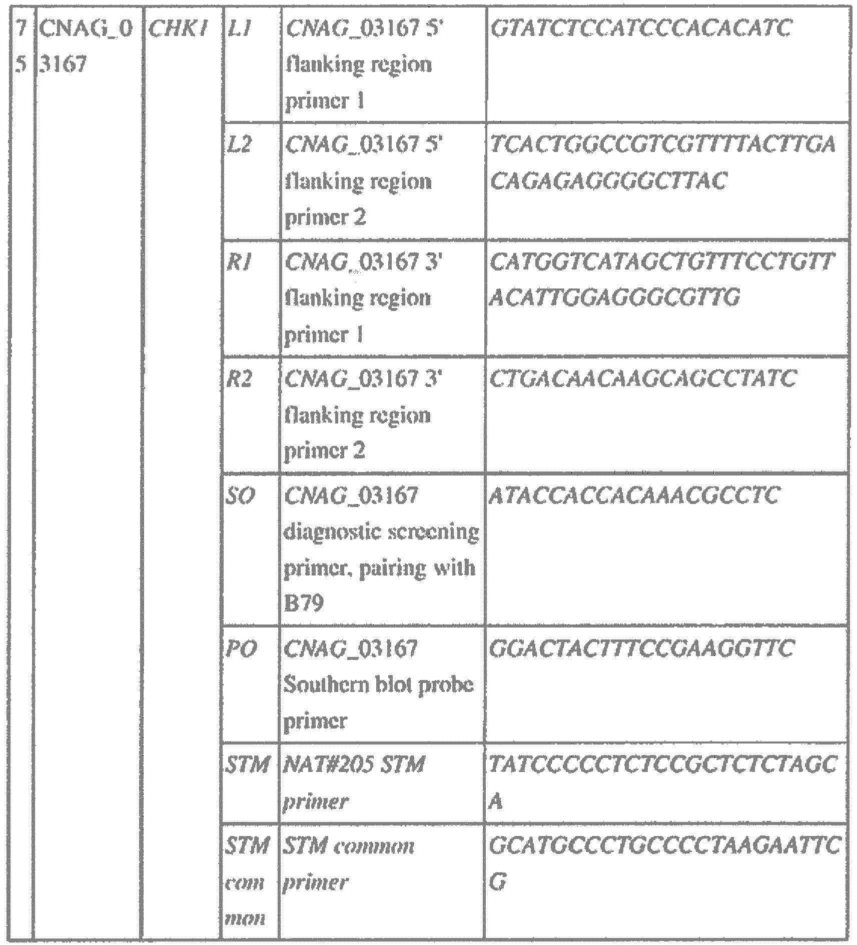

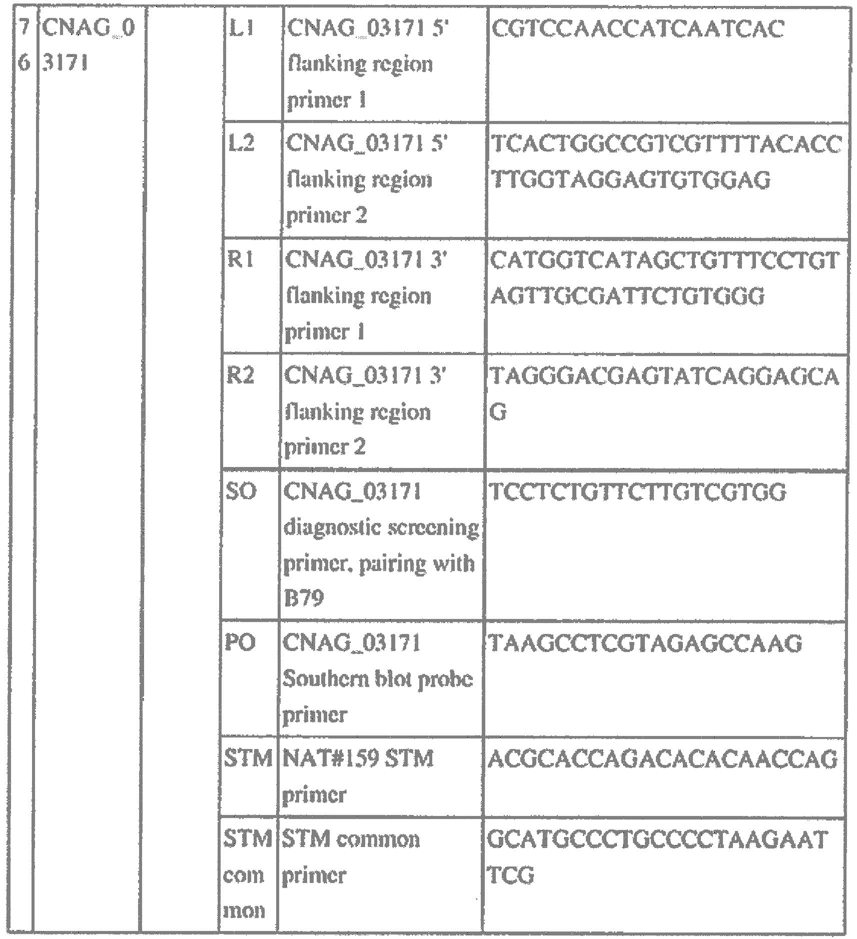

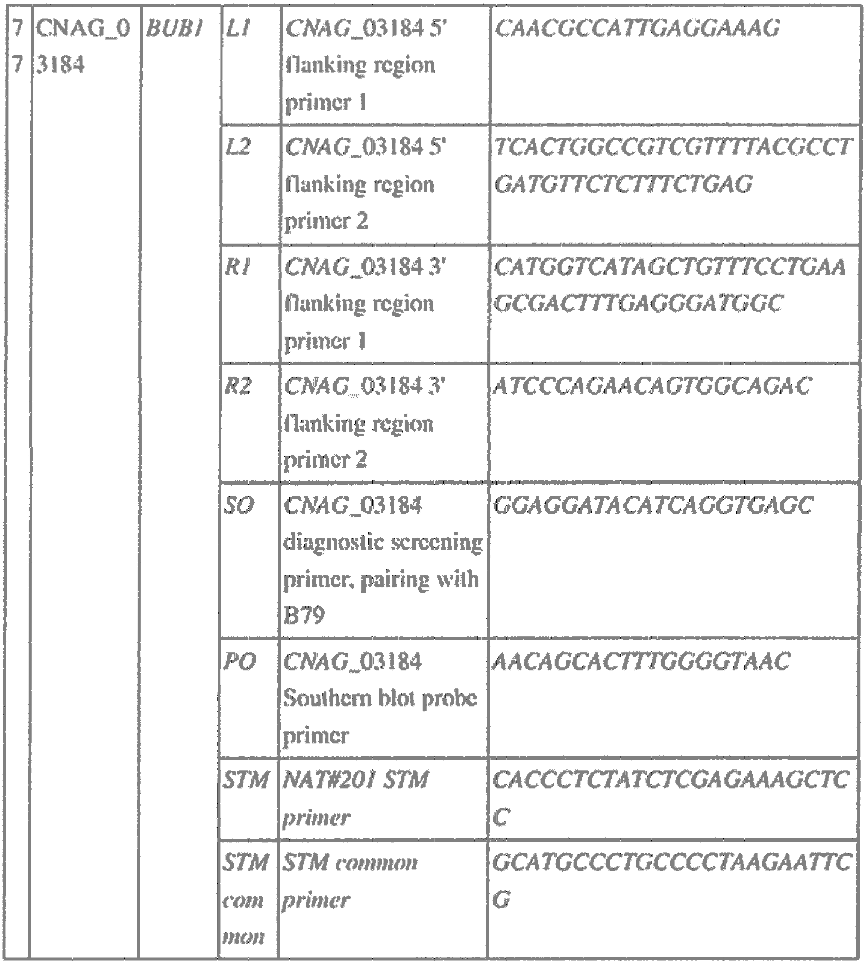

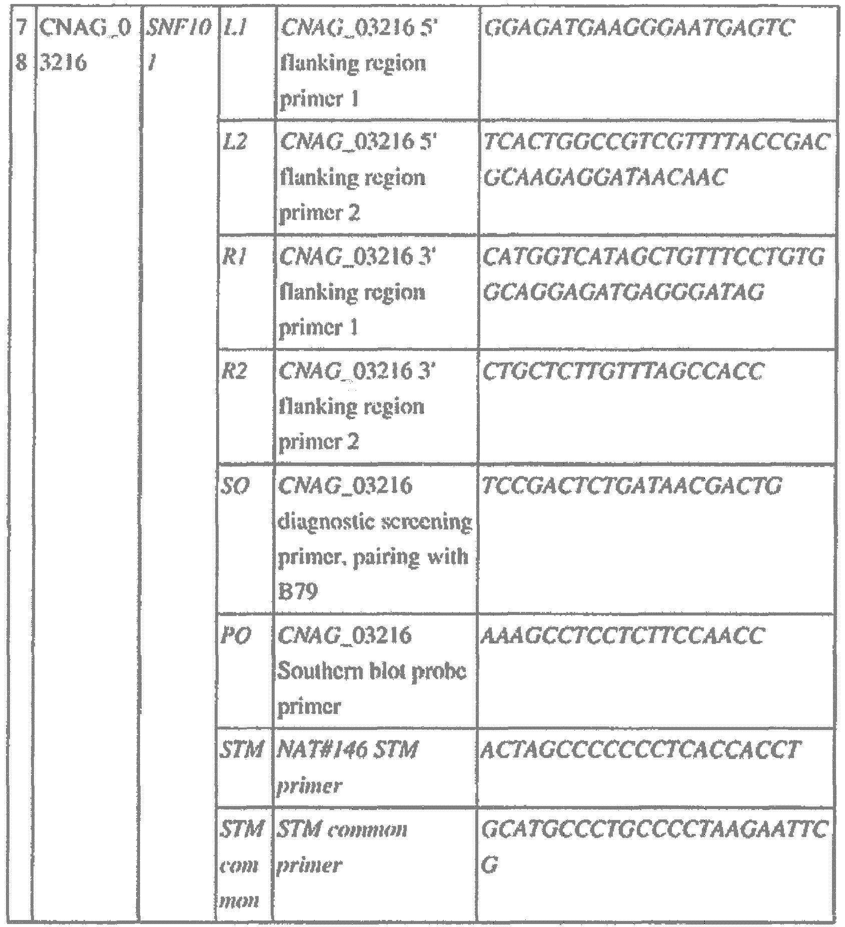

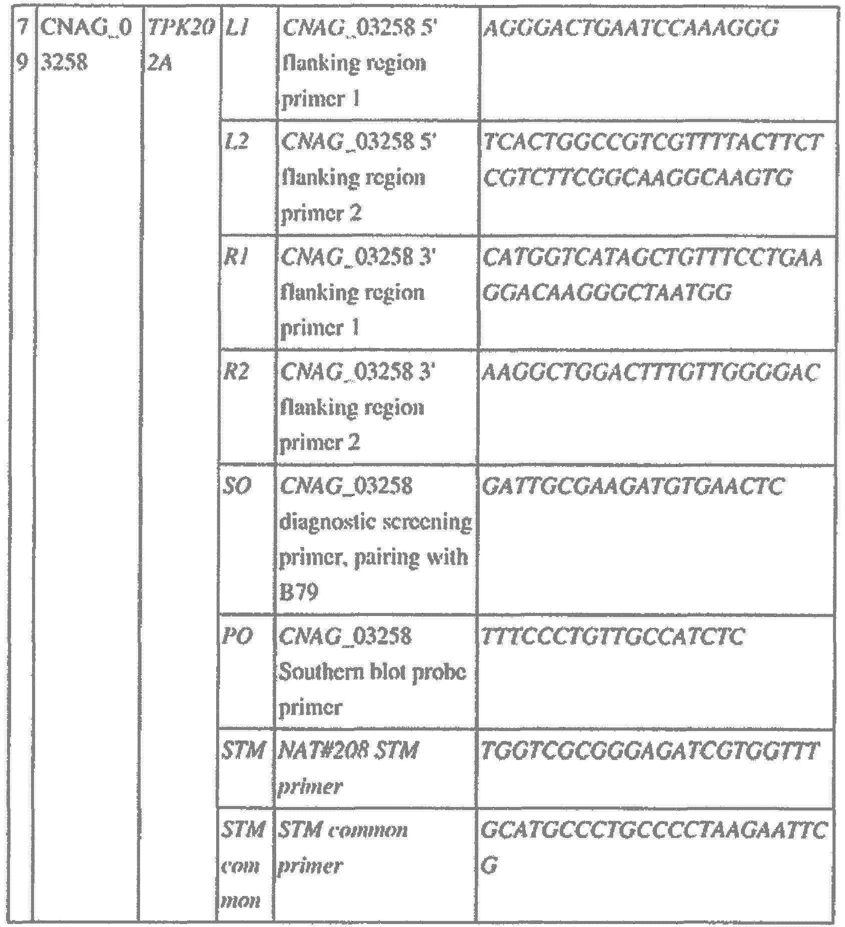

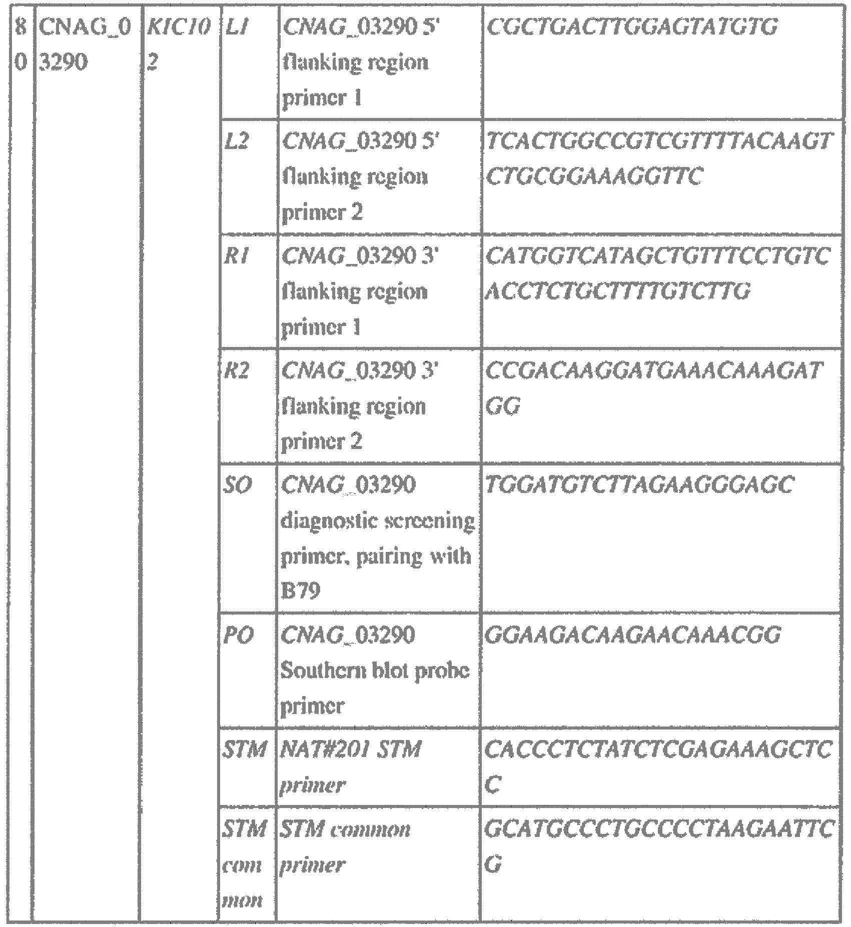

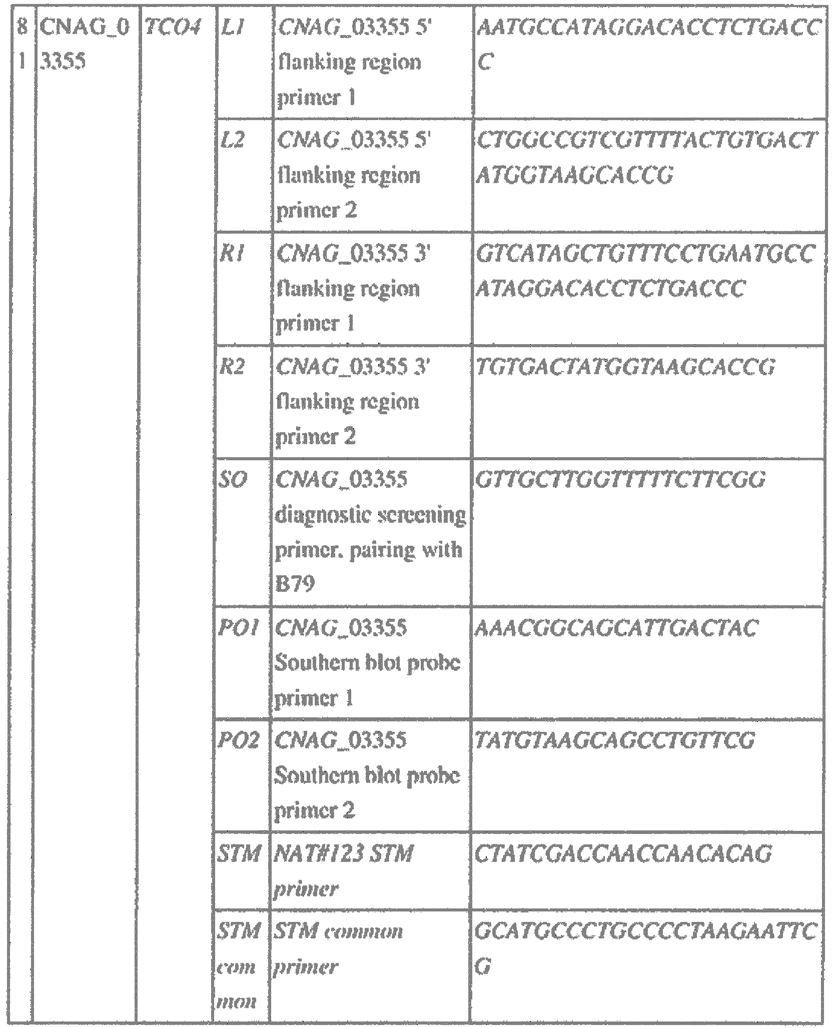

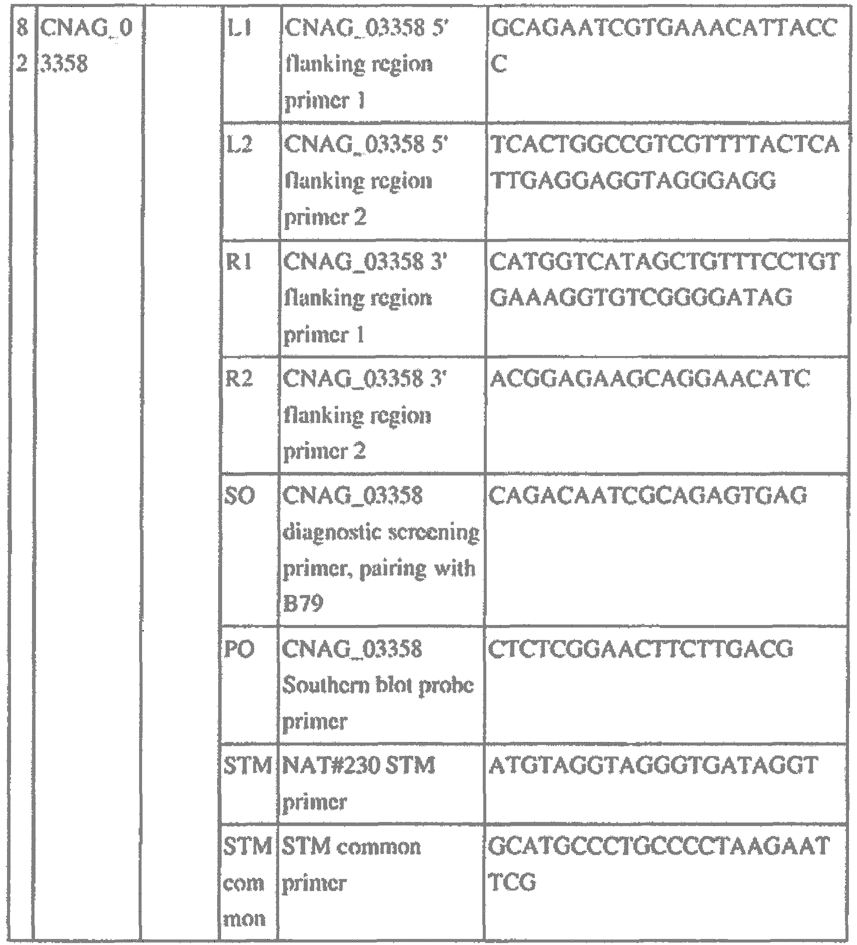

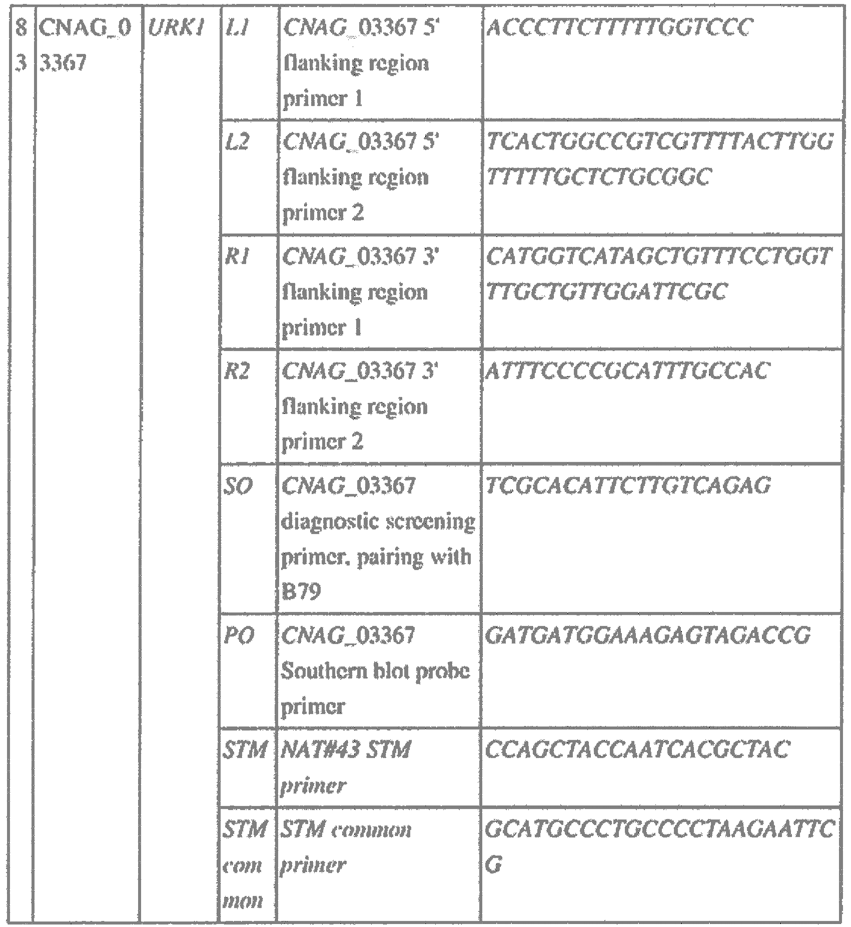

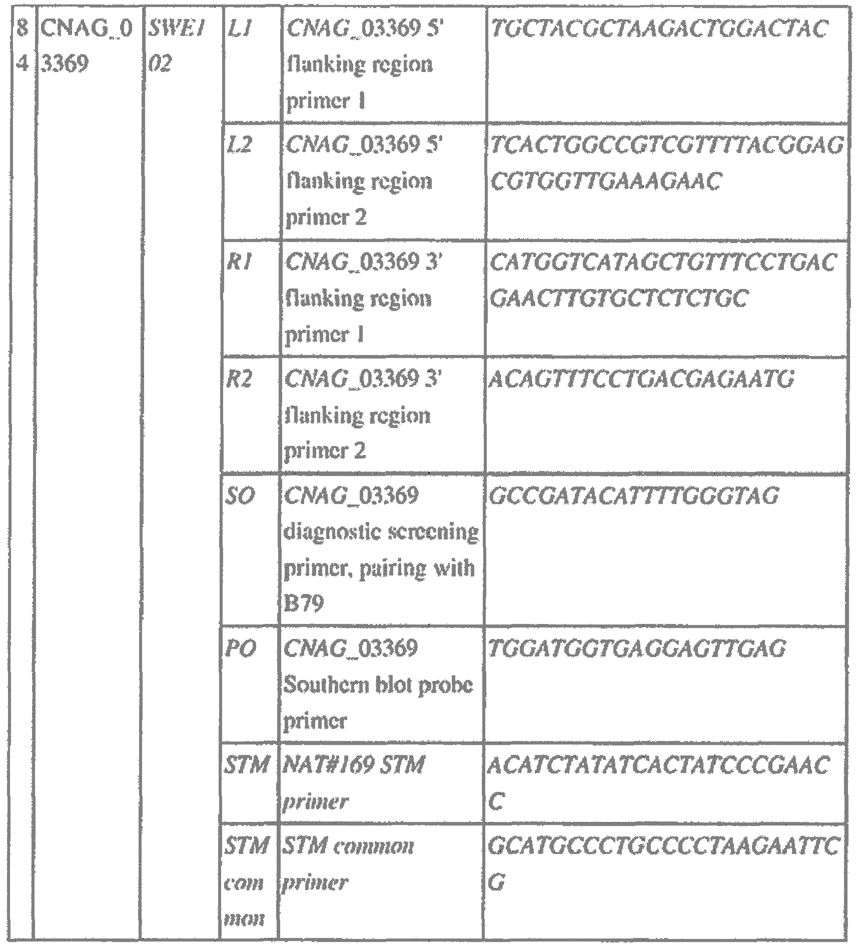

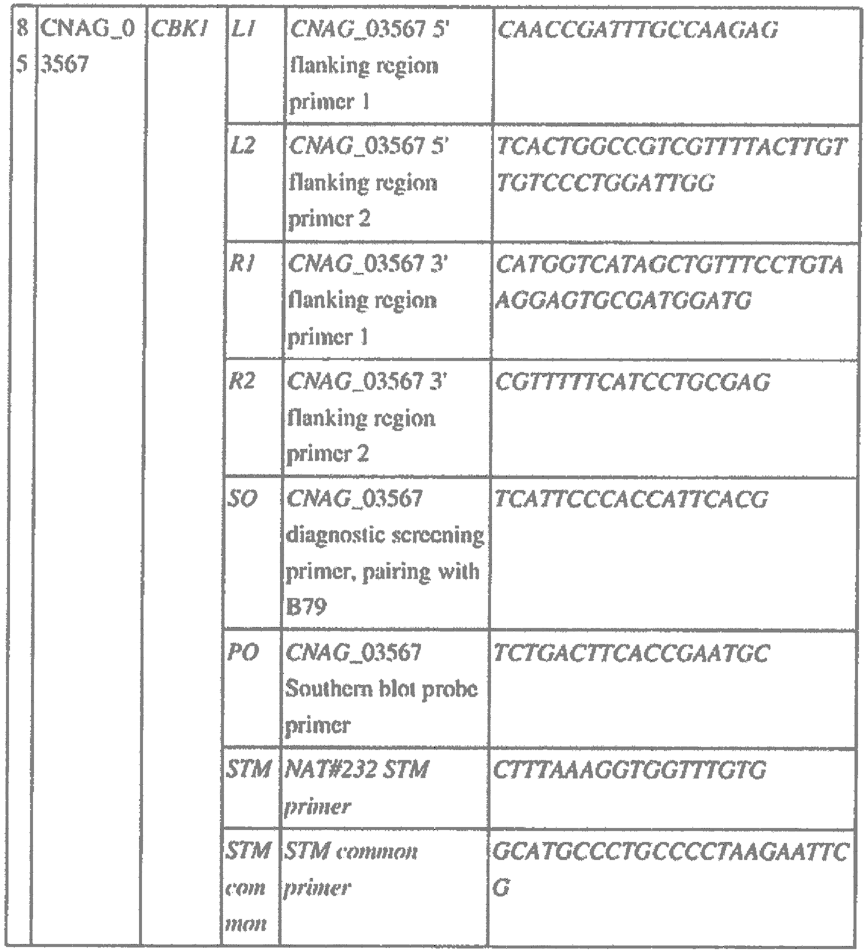

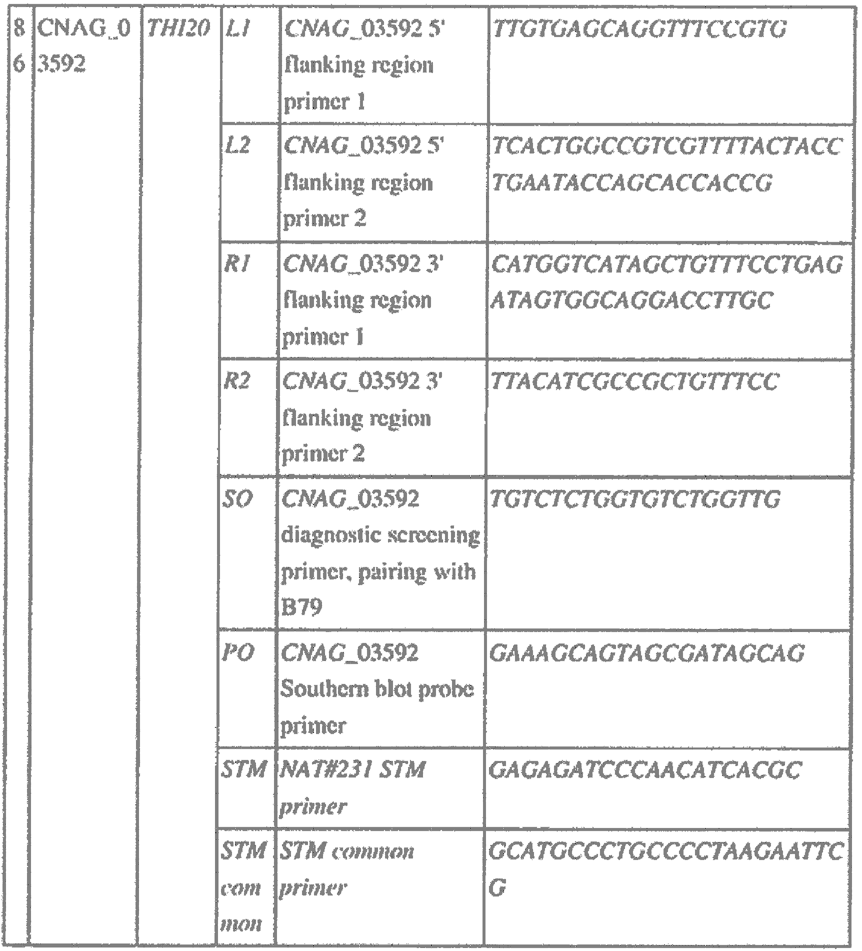

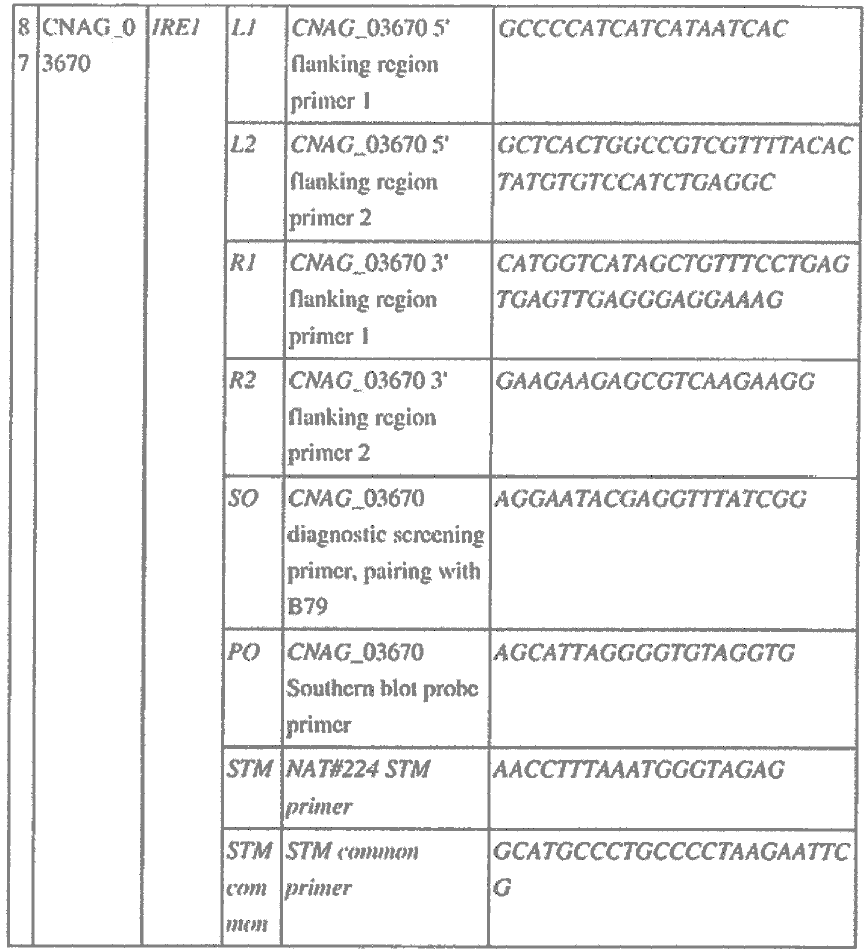

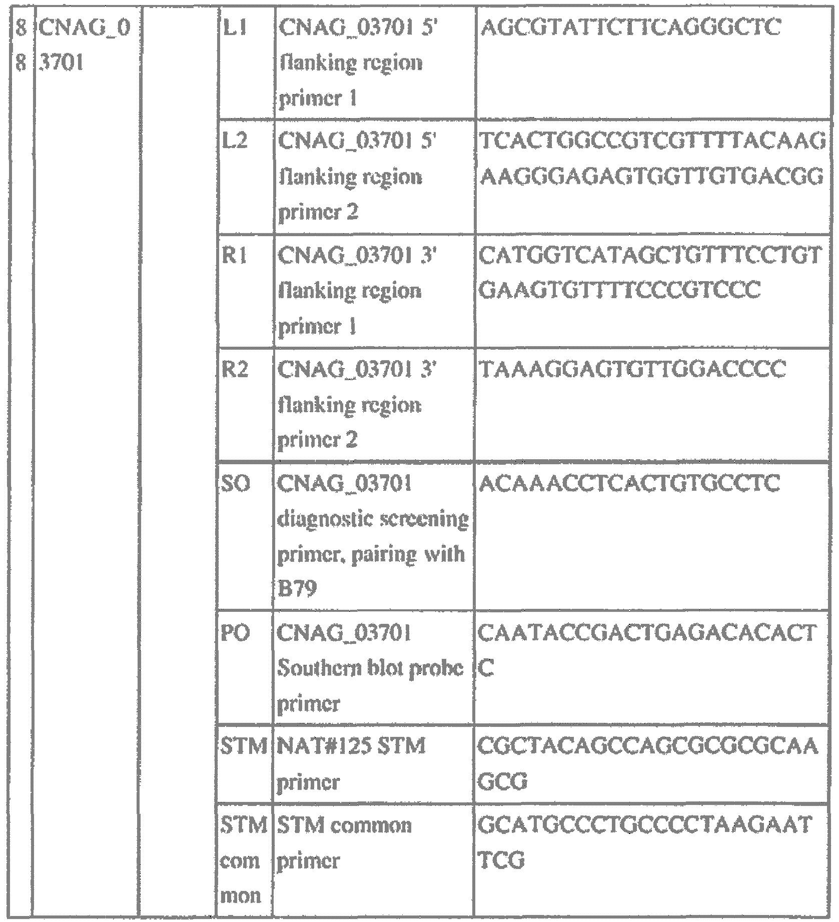

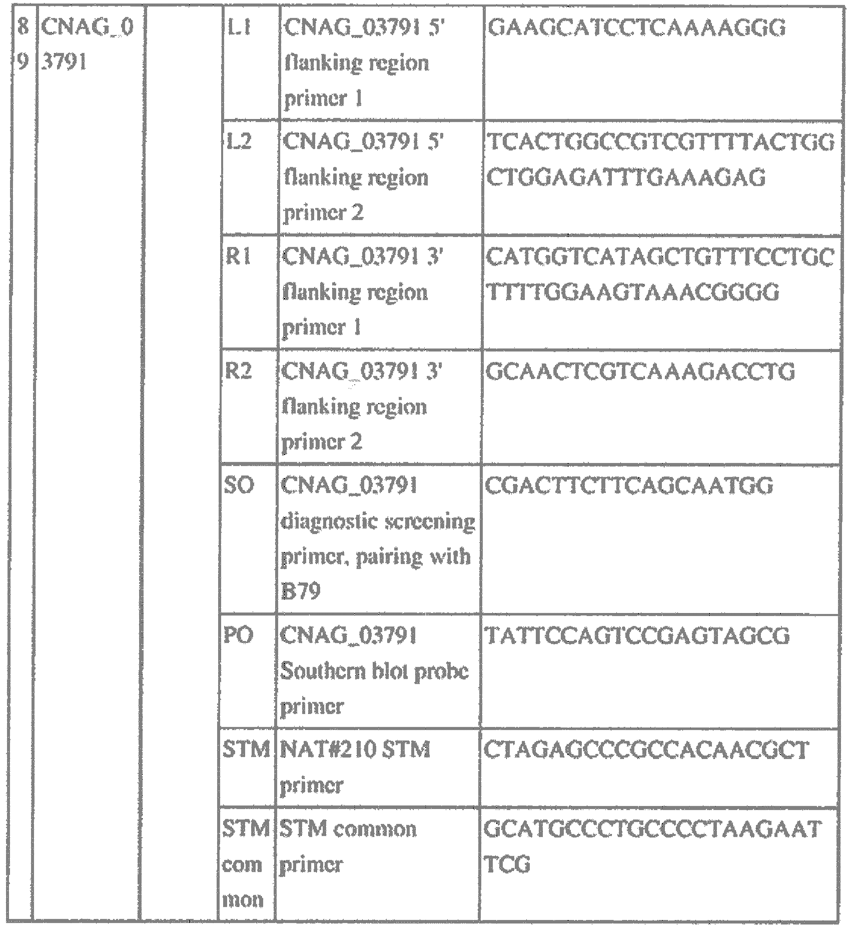

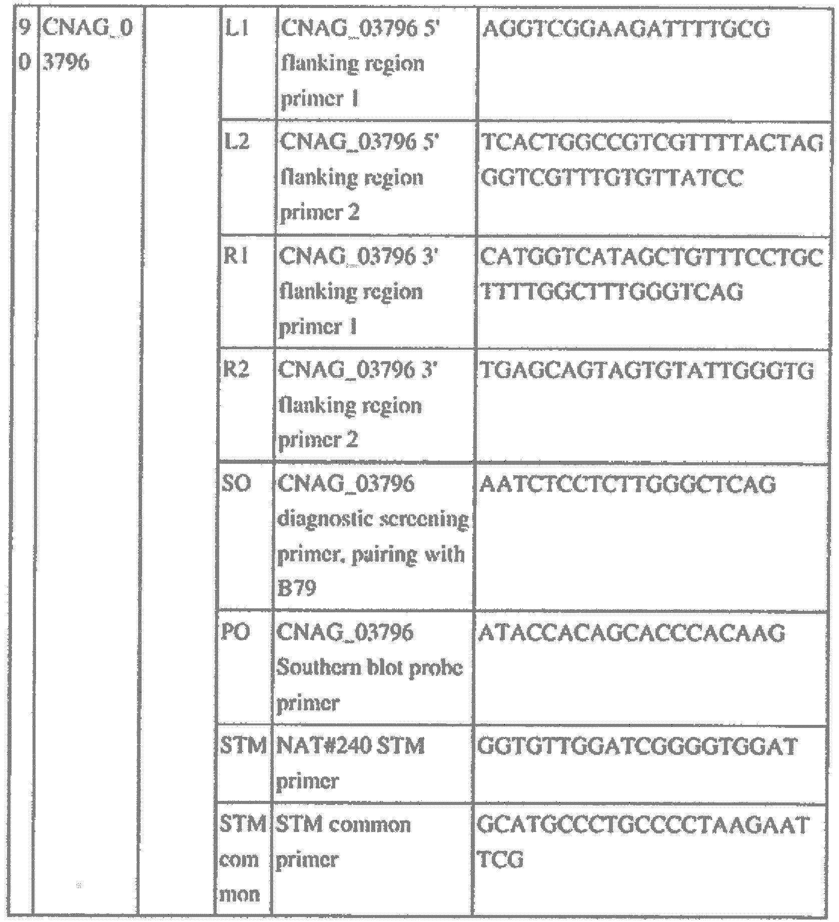

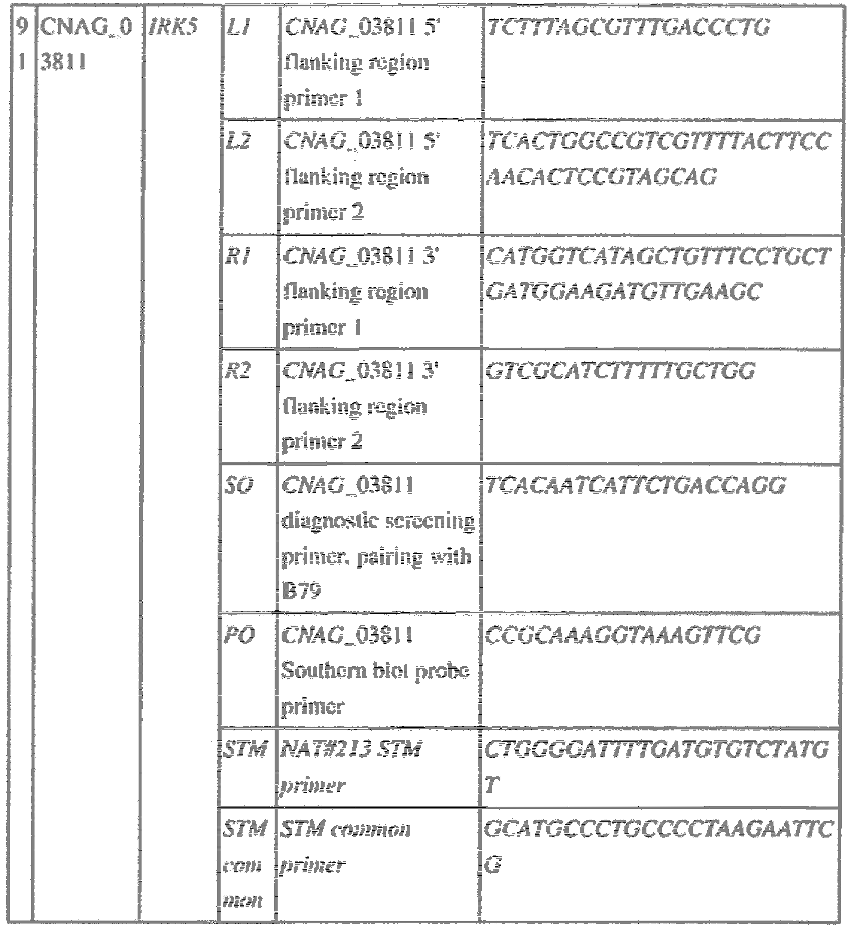

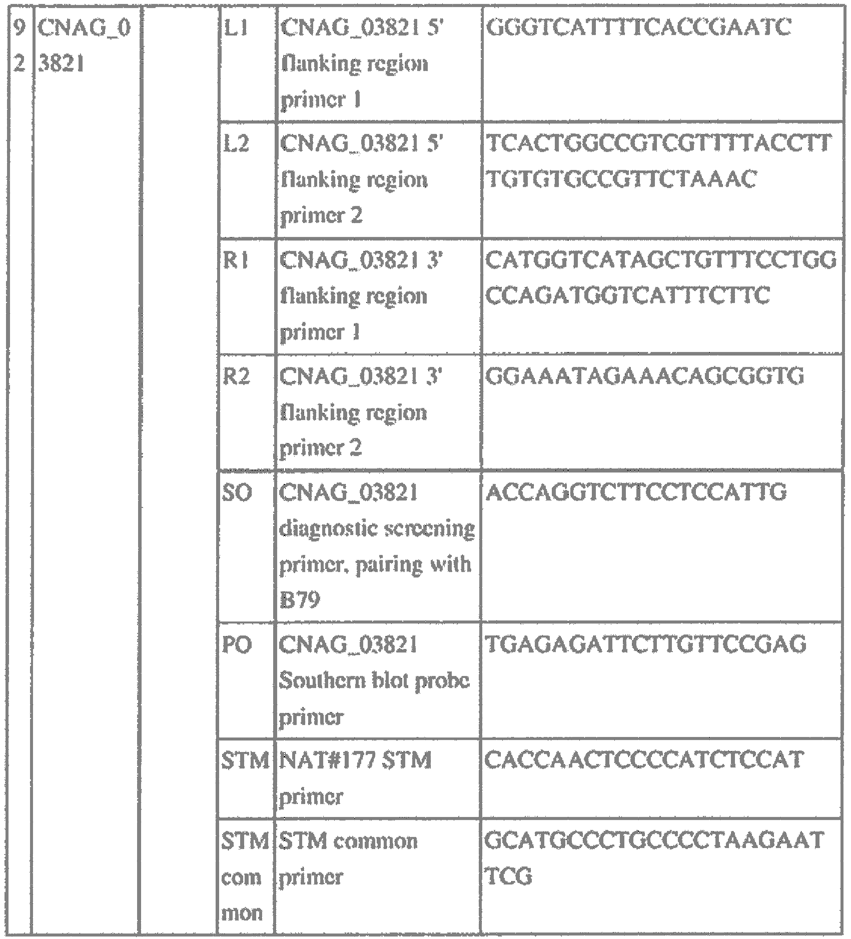

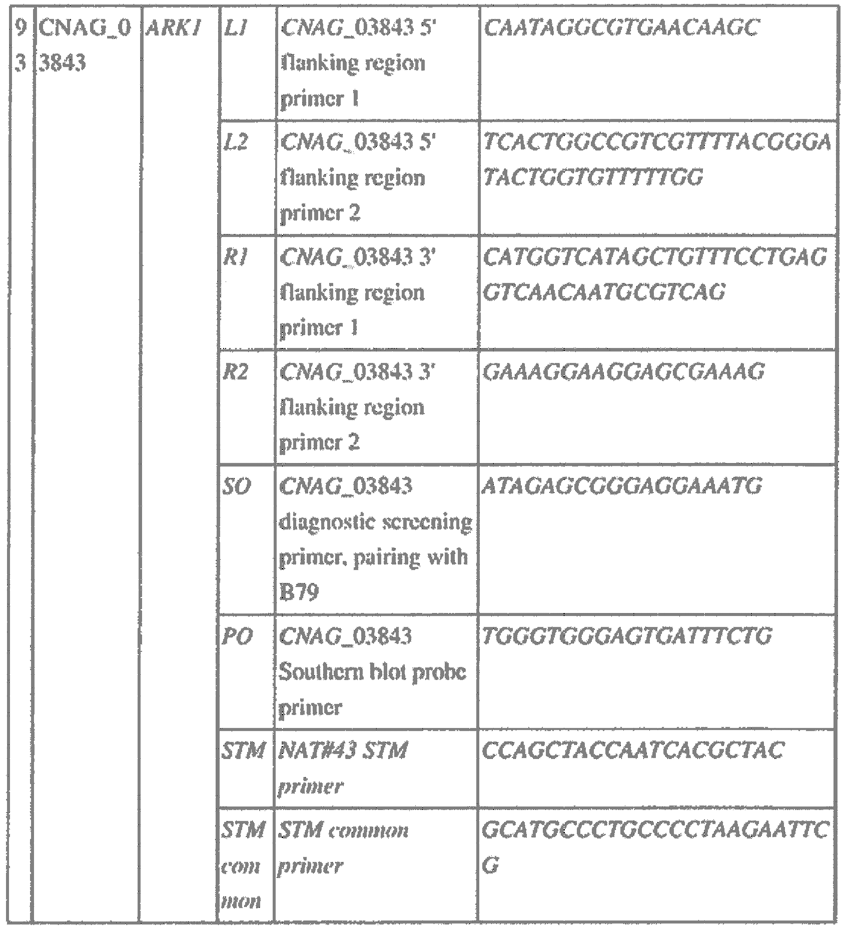

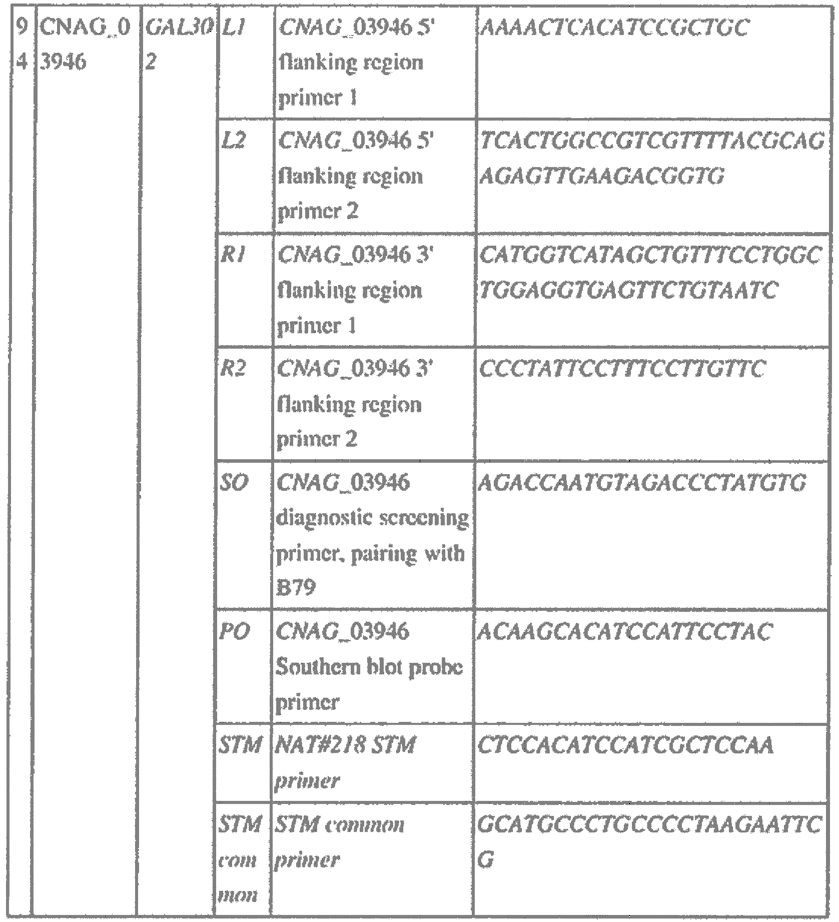

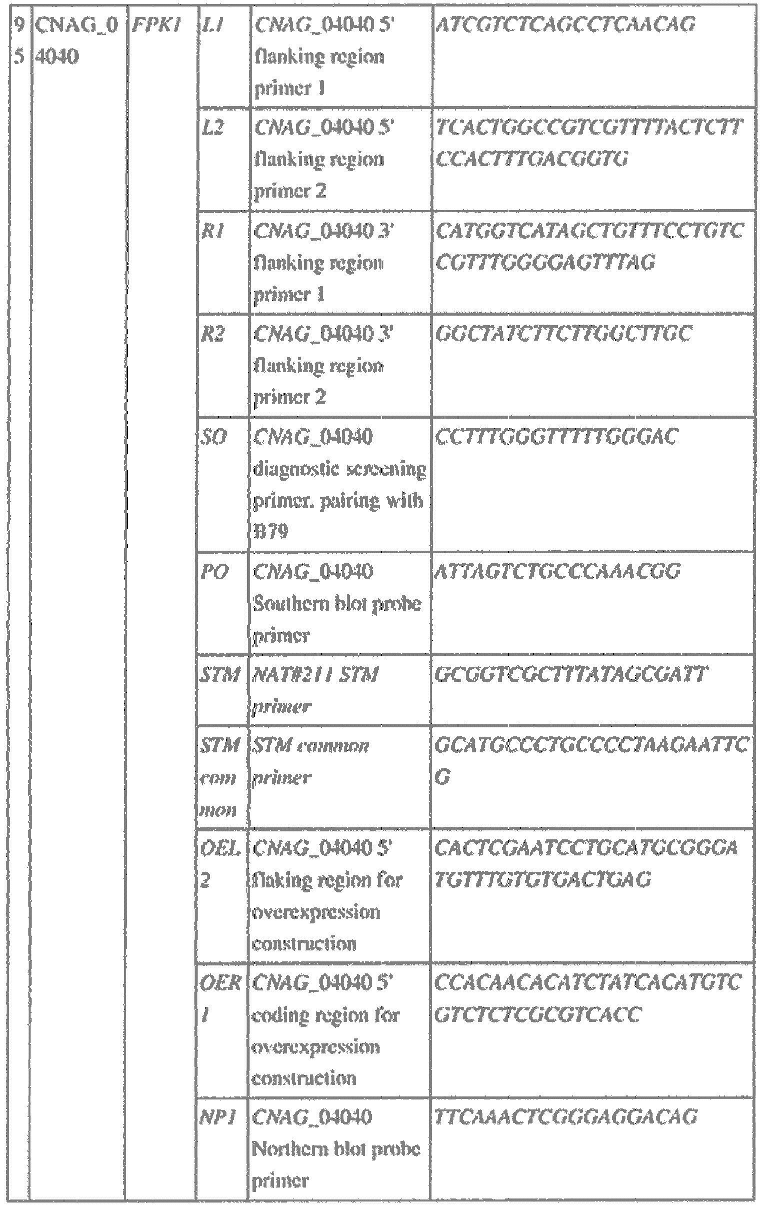

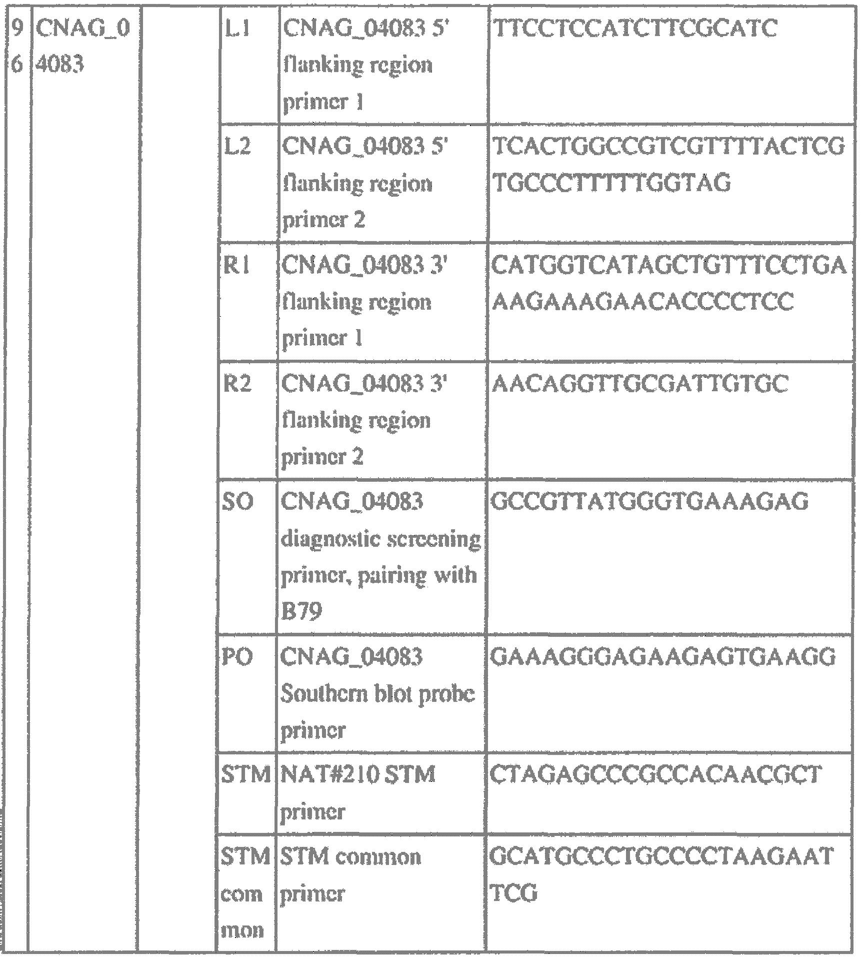

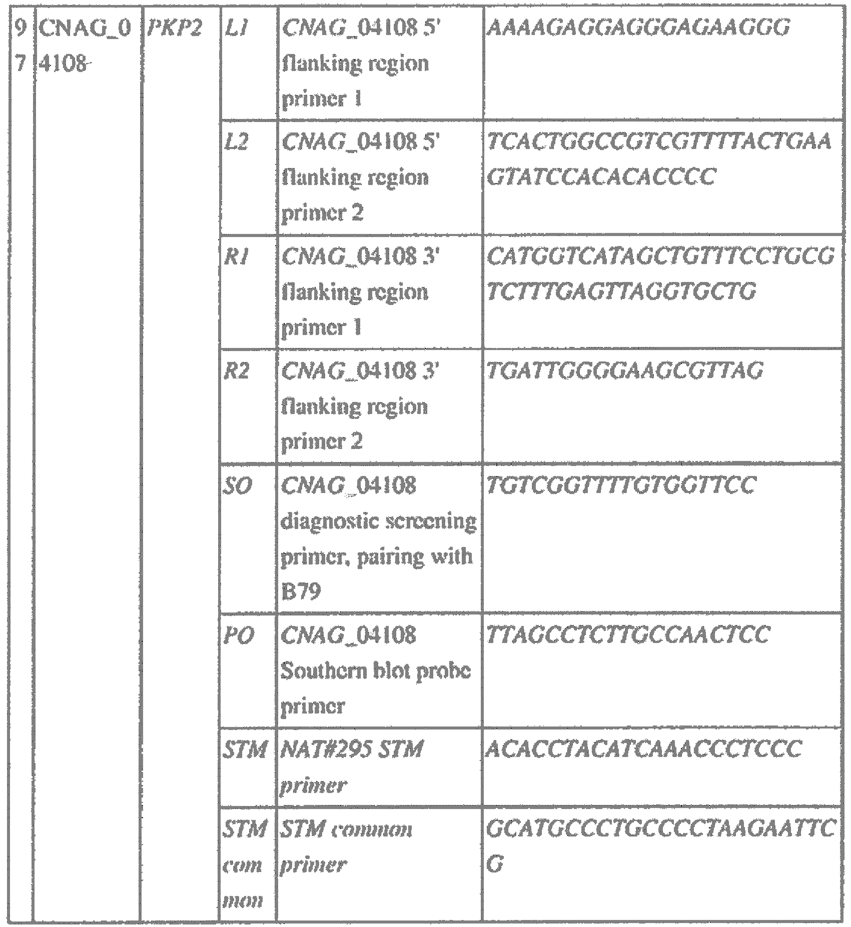

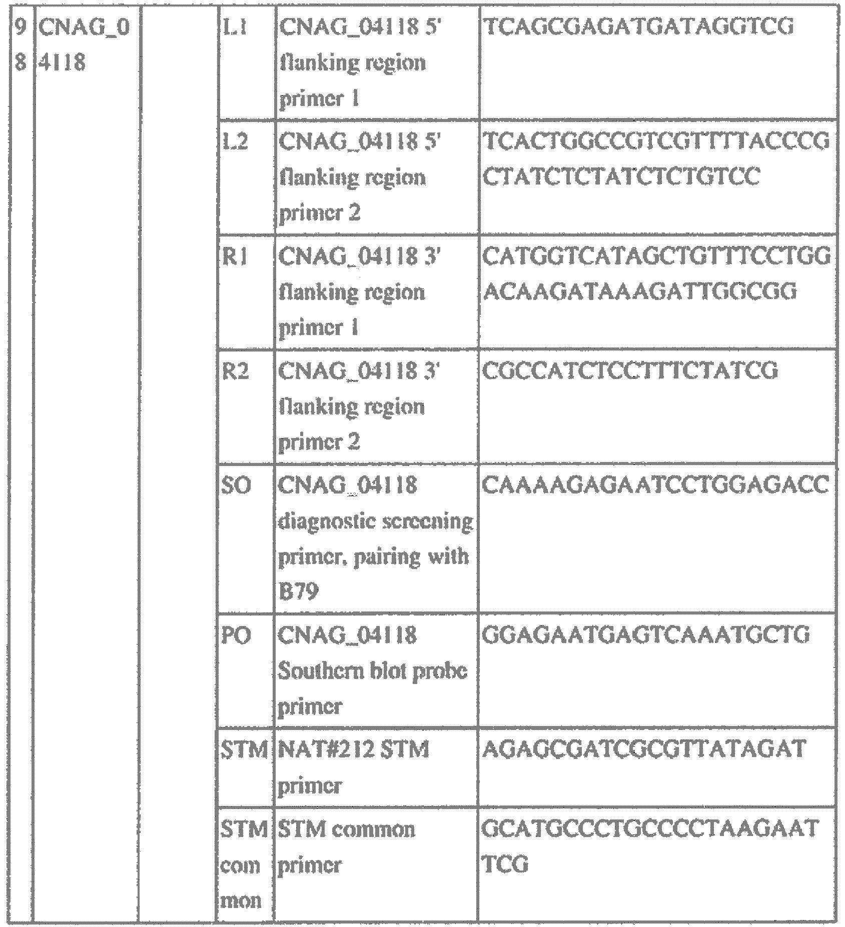

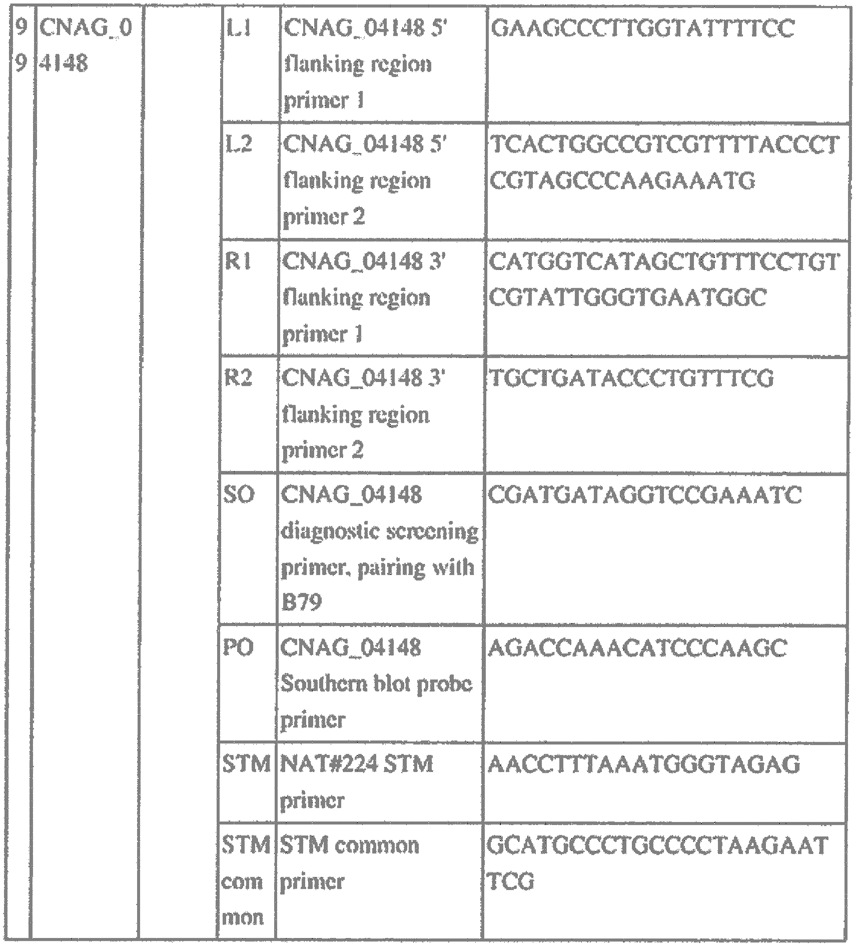

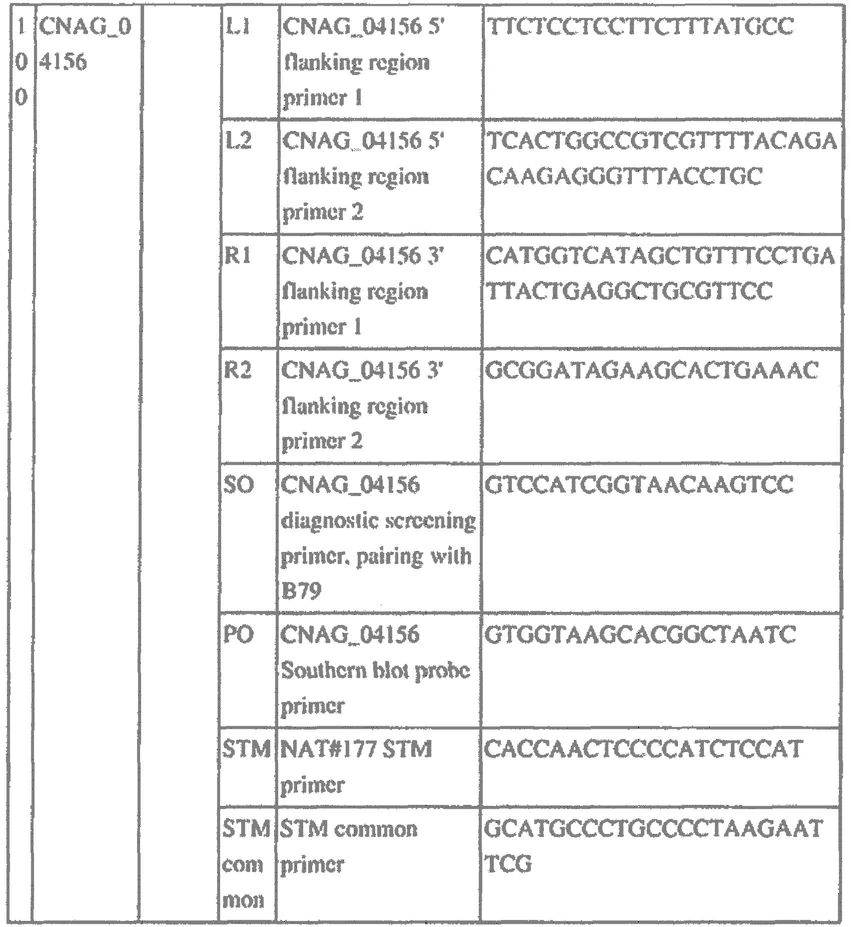

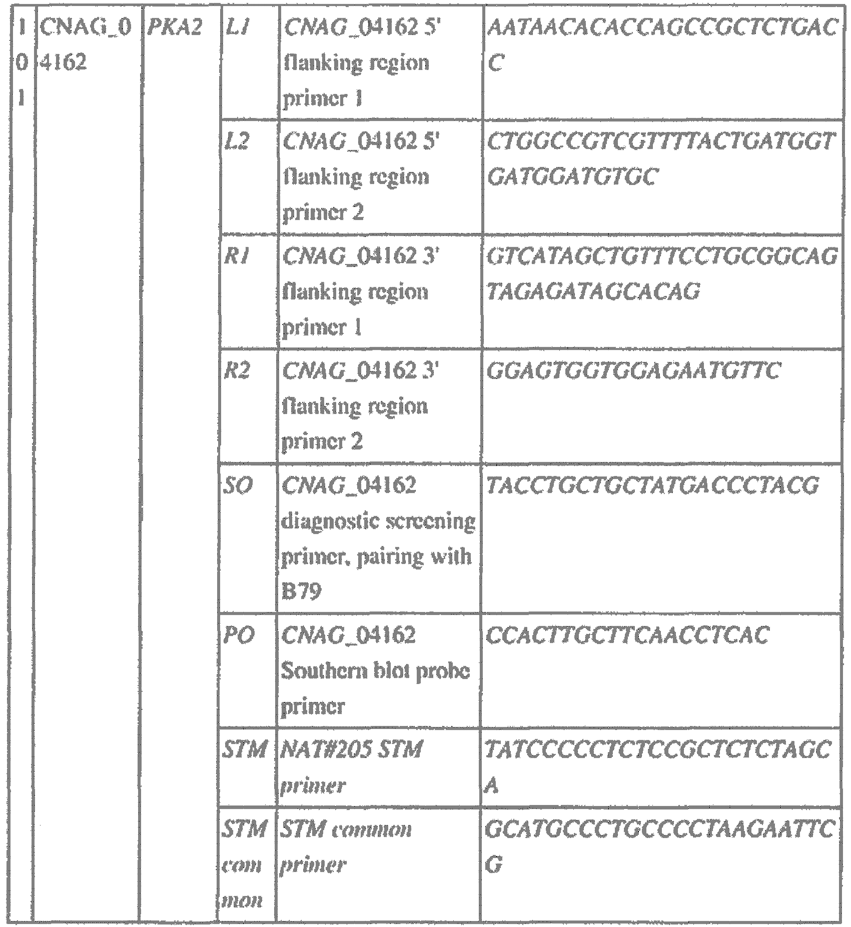

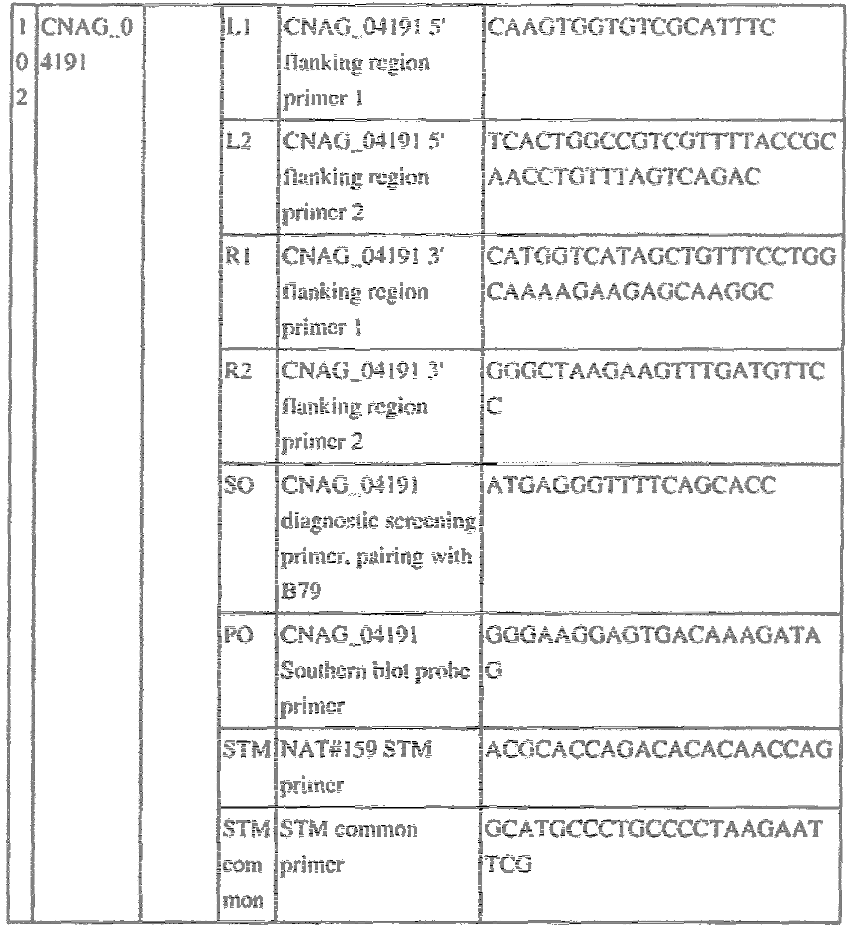

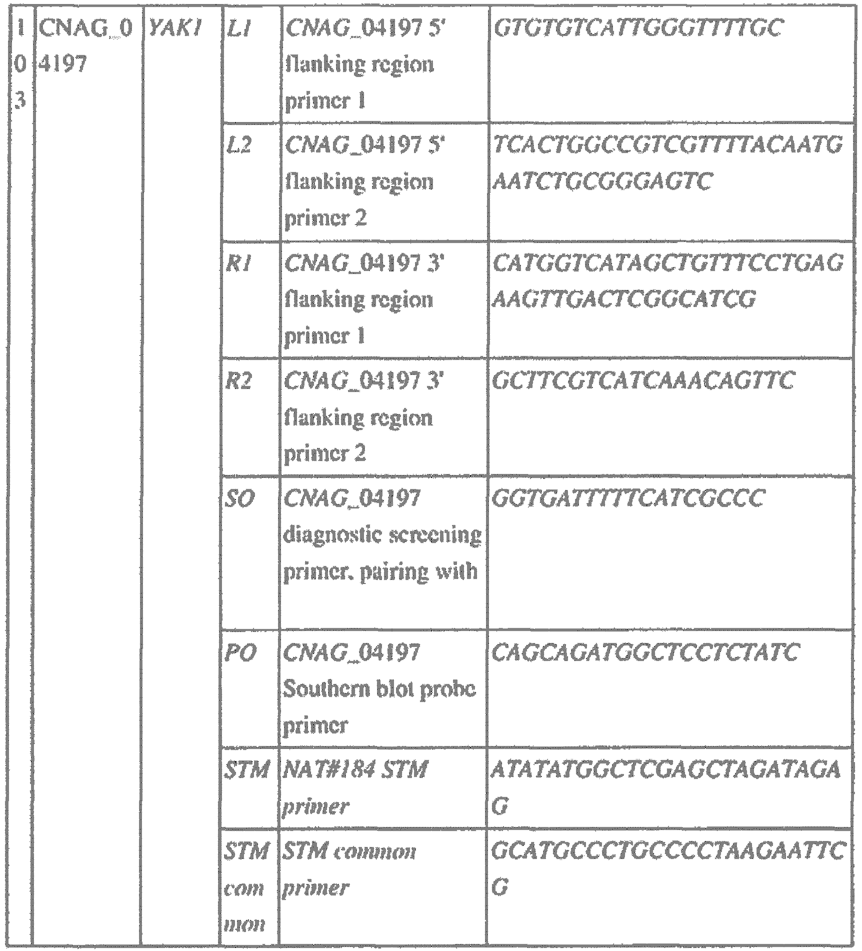

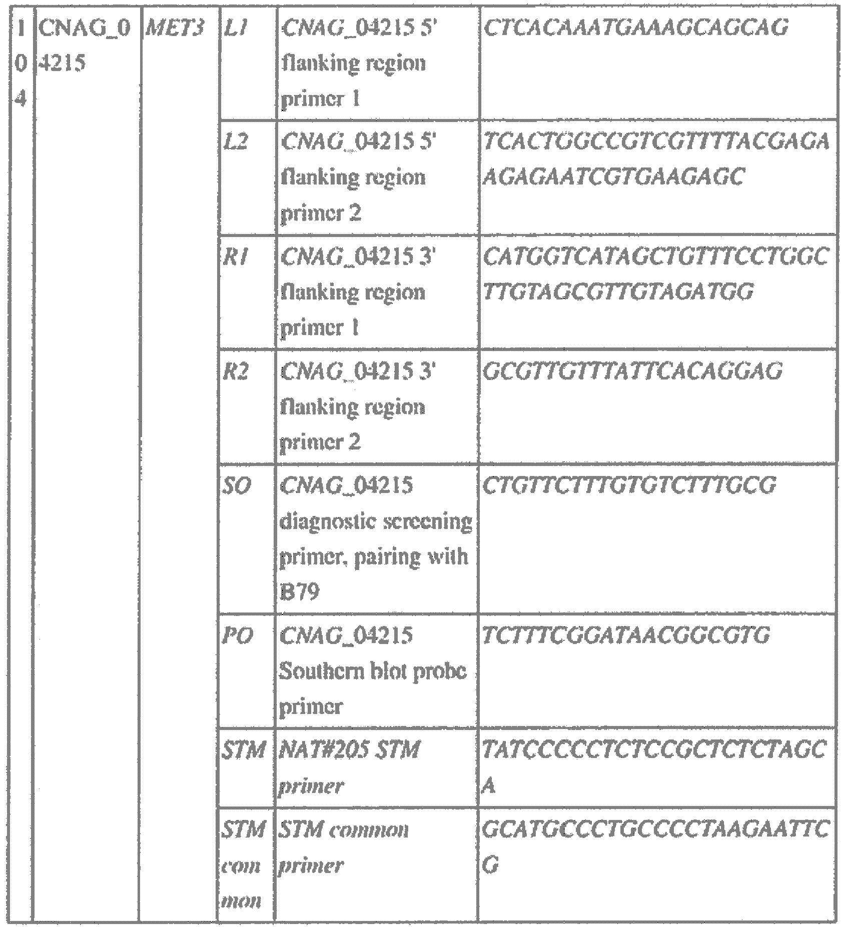

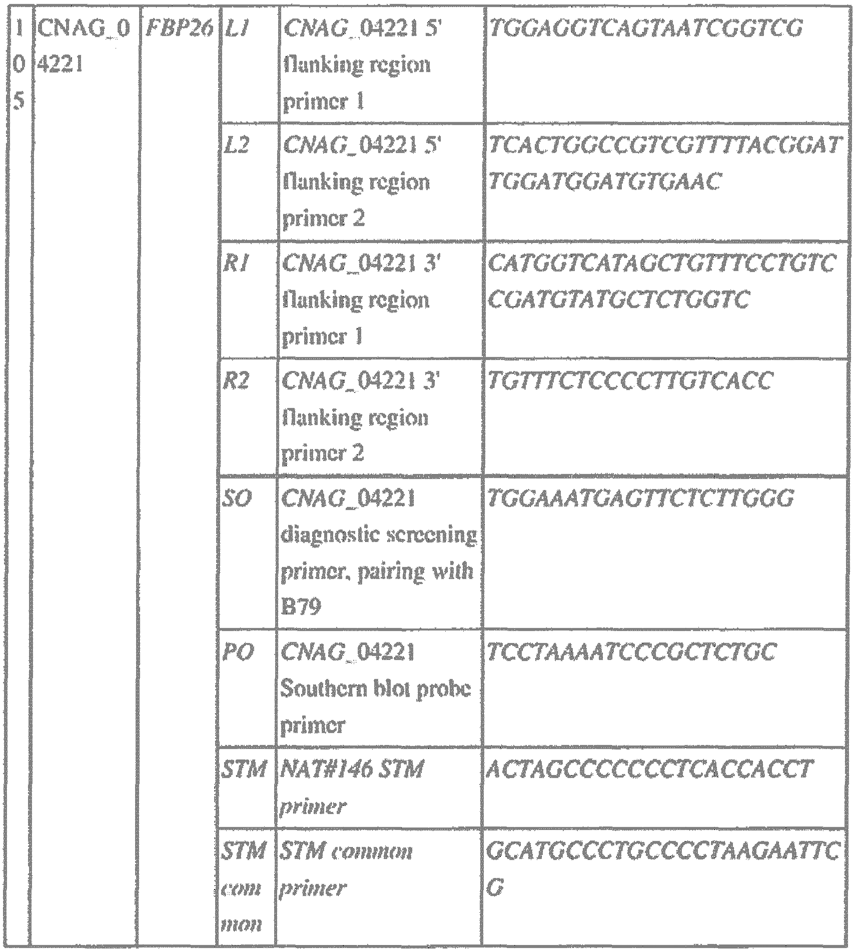

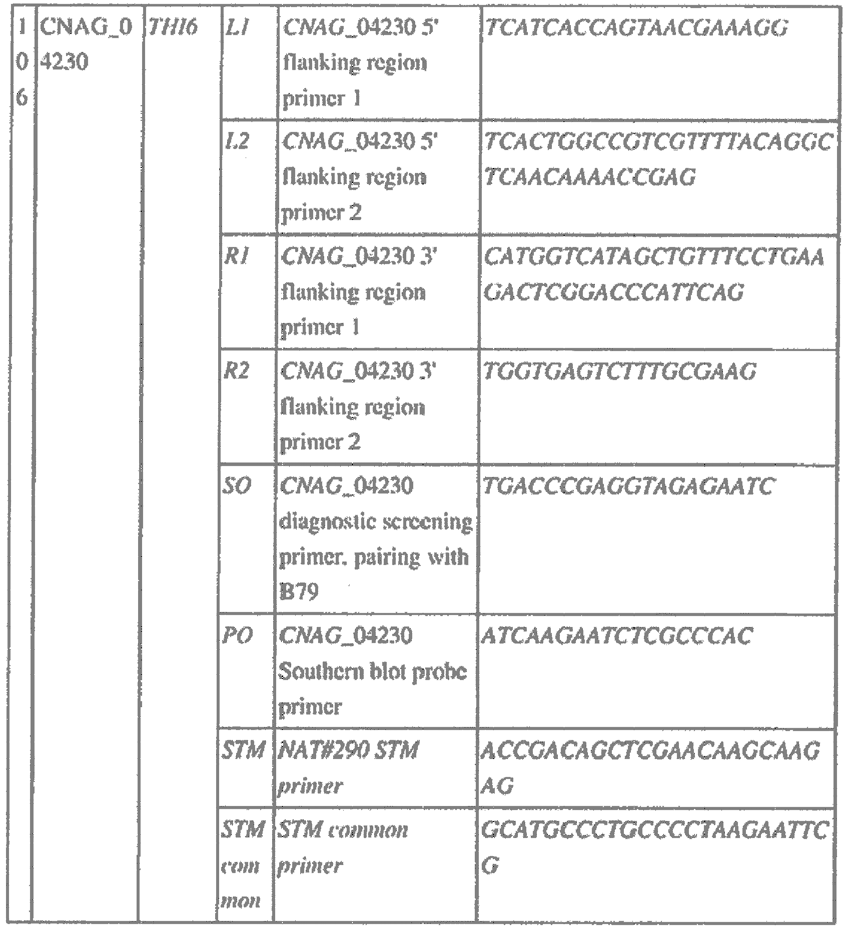

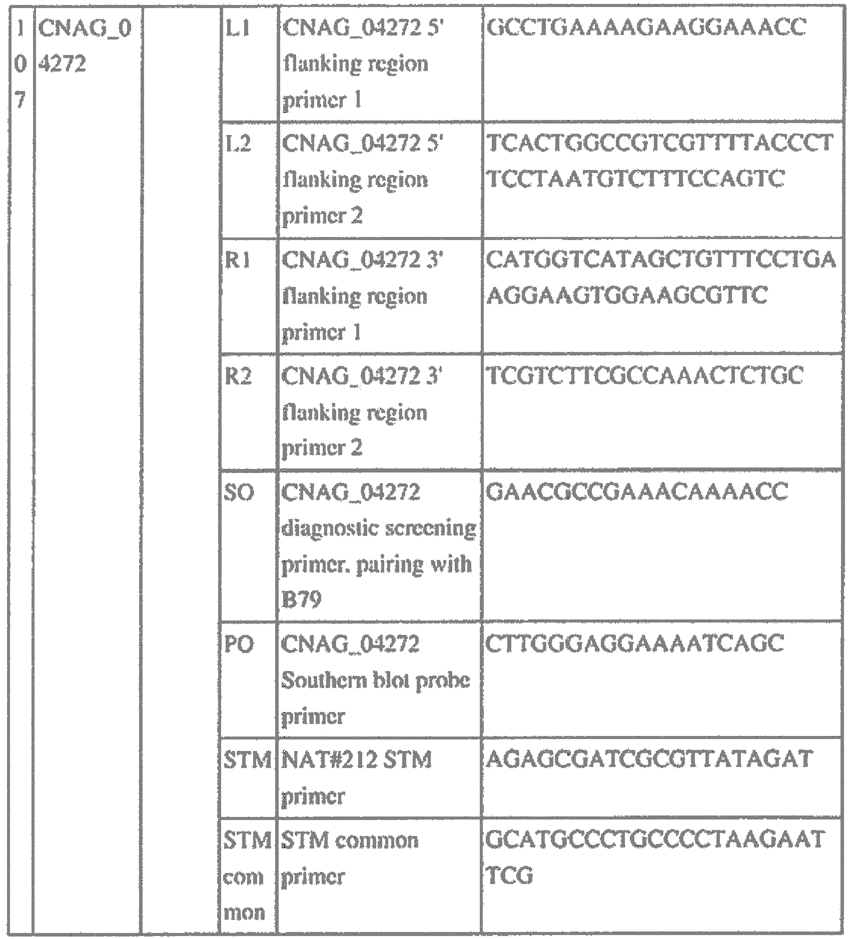

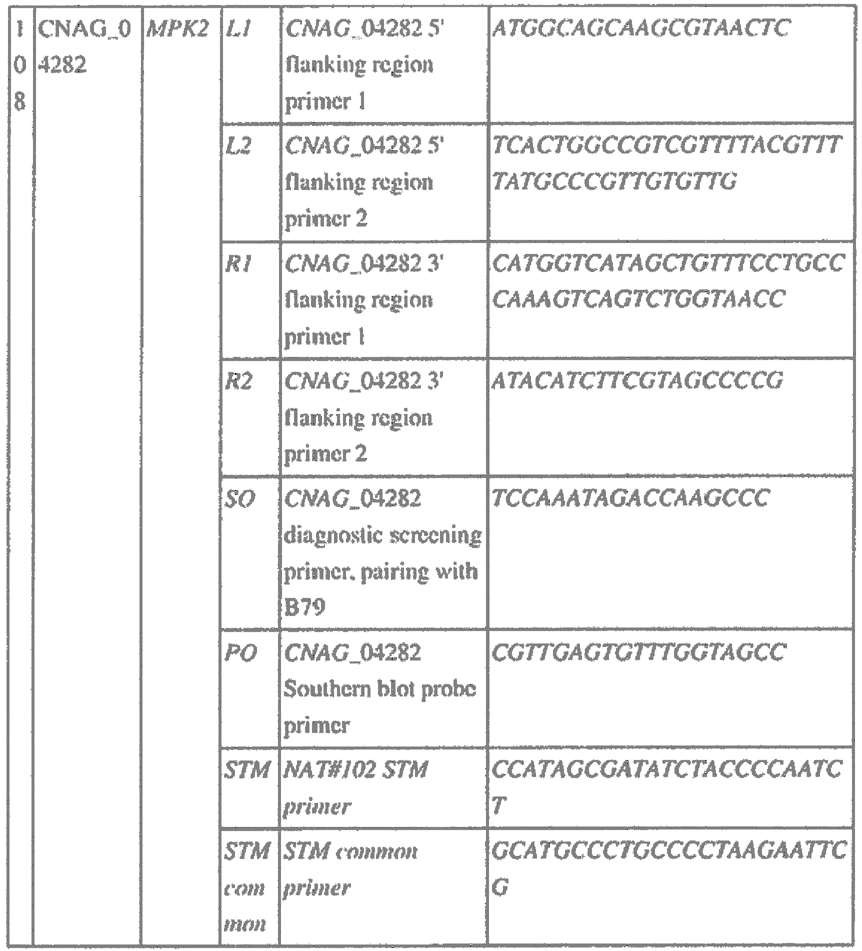

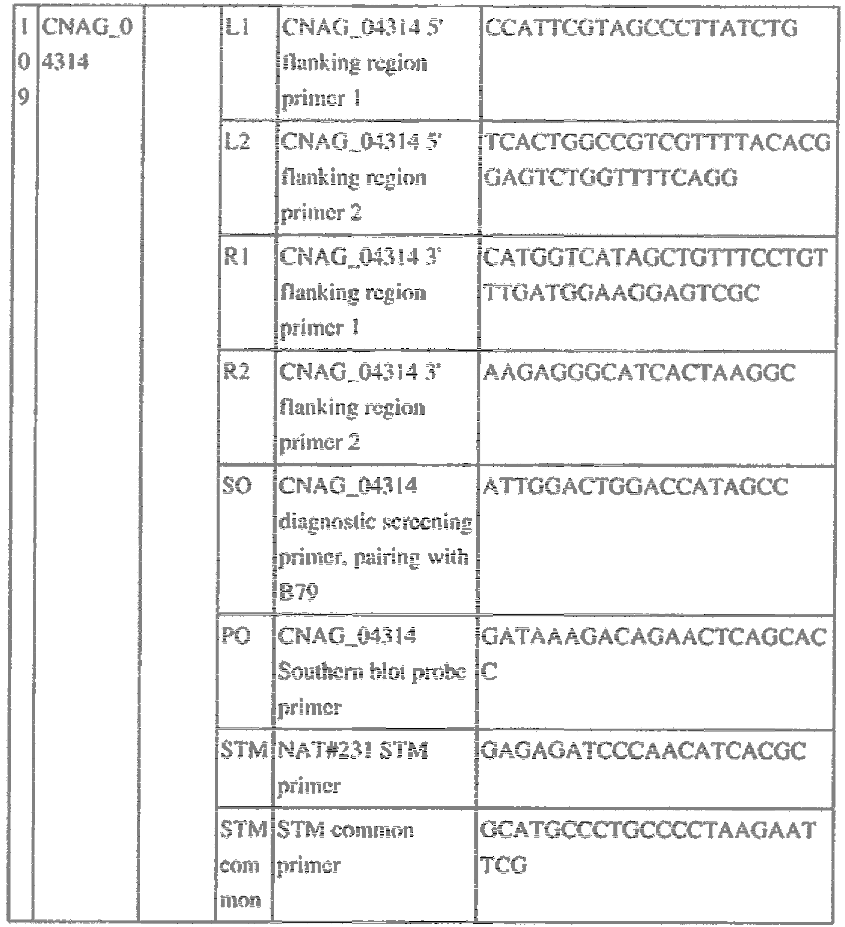

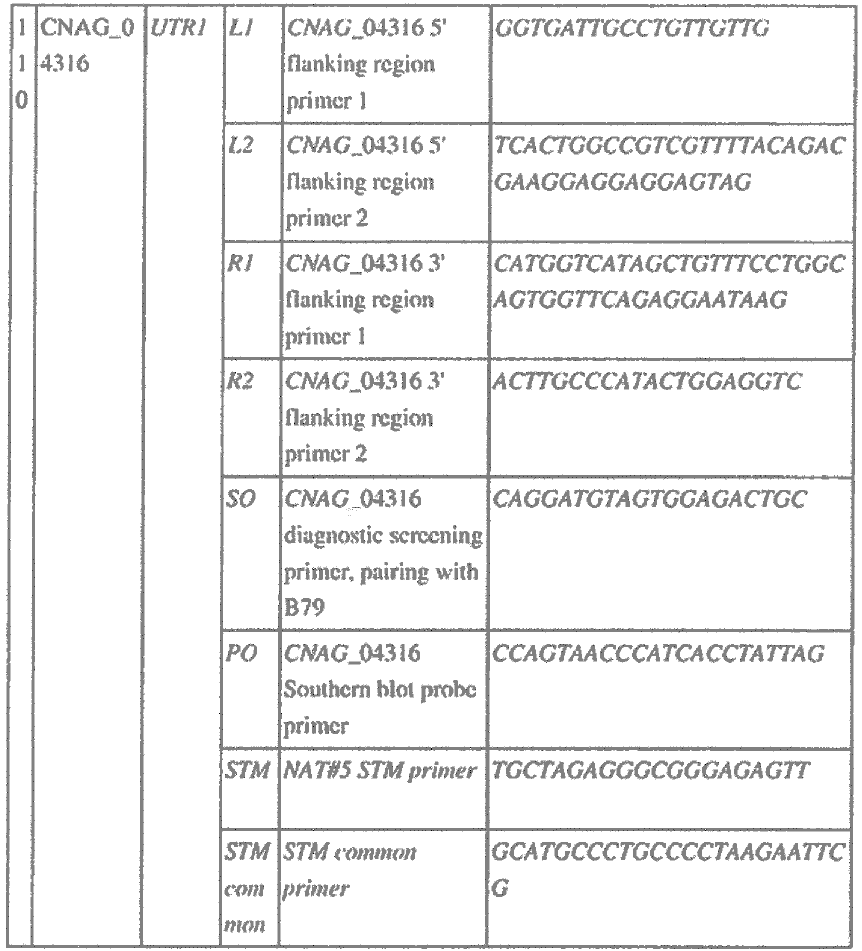

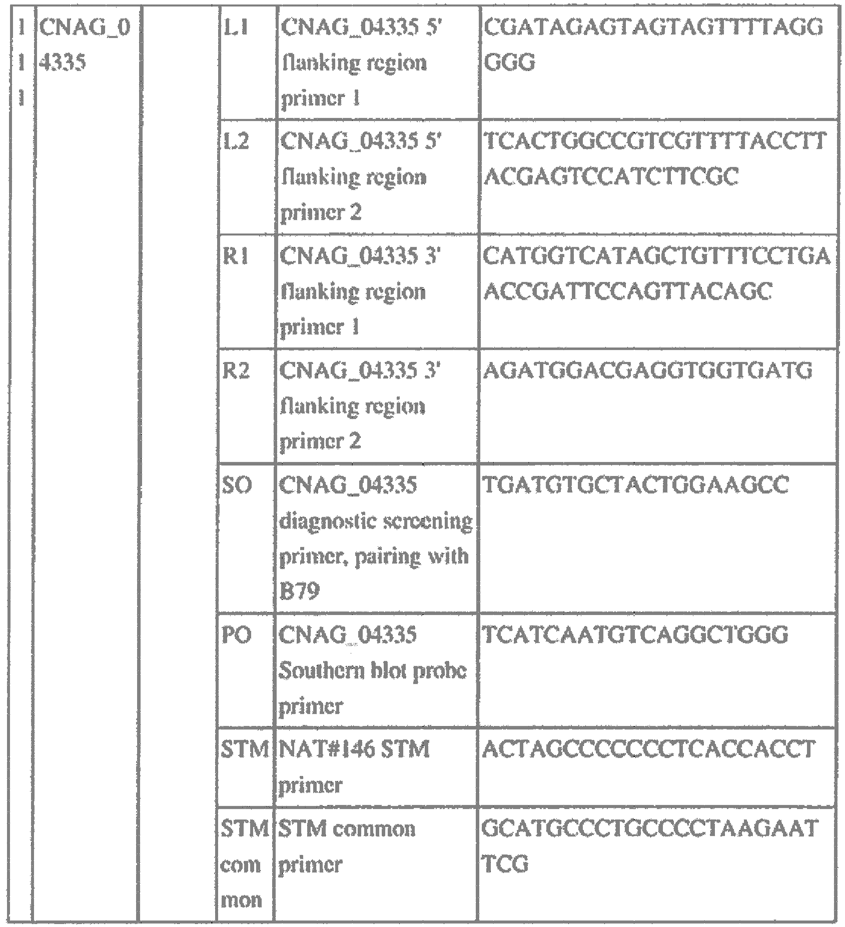

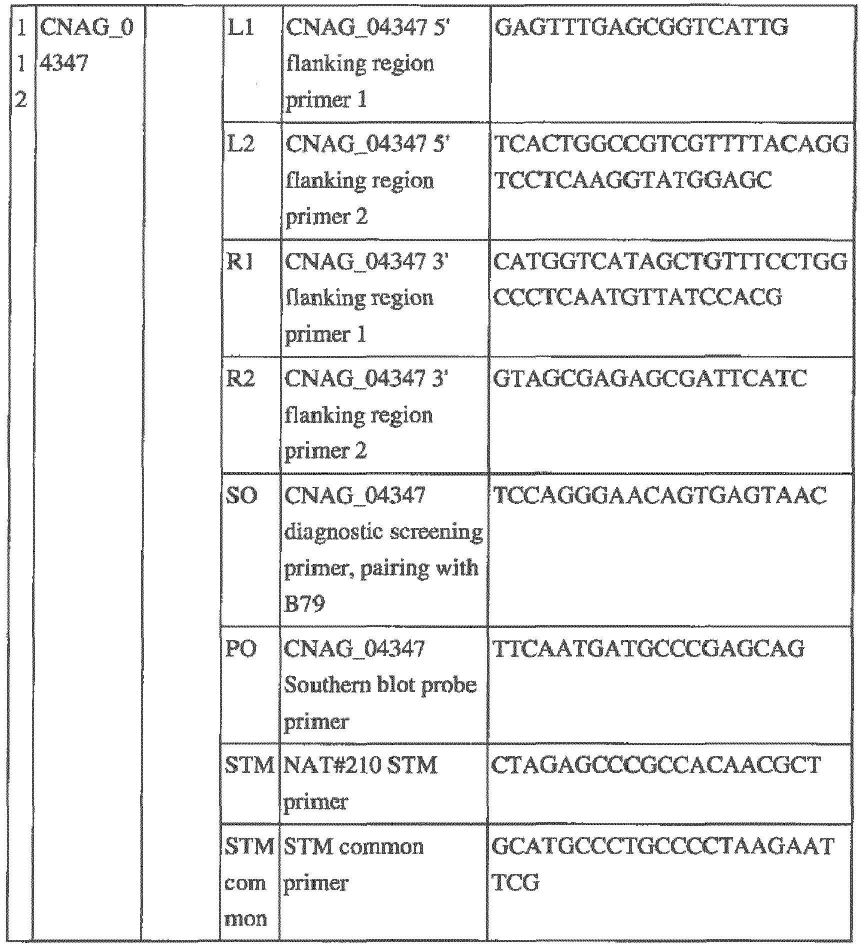

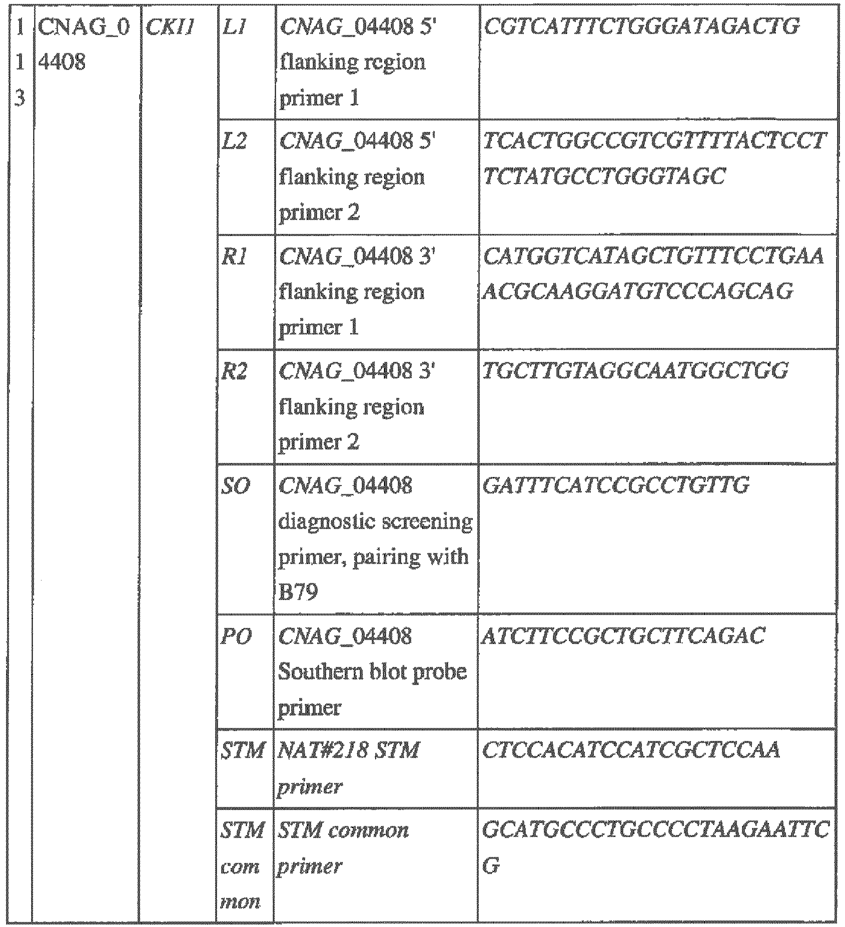

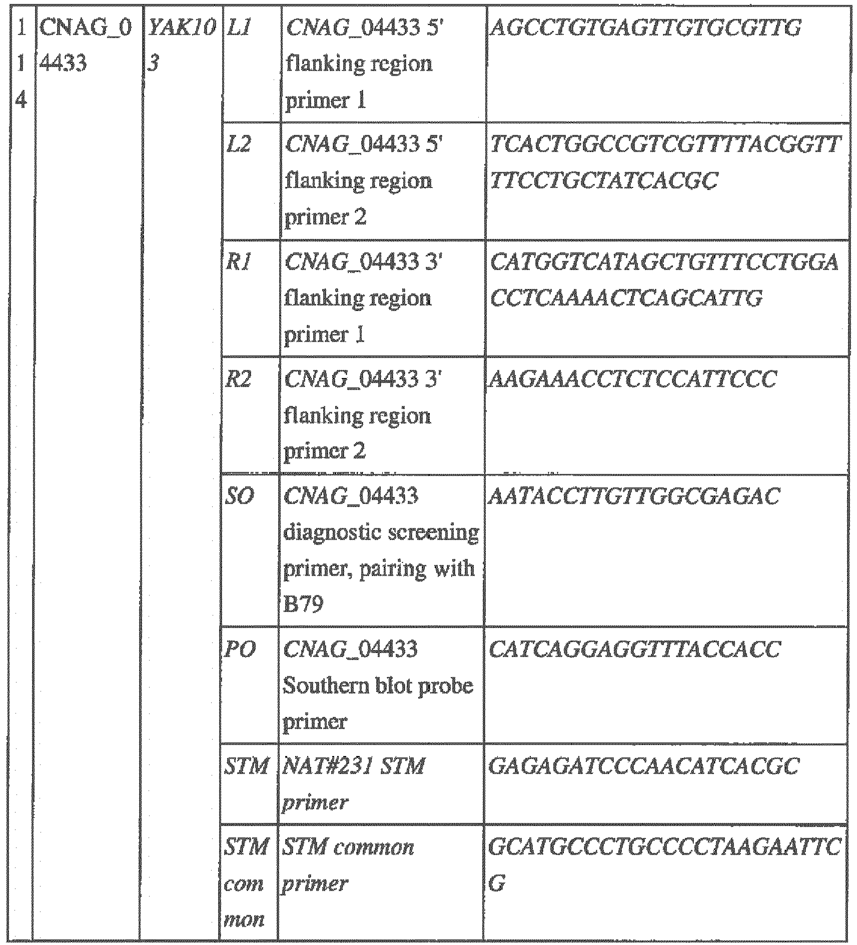

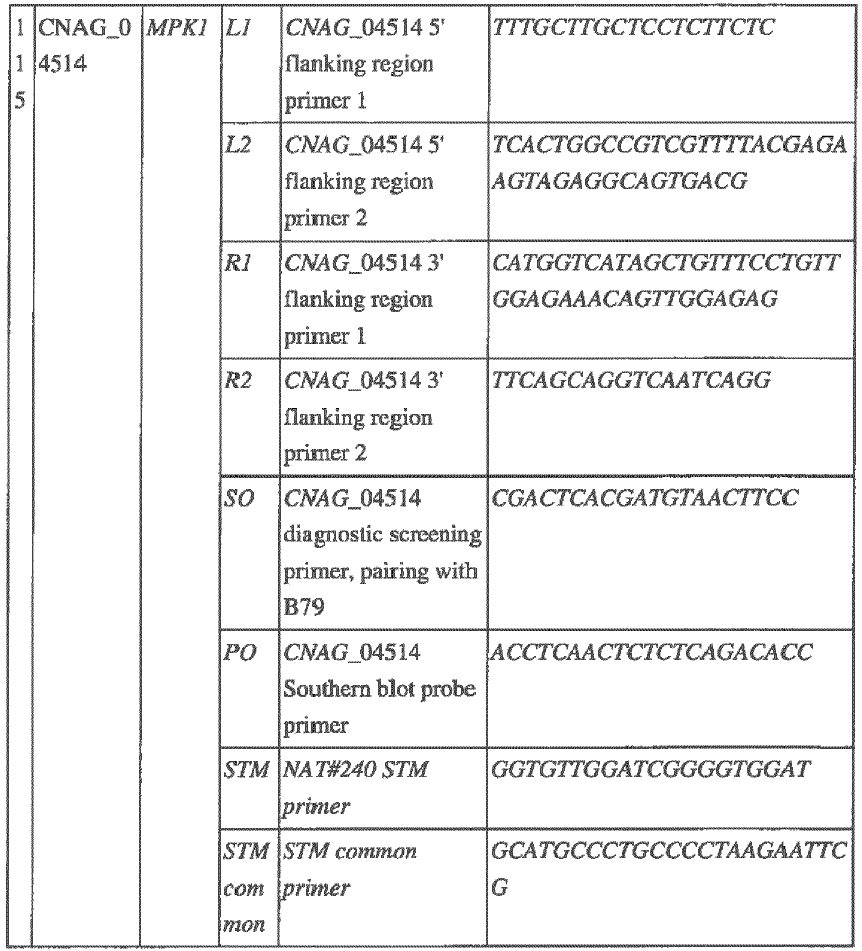

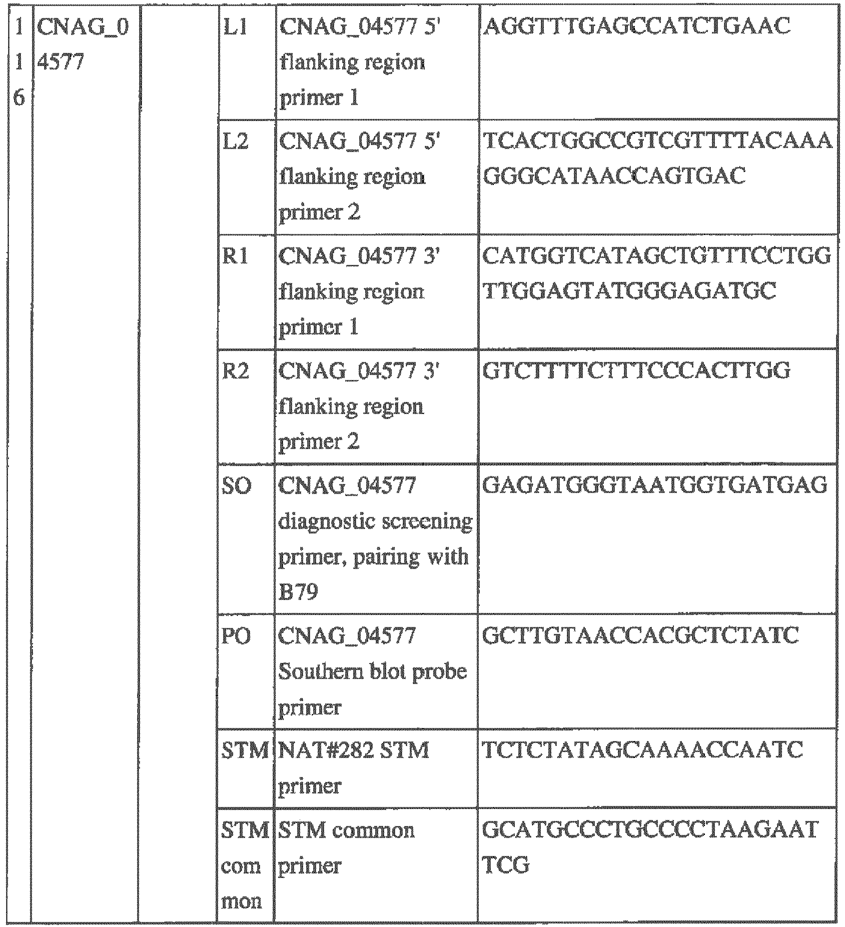

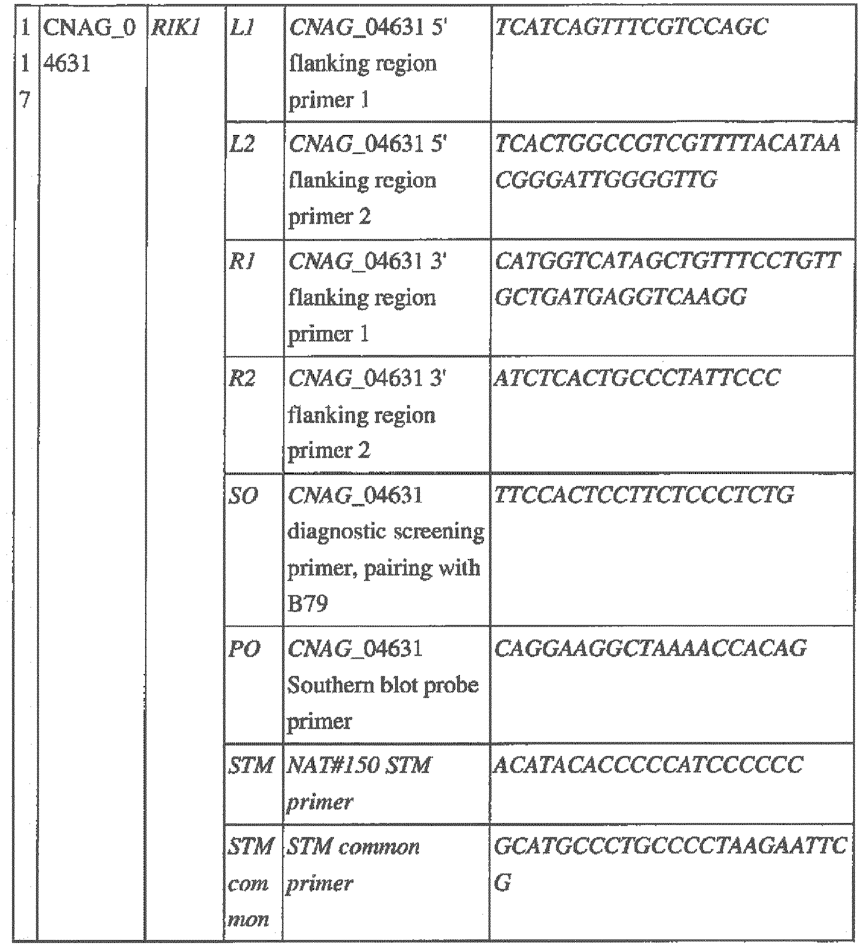

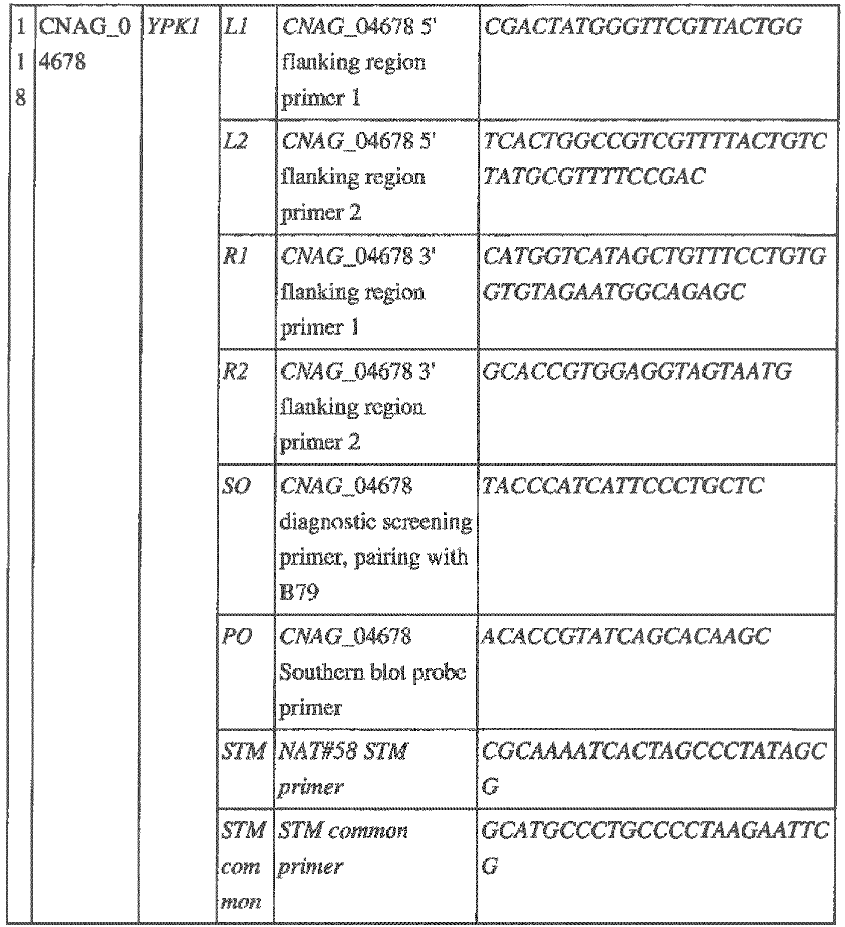

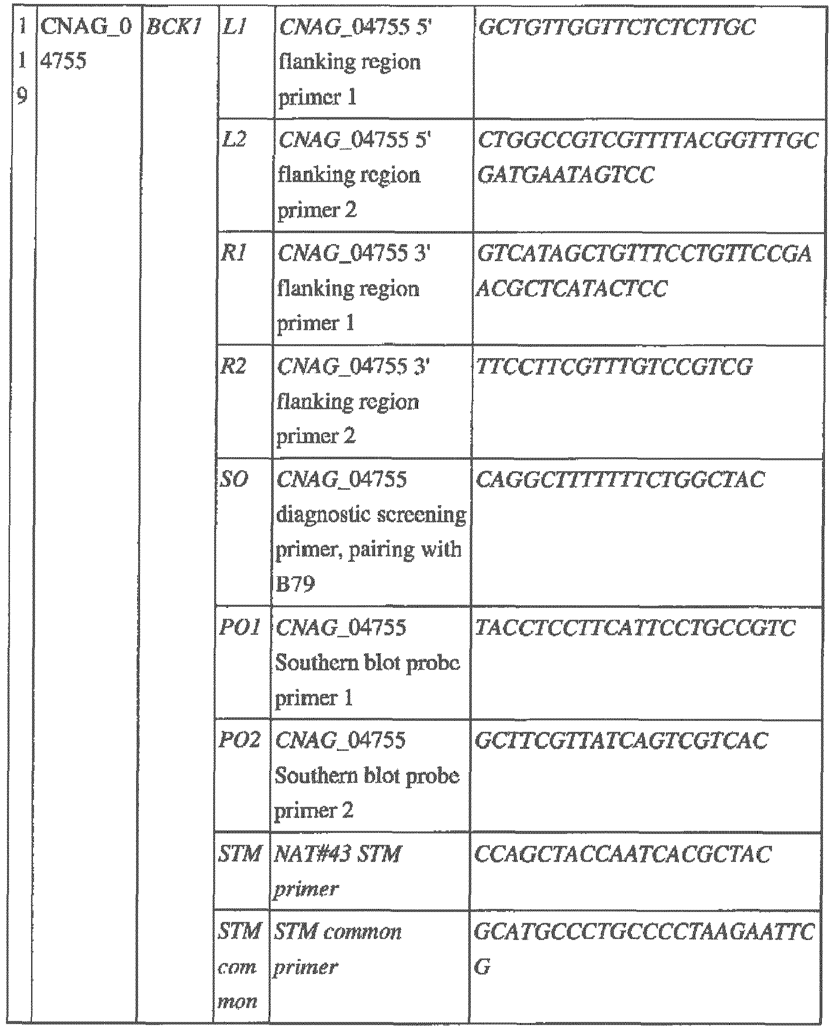

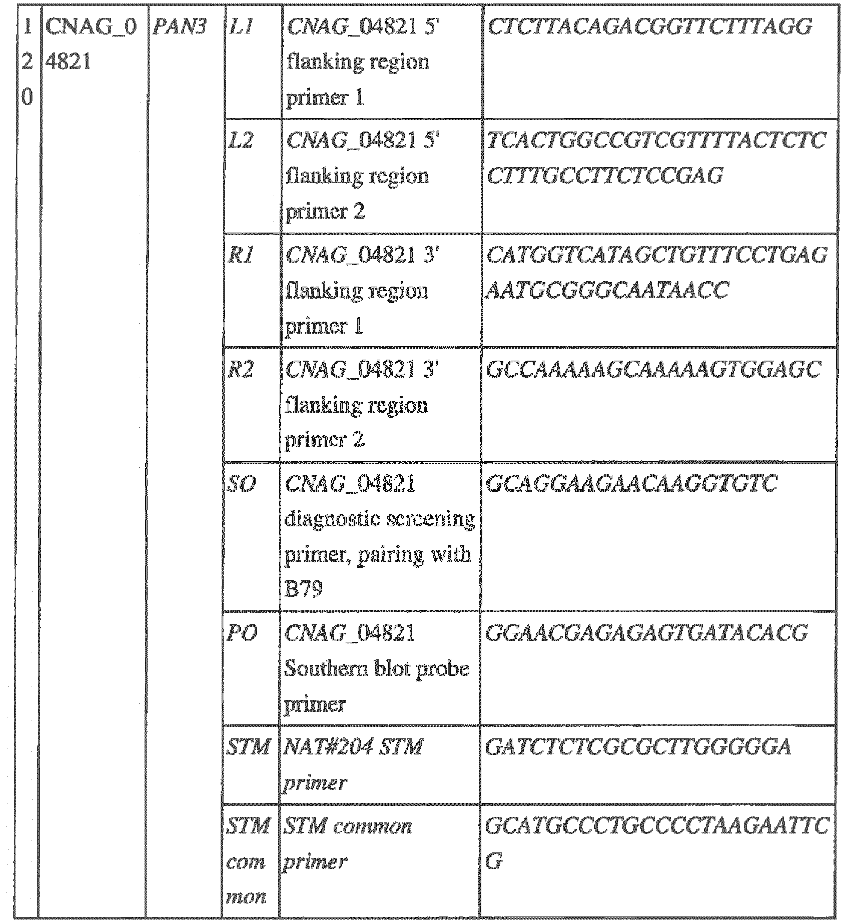

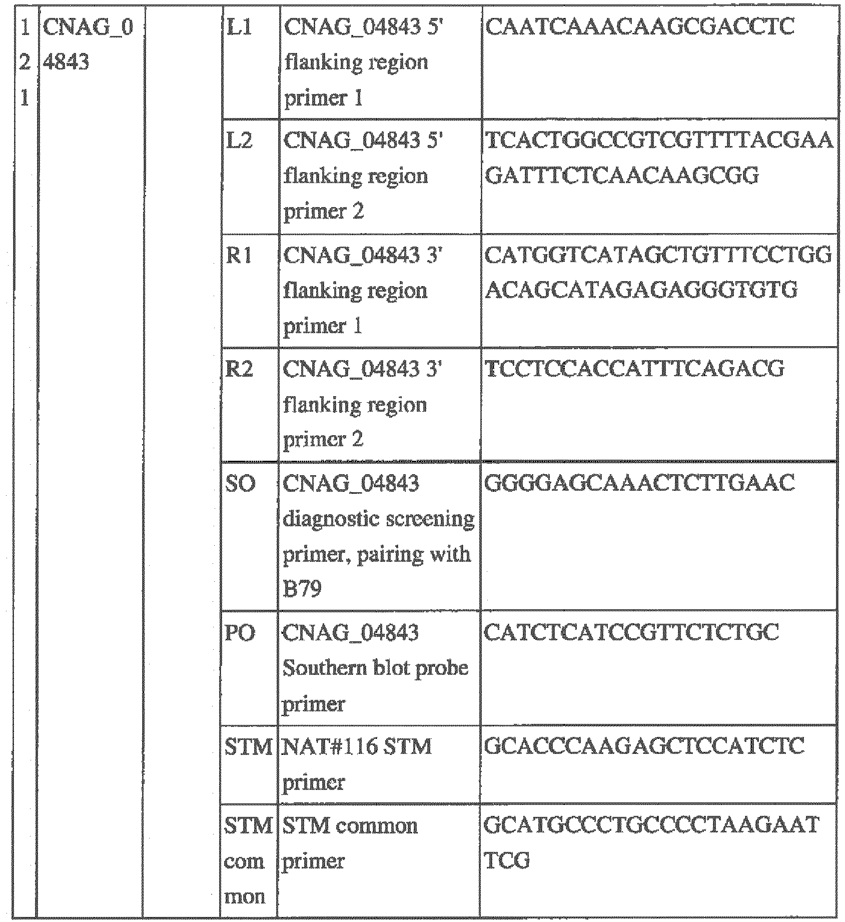

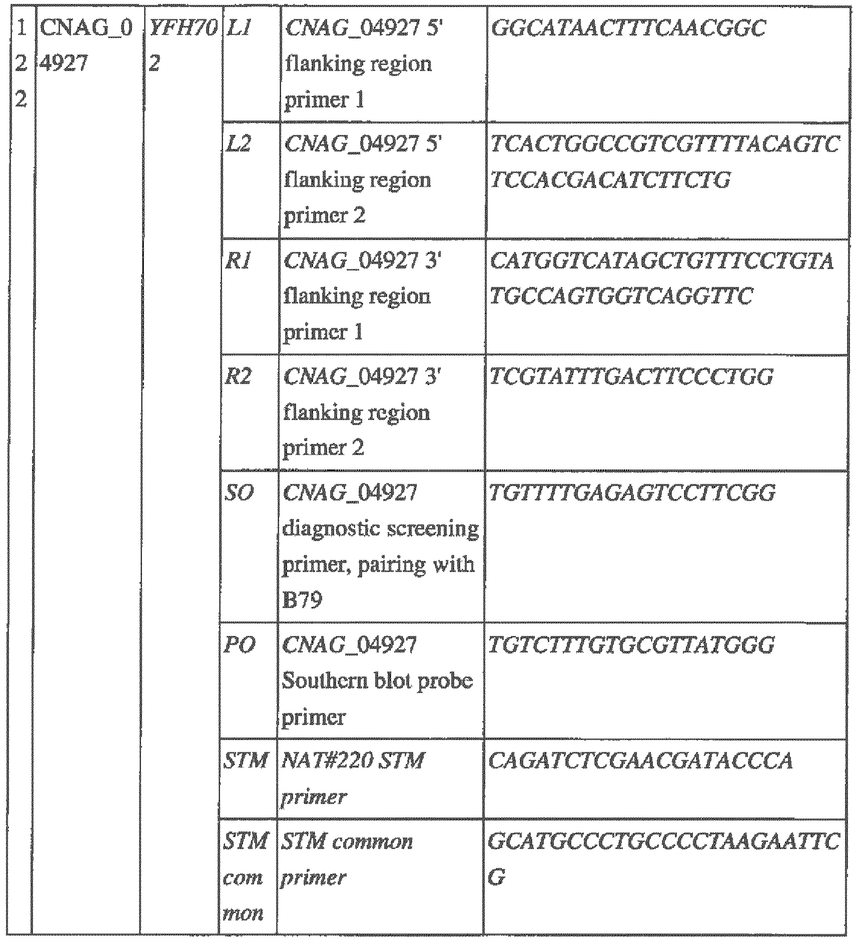

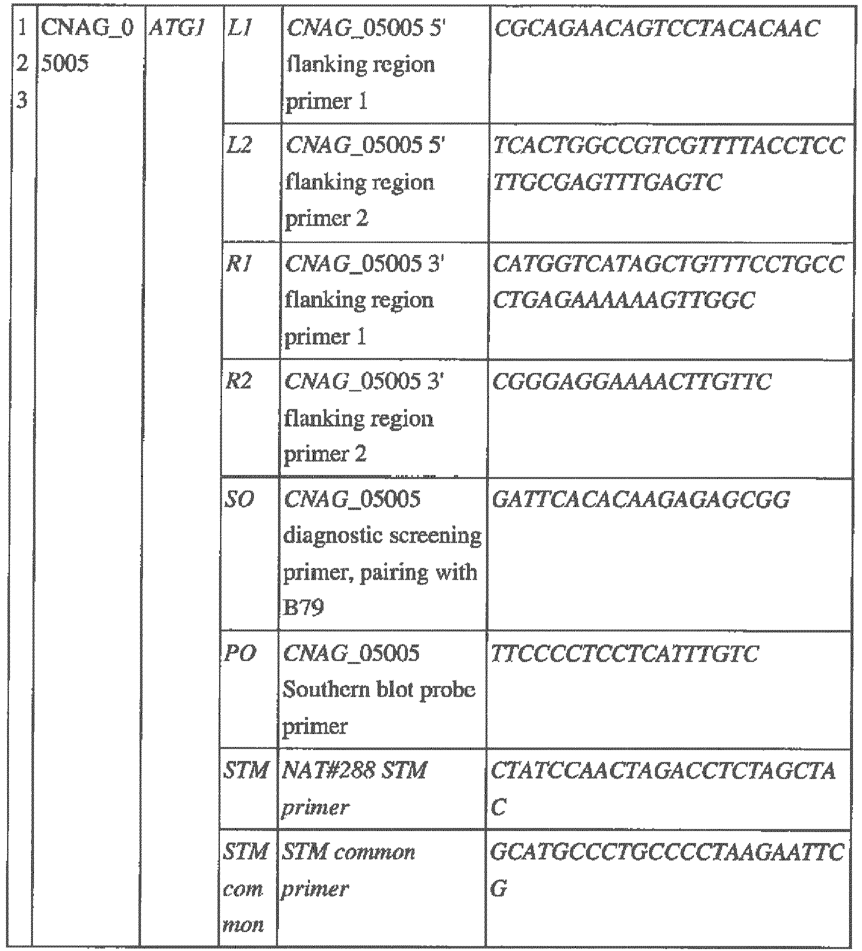

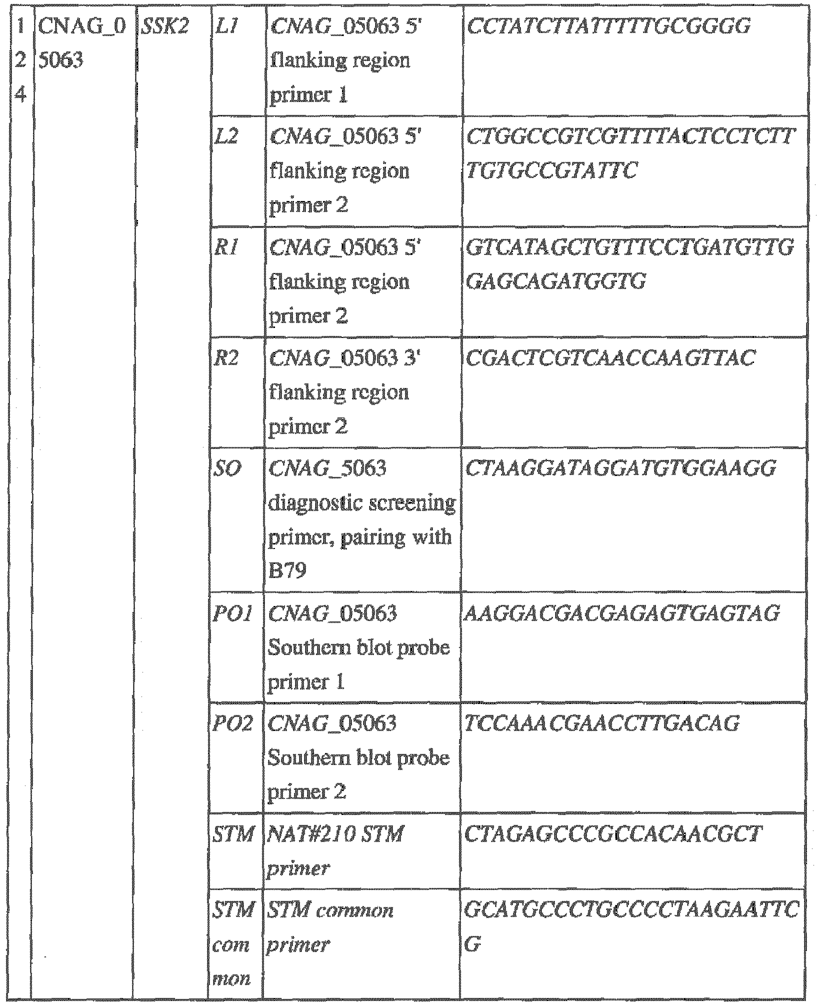

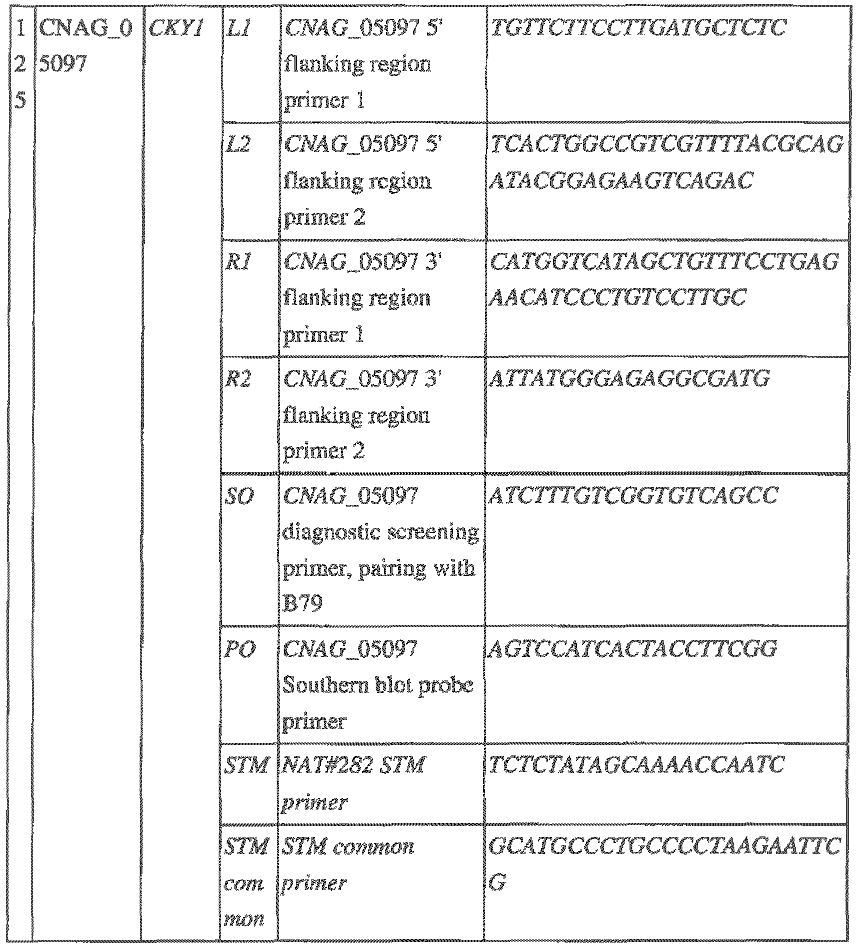

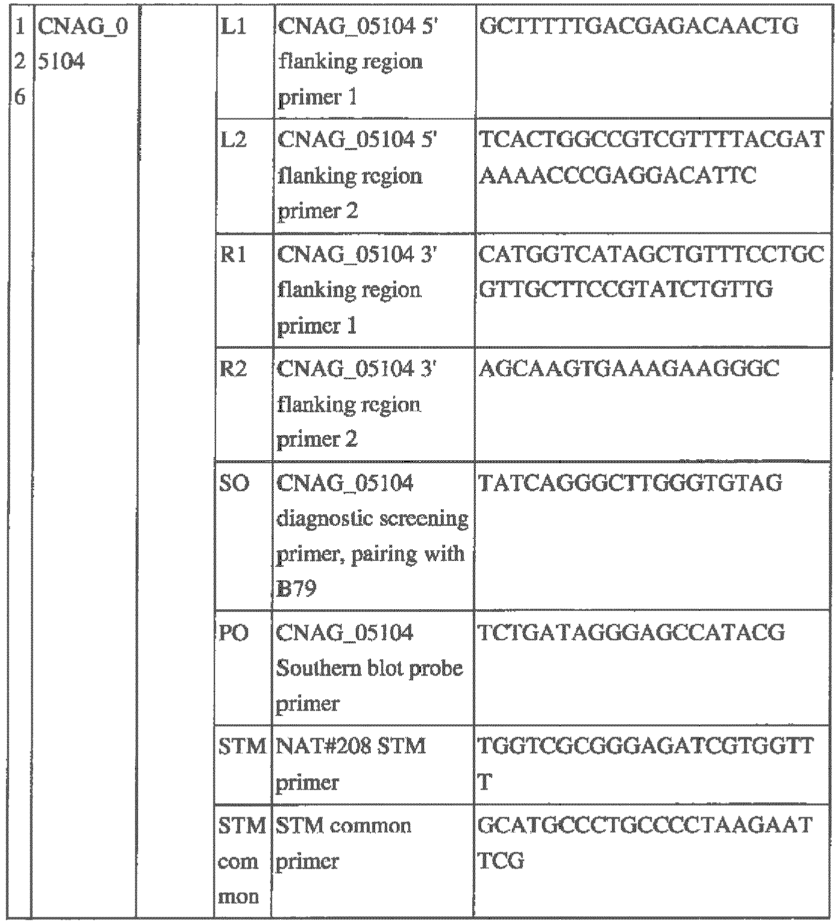

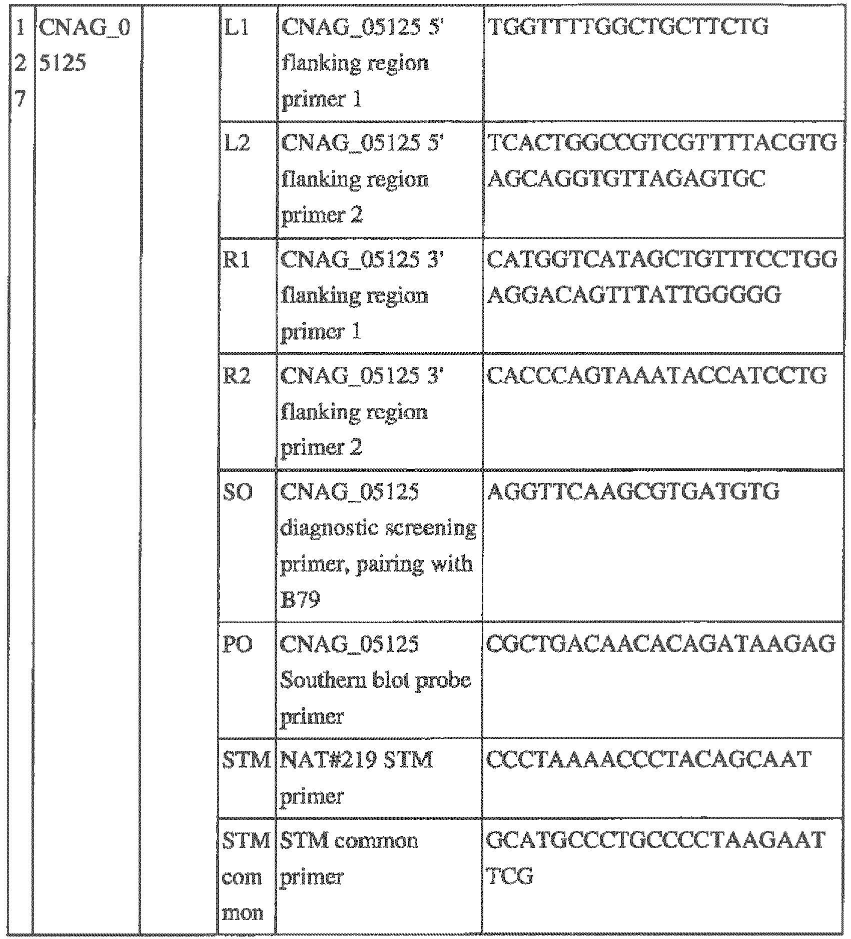

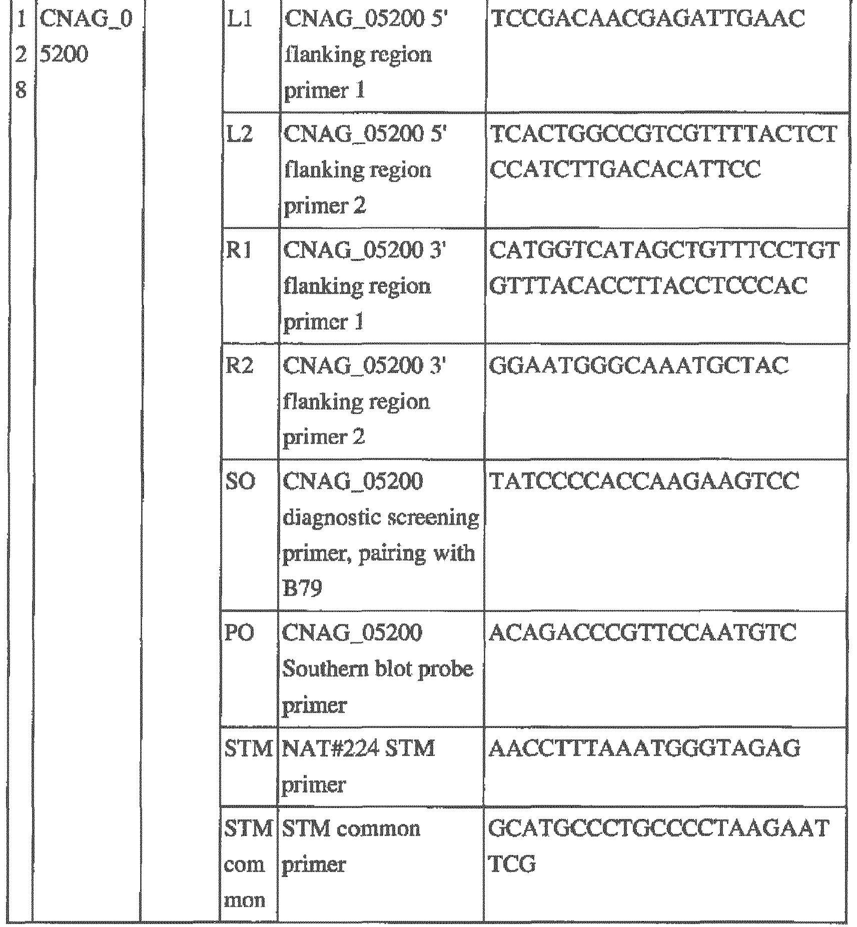

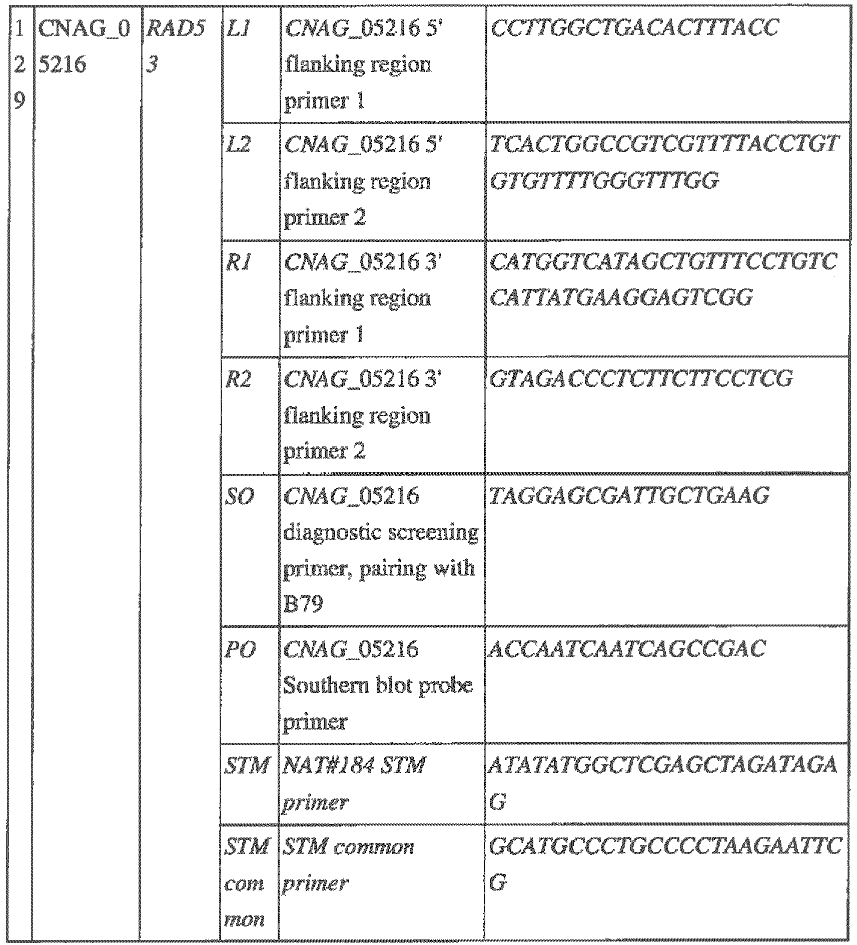

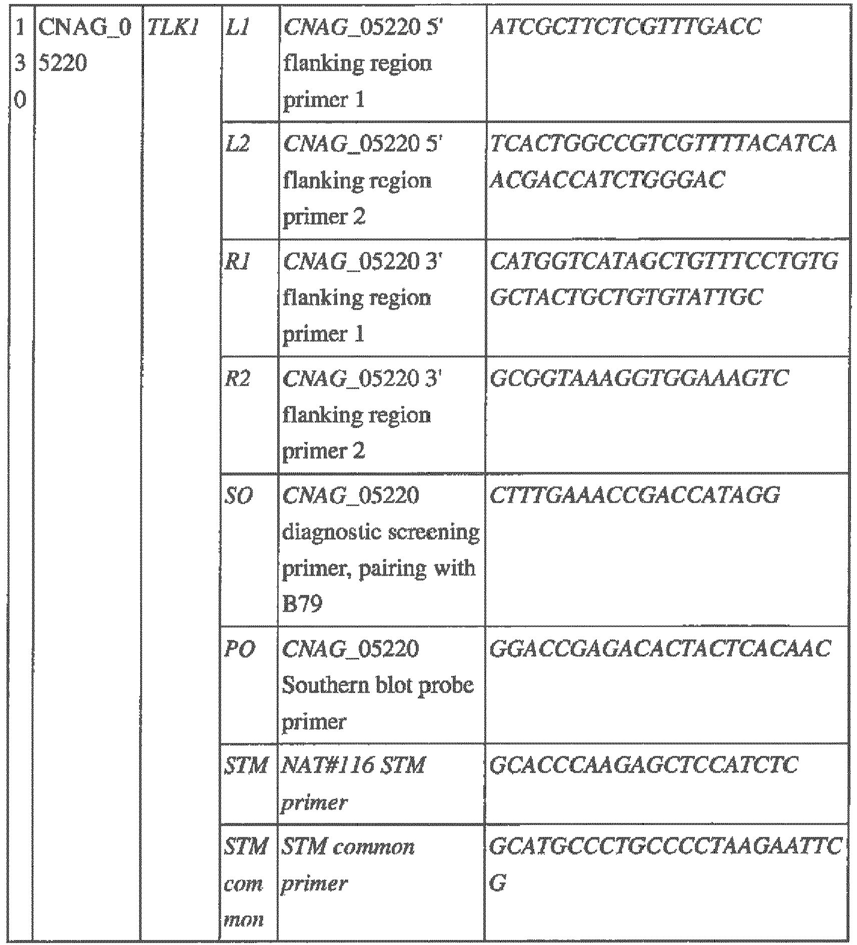

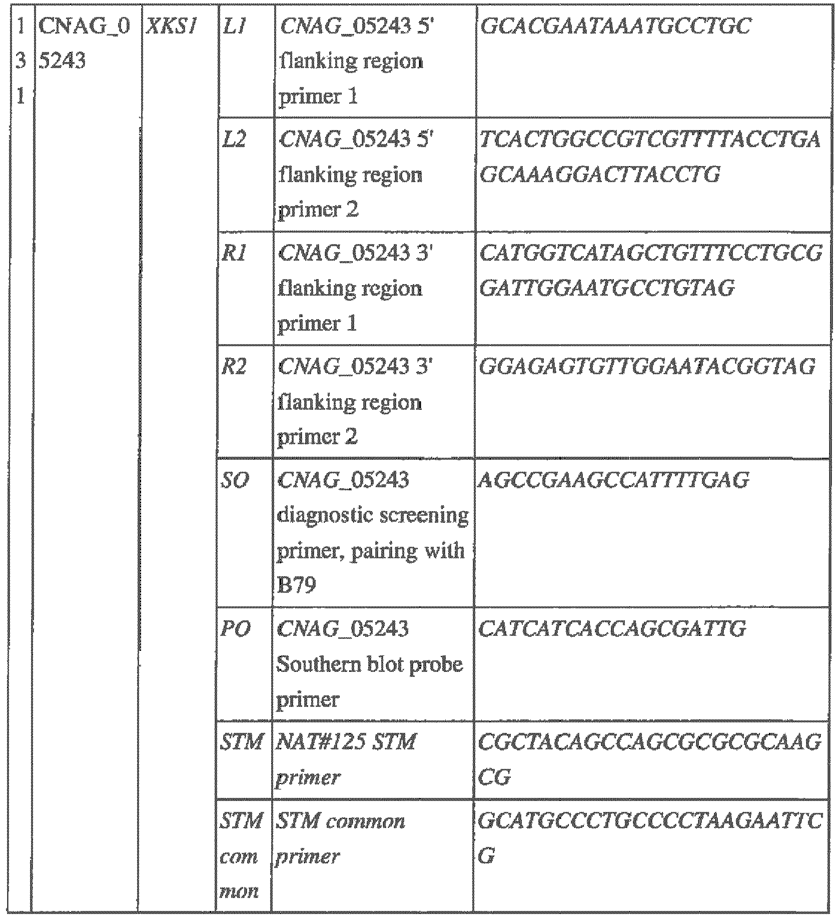

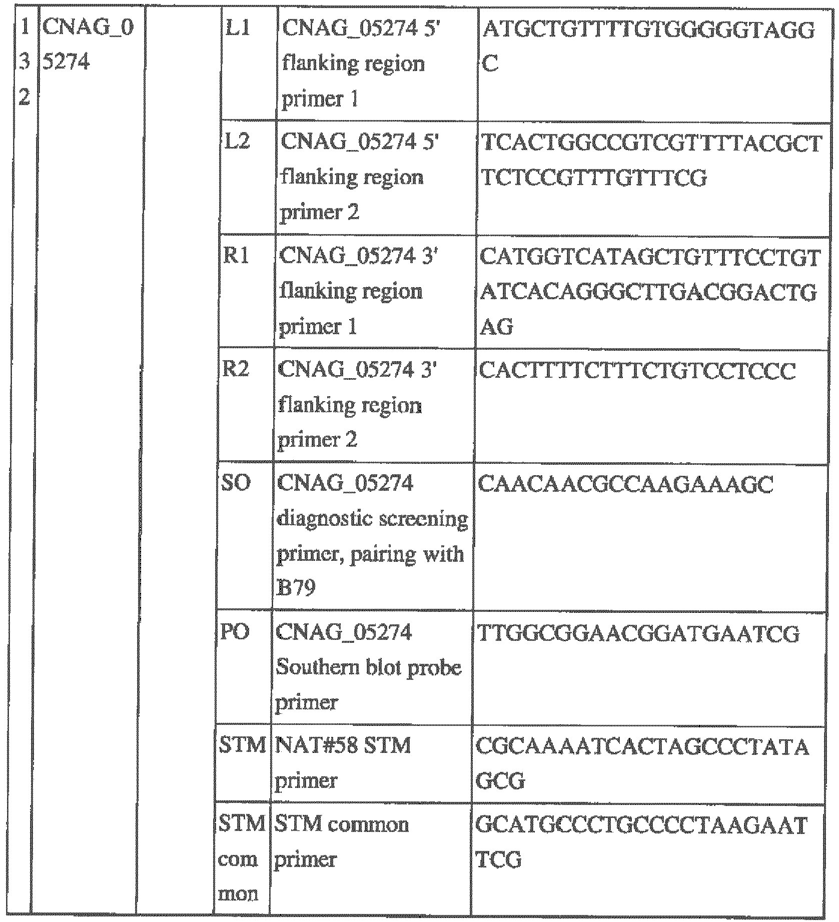

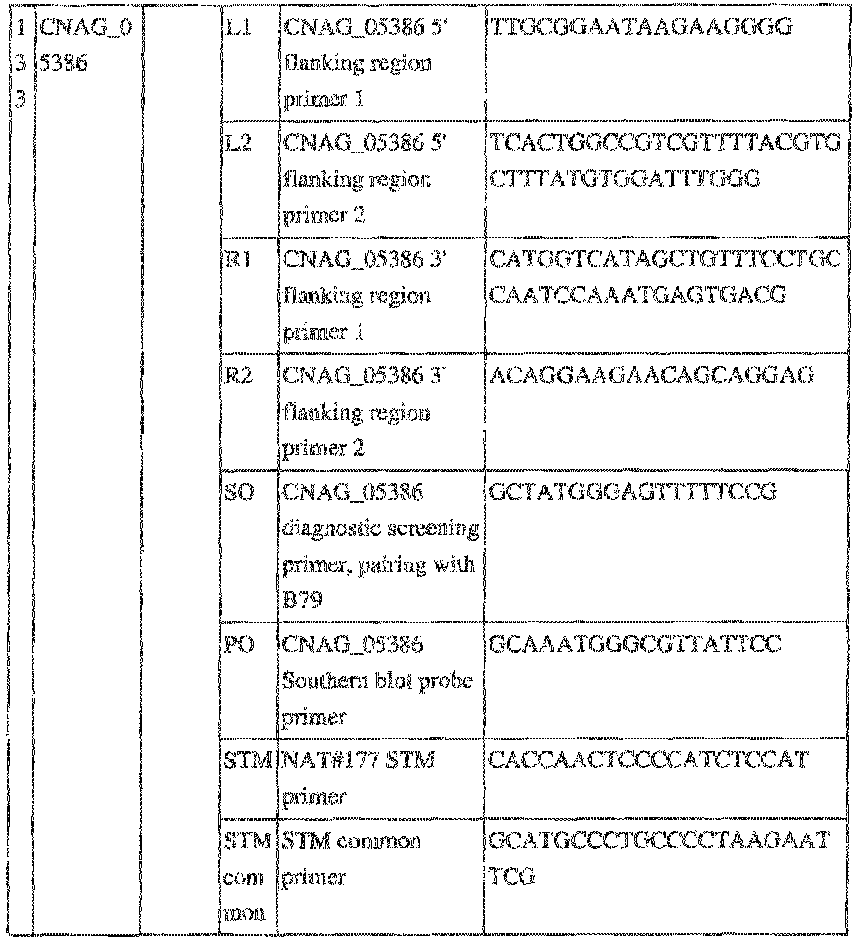

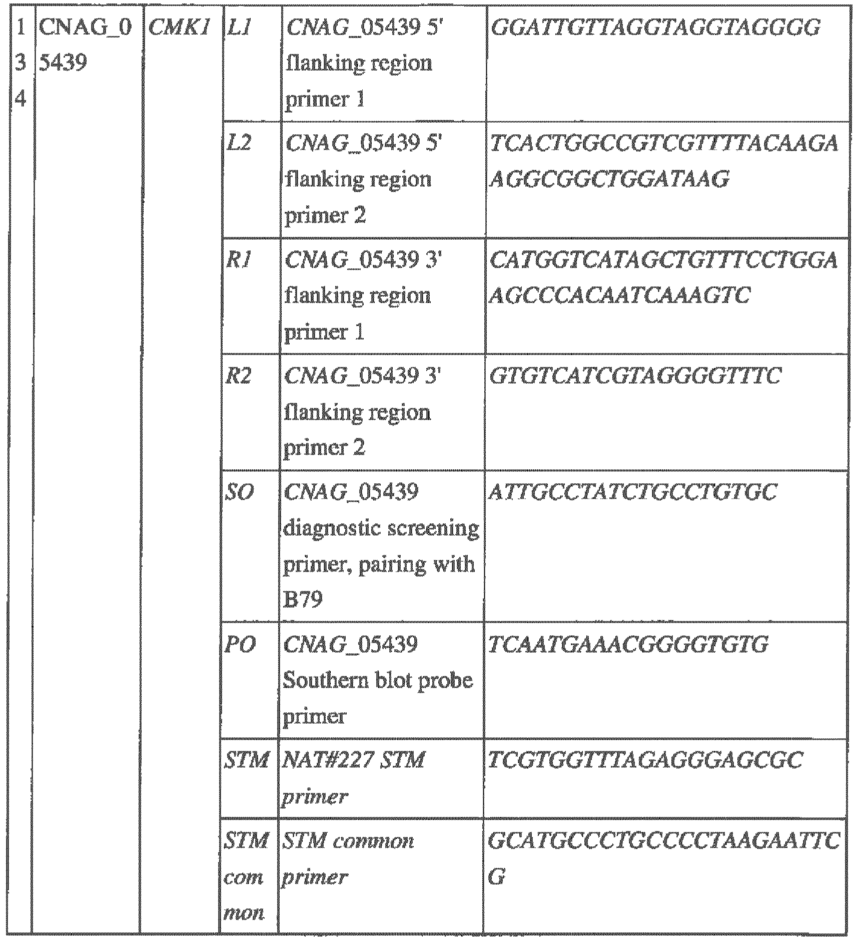

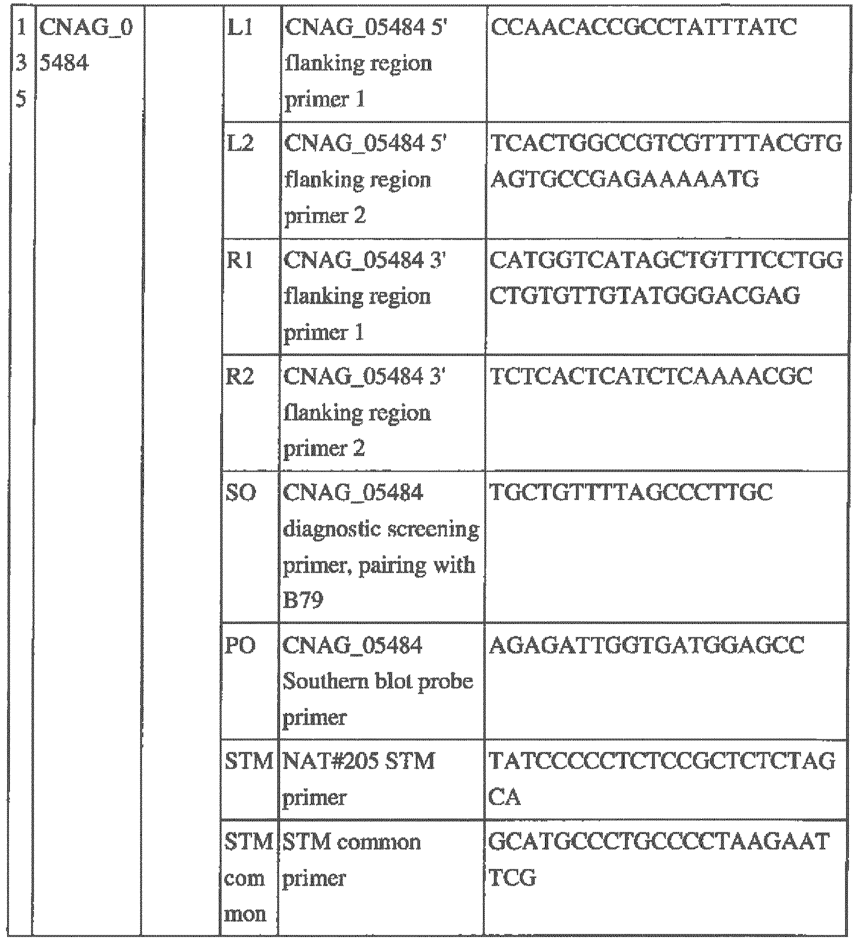

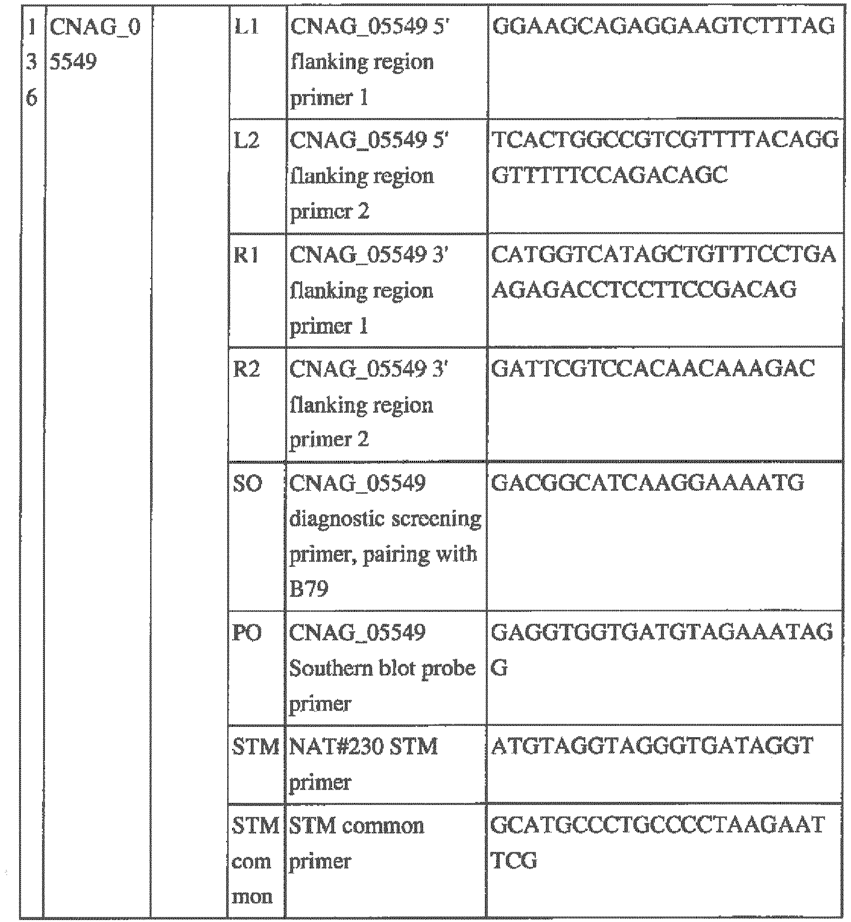

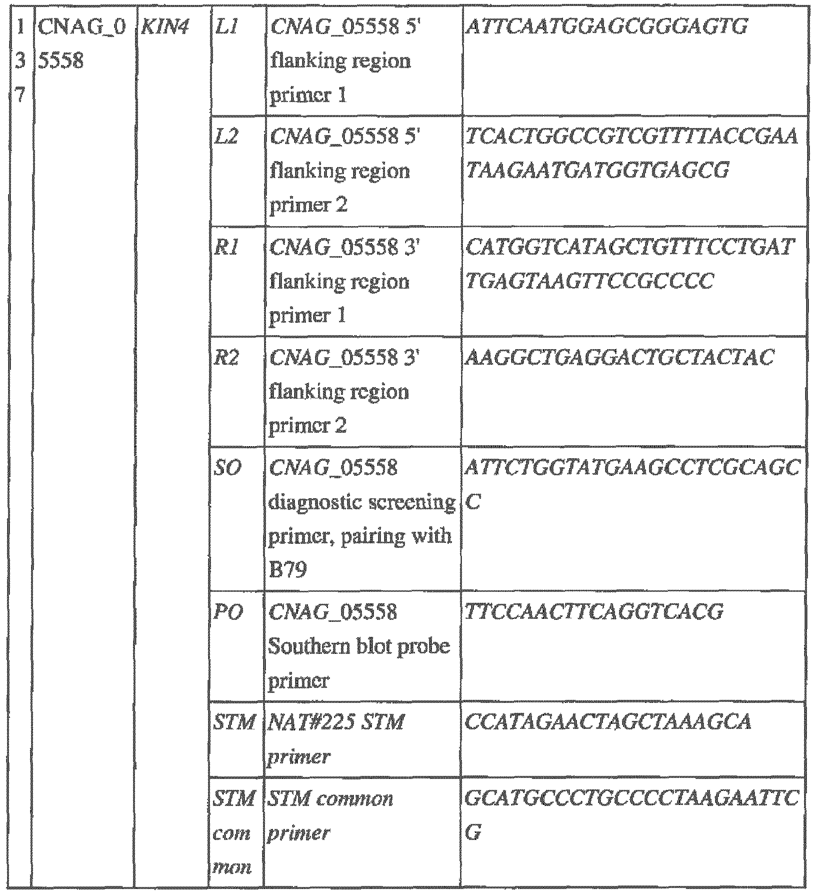

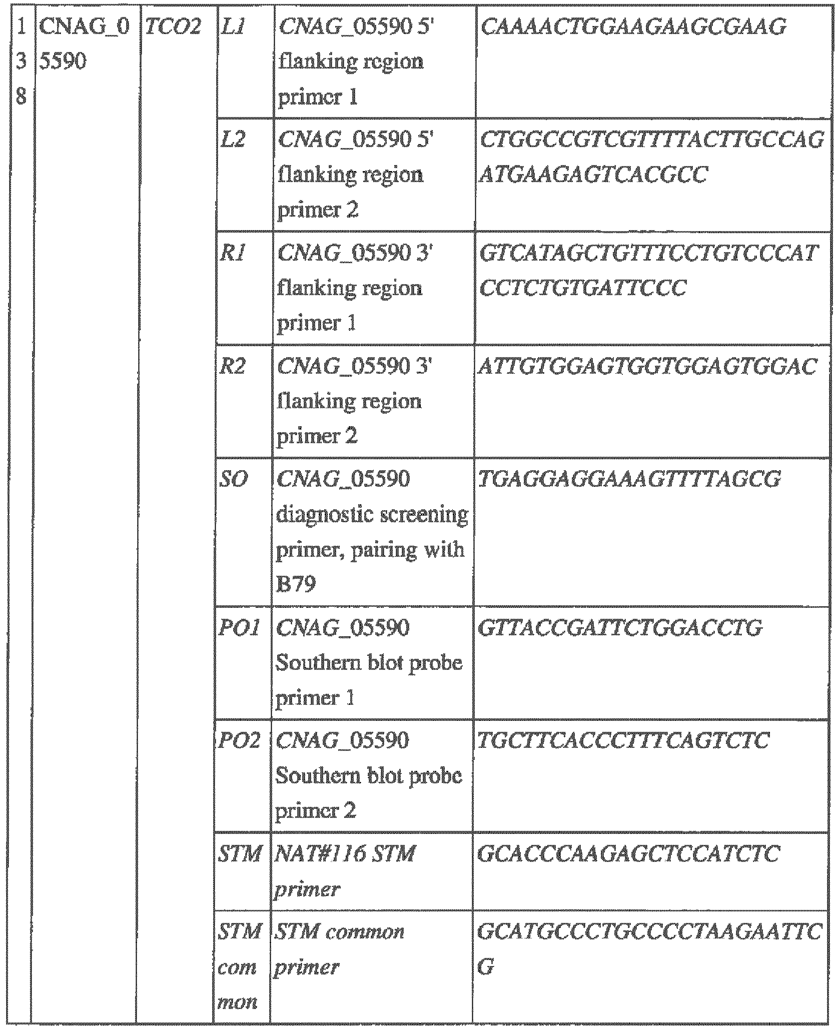

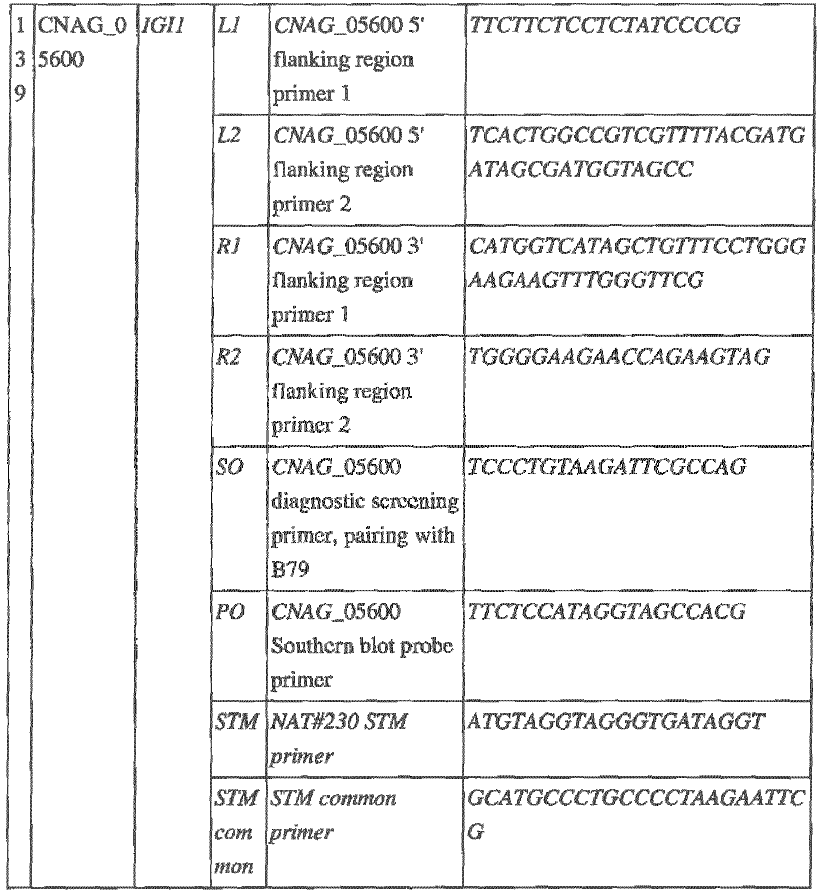

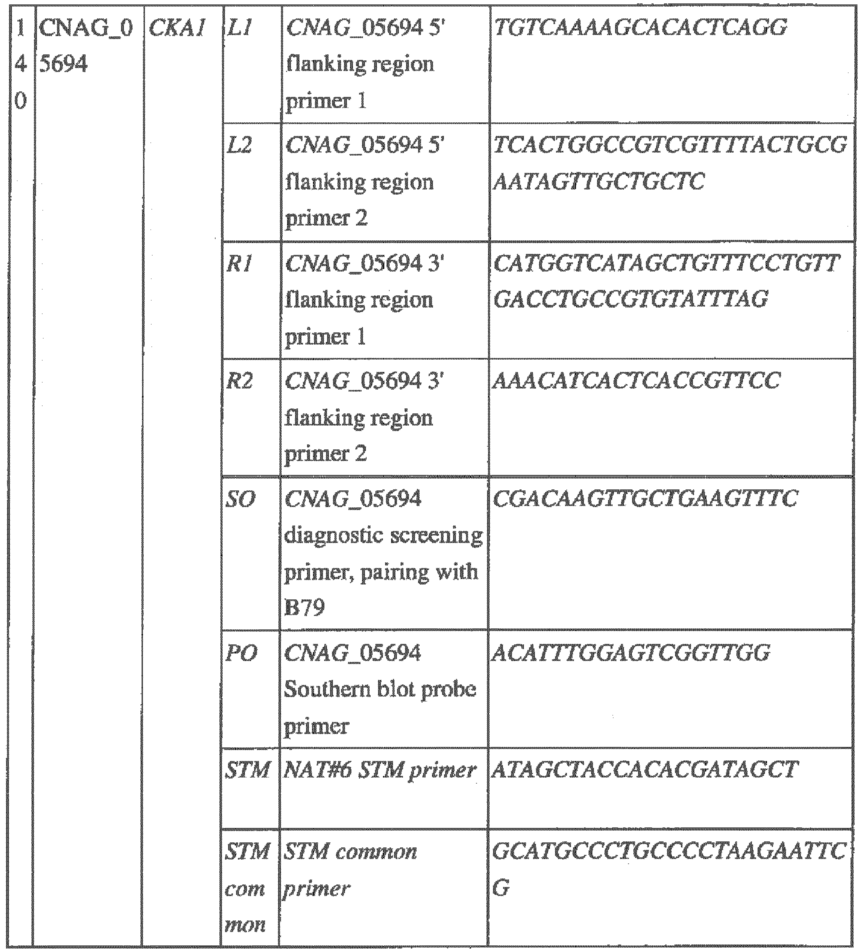

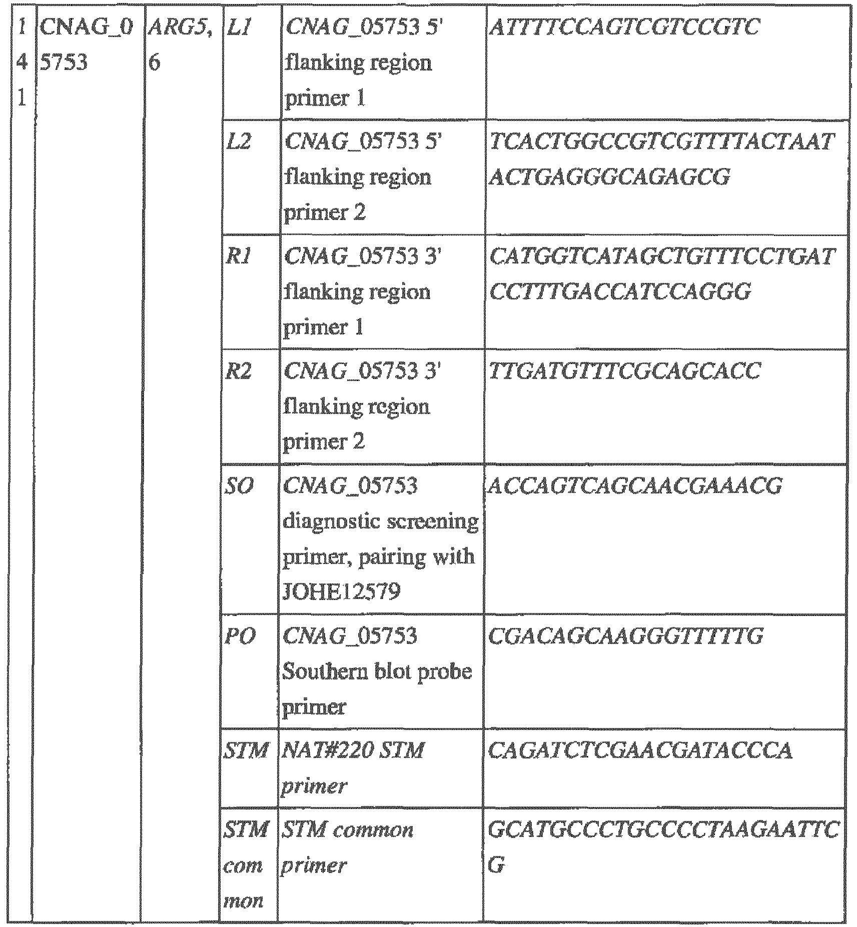

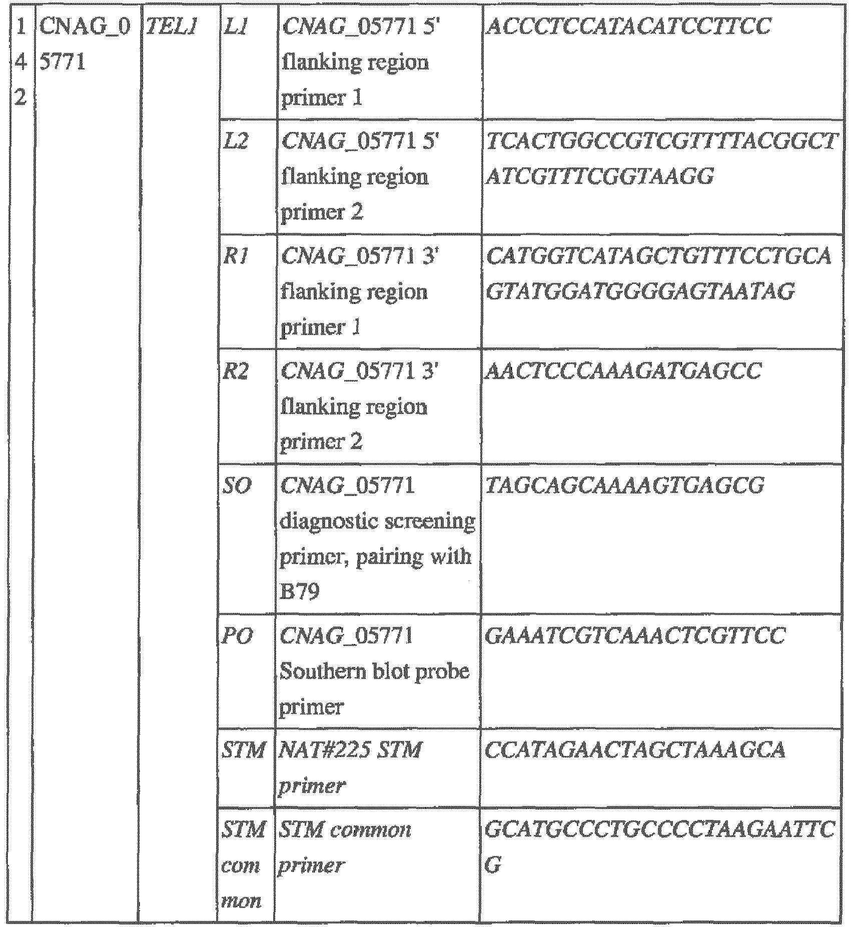

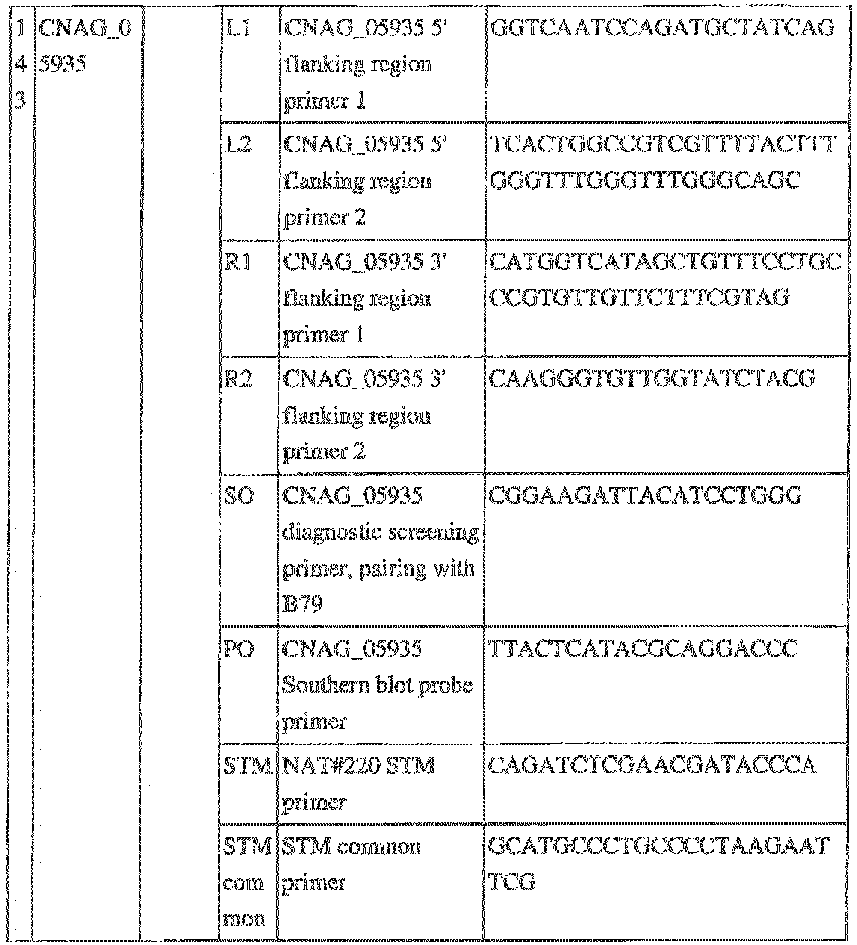

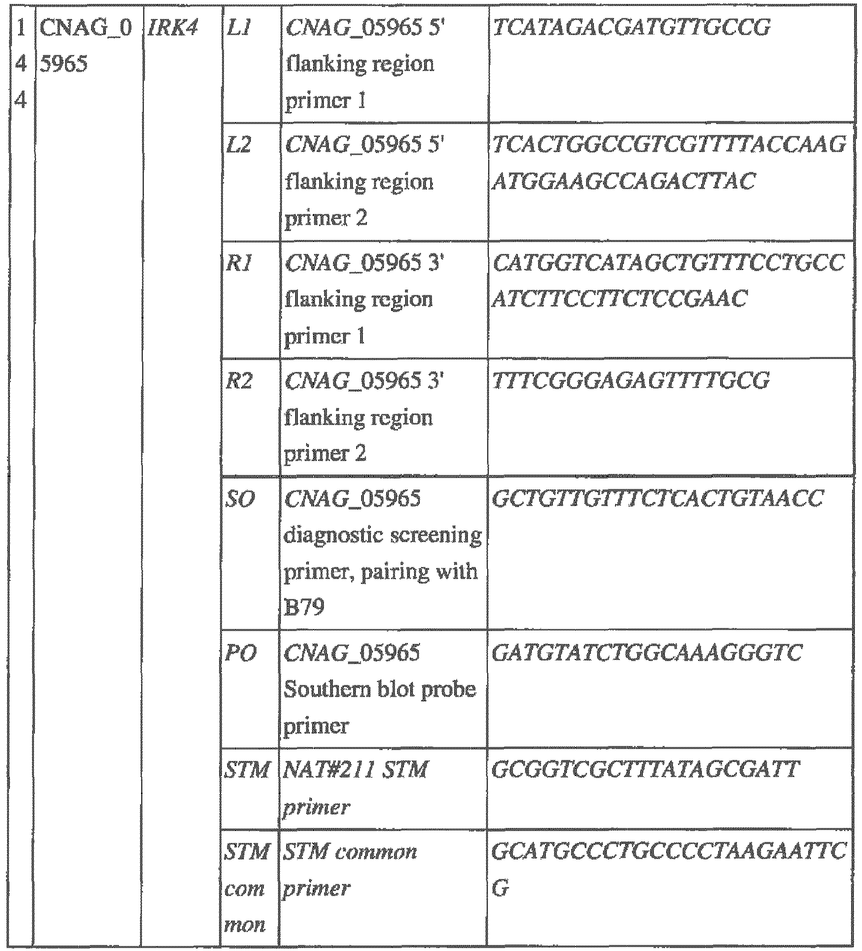

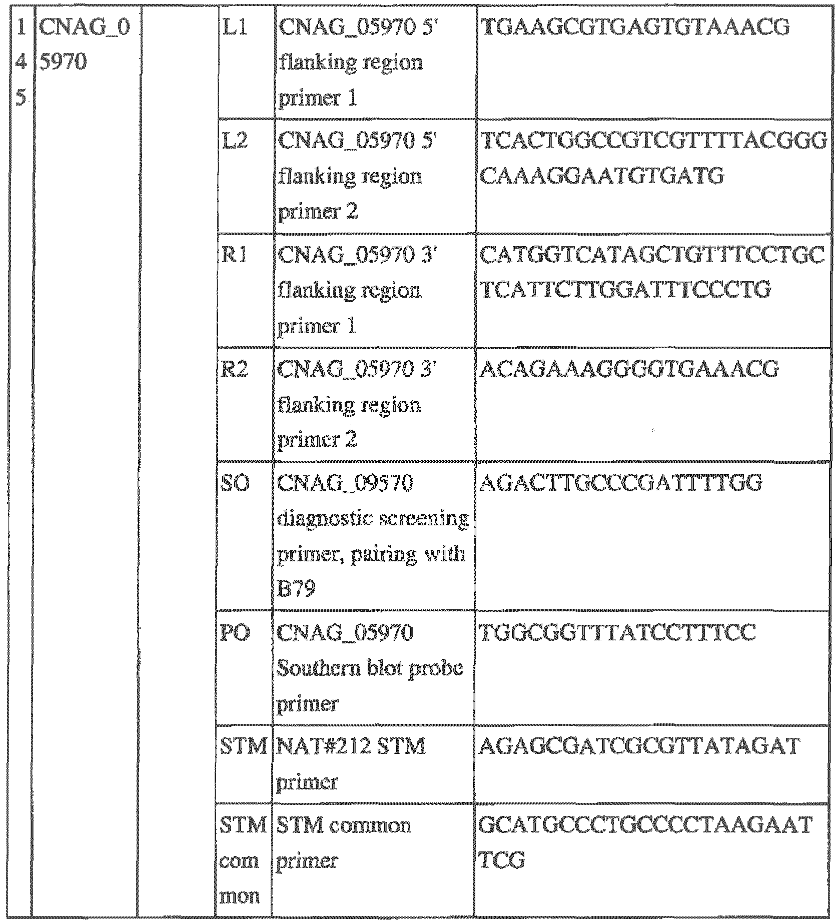

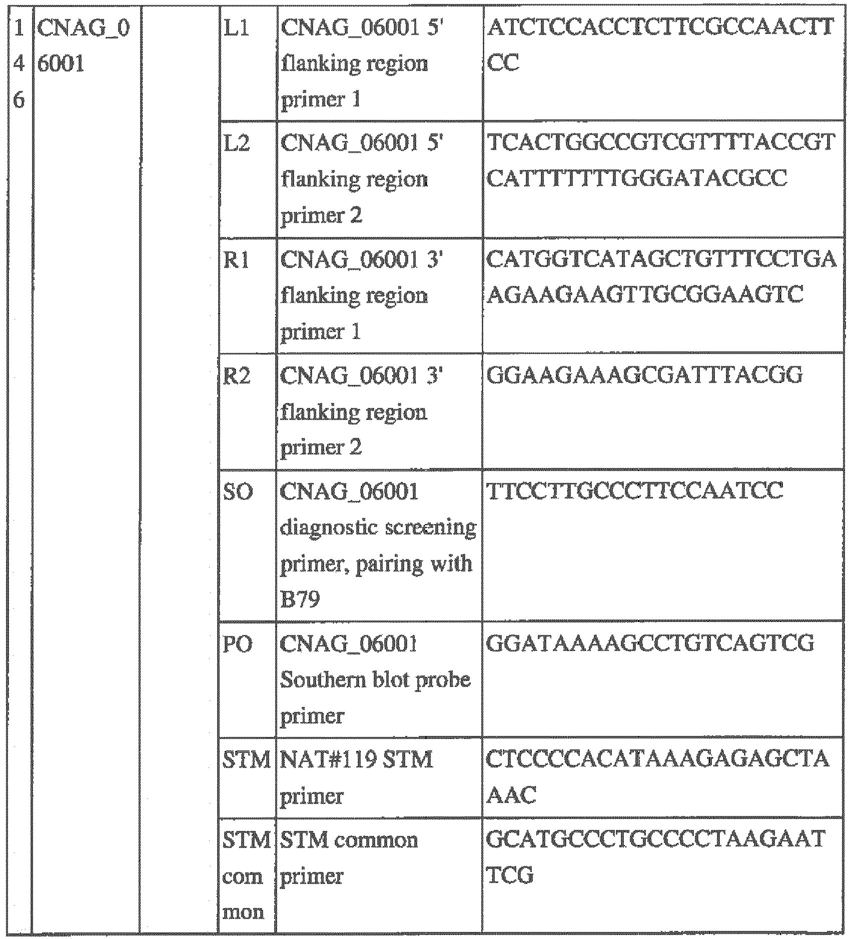

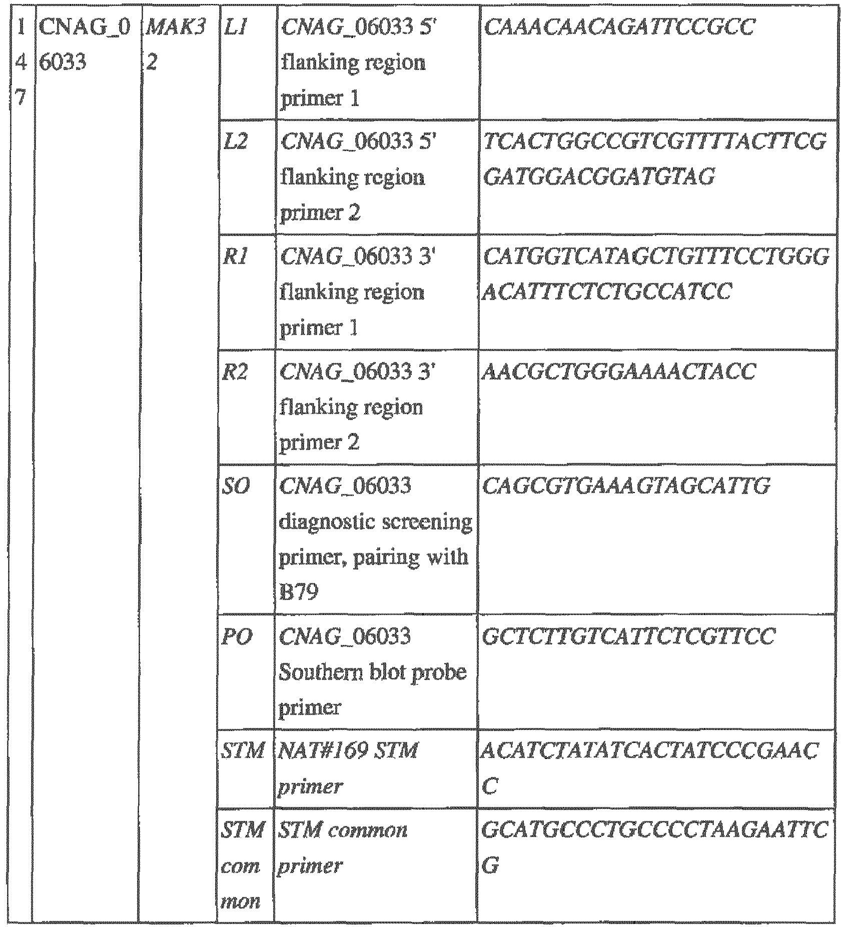

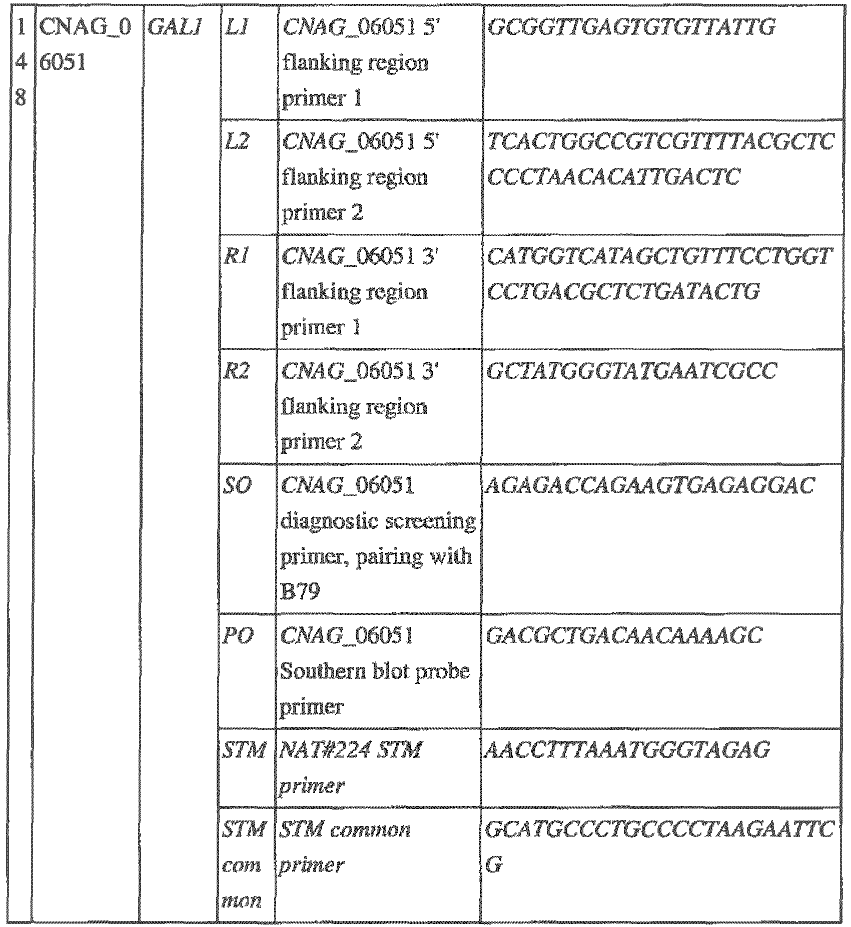

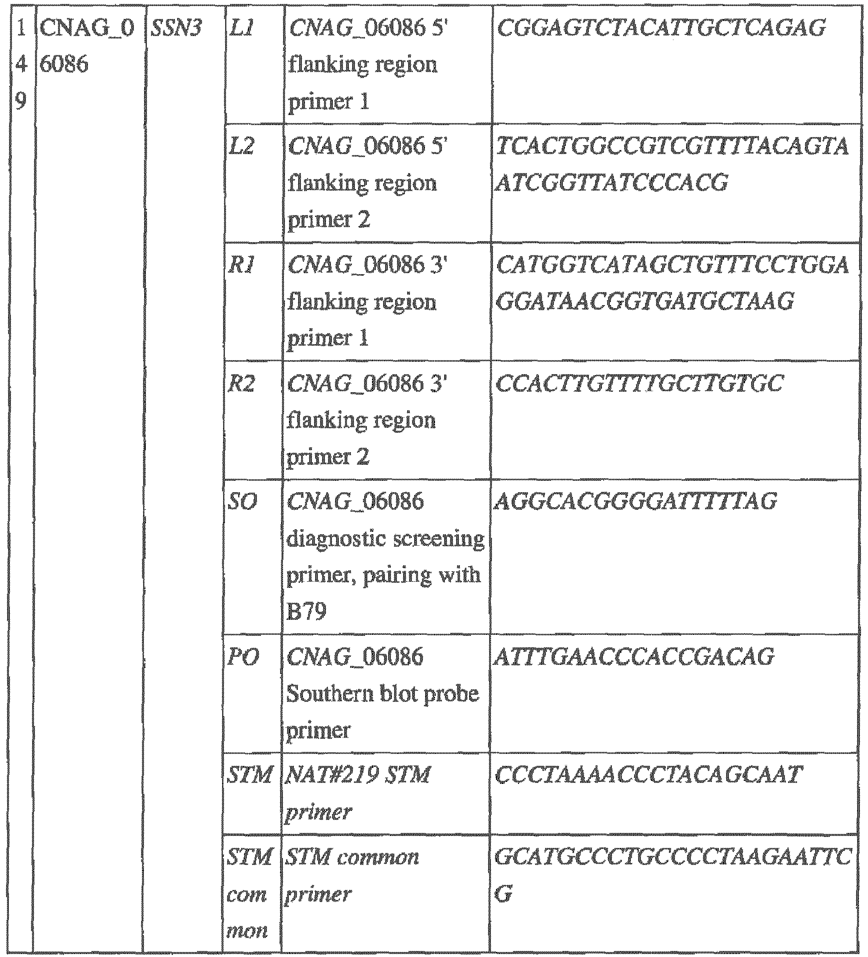

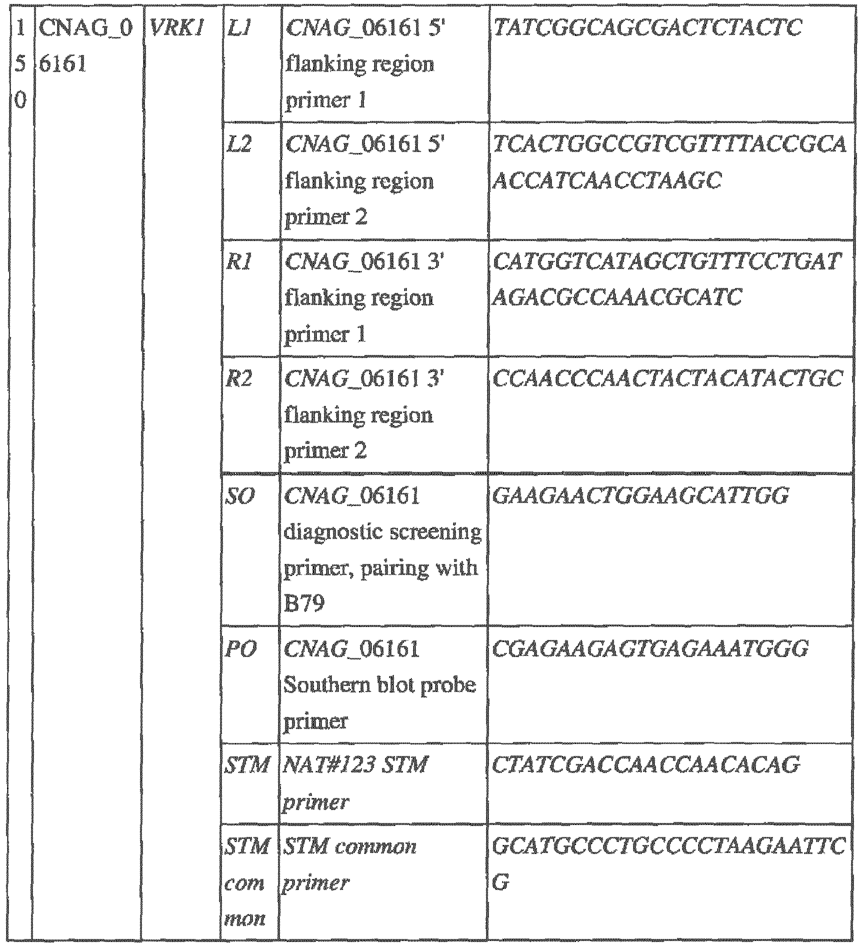

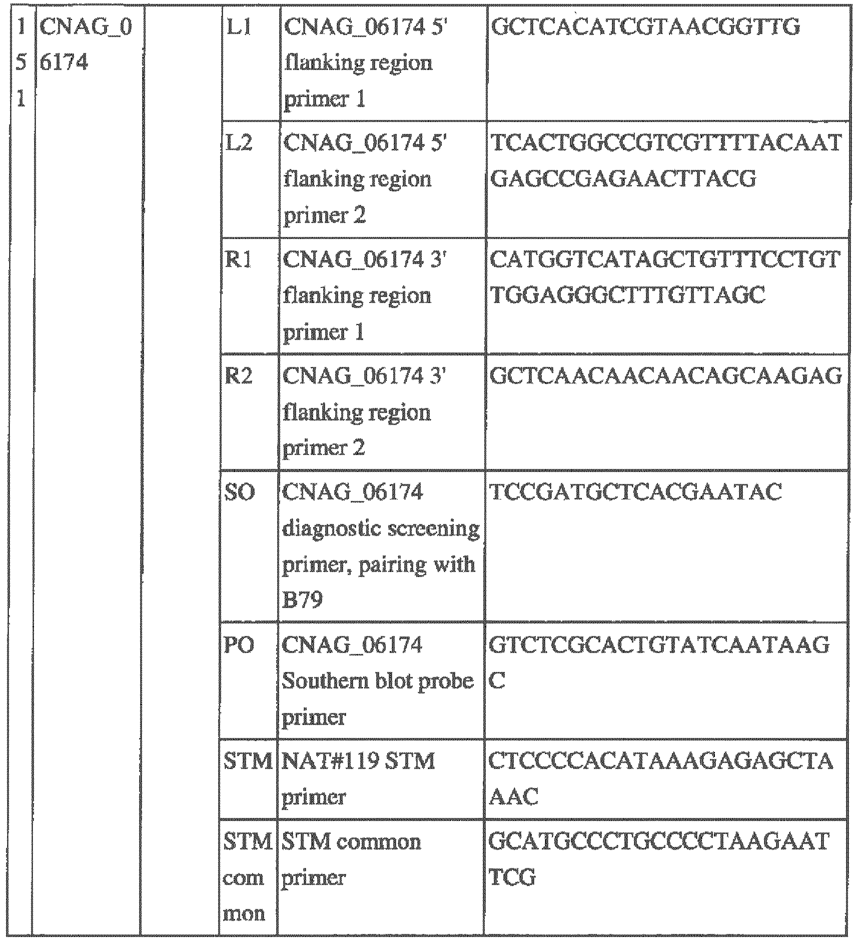

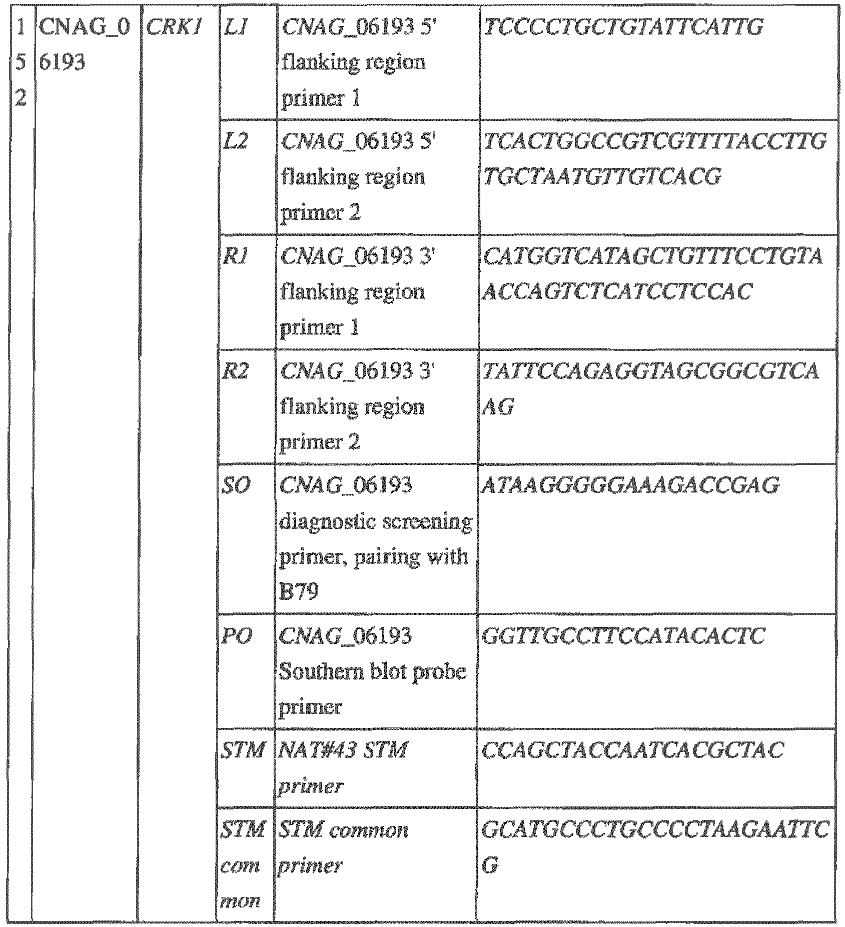

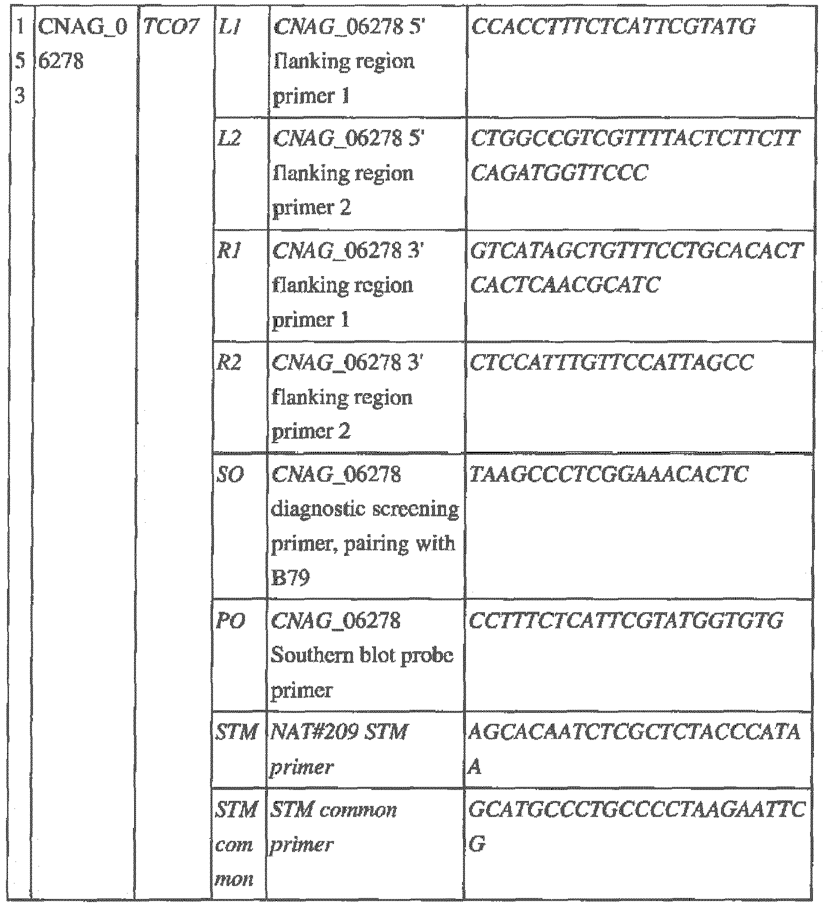

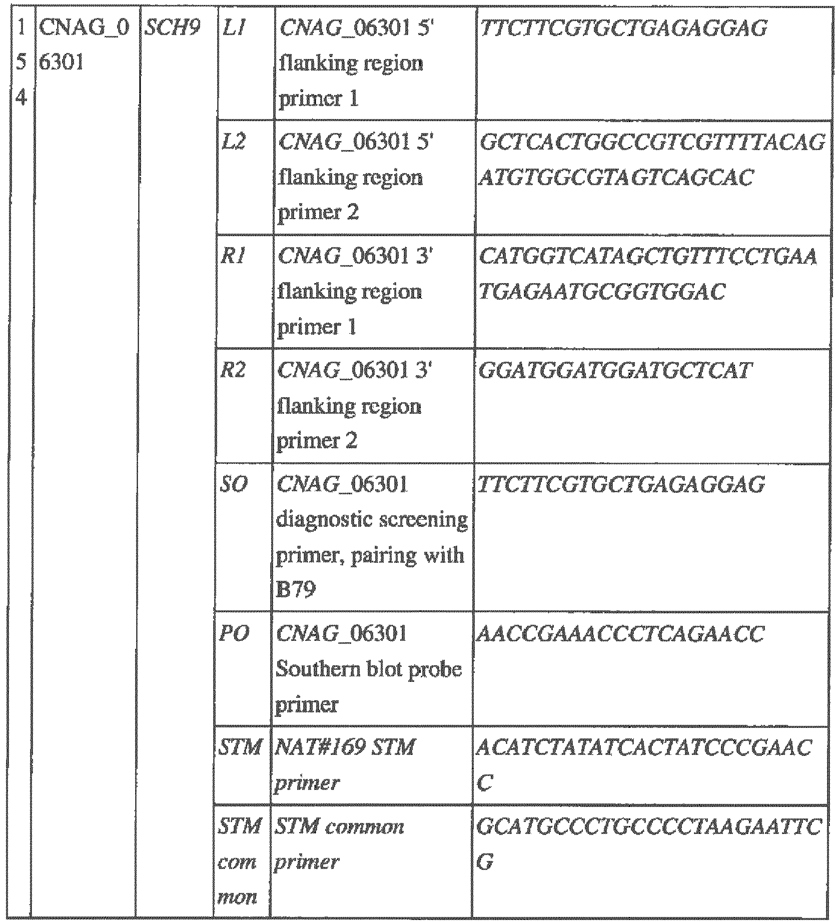

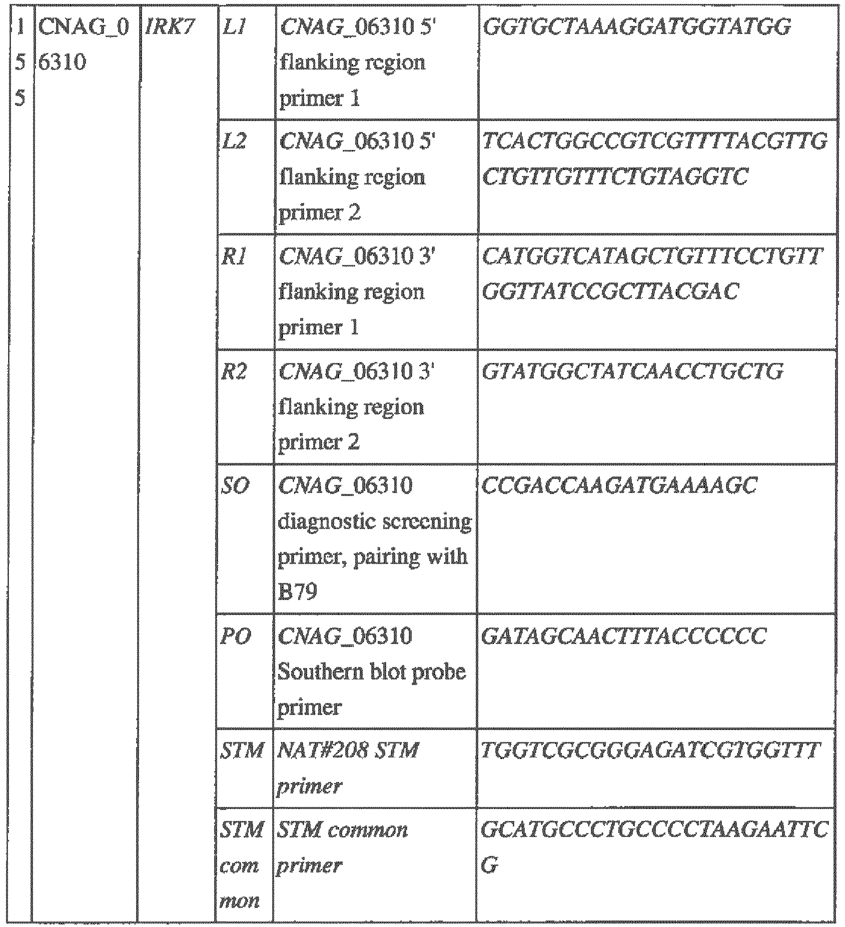

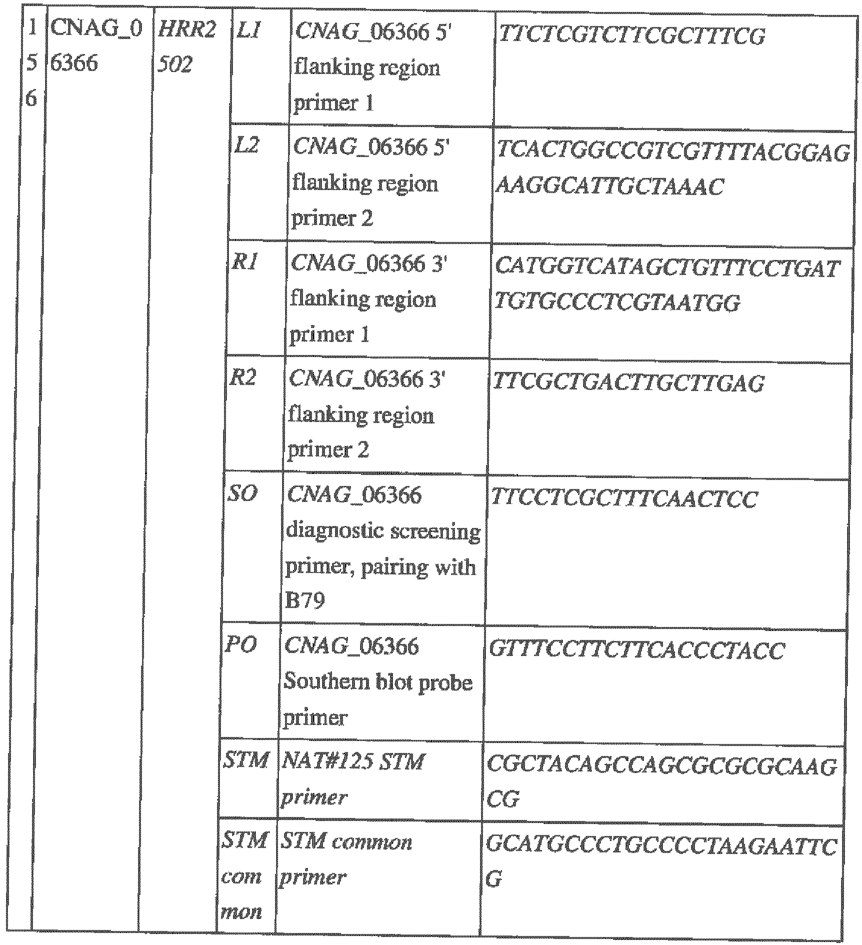

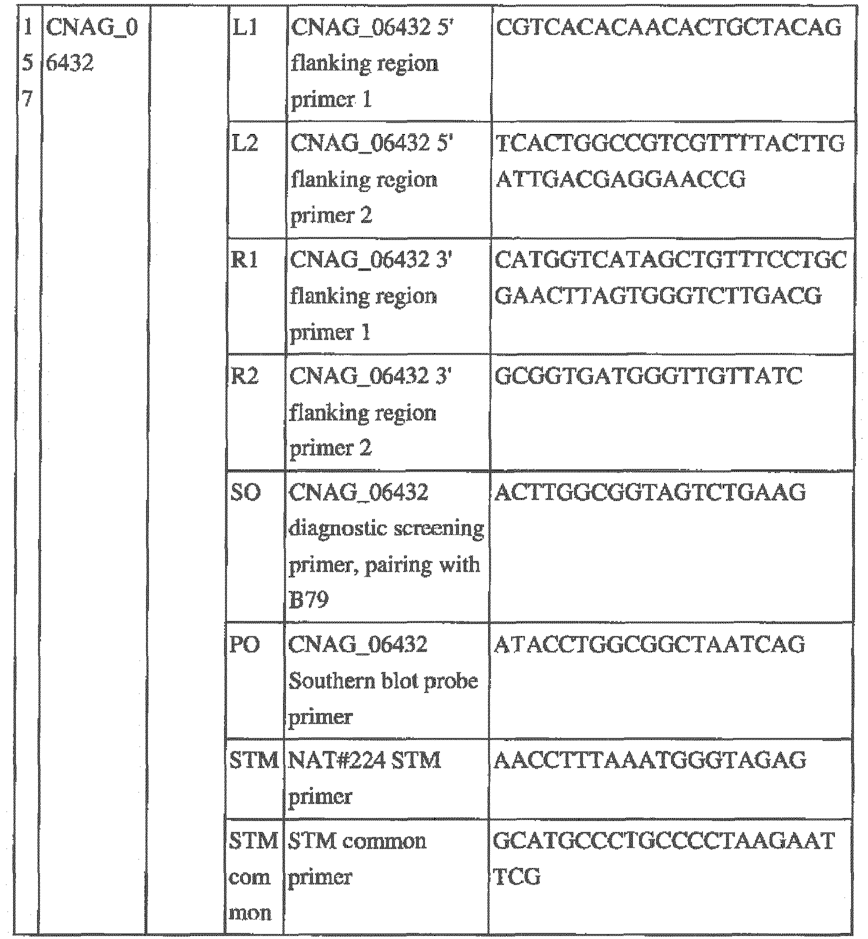

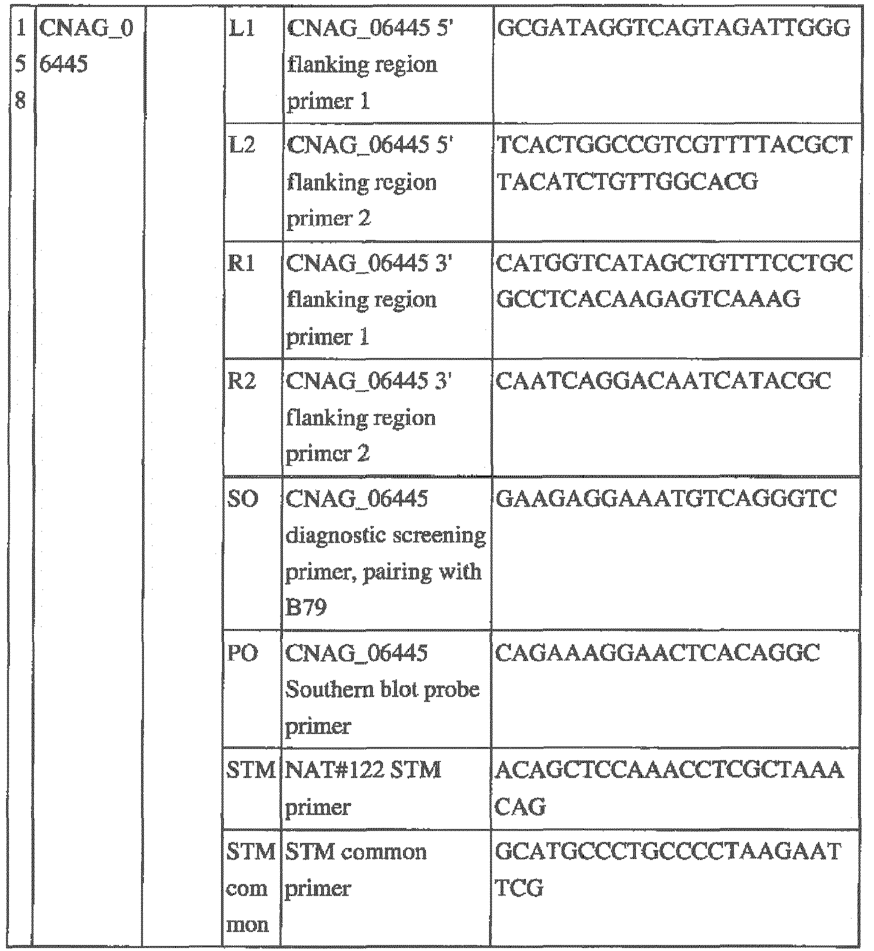

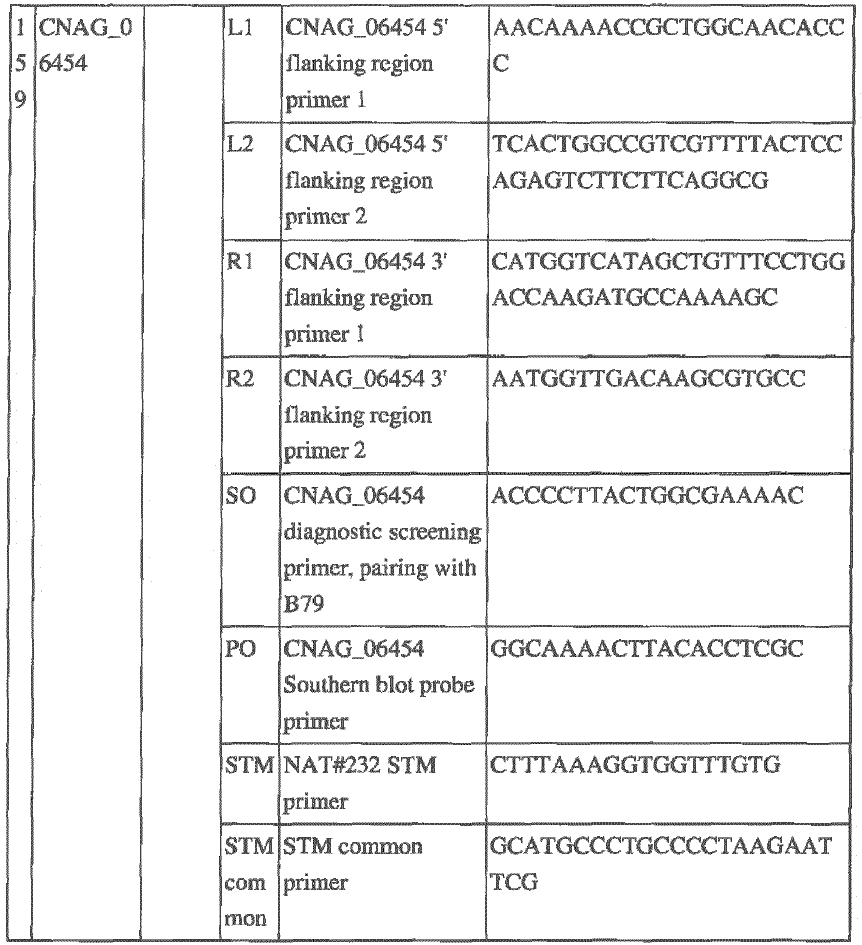

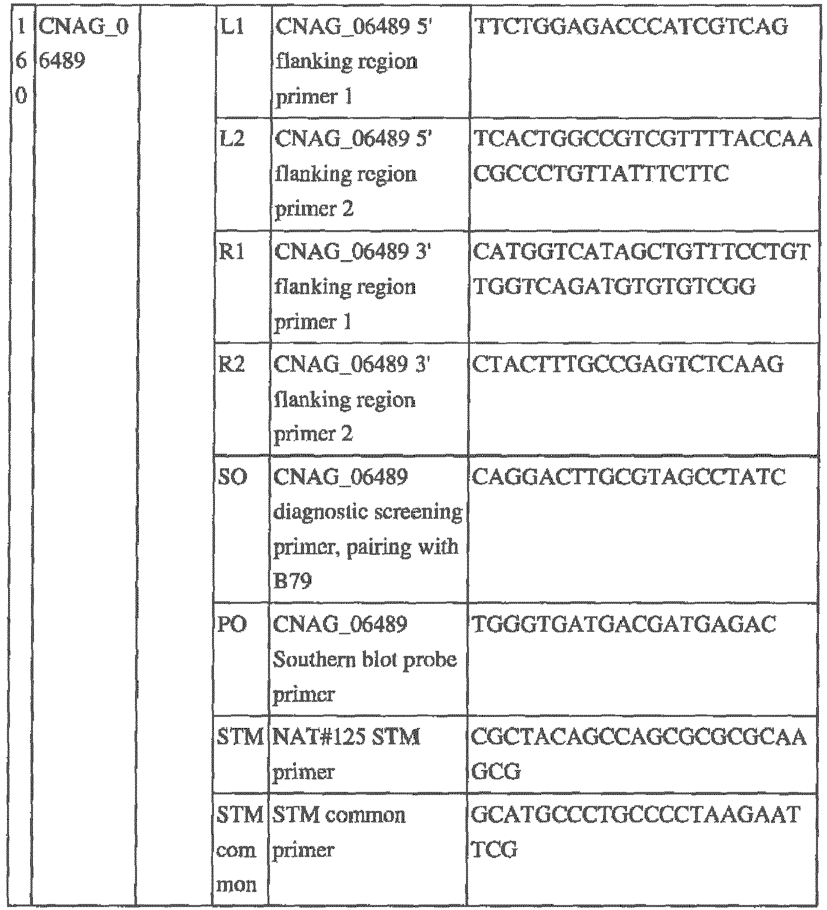

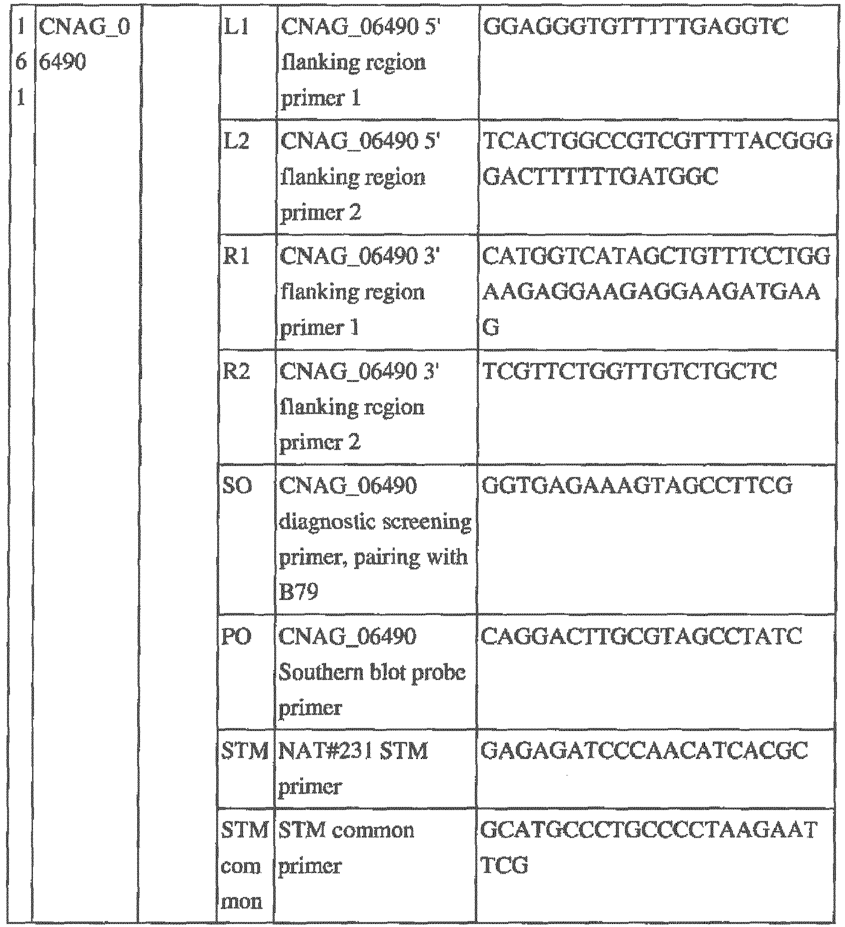

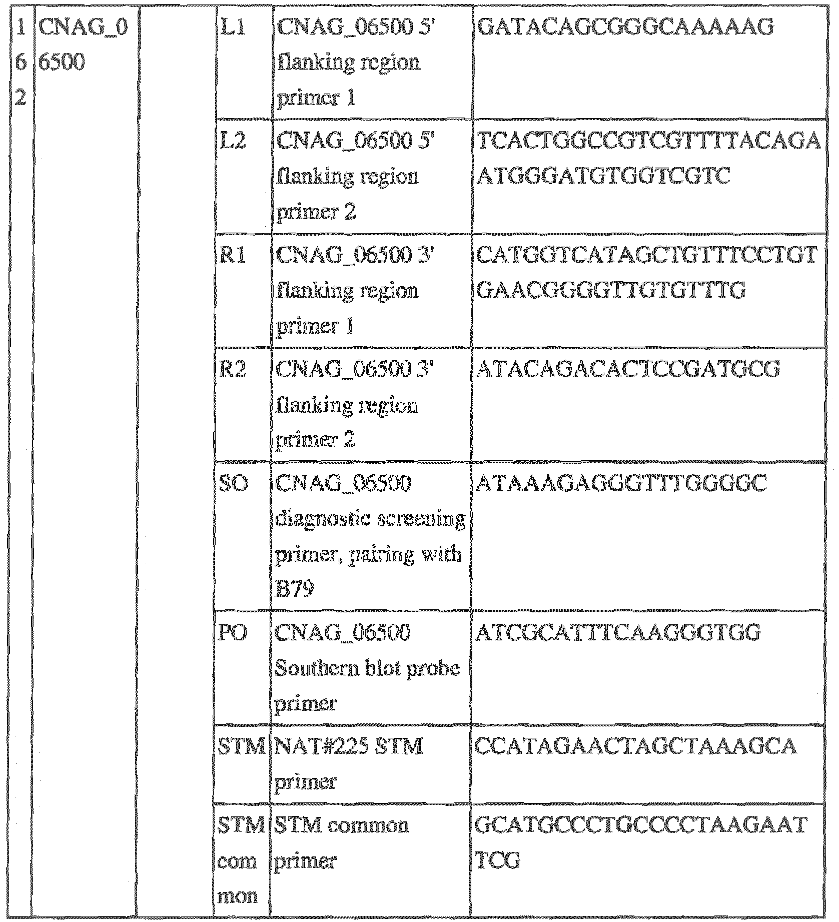

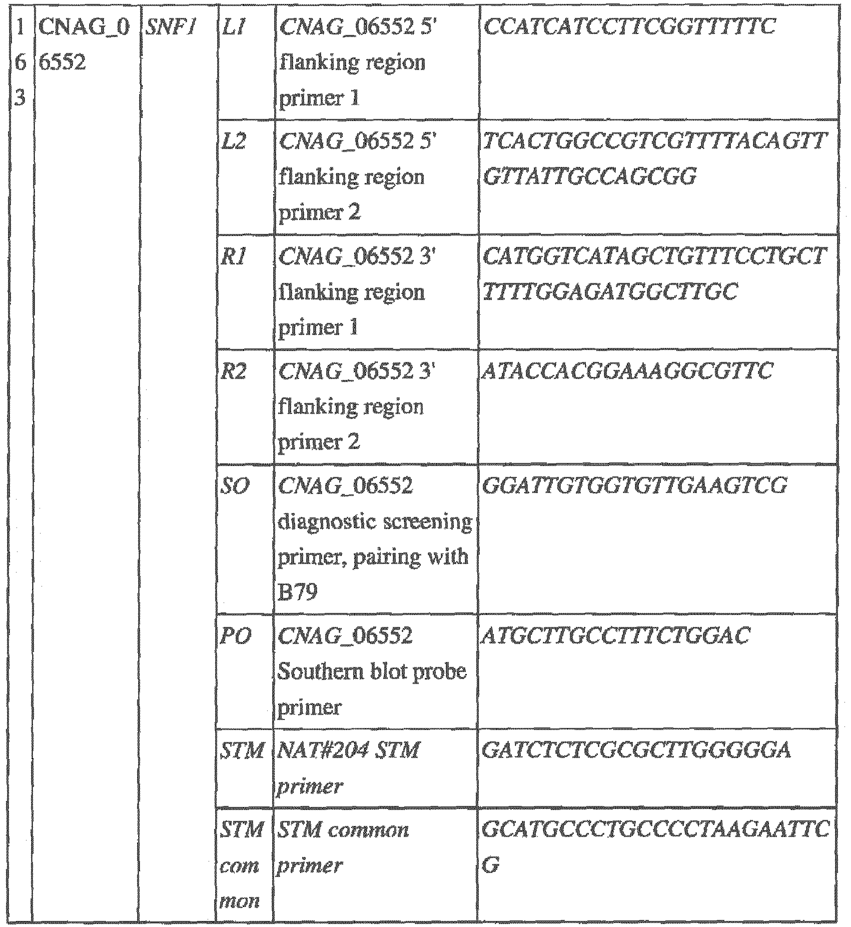

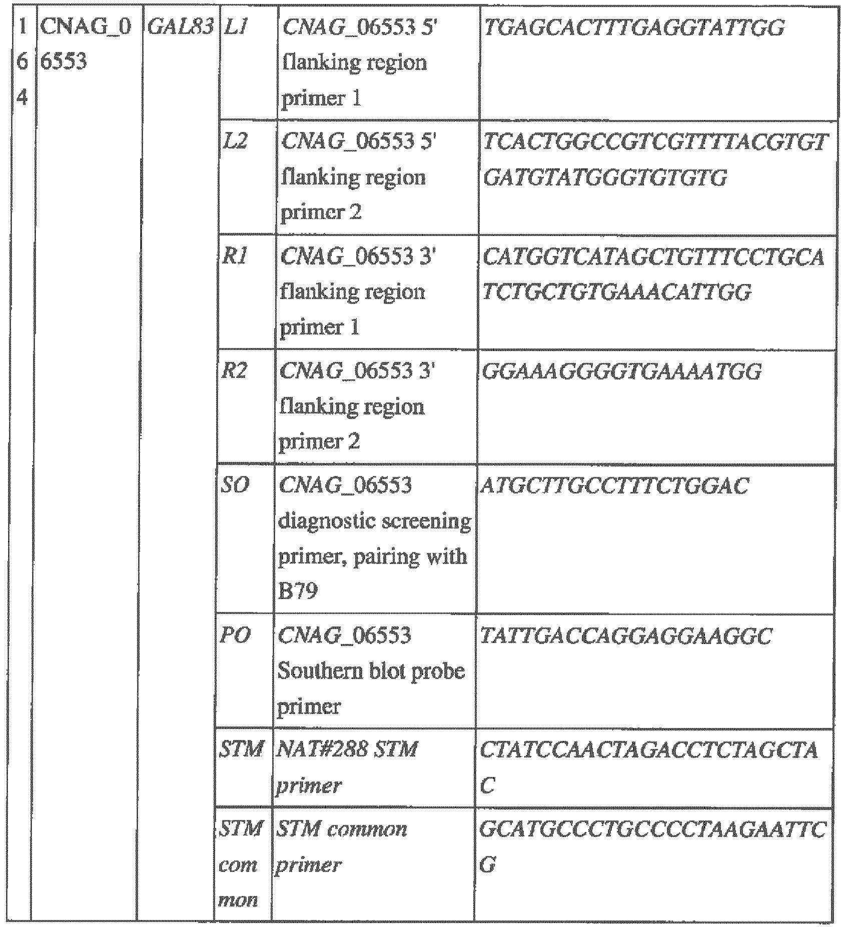

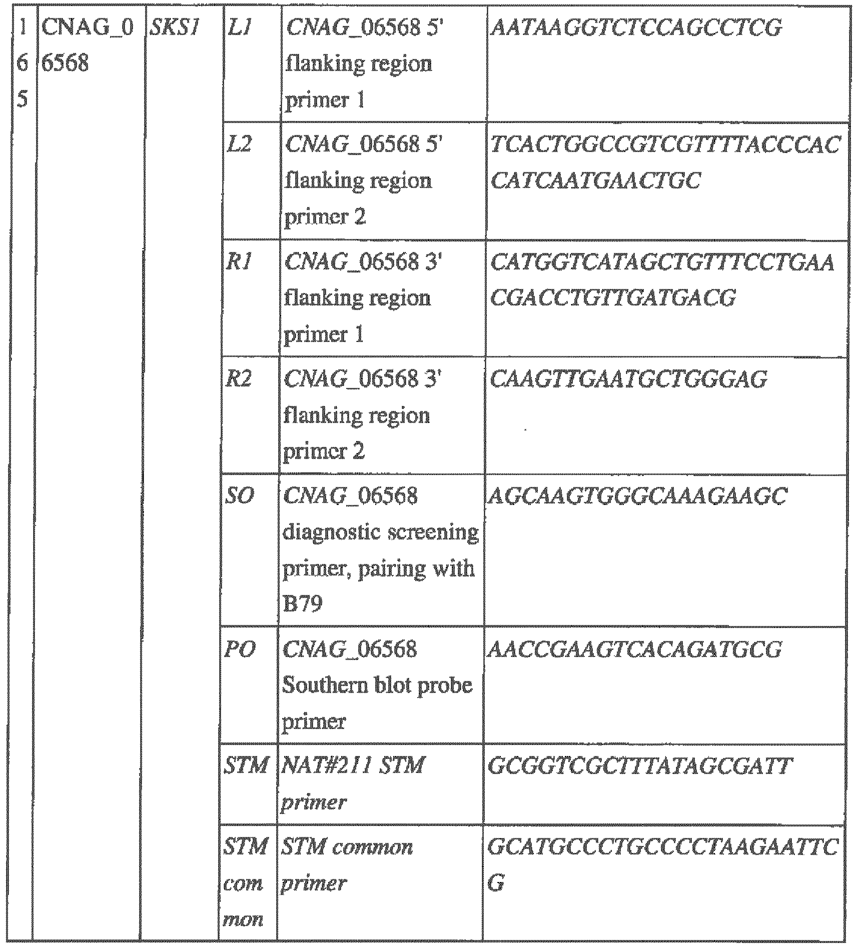

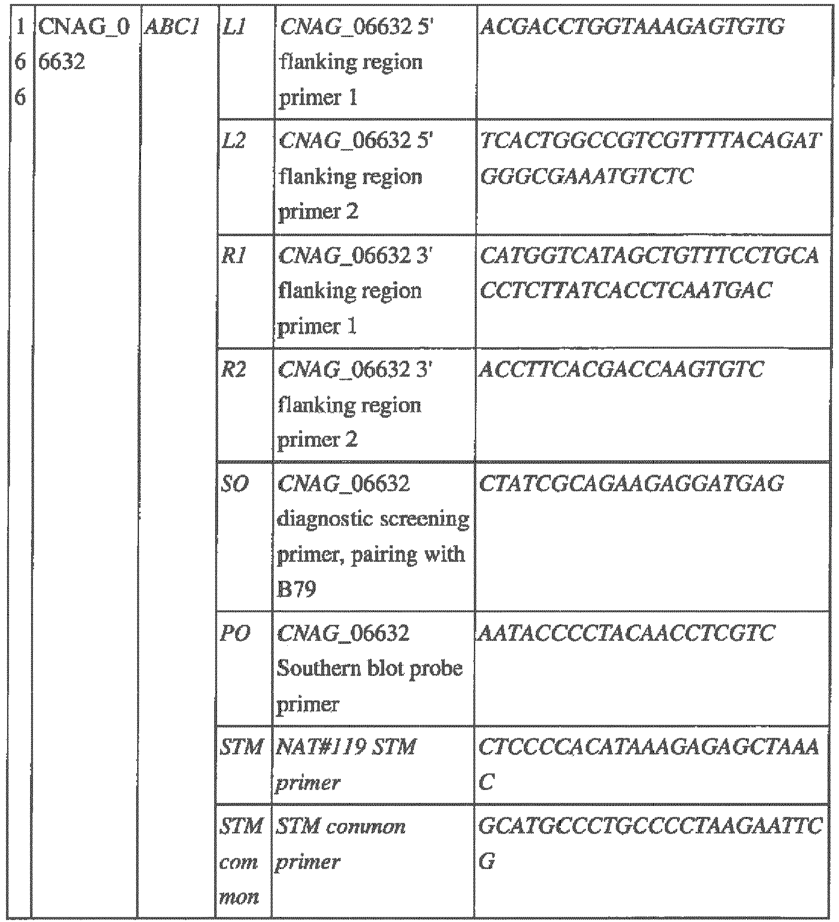

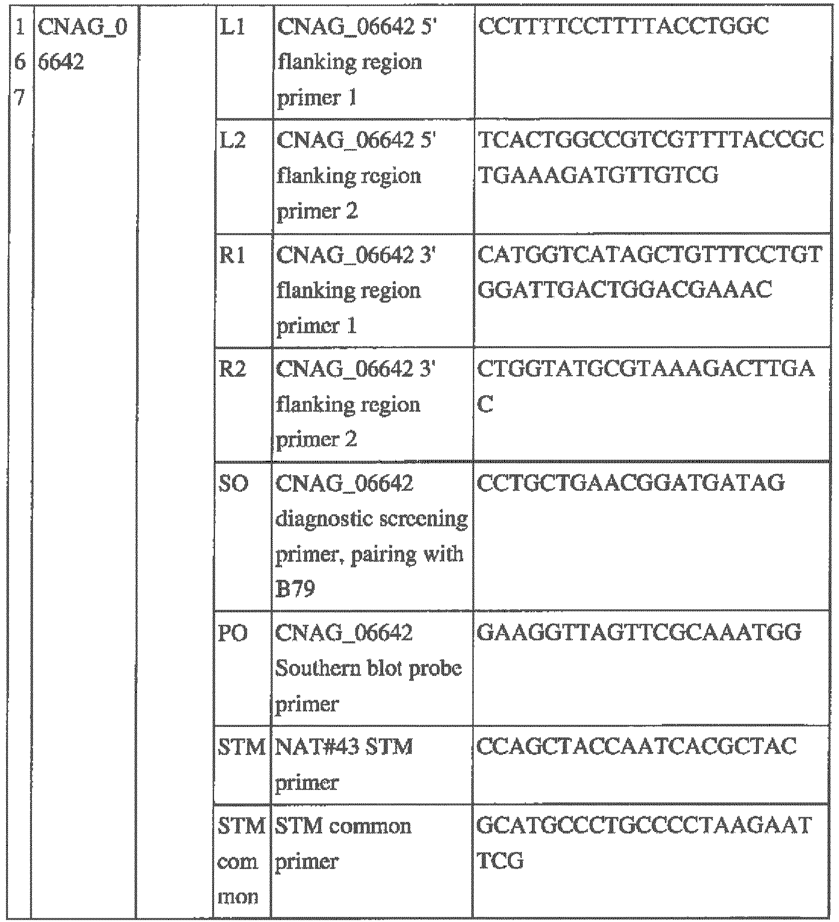

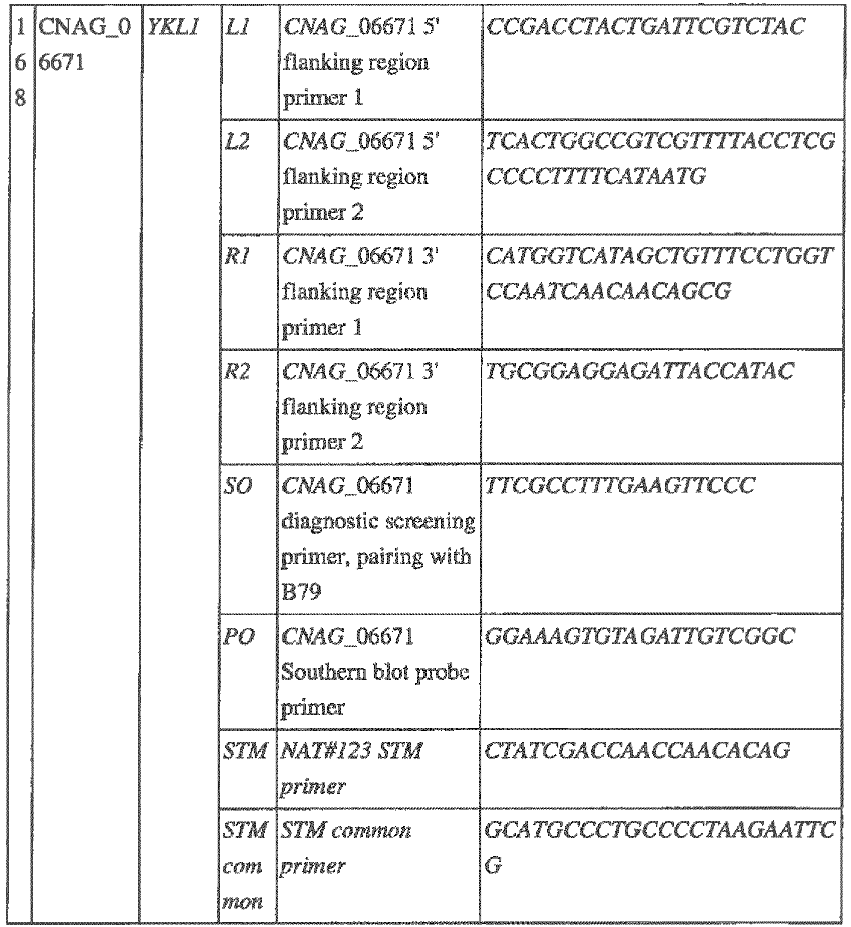

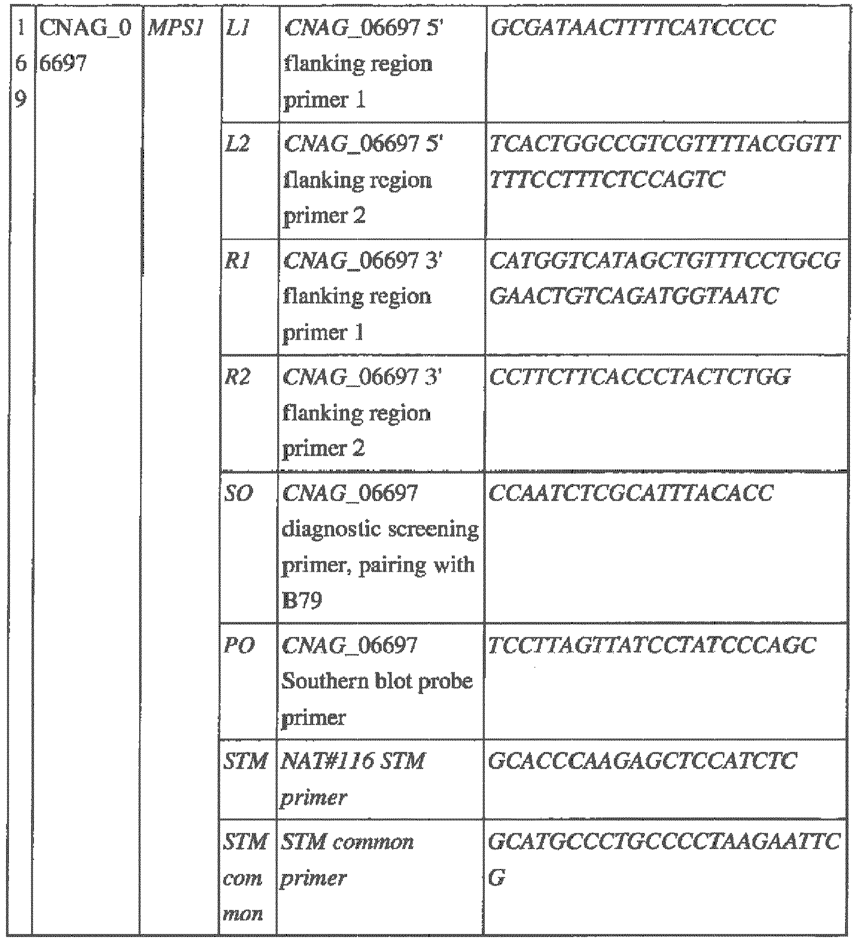

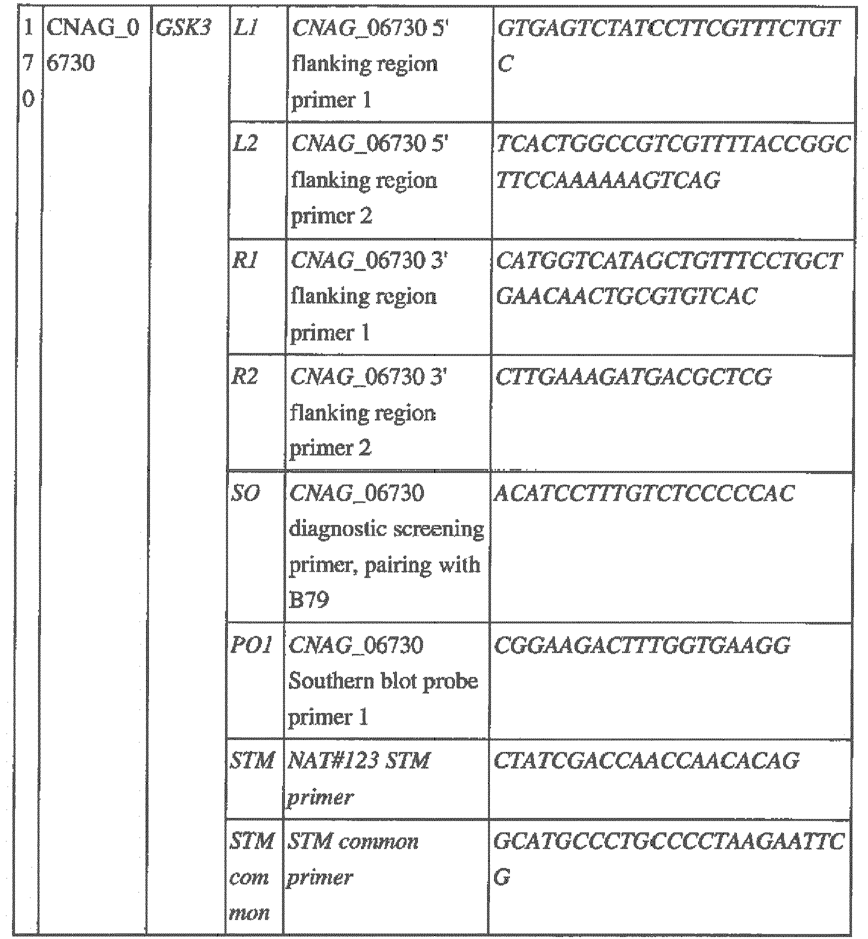

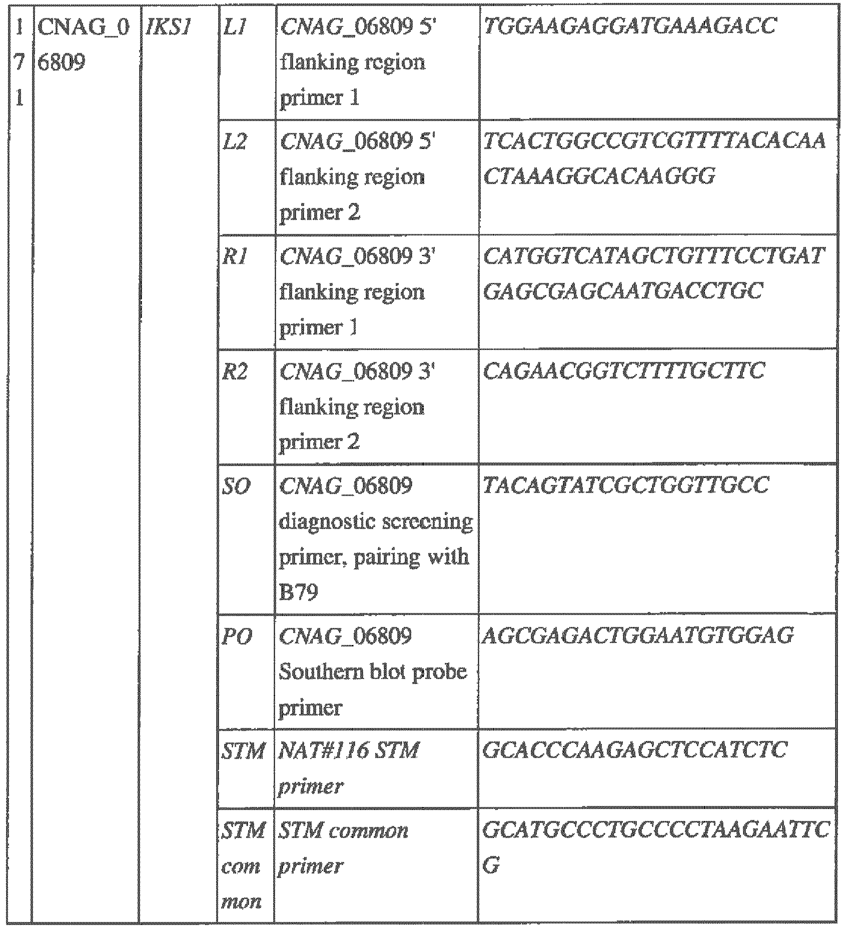

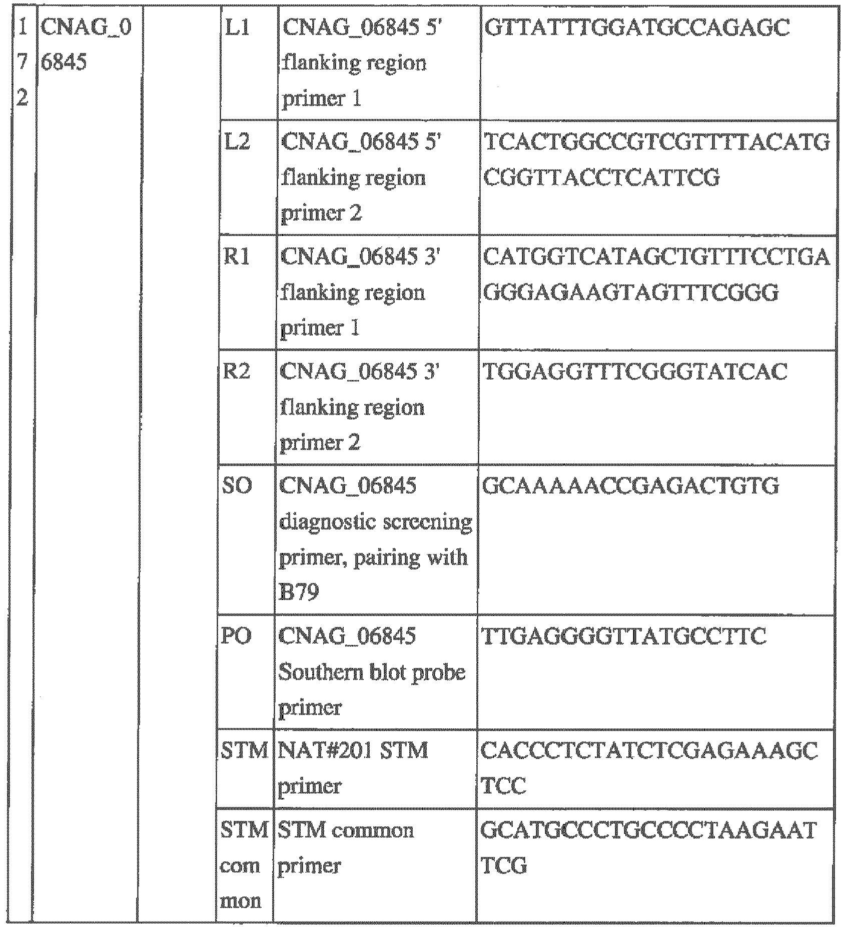

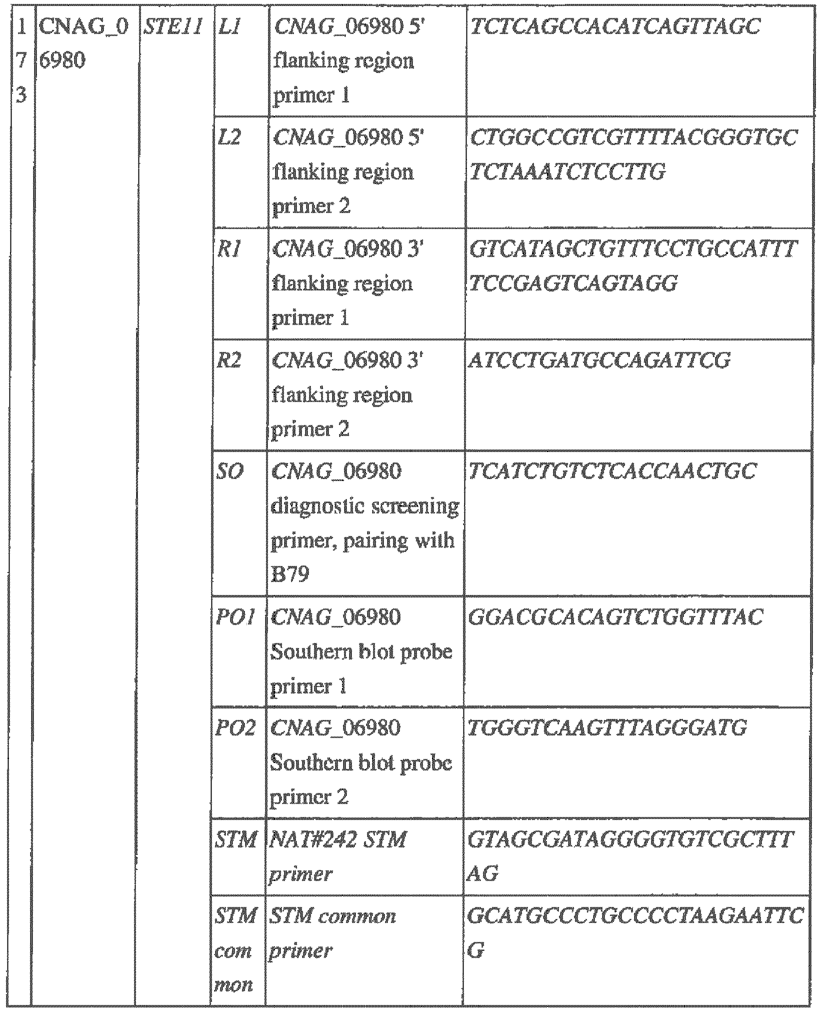

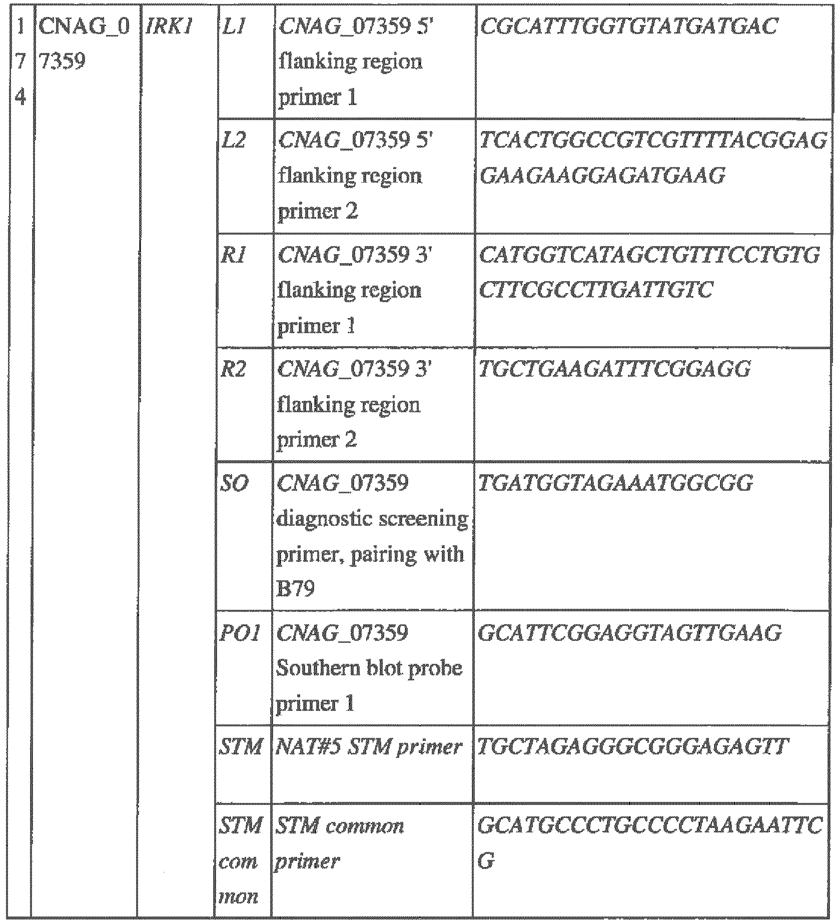

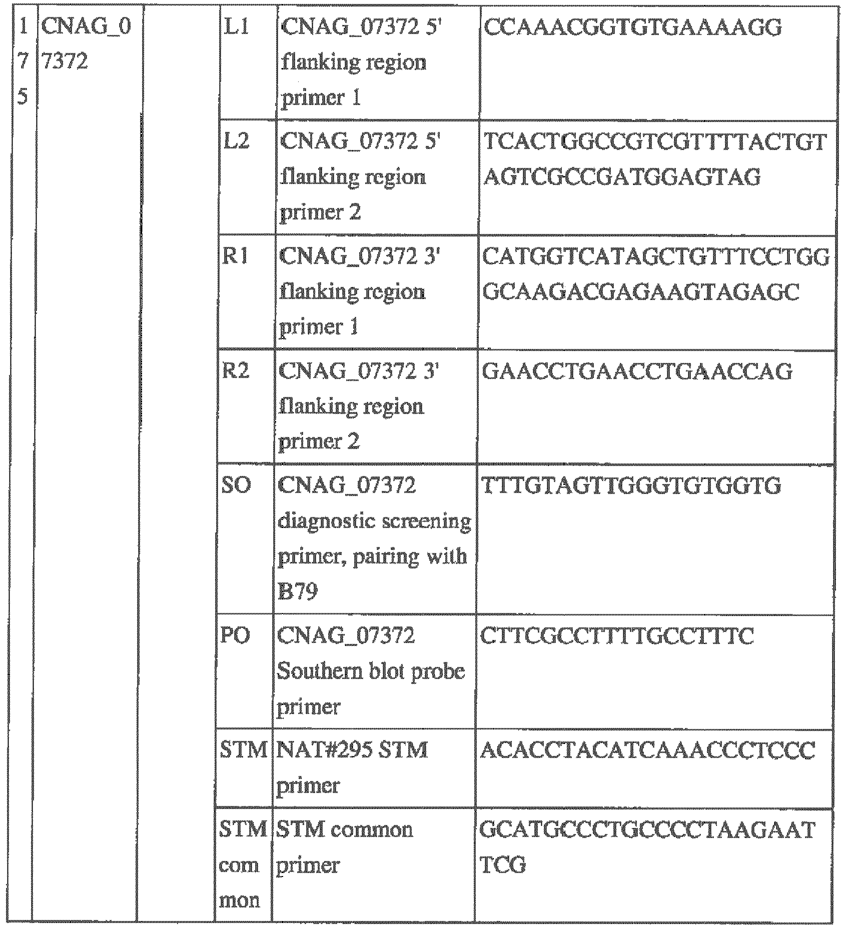

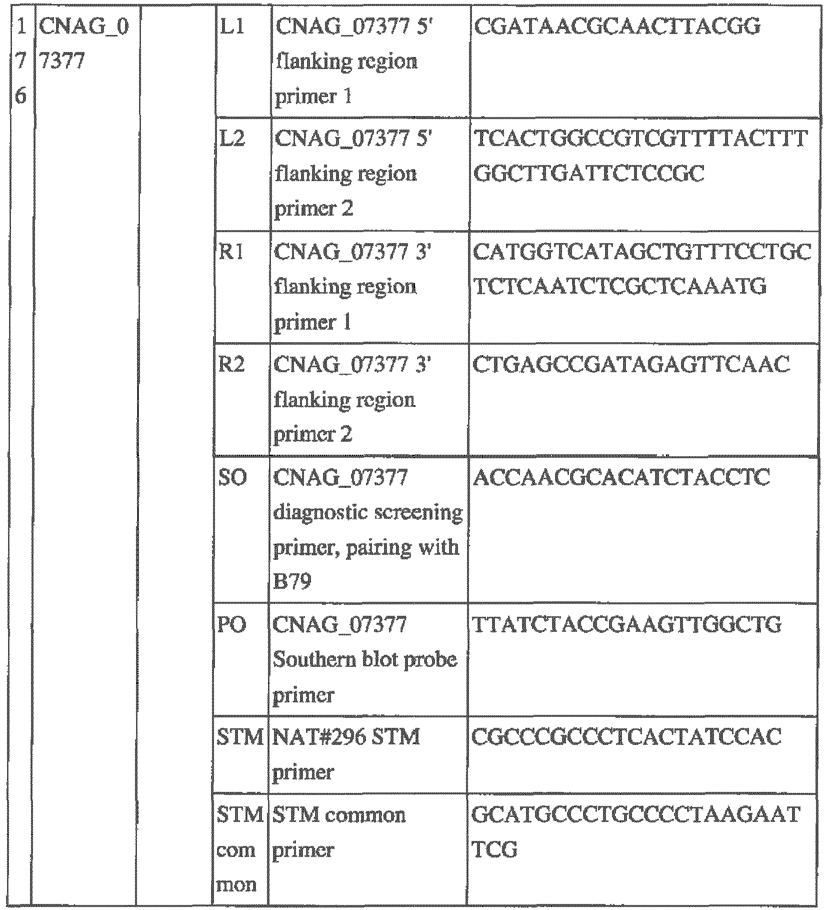

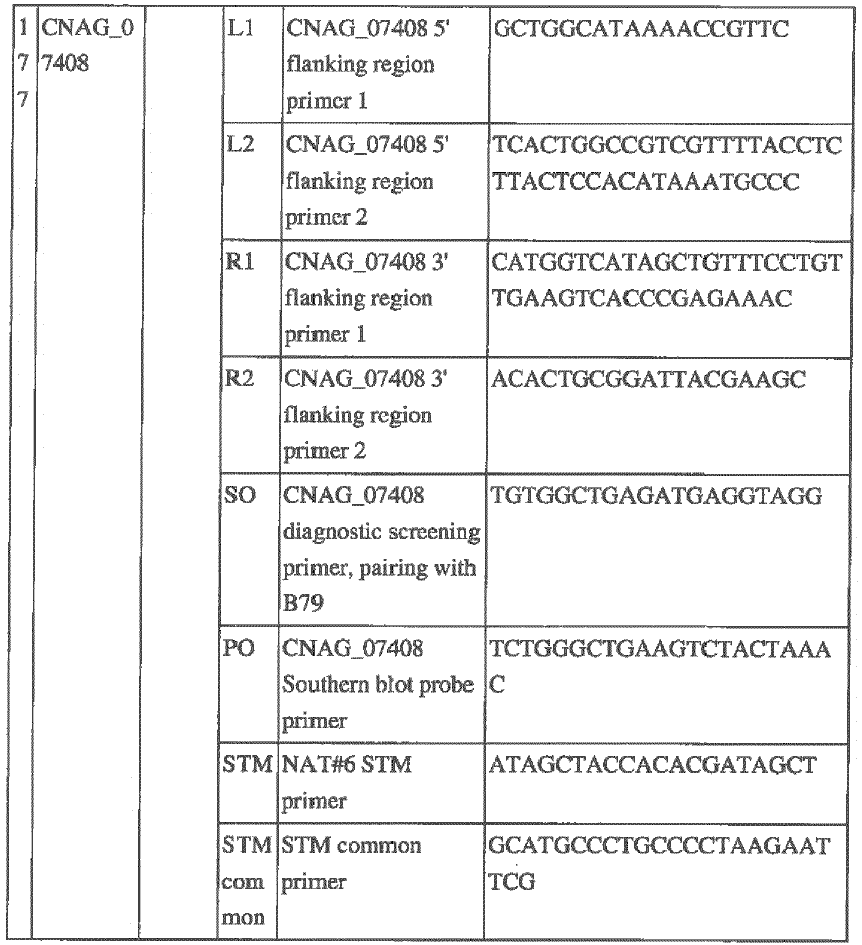

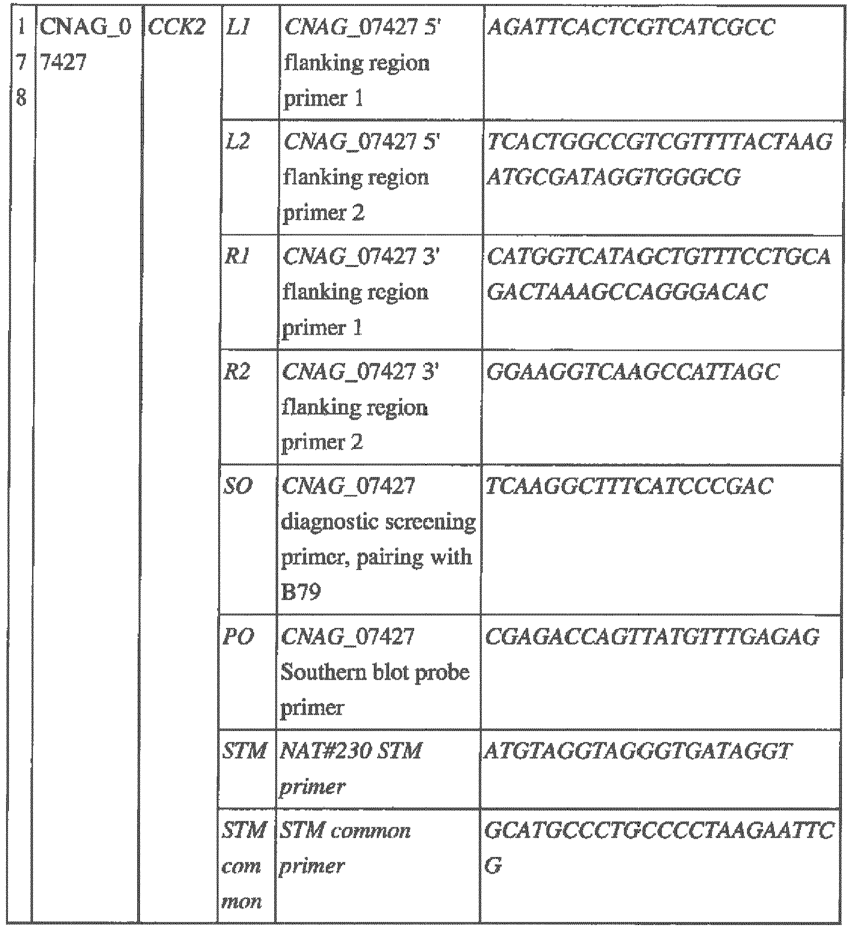

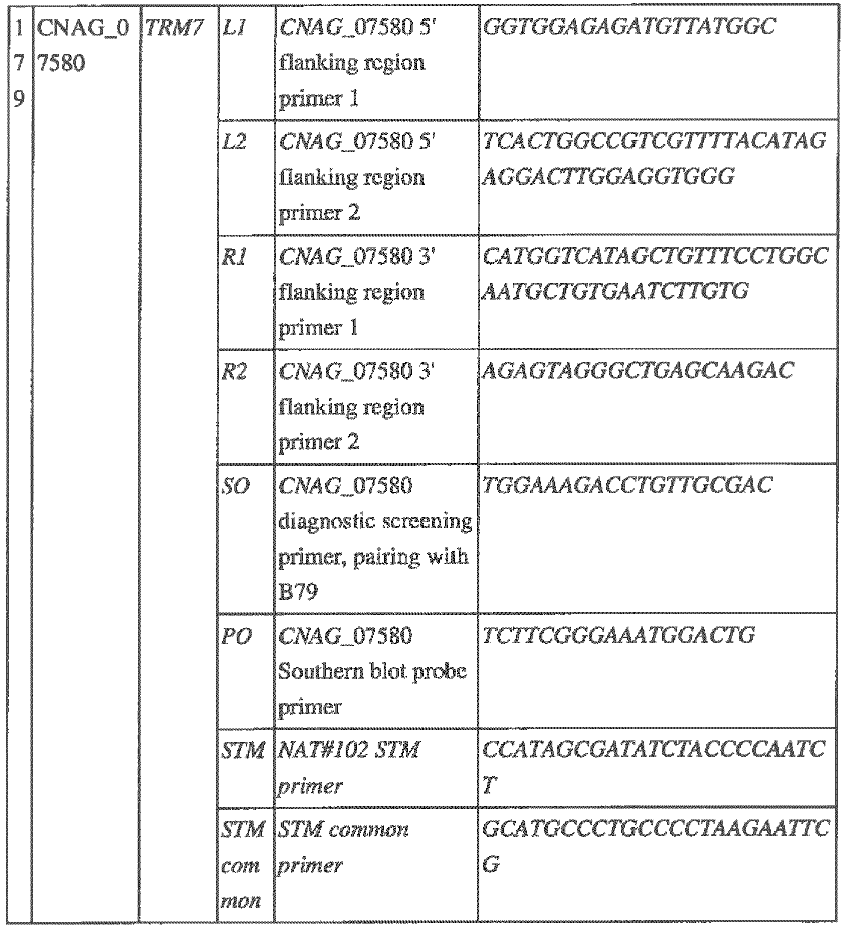

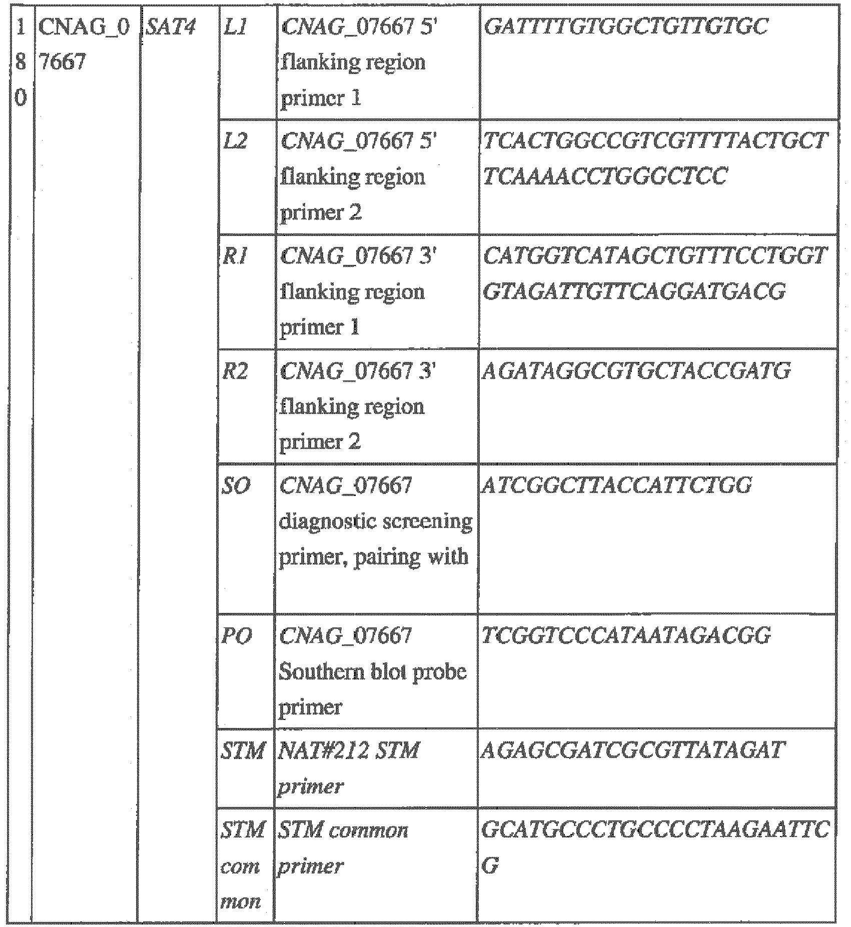

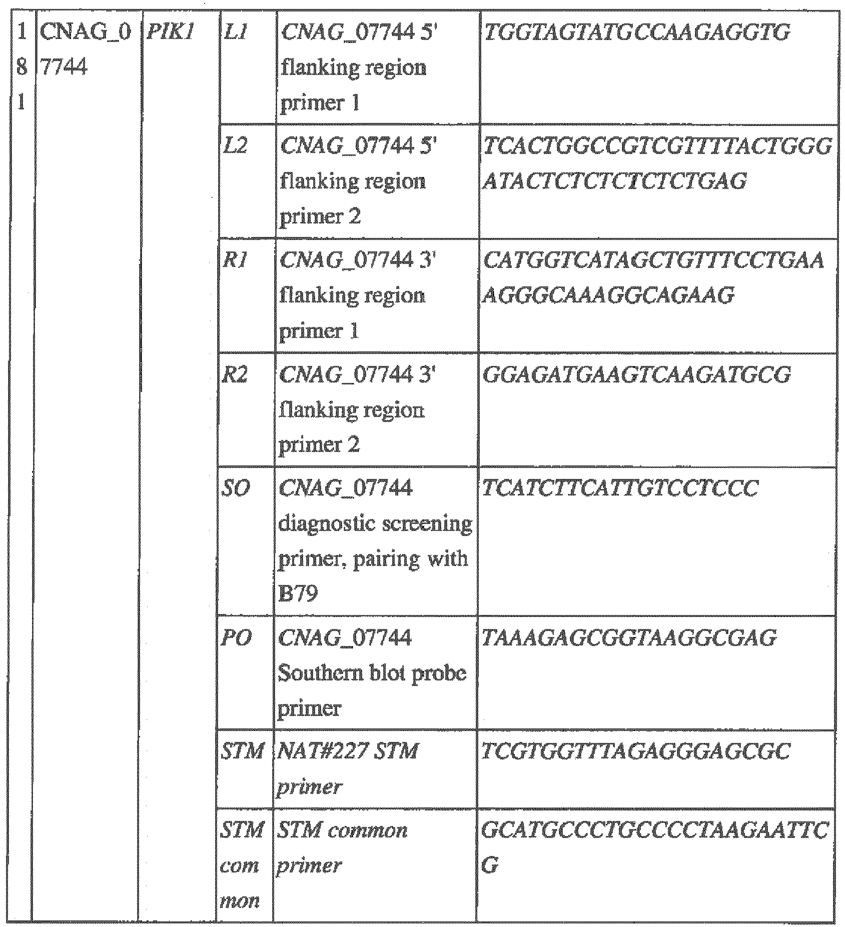

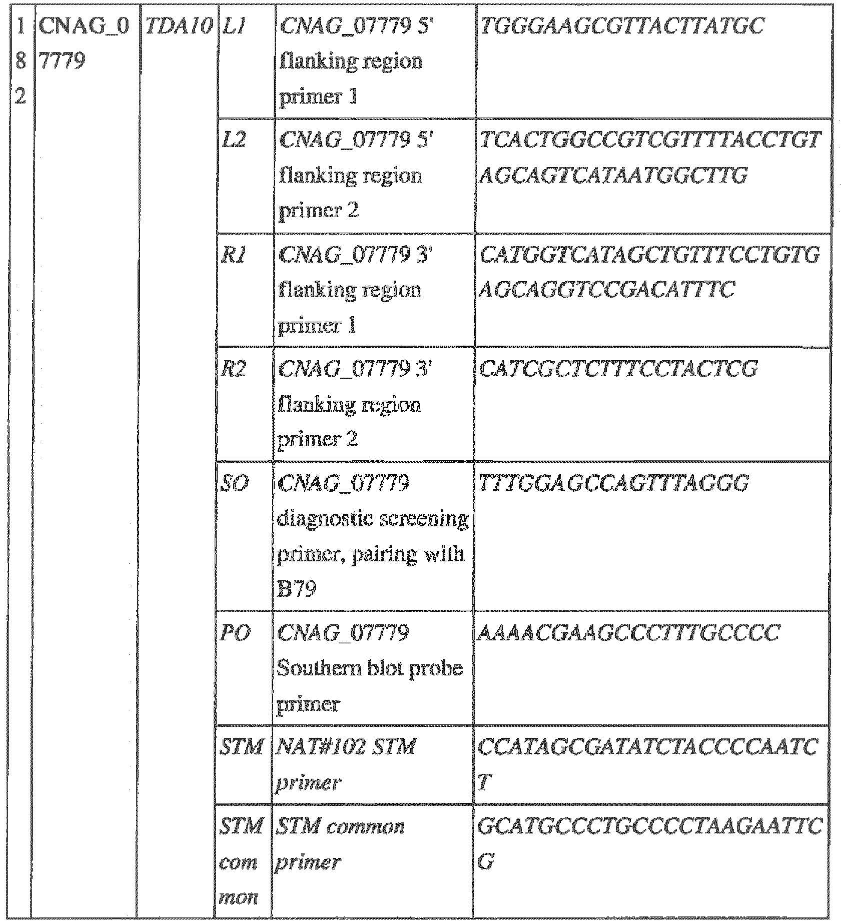

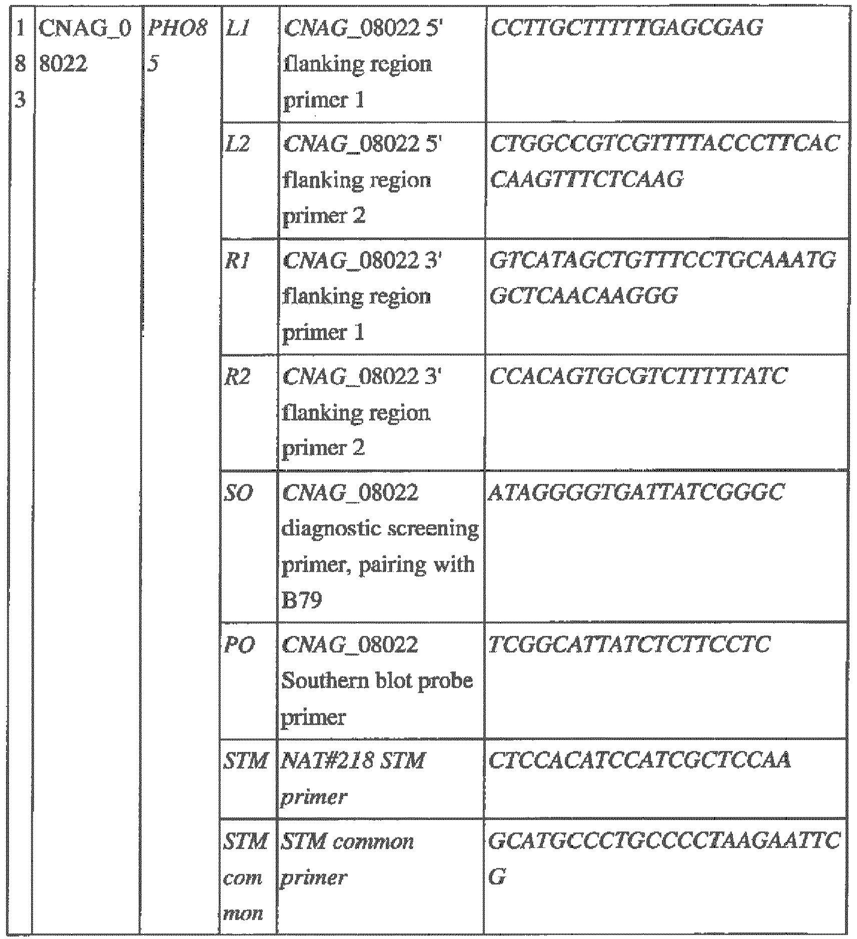

**[Table 3]**

| Primers used in the construction and functional characterization of kinase mutant library | | |
|---|---|---|
| Primer name | Primer description | Primer sequence (5'-3') |
| B1026 | M13 Forward extended | GTAAAACGACGGCCAGTGAGC |
| B1027 | M13 Reverse extended | CAGGAAACAGCTATGACCATG |
| B1454 | NAT split marker primer (NSR) | AAGGTGTTCCCCOACGACGAATCG |
| B1455 | NAT split marker primer (NSL) | AACTCCGTCGCGAGCCCCATCAAC |
| B1886 | NEO split marker primer (GSR) | TGGAAGAGATGGATGTGC |
| B1887 | NEO split marker primer (GSL) | ATTGTCTGTTGTGCCCAG |
| B4017 | Primer 1 for overexpression promoter with NEO marker | GCATGCAGGATTCGAGTG |
| B4018 | Primer 2 for overexpression promoter with NEO marker | GTGATAGATGTGTTGTGGTG |
| B678 | Northern probe primer1 for *ERG11* | TTCAGGGAACTTGGGAACAGC |
| B1598 | Northern probe primer2 for *ERG11* | CAGGAGCAGAAACAAAGC |
| B3294 | Northern probe primer1 for *ACT1* | GCACCATACCTTCTACAATGAG |
| B3295 | Northern probe primer2 for *ACT1* | ACTTTCGGTGGACGATTG |
| B5251 | RT-PCR primer for *HXL1* of H99 | CACTCCATTCCTTTCTGC |
| B5252 | RT-PCR primer for *HXL1* of H99 | CGTAACTCCACTGTGTCC |
| B7030 | qRT-PCR primer for *CNA1* of H99 | AGACTGTTTACAATGCCTGC |
| B7031 | qRT-PCR primer for *CNA1* of H99 | TCTGGCGACAAGCCACCATG |
| B7032 | qRT-PCR primer for *CNB1* of H99 | AAGATGGAAGTGGAACGG |
| B7033 | qRT-PCR primer for *CNB1* of H99 | TTGAAAGCGAATCTCAGCTT |
| B7034 | qRT-PCR primer for *CRZ1* of H99 | ACCACGGACATTATCTTCAG |
| B7035 | qRT-PCR primer for *CRZ1* of H99 | AGCCCAGCCTTGCTGTTCGT |
| B7036 | qRT-PCR primer for *UTR2* of H99 | TTTCTATGCCCATCTACAGC |
| B7037 | qRT-PCR primer for *UTR2* of H99 | CTTCGTGGGAGTACAGTGGC |
| B679 | qRT-PCR primer for *ACT1* of H99 | CGCCCTTGCTCCTTCTTCTATG |
| B680 | qRT-PCR primer for *ACT1* of H99 | GACTCGTCGTATTCGCTCTTCG |

### Example 3: Systematic Phenotypic Profiling and Clustering of Cryptococcus neoformans kinom network

With the kinase mutant library constructed in the above Example, the present inventors performed a series of *in vitro* phenotypic analyses (a total of 30 phenotypic traits) under distinct growth conditions covering six major phenotypic classes (growth, differentiation, stress responses and adaptations, antifungal drug resistance and production of virulence factors), thereby making more than 6,600 phenotype data. Such comprehensive kinase phenome data are freely accessible to the public through the *Cryptococcus neoformans* kinome database (http://kinase.cryptococcus.org). To gain insights into the functional and regulatory connectivity among kinases, the present inventors attempted to group kinases by phenotypic clustering through Pearson correlation analysis (see FIG. 3). The rationale behind this analysis was that a group of kinases in a given signaling pathway tended to cluster together in terms of shared phenotypic traits. For example, mutants in three-tier mitogen-activated protein kinase (MAPK) cascades should cluster together because they exhibit almost identical phenotypic traits. In fact, the present inventors found that the three-tier kinase mutants in the cell wall integrity MAPK (*bck1Δ, mkk1Δ, mpk1Δ*), the high osmolarity glycerol response (HOG) MAPK (*ssk2Δ, pbs2Δ, hog1Δ*), and the pheromone-responsive MAPK (*ste11Δ, ste7Δ, cpk1Δ*) pathways were clustered together based on their shared functions (FIG. 4). Therefore, groups of kinases clustered together by this analysis are highly likely to function in the same or related signaling cascades. The present inventors identified several hitherto uncharacterized kinases that are functionally correlated with these known signaling pathways. First, the present inventors identified CNAG_06553, encoding a protein orthologous to yeast Gal83 that is one of three possible β-subunits of the Snfl kinase complex in *S. cerevisiae.* The yeast Snfl kinase complex consists of Snf1, catalytic α-subunit, Snf4, regulatory γ subunit, and one of three possible β-subunits (Gal83, Sip1 and Sip2), and controls the transcriptional changes under glucose derepression (Jiang, R. & Carlson, M. The Snfl protein kinase and its activating subunit, Snf4, interact with distinct domains of the Sipl/Sip2/Gal83 component in the kinase complex. Mol Cell Biol 17, 2099-2106, 1997; Schuller, H. J. Transcriptional control of nonfermentative metabolism in the yeast Saccharomyces cerevisiae. Curr Genet 43, 139-160, doi:10.1007/s00294-003-0381-8, 2003). In *C. neoformans,* Snf1 functions have been previously characterized (Hu, G., Cheng, P. Y., Sham, A., Perfect, J. R. & Kronstad, J. W. Metabolic adaptation in Cryptococcus neoformans during early murine pulmonary infection. Molecular microbiology 69, 1456-1475, doi:10.1111/j.1365-2958.2008.06374.x, 2008). Several lines of experimental evidence showed that Gal83 is likely to function in association with Snf1 in *C. neoformans.* First, the *in vitro* phenotypic traits of the *gal83*Δ mutant were almost equivalent to those of the *snf1Δ* mutant (FIG. 3). Both *snf1Δ* and *gal83Δ* mutants exhibited increased susceptibility to fludioxonil and increased resistance to organic peroxide (tert-butyl hydroperoxide). Second, growth defects in the *snf1Δ* mutant in alternative carbon sources (for example, potassium acetate, sodium acetate and ethanol) were also observed in *gal83Δ* mutants (FIG. 4). Therefore, Gal83 is likely to be one of the possible β-subunits of the Snf1 kinase complex in *C. neoformans.*

The present inventors also identified several kinases that potentially work upstream or downstream of the TOR kinase complex. Although the present inventors were not able to disrupt Tor1 kinase, which has been suggested to be essential in *C. neoformans*, the present inventors found three kinases (Ipk1, Ypk1 and Gsk3 found to be clustered in most eukaryotes) that are potentially related to Tor1-dependent signaling cascades clustered in *C. neoformans.* Recently, Lev et *al*. proposed that Ipk1 could be involved in the production of inositol hexaphosphate (IP₆) based on its limited sequence homology to *S. cerevisiae* Ipk1 (Lev, S. et al. Fungal Inositol Pyrophosphate IP7 Is Crucial for Metabolic Adaptation to the Host Environment and Pathogenicity. MBio 6, e00531-00515, doi:10.1128/mBio.00531-15 (2015)). In mammals, inositol polyphosphate multikinase (IPMK), identified as Arg82 in yeast, produces IP₆, a precursor of 5-IP₇ that inhibits Akt activity and thereby decreases mTORC1-mediated protein translation and increases GSK3 -mediated glucose homeostasis, adipogenesis, and activity (Chakraborty, A., Kim, S. & Snyder, S. H. Inositol pyrophosphates as mammalian cell signals. Sci Signal 4, rel, doi:10.1126/scisignal.2001958 (2011)). It was reported that in *S. cerevisiae,* Ypk1 is the direct target of TORC2 by promoting autophagy during amino acid starvation (Vlahakis, A. & Powers, T. A role for TOR complex 2 signaling in promoting autophagy. Autophagy 10, 2085-2086, doi:10.4161/auto.36262 (2014)). In *C. neoformans, Ypk1,* which is a potential downstream target of Tor1, is involved in sphingolipid synthesis and deletion of *YPK1* resulted in a significant reduction in virulence (Lee, H., Khanal Lamichhane, A., Garraffo, H. M., Kwon-Chung, K. J. & Chang, Y. C. Involvement of PDK1, PKC and TOR signalling pathways in basal fluconazole tolerance in Cryptococcus neoformans. Mol. Microbiol. 84, 130-146, doi:10.1111/j.1365-2958.2012.08016.x (2012)). Reflecting the essential role of Tor1, all of the mutants (*ipk1Δ*, *ypk1Δ,* and *gsk3*Δ) exhibited growth defects, particularly at high temperature.

However, there are two major limitations in this phenotypic clustering analysis. First, kinases that are oppositely regulated in the same pathway cannot be clustered. Second, a kinase that regulates a subset of phenotypes governed by a signaling pathway may not be clustered with its upstream kinases; this is the case of the Hog1-regulated kinase 1 (CNAG_00130; Hrk1). Although the present inventors previously demonstrated that Hrk1 is regulated by Hog1, Hrk1 and Hog1 are not clustered together as Hrk1 regulates only subsets of Hog1-dependent phenotypes. Phospholipid flippase kinase 1 (Fpk1) is another example. In *S. cerevisiae,* the activity of Fpk1 is inhibited by direct phosphorylation by Ypk1. As expected, Fpk1 and Ypk1 were clustered together. To examine whether Fpk1 regulates Ypk1-dependent phenotypic traits in C. *neoformans,* the present inventors performed epistatic analyses by constructing and analyzing FPK1 overexpression strains constructed in the *ypk1*Δ and wild-type strain backgrounds. As expected, overexpression of *FPK1* partly restored normal growth, resistance to some stresses (osmotic, oxidative, genotoxic, and cell wall/membrane stresses) and antifungal drug (amphotericin B) in *ypk1Δ* mutants (FIG. 5). However, azole susceptibility of *ypk1*Δ mutants could not be restored by *FPK1* overexpression (see FIG. 5). These results suggest that Fpk1 could be one of the downstream targets of Ypk1 and may be positively regulated by Ypk1.

### Example 4: Pathogenic Kinome Networks in C. neoformans

To identify pathogenicity-regulating kinases which are controlled by both infectivity and virulence, the present inventors two large-scale in *vivo* animal studies: a wax moth-killing virulence assay and a signature-tagged mutagenesis (STM)-based murine infectivity assay. In the two assays, two independent mutants for each of kinases, excluding kinases with single mutants, were monitored. As a result, 31 virulence-regulating kinases in the insect killing assay (FIGS. 6 and 7) and 54 infectivity-regulating kinases in the STM-based murine infectivity assay were found (FIGS. 9 and 10). Among these kinases, 25 kinases were co-identified by both assays (FIG. 11a), indicating that virulence in the insect host and infectivity in the murine host are closely related to each other as reported previously (Jung, K. W. et al. Systematic functional profiling of transcription factor networks in Cryptococcus neoformans. Nat Comms 6, 6757, doi:10.1038/ncomms7757, 2015). Only 6 kinase mutants were identified by the insect killing assay (FIG. 11b). The present inventors discovered a total of 60 kinase mutants involved in the pathogenicity of C. neoformans.

Additionally, a large number of known virulence-regulating kinases (a total of 15 kinases) were rediscovered in the present invention (kinases indicated in black in FIG. 11a). These kinases include Mpk1 MAPK (Gerik, K. J., Bhimireddy, S. R., Ryerse, J. S., Specht, C. A. & Lodge, J. K. PKC1 is essential for protection against both oxidative and nitrosative stresses, cell integrity, and normal manifestation of virulence factors in the pathogenic fungus Cryptococcus neoformans. Eukaryot. Cell 7, 1685-1698, 2008; Kraus, P. R., Fox, D. S., Cox, G. M. & Heitman, J. The Cryptococcus neoformans MAP kinase Mpk1 regulates cell integrity in response to antifungal drugs and loss of calcineurin function. Mol. Microbiol. 48, 1377-1387, 2003); Ssk2 in the high osmolarity glycerol response (HOG) pathway (Bahn, Y. S., Geunes-Boyer, S. & Heitman, J. Ssk2 mitogen-activated protein kinase governs divergent patterns of the stress-activated Hog1 signaling pathway in Cryptococcus neoformans. Eukaryot. Cell 6, 2278-2289, 2007), an essential catalytic subunit (Pka1) of protein kinase A in the cAMP pathway (D'Souza, C. A. et al. Cyclic AMP-dependent protein kinase controls virulence of the fungal pathogen Cryptococcus neoformans. Mol. Cell. Biol. 21, 3179-3191, 2001); Ire1 kinase/endoribonuclease in the unfolded protein response (UPR) pathway (Cheon, S. A. et al. Unique evolution of the UPR pathway with a novel bZIP transcription factor, Hxll, for controlling pathogenicity of Cryptococcus neoformans. PLoS Pathog. 7, e1002177, doi:10.1371/journal.ppat.1002177, 2011); Ypk1 (Kim, H. et al. Network-assisted genetic dissection of pathogenicity and drug resistance in the opportunistic human pathogenic fungus Cryptococcus neoformans. Scientific reports 5, 8767, doi:10.1038/srep08767, 2015; Lee, H., Khanal Lamichhane, A., Garraffo, H. M., Kwon-Chung, K. J. & Chang, Y. C. Involvement of PDK1, PKC and TOR signalling pathways in basal fluconazole tolerance in Cryptococcus neoformans. Mol. Microbiol. 84, 130-146, doi:10.1111/j.1365-2958.2012.08016.x, 2012); and Snf1 (Hu, G., Cheng, P. Y., Sham, A., Perfect, J. R. & Kronstad, J. W. Metabolic adaptation in Cryptococcus neoformans during early murine pulmonary infection. Molecular microbiology 69, 1456-1475, doi:10.1111/j.1365-2958.2008.06374.x, 2008. The function of (B3501A) Gsk3 in serotype D was examined, and it was demonstrated that Gsk3 survives at low oxygen partial pressure (1%) in *C. neoformans* and is required for the virulence of serotype D in a murine model system (Chang, Y. C., Ingavale, S. S., Bien, C., Espenshade, P. & Kwon-Chung, K. J. Conservation of the sterol regulatory element-binding protein pathway and its pathobiological importance in Cryptococcus neoformans. Eukaryot Cell 8, 1770-1779, doi:10.1128/EC.00207-09, 2009). The present inventors found that Gsk3 is also required for the virulence of serotype A C. *neoformans* (H99S). Although not previously reported, deletion mutants of kinases functionally connected to these known virulence-regulating kinases were also found to be attenuated in virulence or infectivity. These include *bck1*Δ and *mkk1*/*2*Δ mutants (related to Mpkl) and the *gal83*Δ mutant (related to Snf1). Notably, among them, 44 kinases have been for the first time identified to be involved in the fungal pathogenicity of C. *neoformans.*

For the 60 pathogenicity-related kinases in C. *neoformans,* the present inventors analyzed phylogenetic relationships among orthologs, if any, in fungal species and other eukaryotic kingdoms. To inhibit a broad spectrum of fungal pathogens, it is ideal to target kinases which are not present in humans and are required in a number of fungal pathogens (broad-spectrum antifungal targets). The present inventors compared these large-scale virulence data of C. neoformans with those of other fungal pathogens. A large-scale kinome analysis was performed for the pathogenic fungus *Fusarium graminearum,* which causes scab in wheat plants, and 42 virulence-related protein kinases were identified (Wang, C. et al. Functional analysis of the kinome of the wheat scab fungus Fusarium graminearum. PLoS Pathog 7, e1002460, doi:10.1371/journal.ppat.1002460, 2011). Among them, a total of 21 were involved in the pathogenicity of both types of fungi, and thus were regarded as broad-spectrum antifungal targets: *BUD32* (Fg10037), ATG1 (Fg05547), *CDC28* (Fg08468), *KIC1* (Fg05734), *MEC1* (Fg13318), *KIN4* (Fg11812), *MKK1*/*2* (Fg07295), *BCK1* (Fb06326), *SNF1* (Fg09897), *SSK2* (Fg00408), *PKA1* (Fg07251), *GSK3* (Fg07329), *CBK1* (Fg01188), *KIN1* (Fg09274), *SCH9* (Fg00472), RIM15 (Fg01312), *HOG1* (Fg09612), and *YAK1* (Fg05418). In another human fungal pathogen C. *albicans,* genome-wide pathogenic kinome analysis has not been performed. Based on information from the Candida genome database (http://www.candidagenome.org/), 33 kinases are known to be involved in the pathogenicity of C. *albicans.* Among them, 13 were involved in the pathogenicity of both C. *neoformans* and C. *albicans.* Notably, five kinases (Sch9, Snfl, Pka1, Hogl, and Swe1) appear to be core-pathogenicity kinases as they are involved in the pathogenicity of all three fungal pathogens.

On the contrary, to selectively inhibit C. *neoformans,* it is ideal to target pathogenicity-related kinases which are present in C. *neoformans* but are not present in other fungi or humans (narrow-spectrum anti-cryptococcosis targets). Among them, CNAG_01294 (named *IPK1*), encoding a protein similar to inositol 1,3,4,5,6-pentakisphosphate 2-kinase from plants, is either not present or distantly related to those in ascomycete fungi and humans, and is considered a potential anticryptococcal target. In addition to lacking virulence, the *ipk1*Δ mutants exhibited pleiotropic phenotypes (FIG. 12). Deletion of *IPK1* increased slightly capsule production, but inhibited melanin and urease production. Its deletion also rendered cells to be defective in sexual differentiation and hypersensitive to high temperature and multiple stresses, and enhances susceptibility to multiple antifungal drugs. In particular, Ipk1 can be an useful target in combination therapy, because its deletion significantly increases susceptibility to various kinds of antifungal drugs. Therefore, the present inventors revealed narrow- and broad-spectrum anticryptococcal and antifungal drug targets by kinome analysis of *C*. *neoformans* pathogenicity.

### Example 5: Biological Functions of Kinases Regulating Pathogenicity of C. neoformans

To further clarify a functional network of pathogenicity-related kinases, the present inventors employed a genome-scale co-functional network CryptoNet (www.inetbio.org/cryptonet) for *C*. *neoformans,* recently constructed by the present inventors (Kim, H. et al. Network-assisted genetic dissection of pathogenicity and drug resistance in the opportunistic human pathogenic fungus Cryptococcus neoformans. Scientific reports 5, 8767, doi:10.1038/srep08767 (2015)). To search for any proteins functionally linked to the pathogenicity-related kinases, previously reported information on C. *neoformans* and the Gene Ontology (GO) terms of corresponding kinase orthologs and its interacting proteins in *S. cerevisiae* and other fungi were used. This analysis revealed that the biological functions of pathogenicity-related kinases include cell cycle regulation, metabolic process, cell wall biogenesis and organization, DNA damage repair, histone modification, transmembrane transport and vacuole trafficking, tRNA processing, cytoskeleton organization, stress response and signal transduction, protein folding, mRNA processing, and transcriptional regulation, suggesting that various biological and physiological functions affect virulence of *C*. *neoformans.* Among pathogenicity-related kinases, kinases involved in the cell cycle and growth control were identified most frequently. These include *CDC7, SSN3, CKA1,* and *MEC1.* In particular, Cdc7 is an essential catalytic subunit of the Dbf4-dependent protein kinase in *S. cerevisiae,* and Cdc7-Dbf4 is required for firing of the replication of origin throughout the S phase in *S. cerevisiae* (Diffley, J. F., Cocker, J. H., Dowell, S. J., Harwood, J. & Rowley, A. Stepwise assembly of initiation complexes at budding yeast replication origins during the cell cycle. J Cell Sci Suppl 19, 67-72, 1995). Although not essential at ambient temperature, *cdc7Δ* mutants exhibit serious growth effects at high temperature (FIG. 13a), indicating that they are likely to affect virulence of *C. neoformans.* The *cdc7Δ* mutants in *C. neoformans* are very susceptible to genotoxic agents such as methyl methanesulfonate (MMS) and hydroxyurea (HU), suggesting that Cdc7 can cause DNA replication and repair (FIG. 13a). Mecl is required for cell cycle checkpoint, telomere maintenance and silencing and DNA damage repair in *S. cerevisiae* (Mills, K. D., Sinclair, D. A. & Guarente, L. MEC1-dependent redistribution of the Sir3 silencing protein from telomeres to DNA double-strand breaks. Cell 97, 609-620, 1999). Reflecting these roles, deletion of MEC1 increased cellular sensitivity to genotoxic agents in *C. neoformans* (FIG. 13b), indicating that the role of Mec1 in chromosome integrity can be retained. Deletion of MEC1 did not cause any lethality or growth defects in *C. neoformans,* as was the case in C. *albicans* (Legrand, M., Chan, C. L., Jauert, P. A. & Kirkpatrick, D. T. The contribution of the S-phase checkpoint genes MEC1 and SGS1 to genome stability maintenance in Candida albicans. Fungal Genet Biol 48, 823-830, doi:10.1016/j.fgb.2011.04.005, 2011). Ckal and Cka2 are catalytic α-subunits of protein kinase CK2, which have essential roles in growth and proliferation of *S. cerevisiae;* deletion of both kinases causes lethality (Padmanabha, R., Chen-Wu, J. L., Hanna, D. E. & Glover, C. V. Isolation, sequencing, and disruption of the yeast CKA2 gene: casein kinase II is essential for viability in Saccharomyces cerevisiae. Mol Cell Biol 10, 4089-4099, 1990). Interestingly, *C. neoformans* appears to have a single protein (CKA1) that is orthologous to both Ckal and Cka2. Although deletion of CKA1 is not essential, it severely affected the growth of *C. neoformans* (FIG. 13c). Notably, the *cka1*Δ mutant showed elongated, abnormal cell morphology (FIG. 13d), which is comparable to that of two kinase mutants in the RAM pathway (*cbk1*Δ and *kic1*Δ)*.* Cbkl and Kic1 are known to control the cellular polarity and morphology of *C. neoforman,* but their correlation with virulence is not yet known (Walton, F. J., Heitman, J. & Idnurm, A. Conserved Elements of the RAM Signaling Pathway Establish Cell Polarity in the Basidiomycete Cryptococcus neoformans in a Divergent Fashion from Other Fungi. Mol. Biol. Cell, 2006). The present inventors revealed that the cellular polarity and morphology of *C. neoforman* is related to virulence.

Bud32 is also required for growth, potentially through involvement of tRNA modification. Bud32 belongs to the piD261 family of atypical protein kinases, which are conversed in bacteria, Archaea and eukaryotes, and it recognizes acidic agents, unlike other eukaryotic protein kinases that recognize basic agents (Stocchetto, S., Marin, O., Carignani, G. & Pinna, L. A. Biochemical evidence that Saccharomyces cerevisiae YGR262c gene, required for normal growth, encodes a novel Ser/Thr-specific protein kinase. FEBS Lett 414, 171-175, 1997). In *S. cerevisiae,* Bud32 is a component of the highly conserved EKC (Endopetidase-like and Kinase-associated to transcribed Chromatin)/KEOPS (Kinase, putative endopetidase and other proteins of small size) complex. This complex is required for N⁶-threonylcarbamoyladenosine (t⁶A) tRNA modification, which is important in maintaining codon-anticodon interactions for all tRNAs. Therefore, damaged cells in the EKC/KEOPS complex are likely to have increased frameshift mutation rate and low growth rate (Srinivasan, M. et al. The highly conserved KEOPS/EKC complex is essential for a universal tRNA modification, t6A. EMBO J 30, 873-881, doi:10.1038/emboj.2010.343, 2011). As expected, these defects in tRNA modification had dramatic effects on various biological aspects of *C. neoformans,* and thus affected virulence. The *bud32*Δ mutants exhibited very defective growth under basal and most of the stress conditions (FIG. 12a), and also produced smaller amounts of capsule, melanin and urease(FIG. 12b). In addition, the bud32 mutant was significantly defective in mating (FIG. 14c). One exception was fluconazole resistance (FIG. 14a). Interestingly, the present inventors found that deletion of *BUD32* abolished the induction of *ERG11* upon sterol depletion by fluconazole treatment (FIG. 14d), suggesting a potential role of Bud32 in ergosterol gene expression and sterol biosynthesis in *C. neoformans.*

Kinases involved in nutrient metabolism are also involved in the pathogenicity of *C. neoformans.* In *S. cerevisiae,* Arg5,6p is synthesized as a single protein and is subsequently processed into two separate enzymes (acetylglutamate kinase and *N*-acetyl-γ-glutamyl-phosphate reductase) (Boonchird, C., Messenguy, F. & Dubois, E. Determination of amino acid sequences involved in the processing of the ARG5/ARG6 precursor in Saccharomyces cerevisiae. Eur J Biochem 199, 325-335, 1991). These enzymes catalyze biosynthesis of ornithine, an arginine intermediate. Consistent with this, the present inventors found that the *arg5, 6pΔ* mutant was auxotrophic for arginine (FIG. 15a). In *S. cerevisiae,* MET3, encoding ATP sulfurylase, catalyzes the initial state of the sulfur assimilation pathway that produces hydrogen sulfide, a precursor for biosynthesis of homocysteine, cysteine and methionine (Cherest, H., Nguyen, N. T. & Surdin-Kerjan, Y. Transcriptional regulation of the MET3 gene of Saccharomyces cerevisiae. Gene 34, 269-281, 1985; Ullrich, T. C., Blaesse, M. & Huber, R. Crystal structure of ATP sulfurylase from Saccharomyces cerevisiae, a key enzyme in sulfate activation. EMBO J 20, 316-329, doi:10.1093/emboj/20.3.316, 2001). In fact, the *met3Δ* mutant was found to be auxotrophic for both methionine and cysteine (FIG. 15b). Notably, both *arg5, 6p*Δ and *met3*Δ mutants did not exhibit growth defects in nutrient-rich media (YPD), but exhibited severe growth defects under various stress conditions (FIG. 15c), which may contribute to virulence defects observed in the *arg5, 6p*Δ and *met3*Δ mutants.

### Example 6: Retrograde Vacuole Trafficking Affecting Pathogenicity of C. neoformans

A notable biological function unknown as a cause of the pathogenicity of *C. neoformans* is retrograde vacuole trafficking. It was already reported that, in *C. neoformans,* the ESCRT complex-mediated vacuolar sorting process is involved in virulence, because some virulence factors such as capsule and melanin need to be secreted extracellularly (Godinho, R. M. et al. The vacuolar-sorting protein Snf7 is required for export of virulence determinants in members of the Cryptococcus neoformans complex. Scientific reports 4, 6198, doi:10.1038/srep06198, 2014; Hu, G. et al. Cryptococcus neoformans requires the ESCRT protein Vps23 for iron acquisition from heme, for capsule formation, and for virulence. Infect Immun 81, 292-302, doi:10.1128/IAI.01037-12, 2013). However, the role of endosome-to-Golgi retrograde transport in the virulence of *C. neoformans* has not previously been characterized. Here the present inventors discovered that deletion of CNAG_02680, encoding a *VPS15* orthologue involved in the vacuolar sorting process, significantly reduced virulence (FIG. 16a). This result is consistent with the finding that mutation of *VPS15* also attenuates virulence of *C. albicans* (Liu, Y. et al. Role of retrograde trafficking in stress response, host cell interactions, and virulence of Candida albicans. Eukaryot Cell 13, 279-287, doi:10.1128/EC.00295-13, 2014), strongly suggesting that the role of Vps15 in fungal virulence is evolutionarily conserved. In *S. cerevisiae,* Vpsl5 constitutes the vacuolar protein sorting complex (Vps15/30/34/38) that mediates endosome-to-Golgi retrograde protein trafficking (Stack, J. H., Horazdovsky, B. & Emr, S. D. Receptor-mediated protein sorting to the vacuole in yeast: roles for a protein kinase, a lipid kinase and GTP-binding proteins. Annu Rev Cell Dev Biol 11, 1-33, doi:10.1146/annurev.cb.11.110195.000245, 1995).

To examine the role of Vps15 in vacuolar sorting and retrograde protein trafficking, the vacuolar morphology of the *vps15*Δ mutant was examined comparatively with that of the wild-type strain. Similar to the *vps15*Δ null mutant in C. *albicans,* the *C. neoformans vps15*Δ mutant also exhibited highly enlarged vacuole morphology (FIG. 16b). It is known that defects in retrograde vacuole trafficking can cause extracellular secretion of an endoplasmic reticulum (ER)-resident chaperon protein, Kar2 (Liu, Y. et al. Role of retrograde trafficking in stress response, host cell interactions, and virulence of Candida albicans. Eukaryot Cell 13, 279-287, doi:10.1128/EC.00295-13 (2014)). Supporting this, the present inventors found that *vps15*Δ mutants were highly susceptible to ER stress agents, such as dithiothreitol (DTT) and tunicamycin (TM) (FIG. 16c). Growth defects at 37°C strongly attenuated the virulence and infectivity of the *v.ps15*Δ mutant (FIG. 16d). This may result from increased cell wall and membrane instability by the *vps15*Δ mutant. In *C. albicans,* impaired retrograde trafficking in the *vps15*Δ mutant also causes cell wall stress, activating the calcineurin signaling pathway by transcriptionally up-regulating *CRZ1, CHR1* and *UTR2* (Liu, Y. et al. Role of retrograde trafficking in stress response, host cell interactions, and virulence of Candida albicans. Eukaryot Cell 13, 279-287, doi:10.1128/EC.00295-13, 2014). In C. *neoformans,* however, the present inventors did not observe such activation of signaling components in the calcineurin pathway of the *vps15*Δ mutant (FIG. 16e). Expression levels of *CHR1, CRZ1* and UTR2 in the *vps15*Δ mutant were equivalent to those in the wild-type strain. In *C. neoformans,* cell wall integrity is also governed by the unfolded protein response (UPR) pathway (Cheon, S. A. et al. Unique evolution of the UPR pathway with a novel bZIP transcription factor, Hxll, for controlling pathogenicity of Cryptococcus neoformans. PLoS Pathog. 7, e1002177, doi:10.1371/journal.ppat.1002177 (2011)). Previously, the present inventors demonstrated that activation of the UPR pathway through Ire1 kinase results in an unconventional splicing event in *HXL1* mRNA, which subsequently controls an ER stress response (Cheon, S. A. et al. Unique evolution of the UPR pathway with a novel bZIP transcription factor, Hxll, for controlling pathogenicity of Cryptococcus neoformans. PLoS Pathog. 7, e1002177 (2011)). Indeed, the present inventors found that cells with the *VPS15* deletion were more enriched with spliced *HXL1* mRNA (*HXL1s*) under basal conditions than the wild-type strain, indicating that the UPR pathway may be activated instead of the calcineurin pathway in *C. neoformans* when retrograde vacuole trafficking is perturbed.

### Example 7: Novel Virulence- and Infectivity-Regulating Kinases in C. neoformans

Eight of the 60 pathogenicity-related kinases did not appear to have apparent orthologs in model yeasts, and thus were named virulence-regulating kinase (Vrk1) or infectivity-regulating kinase 1-7 (Irk1-7). Particularly, the present inventors paid attention to Vrk1 (CNAG_06161) (FIG. 17) because its deletion reduced the virulence of *C. neoformans* in the insect host model (FIGS. 6 to 8) and diminished infectivity in the murine host model (FIGS. 9 and 10). A yeast ortholog closest thereto is Fab1 (score: 140.9, e-value: 3.2E-34), but the closest Fab1 ortholog in *C. neoformans* is CNAG_01209 (score: 349.7, e-value: 0.0). Surprisingly, deletion of VRK1 increased cellular resistance to hydrogen peroxide and capsule production (FIGS. 17a and 17b). In addition, it increased cellular resistance to 5-flucytosine and increased fludioxonil susceptibility (FIG. 17a). Based on the kinase mutant phenome clustering data of the present inventors, Vrk1 was not clearly grouped with other kinases.

To gain further insight into the regulatory mechanism of Vrk1, the present inventors performed comparative phosphoproteomic analysis of the wild-type and *vrk1*Δ strains to identify Vrk1-specific phospho-target proteins. TiO₂ enrichment-based phosphoproteomic analysis showed eight potential Vrk1 substrates: CNAG_04190 (*TOP1*, Topoisomerase I), CNAG_01744 (*GPP2,* a DL-glycerol-3-phosphate phosphatase), CNAG_05661 (POB3, heterodimeric FACT complex subunit), CNAG_01972, CNAG_07381, CNAG_00055, CNAG_02943 (*SLM1*, a phosphatidylinositol-4,5-bisphosphate binding protein), and CNAG_07878 *(NOC2,* a nucleolar complex associated protein). CNAG_01972, 07381 and 00055 did not have clear fungal orthologues. Although it is not clear whether candidate proteins are phosphorylated by Vrk1 directly or indirectly, it was found that five candidate proteins (TOP1, GPP2, POB3, CNAG_01972 and CNAG_07381) in the *vrk1Δ* mutant were damaged (FIG. 17c), suggesting that these proteins can be phosphorylated directly by Vrk1. To gain further insight into Vrkl-dependent functional networks, the present inventors used CryptoNet to search for any proteins that were functionally linked to the Vrk1-regulated target proteins and Vrk1 itself, and constructed the functional networks for those proteins. CNAG_01972 and 00055 did not have meaningful connections with any known proteins. Among a variety of potential biological functions connected to Vrk1 and its substrates, rRNA processing were mostly over-represented, suggesting that Vrk1 could be involved in the ribosome biosynthesis and trafficking, either directly or indirectly (FIG. 17d).

### Example 8: Analysis of Antifungal Drug Resistance-Related Kinases in C. neoformans

Based on antifungal drug analysis using the kinas mutant library, 43, 38 and 42 kinases showed increased or reduced susceptibility to amphotericin B (a polyene), fluconazole (an azole) and flucytosine (a nucleotide analog), respectively, which are antifungal drugs used in clinical applications (Table 4). For kinases with deletions that increase susceptibility to these drugs, the present inventors discovered 39 kinases (to amphotericin B), 24 kinases (to fluconazole) and 28 kinases (to flucytosine), which can be developed as targets of drugs in combination therapy.

**[Table 4]**

| Analysis of Antifungal Drug Resistance-Related Kinases in *C. neoformans* | | |
|---|---|---|
| Antifungal agents | Kinase mutants showingincreased resistance | Kinase mutants showingincreased susceptibility |
| Polyene(Amph otericin B) | *HRK1*/NPH1, SPS1, ***SWE192**,* TCO4 | ***YPK1,** VPS15, CBK1, HOG1, SSK2,* PBS2, ***ARG5,6,** GAL83, SNF1*, *MKK2, MPK1,* *BUD32*, *CKA1,* *IPK1 , IRF1, CDC7, KIC1, PKA1,* CRK1, ***BCX1,*** TCO2, ***IRK5*,** *IGI1, **GSK3,** UTR1, MEC1, MET3, PAN3, MPS1, PKH201, **PIK1,*** HRK1, ***KIC102,*** ALK1, TLK1, ARK1, ***IRK3,** KIN1, **POS5*** |
| Azole(Fluconaz ole) | ***GAL83,** PAN3,* ALK1, TCO1, STE11, TCO2, ***SCH9*, *SSK2,*** PBS2, ***HOG1, BUD32, PKA1,*** CHK1, ***YAK1*** | ***YPK1,** VPS15, CBK1, MKK2, MPK1, IPK1, IRE1, BCK1, IGI1, **GSK3,** UTRI, PIK1, HRK1*/*NPH1, **CDC7.*** HRK1, ***PSK201,** MPK2, RAD53, **ARG5,6** KIC1, KIC102, SPS1, **IRK6,*** MAK322 |
| 5-flucyotosine | ***BCK1,*** *PSK201, ARG5,6, GAL83, TCO2, **SNF1,** IRK5,* PKH201, ***VRK1,** CKI1,* TCO5 , STE7, IGI1, ***URK1*** | ***YPK1,** VPS15, GSK3. UTR1,* HRK1 /NPH1, ***SCH9,*** *BUD32, CKA1, MEC1, FBP26, CBK1, **HOG1,** IPK1 IRE1, SSK2,* PBS2, ***MET3,** CDC7, KIC1, PAN3,* TCO1, ***PKA1,*** CHK1, CRK1, ***MPS1,** CDC2801, TCO6, BUB1* |

| | | |
|---|---|---|
| * Underlined kinases are those identified for the first time in the present invention. | | |

### Example 9: Growth and Chemical Susceptibility Test

To analyze the growth and chemical susceptibility of the kinase mutant library, *C. neoformans* cells grown overnight at 30°C were serially diluted tenfold (1 to 10⁴) and spotted on YPD media containing the indicated concentrations of chemical agents as follows: 2M sorbitol for osmotic stress and 1-1.5M NaCl and KCl for cation/salt stresses under either glucose-rich (YPD) or glucose-starved (YPD without dextrose; YP) conditions; hydrogen peroxide (H₂O₂), tert-butyl hydroperoxide (an organic peroxide), menadione (a superoxide anion generator), diamide (a thiol-specific oxidant) for oxidative stress; cadmium sulphate (CdSO₄) for toxic heavy metal stress; methyl methanesulphonate and hydroxyurea for genotoxic stress; sodium dodecyl sulphate (SDS) for membrane destabilizing stress; calcofluor white and Congo red for cell wall destabilizing stress; tunicamycin (TM) and dithiothreitol (DTT) for ER stress and reducing stress; fludioxonil, fluconazole, amphotericin B, flucytosine for antifungal drug susceptibility. Cells were incubated at 30°C and photographed post-treatment from day 2 to day 5. To test the growth rate of each mutant at distinct temperatures, YPD plates spotted with serially diluted cells were incubated at 25°C, 30°C, 37°C, and 39°C, and photographed after 2 to 4 days.

### Example 10: Mating Assay

To examine the mating efficiency of each kinase mutant, the *MAT*α kinase mutant in Table 1 above was co-cultured with serotype A *MAT*α wild-type strain KN99a as a unilateral mating partner. Each kinase mutant *MATα* strain and *MAT*α WT KN99a strain (obtained from the Joeseph Heitman Laboratory at Duke University in USA) was cultured in YPD medium at 30°C for 16hours, pelleted, washed and resuspended in distilled water. The resuspended α and a cells were mixed at equal concentrations (10⁷ cells per ml) and 5µl of the mixture was spotted on V8 mating media (pH 5). The mating plate was incubated at room temperature in the dark for 7 to 14 days and was observed weekly.

### Example 11: In Vitro Virulence-Factor Production Assay

For virulence-factor production assay, capsule production, melanin production and urease production were examined for each kinase mutant. Capsule production was examined qualitatively by India ink staining (Bahn, Y. S., Hicks, J. K., Giles, S. S., Cox, G. M. & Heitman, J. Adenylyl cyclase-associated protein Aca1 regulates virulence and differentiation of Cryptococcus neoformans via the cyclic AMP-protein kinase A cascade. Eukaryot. Cell 3, 1476-1491 (2004). To measure the capsule production levels quantitatively by Cryptocrit, each kinase mutant was grown overnight in YPD medium at 30°C, spotted onto Dulbecco's Modified Eagle's (DME) solid medium, and then incubated at 37°C for 2 days for capsule induction. The cells were scraped, washed with phosphate buffered saline (PBS), fixed with 10% of formalin solution, and washed again with PBS. The cell concentration was adjusted to 3 × 10⁸ cells per ml for each mutant and 50µl of the cell suspension was injected into microhaematocrit capillary tubes (Kimble Chase) in triplicates. All capillary tubes were placed in an upright vertical position for 3 days. The packed cell volume ratio was measured by calculating the ratio of the lengths of the packed cell phase to the total phase (cells plus liquid phases). The relative packed cell volume ratio was calculated by normalizing the packed cell volume ratio of each mutant with that of the wild-type strain. Statistical differences in relative packed cell volume ratios were determined by one-way analysis of variance tests employing the Bonferroni correction method by using the Prism 6 (GraphPad) software.

To examine melanin production, each kinase mutant was grown overnight in YPD medium at 30 °C; 5µl of each culture was spotted on Niger seed media containing 0.1% or 0.2% glucose. The Niger seed plates were incubated at 37°C and photographed after 3-4 days. For kinase mutants showing growth defects at 37°C, the melanin and capsule production were assessed at 30°C. To examine urease production, each kinase mutant was grown in YPD medium at 30°C overnight, washed with distilled water, and then an equal number of cells (5 × 10⁴) was spotted onto Christensen's agar media. The plates were incubated for 2-3 days at 30°C and photographed.

### Example 12: Insect-Based In Vivo Virulence Assay

For each tested *C. neoformans* strain, the present inventors randomly selected a group of 15 *Galleria mellonella* caterpillars in the final instar larval stage with a body weight of 200-300mg, which arrived within 7 days from the day of shipment (Vanderhorst Inc. St Marys, OH, USA) . Each *C. neoformans* strain was grown overnight at 30°C in YPD liquid medium, washed three times with PBS, pelleted and resuspended in PBS at equal concentrations (10⁶ cells per ml). A total of 4,000 *C. neoformans* cells in a 4-µl volume per larva was inoculated through the second to last prolegs by using a 100-µl Hamilton syringe equipped with a 10µl-size needle and a repeating dispenser (PB600-1, Hamilton). The same volume (4 µl) of PBS was injected as a non-infectious control. Infected larvae were placed in petri dishes in a humidified chamber, incubated at 37°C, and monitored daily. Larvae were considered dead when they showed a lack of movement upon touching. Larvae that pupated during experiments were censored for statistical analysis. Survival curves were illustrated using the Prism 6 software (GraphPad). The Log-rank (Mantel-Cox) test was used for statistical analysis. The present inventors examined two independent mutant strains for each kinase mutant. For kinase mutants with single strains, the experiment was performed in duplicate.

### Example 13: Signature-Tagged Mutagenesis (STM)-Based Murine Infectivity Assay

For the high-throughput murine infectivity test, a group of kinase mutant strains with the *NAT* selection marker containing 45 unique signature-tags (a total of four groups) was pooled. The *ste50*Δ and *hxl1Δ* mutants were used as virulent and avirulent control strains, respectively (Cheon, S. A. et al. Unique evolution of the UPR pathway with a novel bZIP transcription factor, Hxl1, for controlling pathogenicity of Cryptococcus neoformans. PLoS Pathog. 7, e1002177, doi:10.1371/journal.ppat.1002177 (2011), Jung, K. W., Kim, S. Y., Okagaki, L. H., Nielsen, K. & Bahn, Y. S. Ste50 adaptor protein governs sexual differentiation of Cryptococcus neoformans via the pheromone-response MAPK signaling pathway. Fungal Genet. Biol. 48, 154-165, doi:S1087-1845(10)00191-X [pii]10.1016/j.fgb.2010.10.006 (2011)). Each group of the kinase mutant library was grown at 30°C in YPD medium for 16 hours separately and washed three times with PBS. The concentration of each mutant was adjusted to 10⁷ cells per ml and 50µl of each sample was pooled into a tube. For preparation of the input genomic DNA of each kinase mutant pool, 200µl of the mutant pool was spread on YPD plate, incubated at 30°C for 2 days, and then scraped. For preparation of the output genomic DNA samples, 50µl of the mutant pool (5 × 10⁵ cells per mouse) was infected into seven-week-old female A/J mice (Jackson Laboratory) through intranasal inhalation. The infected mice were sacrificed with an overdose of Avertin 15 days post-infection, their infected lungs were recovered and homogenized in 4ml PBS, spread onto the YPD plates containing 100µg/ml of chloramphenicol, incubated at 30°C for 2 days, and then scraped. Total genomic DNA was extracted from scraped input and output cells by the CTAB method (Jung, K. W., Kim, S. Y., Okagaki, L. H., Nielsen, K. & Bahn, Y. S. Ste50 adaptor protein governs sexual differentiation of Cryptococcus neoformans via the pheromone-response MAPK signaling pathway. Fungal Genet. Biol. 48, 154-165, doi:S1087-1845(10)00191-X [pii]10.1016/j.fgb.2010.10.006 (2011)). Quantitative PCR was performed with the tag-specific primers listed in Tables 2 and 3 above by using MyiQ2 Real-Time PCR detection system (Bio-Rad). The STM score was calculated (Jung, K. W. et al. Systematic functional profiling of transcription factor networks in Cryptococcus neoformans. Nat Comms 6, 6757, doi:10.1038/ncomms7757 (2015)). To determine the STM score, relative changes in genomic DNA amounts were calculated by the 2^{-ΔΔCT} method (Choi, J. et al. CFGP 2.0: a versatile web-based platform for supporting comparative and evolutionary genomics of fungi and Oomycetes. Nucleic Acids Res 41, D714-719, doi:10.1093/nar/gks1163 (2013)). The mean fold changes in input verses output samples were calculated in Log score (Log₂ 2^{-(*C*t, Target-*C*t,Actin) output- (*C*t, Target-*C*t,Actin)input}).

### Example 14: Vacuole Staining

To visualize vacuole morphology, the wild-type H99S strain and *vsp15*Δ strains (YSB1500 and YSB1501) (obtained from the Joeseph Heitman Laboratory at Duke University in USA) were cultured in liquid YPD medium at 30°C for 16hours. FM4-64 dye (Life Technologies) was added to each culture at a final concentration of 10 µM and further incubated at 30°C for 30minutes. The cells were pelleted by centrifugation, resuspended with fresh liquid YPD medium, and further incubated at 30°C for 30minutes. The cells were pelleted again, washed three times with PBS, and then resuspended in 1ml of PBS. On the glass slide, 10ml of the cells and 10ml of mounting solution (Biomeda) were mixed and spotted. The glass slides were observed by confocal microscope (Olympus BX51 microscope).

### Example 15: TiO₂ Enrichment-Based Phosphoproteomics

To identify the phosphorylated targets of Vrk1 on a genome-wide scale, the H99S and *vrk1Δ* mutant strains were incubated in YPD liquid medium at 30°C for 16 hours, sub-cultured into 1 liter of fresh YPD liquid medium, and further incubated at 30°C until it approximately reached an optical density at 600nm (OD₆₀₀) of 0.9. Each whole-cell lysate was prepared with lysis buffer (Calbiochem) containing 50mM Tris-Cl (pH 7.5), 1% sodium deoxycholate, 5mM sodium pyrophosphate, 0.2mM sodium orthovanadate, 50mM sodium fluoride (NaF), 0.1% sodium dodecyl sulphate, 1% Triton X-100, 0.5mM phenylmethylsulfonyl fluoride (PMSF) and 2.5x protease inhibitor cocktail solution (Merck Millipore). The protein concentration of each cell lysate was measured using a Pierce BCA protein kit (Life Technologies). Sulfhydryl bonds between cysteine residues in protein lysates were reduced by incubating 10mg of total protein lysate with 10mM DTT at room temperature for 1hour and then alkylated with 50 mM iodoacetamide in the dark at room temperature for 1hour . These samples were treated again with 40mM DTT at room temperature for 30min and then digested using trypsin (Sequencing grade trypsin, Promega) at an enzyme: substrate ratio of 1:50 (w/w) with overnight incubation at 37°C. The trypsin-digested protein lysates were then purified with Sep-Pak C18 columns (Waters Corporation, Milford, MA), lyophilized and stored at -80°C. Phosphopeptides were enriched using TiO₂Mag Sepharose beads (GE Healthcare) and then lyophilized for LC-MS/MS. Mass spectrometric analyses were performed using a Q Exactive Hybrid Quadrupole-Orbitrap mass spectrometer (Thermo Scientific, MA, USA) equipped with Dionex U 3000 RSLC nano high-performance liquid chromatography system, a nano-electrospray ionization source and fitted with a fused silica emitter tip (New Objective, Wobum, MA). All phosphopeptide samples were reconstituted in solution A (water/acetonitrile (98:2, v/v), 0.1% formic acid), and then injected into an LC-nano ESI-MS/MS system. Samples were first trapped on a Acclaim PepMap 100 trap column (100 µm i.d. × 2cm, nanoViper C₁₈, 5µm particle size, 100Å pore size, Thermo Scientific) and washed for 6min with 98% solution A at a flow rate of 4µl/min, and then separated on an Acclaim PepMap 100 capillary column (75 µm i.d. × 15cm, nanoViper C₁₈, 3 µm particle size, 100Å pore size, Thermo Scientific) at a flow rate of 400nl/min. Peptides were analyzed with a gradient of 2 to 35% solution B (water/acetonitrile (2:98, v/v), 0.1% formic acid) over 90min, 35 to 90% over 10min, followed by 90% for 5min, and finally 5% for 15min. The resulting peptides were electrosprayed through a coated silica tip (PicoTip emitter, New Objective, MA, USA) at an ion spray voltage of 2,000eV. To assign peptides, MS/MS spectra were searched against the *C. neoformans* var. *grubii* H99S protein database (http://www.uniprot.org) using the SEQUEST search algorithms through the Proteome Discoverer platform (version 1.4, Thermo Scientific). The following search parameters were applied: cysteine carbamidomethylation as fixed modifications, methionine oxidation and serine/threonine/tyrosine phosphorylation as variable modifications. Two missed trypsin cleavages were allowed to identify the peptide. Peptide identification was filtered by a 1% false discovery rate cutoff. Spectral counts were used to estimate relative phosphopeptide abundance between the wild-type and mutant strains. The Student's t-test was used to assess the statistically significant difference between the samples.

### Example 16: ER Stress Assay

To monitor the ER stress-mediated UPR induction, the H99S and *vps15*Δ mutant strains were incubated in YPD at 30°C for 16hours, sub-cultured with fresh YPD liquid medium, and then further incubated at 30°C until they reached the early-logarithmic phase (OD₆₀₀=0.6). The cells were treated with 0.3 µg/ml tunicamycin (TM) for 1hour. The cell pellets were immediately frozen with liquid nitrogen and then lyophilized. Total RNAs were extracted using easy-BLUE (Total RNA Extraction Kit, Intron Biotechnology) and subsequently cDNA was synthesized using an MMLV reverse transcriptase (Invitrogen). *HXL1* splicing patterns (UPR-induced spliced form of *HXL1* (*HXL1S*) and unspliced form of *HXL1* (*HXL1U*)) were analyzed by PCR using cDNA samples of each strain and primers (B5251 and B5252) (Table 3).

### Example 17: Expression Analysis

To measure the expression level of *ERG11,* the H99S strain and *bud32*Δ mutants were incubated in liquid YPD medium at 30°C for 16hours and sub-cultured with fresh liquid YPD medium. When the cells reach the early-logarithmic phase (OD₆₀₀=0.6), the culture was divided into two samples: one was treated with fluconazole (FCZ) for 90 minutes and the other was not treated. The cell pellets were immediately frozen with liquid nitrogen and then lyophilized. Total RNA was extracted and northern blot analysis was performed with the total RNA samples for each strain as previously reported (Jung, K. W., Kim, S. Y., Okagaki, L. H., Nielsen, K. & Bahn, Y. S. Ste50 adaptor protein governs sexual differentiation of Cryptococcus neoformans via the pheromone-response MAPK signaling pathway. Fungal Genet. Biol. 48, 154-165, doi:S1087-1845(10)00191-X [pii]10.1016/j.fgb.2010.10.006 (2011)). For quantitative reverse transcription-PCR (qRT-PCR) analysis of genes involved in the calcineurin pathway, the H99S strain and *vps15*Δ mutants were incubated in liquid YPD medium at 30°C for 16hours and were sub-cultured in fresh liquid YPD medium until they reached to the early-logarithmic phase (OD₆₀₀=0.8). The cells were then pelleted by centrifugation, immediately frozen with liquid nitrogen, and lyophilized. After total RNA was extracted, cDNA was synthesized using RTase (Thermo Scientific). *CNA1*, *CNB1, CRZ1, UTR2* and *ACT1*-specific primer pairs (B7030 and B7031, B7032 and B7033, B7034 and B7035, B7036 and B7037, B679 and B680, respectively) (Table 3) were used for qRT-PCR.

### Example 18: Construction of FPK1 Overexpression Strains

To construct the FPK1 overexpression strain, the native promoter of FPK1 was replaced with histone H3 promoter using an amplified homologous recombination cassette (FIG. 5a). In the first round of PCR, primer pairs L1/OEL2 and OER1/PO were used for amplification of the 5'-flaking region and 5'-coding region of *FPK1*, respectively. The *NEO-H3* promoter region was amplified with the primer pair B4017/B4018. For second-round PCR for the 5' or 3' region of the P*H3*:*FPK1* cassette, the first-round PCR product was overlap-amplified by DJ-PCR with the primer pair L1/GSL or GSR/PO (primers in Tables 2 and 3 above). Then, the PH3:FPK1 cassettes were introduced into the wild-type strain H99S (obtained from the Joeseph Heitman Laboratory at Duke University in USA) and the *ypk1*Δ mutant (YSB1736) by biolistic transformation. Stable transformants selected on YPD medium containing G418 were screened by diagnostic PCR with a primer pair (SO/B79). The correct genotype was verified by Southern blotting using a specific probe amplified by PCR with primers L1/PO. Overexpression of FPK1 was verified using a specific Northern blot probe amplified by PCR with primers NP1 and PO (FIGS. 5b and 5c).

### Example 19: Kinase Phenome Clustering

*In vitro* phenotypic traits of each kinase mutant were scored with the following qualitative scale: -3 (strongly sensitive or defective), -2 (moderately sensitive or defective), -1 (weakly sensitive or defective), 0 (wild-type-like), +1 (weakly resistant or enhanced), +2 (moderately resistant or enhanced), and +3 (strongly resistant or enhanced). The excel file containing the phenotype scores of each kinase mutant was uploaded by Gene-E software (http://www.broadinstitute.org/cancer/software/GENE-E/) and then kinase phenome clustering was drawn using one minus Pearson correlation.

### Example 20: Cryptococcus Kinome Web-Database

For public access to the phenome and genome data for the C. *neoformans* kinase mutant library constructed by the present inventors, the *Cryptococcus* Kinase Phenome Database was developed (http://kinase.cryptococcus.org/). Genome sequences of *C. neoformans var. grubii* H99 were downloaded from the Broad Institute (http://www.broadinstitute.org/annotation/genome/cryptococcus neoformans/MultiHome.html), and incorporated into the standardized genome data warehouse in the Comparative Fungal Genomics Platform database (CFGP 2.0; http://cfgp.snu.ac.kr/) (Choi, J. et al. CFGP 2.0: a versatile web-based platform for supporting comparative and evolutionary genomics of fungi and Oomycetes. Nucleic Acids Res 41, D714-719, doi:10.1093/nar/gksll63 (2013)). Classification of protein kinases was performed by using the hidden Markov model-based sequence profiles of SUPERFAMILY (version 1.73) (Wilson, D. et al. SUPERFAMILY--sophisticated comparative genomics, data mining, visualization and phylogeny. Nucleic Acids Res 37, D380-386, doi:10.1093/nar/gkn762 (2009)). A total of 64 family identifiers belonging to 38 superfamilies were used to predict putative kinases. In addition, the sequence profiles of Kinomer (version 1.0) (Martin, D. M., Miranda-Saavedra, D. & Barton, G. J. Kinomer v. 1.0: a database of systematically classified eukaryotic protein kinases. Nucleic Acids Res 37, D244-250, doi:10.1093/nar/gkn834 (2009); Miranda-Saavedra, D. & Barton, G. J. Classification and functional annotation of eukaryotic protein kinases. Proteins 68, 893-914, doi:10.1002/prot.21444 (2007)) and the Microbial Kinome (Kannan, N., Taylor, S. S., Zhai, Y., Venter, J. C. & Manning, G. Structural and functional diversity of the microbial kinome. PLoS Biol 5, e17, doi:10.1371/journal.pbio.0050017 (2007)) were used to supplement the kinase prediction. Information from genome annotation of C. *neoformans* var. *grubii* H99 and protein domain predictions of InterProScan 62 was also adopted to capture the maximal extent of possible kinase-encoding genes. For each gene, results from the eight bioinformatics programs were also provided to suggest clues for gene annotations. In addition, results from SUPERFAMILY, Kinomer and Microbial Kinome were displayed for supporting robustness of the prediction. If a gene has an orthologue in *C. neoformans* var. *neoformans* JEC21, a link to the KEGG database was also provided. To browse genomic data in context to important biological features, the Seoul National University genome browser (SNUGB; http://genomebrowser.snu.ac.kr/) (Jung, K. et al. SNUGB: a versatile genome browser supporting comparative and functional fungal genomics. BMC Genomics 9, 586, doi:10.1186/1471-2164-9-586 (2008)) was integrated into the *Cryptococcus* kinase phenome database. In kinase browser, a direct link to the SNUGB module was provided for each gene. The *Cryptococcus* kinase phenome database was developed by using MySQL 5.0.81 (source code distribution) for database management and PHP 5.2.6 for web interfaces. The web-based user interface is served through the Apache 2.2.9 web server.

### [industrial Applicability]

The present invention relates to kinases making it possible to effectively screen novel antifungal agent candidates. The use of the kinases according to the present invention makes it possible to effectively screen novel antifungal agent candidates. In addition, the use of an antifungal pharmaceutical composition comprising an agent (antagonist or inhibitor) for the kinase according to the present invention can effectively prevent, treatment and/or diagnose fungal infection.

### [Embodiments]

[Embodiment 1] A method for screening an antifungal agent, comprising the steps of:
   (a) bringing a sample to be analyzed into contact with a cell containing a pathogenicity-regulating kinase protein or a gene encoding the protein;
   (b) measuring an amount or activity of the protein or an expression level of the gene; and
   (c) determining that the sample is an antifungal agent, when the amount or activity of the protein or the expression level of the gene is measured to be down-regulated or up-regulated.
[Embodiment 2] The method of claim 1, wherein the pathogenicity-regulating kinase protein is one or more selected from the group consisting of BUD32, ATG1, CDC28, KIC1, MEC1, KIN4, MKK1/2, BCK1, SNF1, SSK2, PKA1, GSK3, CBK1, KIN1, SCH9, RIM15, HOG1, YAK1, IPK1, CDC7, SSN3, CKA1, MEC1, ARG5, 6P, MET3, *VPS15* and VRK1.
[Embodiment 3] The method of claim 1 or 2, wherein the cell is a *Cryptococcus neoformans* cell.
[Embodiment 4] The method of claim 1 or 2, wherein the antifungal agent is an antifungal agent for treating meningoencephalitis or cryptococcosis.
[Embodiment 5] An antifungal pharmaceutical composition, comprising an antagonist or inhibitor of a *Cryptococcus neoformans* pathogenicity-regulating kinase protein or an antagonist or inhibitor of the gene encoding the protein.
[Embodiment 6] The antifungal pharmaceutical composition of claim 5, wherein pathogenicity-regulating kinase protein is one or more selected from the group consisting of BUD32, ATG1, CDC28, KIC1, MEC1, KIN4, MKK1/2, BCK1, SNF1, SSK2, PKA1, GSK3, CBK1, KIN1, SCH9, RIM15, HOG1, YAK1, IPK1, CDC7, SSN3, CKA1, MEC1, ARG5,6P, MET3, VPS15 and VRK1.
[Embodiment 7] The antifungal pharmaceutical composition of claim 5 or 6, wherein the composition is for treating meningoencephalitis or cryptococcosis.
[Embodiment 8] The antifungal pharmaceutical composition of claim 5 or 6, wherein the antagonist or inhibitor is an antibody against the protein.
[Embodiment 9] The antifungal pharmaceutical composition of claim 5 or 6, wherein the antagonist or inhibitor is an antisense oligonucleotide, siRNA, shRNA, miRNA, or a vector comprising one or more of these, against the gene.
[Embodiment 10] The antifungal pharmaceutical composition of claim 5 or 6, wherein the composition is administered in combination with an azole-based or non-azole-based antifungal agent.
[Embodiment 11] The antifungal pharmaceutical composition of claim 10, wherein the azole-based antifungal agent is one or more selected from the group consisting of fluconazole, itraconazole, voriconazole and ketoconazole.
[Embodiment 12] The antifungal pharmaceutical composition of claim 10, wherein the non-azole-based antifungal agent is one or more selected from the group consisting of amphotericin B, natamycin, rimocidin, nystatin and fludioxonil,
[Embodiment 13] A novel gene-deletion kinase mutant (accession number: KCCM 51297).

## Claims

1. A method for screening an antifungal agent having an increased susceptibility for fungi resistant to one or more antifungal, agents selected from the group consisting of Polyene family antifungals, Azole family antifungals, and 5-flucytocine, comprising the steps of:
(a) bringing a sample to be analyzed into contact with a cell containing a fungal pathogenicity-regulating kinase protein or a gene encoding the protein;
(b) measuring an amount or activity of the protein or an expression level of the gene; and
(c) determining that the sample is an antifungal agent, when the amount or activity of the protein or the expression level of the gene is measured to be down-regulated,
wherein the fungal pathogenicity-regulating kinase protein is one or more selected from the group consisting of YPK1, VPS15, CBK1, HOG1, SSK2, PBS2, ARG5,6, GAL83, SNF1, MKK2, MPK1, BUD32, CKA1, IPK1, IRE1, CDC7, KIC1, PKA1, CRK1, BCK1, TCO2, IRK5, IGI1, GSK3, UTR1, MEC1, MET3, PAN3, MPS1, FKH201, PIK1, HRK1, KIC102, ALK1, TLK1, ARK1, IRK3, KIN1, POS5, HRK1/NPH1, PSK201, MPK2, RAD53, SPS1, IRK6, MAK322, SCH9, FBP26, TCO1, CHK1, CDC2801, TCO6 and BUB1.

2. The method of claim 1, wherein the method is to screen an antifungal agent having an increased susceptibility against Polyene family antifungals, Azole family antifungals and 5-flucytocin, and wherein the fungal pathogenicity-regulating kinase protein is one or more selected from the group consisting of YPK1, VPS15, CBK1, IRE1, CDC7, KIC1, GSK3 and UTR1

3. The method of claim 1 or claim 2, wherein the cell is a *Cryptococcus neoformans* cell.

4. An antifungal composition having an increased susceptibility for fungi resistant to one or more antifungal agents selected from the group consisting of Polyene family antifungals, Azole family antifungals, and 5-flucytocine, comprising
an antagonist or inhibitor of a fungal pathogenicity-regulating kinase protein or an antagonist or inhibitor of the gene encoding the protein,
wherein the fungal pathogenicity-regulating kinase protein is one or more selected from the group consisting of YPK1, VPS15, CBK1, HOG1, SSK2, PBS2, ARG5, 6, GAL83, SNF1, MKK2, MPK1, BUD32, CKA1, IPK1, IRE1, CDC7, KIC1, PKA1, CRK1, BCK1, TCO2, IRK5, IGI1, GSK3, UTR1, MEC1, MET3, PAN3, MPS1, PKH201, PIK1, HRK1, KIC102, ALK1, TLK1, ARK1, IRK3, KIN1, POS5, HRK1/NPH1, PSK201, MPK2, RAD53, SPS1, IRK6, MAK322, SCH9, FBP26, TCO1, CHK1, CDC2801, TCO6 and BUB1

5. The antifungal composition of claim 4, wherein the composition has antifungal activities against Amphotericin B, Fluconazole and 5-flucytocin, and wherein the fungal pathogenicity-regulating kinase protein is one or more selected from the group consisting of YPK1, VPS15, CBK1, IRE1, CDC7, KIC1, GSK3 and UTR1.

6. The antifungal composition of claim 4 or 5, wherein the composition is for treating meningoencephalitis or cryptococcosis.

7. The antifungal composition of claim 4 or 5, wherein the antagonist or inhibitor is an antibody against the protein.

8. The antifungal composition of claim 4 or 5, wherein the antagonist or inhibitor is an antisense oligonucleotide, siRNA, shRNA, miRNA, or a vector comprising one or more of these, against the gene.

9. The antifungal composition of claim 4 or 5, wherein the composition is administered in combination with an azole-based or non-azole-based antifungal agent.

10. The antifungal composition of claim 9, wherein the azole-based antifungal agent is one or more selected from the group consisting of fluconazole, itraconazole voriconazole and ketoconazole.

11. The antifungal composition of claim 9, wherein the non-azole-based antifungal agent is one or more selected from the group consisting of amphotericin B, natamycin, rimocidin, nystatin and fludioxonil.

12. The method of claim 1, wherein the cell is a *Cryptococcus neoformans* selected from a cell line library (accession number: KCCM 51297)
